# EUROPEAN PATENT APPLICATION

(11) **EP 3 444 304 A1**
(43) Date of publication of application: **20.02.2019**
(21) Application number: 17775154.2
(22) Date of filing: 28.03.2017
(51) Int. Cl.: C09B 62/465, A61K 8/49, A61Q 5/06, C08F 20/36, C08L 101/00, C09B 11/28, C09B 67/32, C09B 69/00

(54) **POLYFUNCTIONAL POLYMERIZABLE COMPOUND AND COLORED COMPOSITION**

(30) Priority: 29.03.2016 JP 2016065055
(71) Applicant: FUJIFILM Wako Pure Chemical Corporation, Tokyo 103-0023 (JP)
(72) Inventor: KAWANO Kei, Kawagoe-shi Saitama 350-1101 (JP); TAKANO Masahiro, Kawagoe-shi Saitama 350-1101 (JP)
(74) Representative: Hinkelmann, Klaus
(86) International application number: PCT/JP2017/012769
(87) International publication number: WO 2017/170617

(57) **Abstract**

With a colored composition of the related art, elution resistance within a practical range is not obtained. Therefore, an object of the present invention is to provide a colored composition having better elution resistance (less dye elution) than that of the colored composition of the related art.

The present invention relates to a compound represented by the following general formula (1), a colored composition comprising the compound, and the like.

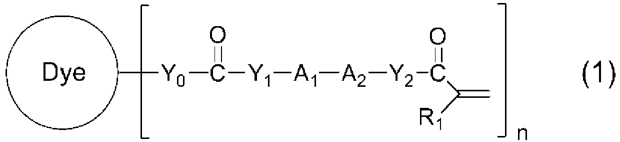

{In the formula, Dye represents a dye residue, R₁ represents a hydrogen atom or a methyl group, Y₀ represents a single bond or the like, Y₁ and Y₂ each independently represent -O- or the like, A₁ represents a specific alkylene group or the like, A₂ represents an alkylene group which may have -O- in a chain and has a group represented by the following general formula (2) in the chain or on the terminal of the chain, and n represents 1 or 2; 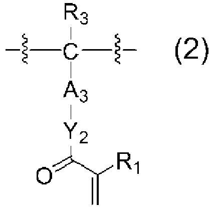 (in the formula, R₁ and Y₂ are the same as described above, R₃ represents a group represented by the following general formula (2-1) or the like, and A₃ represents a specific alkylene group or the like; 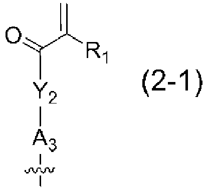 [in the formula, R₁, A₃, and Y₂ are the same as described above.].).}

## Description

### Technical Field

The present invention relates to a polyfunctional polymerizable compound, which is used for forming colored pixels of color filters and the like and used in printing ink, ink jet ink, paint, and the like, and a colored composition comprising the compound.

### Background Art

As color pixel forming methods used in manufacturing color filters of liquid crystal display elements, solid-state imaging devices, and the like, a staining method or a dye dispersion method using a dye as a colorant, a pigment dispersion method using a pigment, an electrodeposition method, a printing method, and the like are known. In recent years, as the characteristics of color filters, brightness and contrast have been particularly required to be improved. According to the pigment dispersion method using a pigment, heat resistance or light fastness of the pigment is higher than that of a dye, and accordingly, deterioration hardly occurs in a heating step at the time of manufacturing a panel, and color pixels having high long-term reliability can be obtained. Therefore, currently, the pigment dispersion method is mainly used. However, in a case where a pigment is used, because the pigment has a relatively large particle diameter, unfortunately, contrast deteriorates due to light scattering. Although an attempt to make the pigment into fine particles has been made, there is a limit to the particle diameter that can be reduced, and dispersion stability needs to be secured for the pigment made into fine particles.

As a method that can solve the above problems, currently, a method for forming color pixels by using a dye is being studied. In a case where a dye is used, light scattering is inhibited, and hence contrast is improved. However, a dye has low heat resistance compared to a pigment, and some dyes have sublimation properties depending on the type thereof. Therefore, problems such as brightness deterioration, fading, and color change occur. Accordingly, in the method using a dye, these problems need to be solved. Various examinations have been carried out regarding dyes having heat resistance, and for example, xanthene-based dyes are reported in WO2014/126167 or WO2015/147285, and triarylmethane-based dyes are reported in WO2010/123071 or WO2013/108591.

### Citation List

### Patent Literature

Patent Literature 1 WO2014/126167
Patent Literature 2 WO2015/147285
Patent Literature 3 WO2010/123071
Patent Literature 4 WO2013/108591

### Summary of Invention

### Technical Problem

As a result of examining colored compositions using the xanthene-based dye or the triarylmethane-based dye of the related art, sufficient elution resistance was not obtained. Therefore, an object of the present invention is to provide a colored composition having better elution resistance (less dye elution) than that of the colored compositions of the related art. Solution to Problem

The inventors of the present invention conducted thorough examinations in consideration of the circumstances described above. As a result, the inventors found that in a case where a compound having a specific functional group is used as a dye, a colored composition having excellent elution resistance (less dye elution) is obtained, and accomplished the present invention.

That is, in a first aspect, the present invention includes inventions (i) and (ii) described below.
(i) A compound represented by the following general formula (1) (hereinafter, simply described as a compound of the present invention in some cases). 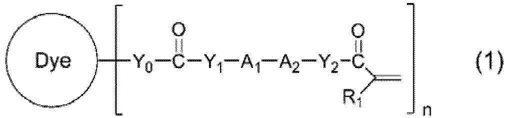 In the formula, Dye represents a dye residue, R₁ represents a hydrogen atom or a methyl group, Y₀ represents a linear alkylene group having 1 to 3 carbon atoms, -NH-, or a single bond, Y₁ and Y₂ each independently represent -O- or -NH-, A₁ represents an alkylene group having 1 to 6 carbon atoms that may have -O-, -OCO-, -COO-, -NHCO-, -CONH-, -NHCONH-, and/or a phenylene group in a chain, or represents a single bond, A₂ represents an alkylene group having 1 to 6 carbon atoms that may have -O- in a chain and has 1 to 6 groups represented by the following general formula (2) in the chain or on the terminal of the chain, and n represents 1 or 2; 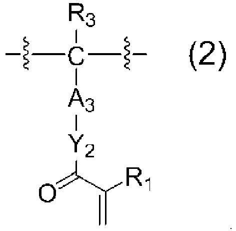 in the formula, R₁ and Y₂ are the same as described above, R₃ represents a hydrogen atom, an alkyl group having 1 to 6 carbon atoms, or a group represented by the following general formula (2-1), and A₃ represents an alkylene group having 1 to 6 carbon atoms that may have -O- in a chain, or represents a single bond; 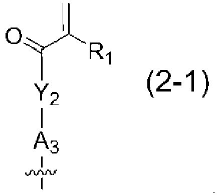 in the formula, R₁, A₃, and Y₂ are the same as described above.
   Here, a plurality of R₁'s, a plurality of Y₀'s, a plurality of Y₁'s, a plurality of Y₂'s, a plurality of A₁'s, and a plurality of A₂'s are the same as or different from each other respectively, and in a case where the number of the groups represented by the general formula (2) in A₂ is 1, R₃ in the general formula (2) represents the group represented by the general formula (2-1).
(ii) A colored composition comprising the compound represented by the general formula (1) (hereinafter, simply described as a colored composition of the present invention in some cases).
   In a second aspect, the present invention includes inventions (iii) to (viii) described below.
(iii) A compound represented by the following general formula (1a). 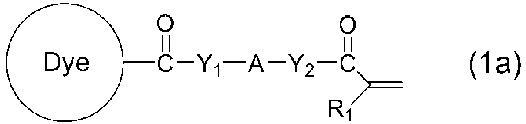
   In the formula, Dye, R₁, Y₁, and Y₂ are the same as described above, and A represents an alkylene group having 1 to 6 carbon atoms that may have -O- in a chain and has 1 to 6 groups represented by the general formula (2) in the chain or on the terminal of the chain. Here, in a case where the number of the groups represented by the general formula (2) in A is 1, R₃ in the general formula (2) represents the group represented by the general formula (2-1).
   It should be noted that "an alkylene group having 1 to 6 carbon atoms that may have -O- in a chain and has 1 to 6 groups represented by the general formula (2) in the chain or on the terminal of the chain" represented by A in the general formula (1a) is the same as A₂ in the general formula (1).
(iv) The compound represented by the general formula (1a), wherein in a case where R₃ in the general formula (2) is a hydrogen atom or an alkyl group having 1 to 6 carbon atoms, the number of the groups represented by the general formula (2) in A in the general formula (1a) is 2 to 6, or in a case where R₃ in the general formula (2) is the group represented by the general formula (2-1), the number of the groups represented by the general formula (2) in A in the general formula (1a) is 1 to 3.
(v) The compound represented by the general formula (1a), wherein A is represented by the following general formula (3a). 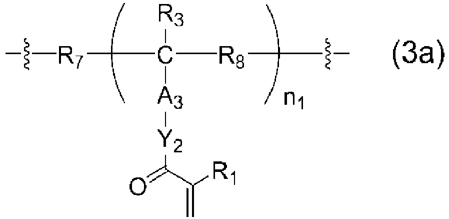
   In the formula, R₁, R₃, A₃, and Y₂ are the same as described above, R₇ represents an alkylene group having 1 to 6 carbon atoms or a single bond, R₈ represents an alkylene group having 1 to 6 carbon atoms that may have -O- in a chain or represents a single bond, and n₁ represents an integer of 1 to 6. Here, a plurality of R₁'s, a plurality of R₃'s, a plurality of R₈'s, a plurality of A₃'s, and a plurality of Y₂'s are the same as or different from each other respectively, the total number of carbon atoms in R₇ and the plurality of R₈'s is 1 to 6, and in a case where n₁ is 1, R₃ represents the group represented by the general formula (2-1).
   It should be noted that "an alkylene group having 1 to 6 carbon atoms" represented by R₇ in the general formula (3a) is equivalent to "an alkylene group having 1 to 6 carbon atoms" represented by A₁ in the general formula (1); "an alkylene group having 1 to 6 carbon atoms that may have -O- in a chain or a single bond" represented by R₈ is equivalent to A₄ in a general formula (3) which will be described later; and n₁ in the general formula (3a) is the same as n₁ in the general formula (3) which will be described later.
(vi) The compound represented by the general formula (1a), wherein A is represented by the following general formula (4a-1) or (4a-2). 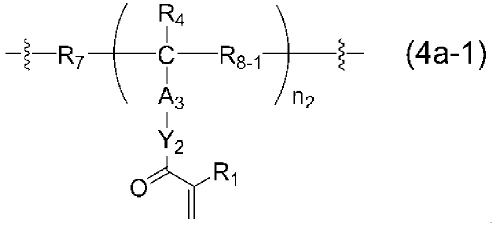
   In the formula, R₁, R₇, A₃, and Y₂ are the same as described above, R₄ represents a hydrogen atom or an alkyl group having 1 to 6 carbon atoms, R₈₋₁ represents an alkylene group having 1 to 6 carbon atoms or a single bond, and n₂ represents an integer of 2 to 6. Here, a plurality of R₁'s, a plurality of R₄'s, a plurality of R₈₋₁'s, a plurality of A₃'s, and a plurality of Y₂'s are the same as or different from each other respectively, and the total number of carbon atoms in R₇ and the plurality of R₈₋₁'s is 1 to 6. 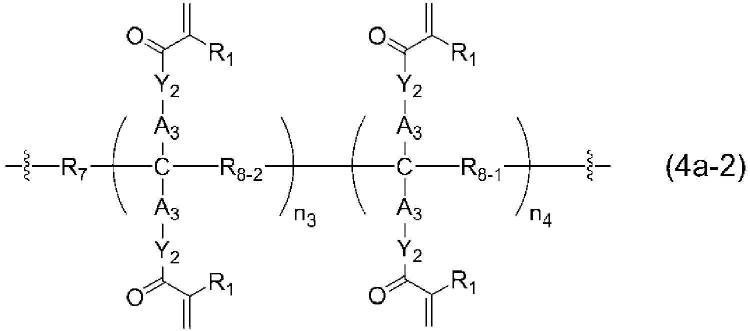
   In the formula, R₁, R₇, R₈₋₁, A₃, and Y₂ are the same as described above, R₈₋₂ represents an alkylene group having 1 to 6 carbon atoms that has -O- in a chain, n₃ and n₄ each independently represent an integer of 0 to 3, and n₃ + n₄ equals an integer of 1 to 3. Here, a plurality of R₁'s, a plurality of R₈₋₁'s, a plurality of R₈₋₂'s, a plurality of A₃'s, and a plurality of Y₂'s are the same as or different from each other respectively, and the total number of carbon atoms in R₇, the plurality of R₈₋₁'s, and the plurality of R₈₋₂'s is 1 to 6.
   It should be noted that R₄ and n₂ in the general formula (4a-1) are the same as R₄ and n₂ in a general formula (4-1) which will be described later, respectively; "an alkylene group having 1 to 6 carbon atoms or a single bond" represented by R₈₋₁ in the general formula (4a-1) is equivalent to A₄₋₁ in the general formula (4-1) which will be described later; "an alkylene group having 1 to 6 carbon atoms that has -O- in a chain" represented by R₈₋₂ in the general formula (4a-2) is equivalent to A₄₋₂ in a general formula (4-2) which will be described later; and n₃ and n₄ in the general formula (4a-2) are the same as n₃ and n₄ in the general formula (4-2) which will be described later, respectively.
(vii) The compound represented by the general formula (1a), wherein A is represented by any one of the following formulae (101a) to (116a). 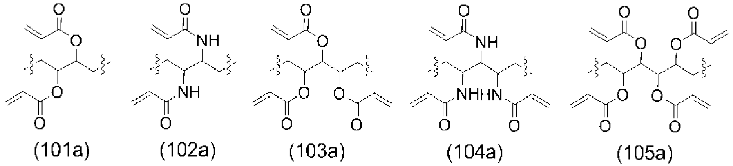 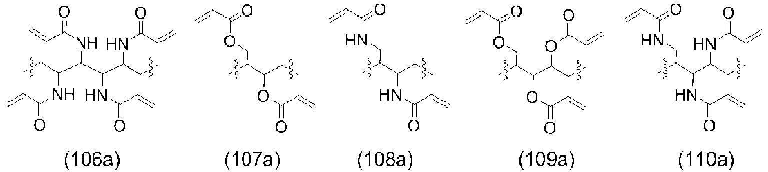 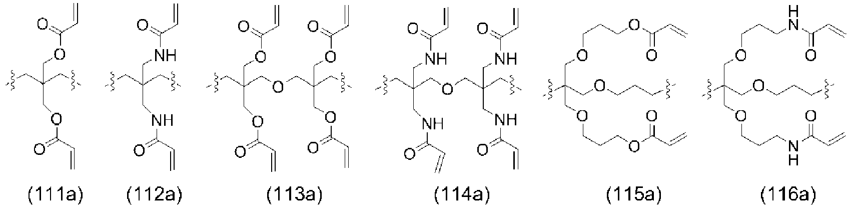
(viii) A colored composition comprising the compound represented by the general formula (1a).

### Advantageous Effects of Invention

In a case where the compound of the present invention is used as a colorant, excellent effects are obtained in which the colorant practically is not eluted even though a colored material is immersed in a solvent such as propylene glycol monomethyl ether acetate (PGMEA) after coloring, and a problem such as a decrease in color density or color mixing does not occur. That is, the colored composition comprising the compound of the present invention can form an excellent colored cured material having higher elution resistance than that of the related art. Therefore, the colored composition of the present invention can be used for forming colored pixels of color filters and the like, which are used in liquid crystal display (LCD), a solid-state imaging device (CCD, CMOS, and the like), an organic electroluminescence display (OLED), and the like, and can be used in printing ink, ink jet ink, paint, and the like. Particularly, the colored composition of the present invention is suitable for color filters of a liquid crystal display. Furthermore, the colored composition of the present invention can be used as a colored resin molded material by being molded into a sheet, a film, a bottle, a cup, and the like, by means of molding methods known in the related art. Accordingly, the colored composition of the present invention can also be used for eyeglasses, contact lenses, colored contact lenses, and the like. By being made into a multilayer structure with a known resin, the colored composition of the present invention can also be used for the same uses. In addition, for example, the colored composition of the present invention can be used for optical films, hair coloring agents, labeling substances for compounds or biological substances, materials for organic solar cells, and the like.

### Description of Embodiments

### Compound of the present invention

The compound of the present invention is a compound represented by a general formula (1).

As R₁ in the general formula (1), a hydrogen atom is preferable.

Specific examples of the linear alkylene group having 1 to 3 carbon atoms that is represented by Y₀ in the general formula (1) include a methylene group, an ethylene group, and a trimethylene group. Among these, the methylene group is preferable.

Those preferred as Y₀ in the general formula (1) vary with the structure of Dye. For example, in a case where Dye is a residue derived from a xanthene-based dye, a single bond is preferable. In a case where Dye is a residue derived from a triarylmethane-based dye, -NH- and a single bond are preferable. In a case where Dye is a residue derived from a cyanine-based dye, a methylene group and a single bond are preferable. More specifically, in a case where Dye is a dye residue represented by a general formula (I) which will be described later, a single bond is preferable. In a case where Dye is a dye residue represented by a general formula (III-1-1) which will be described later, a single bond is preferable. In a case where Dye is a dye residue represented by a general formula (III-1-2) which will be described later, -NH- is preferable. In a case where Dye is a dye residue represented by a general formula (IV) which will be described later, a methylene group and a single bond are preferable.

As Y₁ in the general formula (1), -O- is preferable.

As Y₂ in the general formula (1), -O- is preferable.

In a case where A₁ in the general formula (1) represents "an alkylene group having 1 to 6 carbon atoms that may have -O-, -OCO-, -COO-, -NHCO-, -CONH-, -NHCONH-, and/or a phenylene group in a chain", as the alkylene group having 1 to 6 carbon atoms, a linear or branched alkylene group is preferable, and the linear alkylene group is more preferable. In addition, among the alkylene group having 1 to 6 carbon atoms, an alkylene group having 1 to 3 carbon atoms is preferable. Specific examples thereof include a methylene group, an ethylene group, a methyl methylene group, a trimethylene group, a propylene group, a dimethyl methylene group, an ethyl methylene group, a tetramethylene group, a 1-methyl trimethylene group, a 2-methyl trimethylene group, a 1,2-dimethyl ethylene group, a 1,1-dimethyl ethylene group, an ethyl ethylene group, an ethyl methyl methylene group, a propyl methylene group, a pentamethylene group, a 1-methyl tetramethylene group, a 2-methyl tetramethylene group, a 1-ethyl trimethylene group, a 2-ethyl trimethylene group, an n-propyl ethylene group, an isopropyl ethylene group, an n-butyl methylene group, an isobutyl methylene group, a tert-butyl methylene group, a hexamethylene group, a 1-methyl pentamethylene group, a 2-methyl pentamethylene group, a 3-methyl pentamethylene group, a 1-ethyl tetramethylene group, a 2-ethyl tetramethylene group, a 1-n-propyl trimethylene group, a 1-isopropyl trimethylene group, a 2-n-propyl trimethylene group, a 2-isopropyl trimethylene group, an n-butyl ethylene group, an isobutyl ethylene group, a tert-butyl ethylene group, an n-pentyl methylene group, an isopentyl methylene group, and the like. Among these, the methylene group, the ethylene group, the trimethylene group, the tetramethylene group, the pentamethylene group, and the hexamethylene group are preferable, and the methylene group, the ethylene group, and the trimethylene group are more preferable.

In a case where A₁ in the general formula (1) represents "an alkylene group having 1 to 6 carbon atoms that may have -O-, -OCO-, -COO-, -NHCO-, -CONH-, -NHCONH-, and/or a phenylene group in a chain", the phenylene group is any one of an o-phenylene group, a m-phenylene group, and a p-phenylene group. Among these, the o-phenylene group and the p-phenylene group are preferable, and the o-phenylene group is more preferable.

Specific examples of the alkylene group having 1 to 6 carbon atoms represented by A₁ in the general formula (1) that has -O-, -OCO-, -COO-, -NHCO-, -CONH-, -NHCONH-, and/or a phenylene group in a chain include groups represented by the following general formulae (1-1) to (1-3).

-(R₅₁-O)ₕ₁R₅₂- (1-1)

(In the formula, R₅₁ and R₅₂ each independently represent an alkylene group having 1 to 5 carbon atoms, and h₁ represents an integer of 1 to 5. Here, the total number of carbon atoms in h₁ pieces of R₅₁ and R₅₂ is 2 to 6.)

-R₅₃-Y₃-R₅₄- (1-2)

(In the formula, R₅₃ and R₅₄ each independently represent an alkylene group having 1 to 5 carbon atoms, and Y₃ represents -OCO-, -COO-, -NHCO-, -CONH-, -NHCONH-, or a phenylene group. Here, the total number of carbon atoms in R₅₃ and R₅₄ is 2 to 6.)

-R₅₅-Y₆-R₅₆-Y₇-R₅₇- (1-3)

(In the formula, R₅₅ and R₅₇ each independently represent an alkylene group having 1 to 4 carbon atoms, R₅₆ represents an alkylene group having 1 to 4 carbon atoms or a phenylene group, Y₆ represents -OCO- or -NHCO-, and Y₇ represents -COO- or -CONH-. Here, the total number of carbon atoms in the alkylene groups represented by R₅₅ to R₅₇ is 2 to 6.)

As the alkylene group having 1 to 5 carbon atoms represented by R₅₁ to R₅₄ in the general formulae (1-1) and (1-2), a linear or branched alkylene group is preferable, and the linear alkylene group is more preferable. In addition, among the alkylene group having 1 to 5 carbon atoms, an alkylene group having 1 to 3 carbon atoms is preferable. Specific examples thereof include a methylene group, an ethylene group, a methyl methylene group, a trimethylene group, a propylene group, a dimethyl methylene group, an ethyl methylene group, a tetramethylene group, a 1-methyl trimethylene group, a 2-methyl trimethylene group, a 1,2-dimethyl ethylene group, a 1,1-dimethyl ethylene group, an ethyl ethylene group, an ethyl methyl methylene group, a propyl methylene group, a pentamethylene group, a 1-methyl tetramethylene group, a 2-methyl tetramethylene group, a 1-ethyl trimethylene group, a 2-ethyl trimethylene group, an n-propyl ethylene group, an isopropyl ethylene group, an n-butyl methylene group, an isobutyl methylene group, a tert-butyl methylene group, and the like. Among these, the methylene group, the ethylene group, the trimethylene group, the tetramethylene group, and the pentamethylene group are preferable, and the methylene group, the ethylene group, and the trimethylene group are more preferable.

As the alkylene group having 1 to 4 carbon atoms represented by R₅₅ to R₅₇ in the general formula (1-3), a linear or branched alkylene group is preferable, and the linear alkylene group is more preferable. In addition, among the alkylene group having 1 to 4 carbon atoms, an alkylene group having 1 or 2 carbon atoms is preferable. Specific examples thereof include a methylene group, an ethylene group, a methyl methylene group, a trimethylene group, a propylene group, a dimethyl methylene group, an ethyl methylene group, a tetramethylene group, a 1-methyl trimethylene group, a 2-methyl trimethylene group, a 1,2-dimethyl ethylene group, a 1,1-dimethyl ethylene group, an ethyl ethylene group, an ethyl methyl methylene group, a propyl methylene group, and the like. Among these, the methylene group, the ethylene group, the trimethylene group, and the tetramethylene group are preferable, and the methylene group and the ethylene group are more preferable.

As h₁ in the general formula (1-1), 1 or 2 is preferable, 1 is more preferable.

Specific examples preferred as the group represented by the general formula (1-1) include a group represented by the following general formula (1-4).

-R₅₁-O-R₅₂- (1-4)

(In the formula, R₅₁ and R₅₂ are the same as described above. Here, the total number of carbon atoms in R₅₁ and R₅₂ is 2 to 6.)

Specific examples preferred as the group represented by the general formula (1-4) include a group represented by the following general formula (1-5).

-(CH₂)ₕ₂-O-(CH₂)ₕ₃- (1-5)

(In the formula, h₂ and h₃ each independently represent an integer of 1 to 5, and h₂ + h₃ equals an integer of 2 to 6.)

As h₂ in the general formula (1-5), an integer of 1 to 3 is preferable, and 1 is more preferable.

As h₃ in the general formula (1-5), an integer of 1 to 3 is preferable, and 1 or 3 is more preferable.

As the group represented by the general formula (1-5), -CH₂-O-CH₂-, -CH₂-O-(CH₂)₂-, -CH₂-O-(CH₂)₃-, -(CH₂)₂-O-CH₂-, -(CH₂)₂-O-(CH₂)₂-, -(CH₂)₂-O-(CH₂)₃-, -(CH₂)₃-O-CH₂-, -(CH₂)₃-O-(CH₂)₂-, and -(CH₂)₃-O-(CH₂)₃- are preferable, and -CH₂-O-CH₂- and -CH₂-O-(CH₂)₃- are more preferable.

The phenylene group represented by Y₃ in the general formula (1-2) is any one of an o-phenylene group, a m-phenylene group, and a p-phenylene group. Among these, the o-phenylene group and the p-phenylene group are preferable, and the o-phenylene group is more preferable.

As Y₃ in the general formula (1-2), -OCO-, -NHCO-, and -NHCONH- are preferable.

Specific examples preferred as the group represented by the general formula (1-2) include a group represented by the following general formula (1-6).

-(CH₂)ₕ₄-Y₃-(CH₂)ₕ₅- (1-6)

(In the formula, Y₃ is the same as described above, h₄ and h₅ each independently represent an integer of 1 to 5, and h₄ + h₅ equals an integer of 2 to 6.)

It is preferable that h₄ and h₅ in the general formula (1-6) are the same as each other. In addition, each of h₄ and h₅ is preferably an integer of 1 to 3 and more preferably 2.

Specific examples of the group represented by the general formula (1-6) include -CH₂-OCO-CH₂-, -(CH₂)₂-OCO-(CH₂)₂-, -(CH₂)₃-OCO-(CH₂)₃-, -CH₂-COO-CH₂-, -(CH₂)₂-COO-(CH₂)₂-, -(CH₂)₃-COO-(CH₂)₃-, -CH₂-NHCO-CH₂-, -(CH₂)₂-NHCO-(CH₂)₂-, -(CH₂)₃-NHCO-(CH₂)₃-, -CH₂-CONH-CH₂-, -(CH₂)₂-CONH-(CH₂)₂-, -(CH₂)₃-CONH-(CH₂)₃-, -CH₂-NHCONH-CH₂-, -(CH₂)₂-NHCONH-(CH₂)₂-, -(CH₂)₃-NHCONH-(CH₂)₃-, groups represented by the following formulae, and the like. 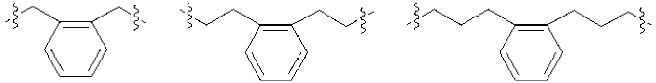 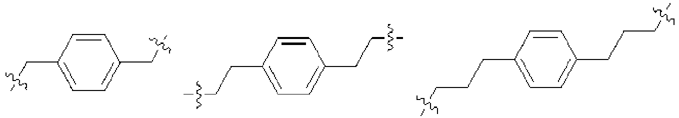 Among these, -(CH₂)₂-OCO-(CH₂)₂-, -(CH₂)₂-NHCO-(CH₂)₂-, and -(CH₂)₂-NHCONH-(CH₂)₂- are preferable.

Examples of the phenylene group represented by R₅₆ in the general formula (1-3) are the same as the examples of the phenylene group represented by Y₃ in the general formula (1-2), and preferred examples are also the same.

"The total number of carbon atoms in the alkylene groups represented by R₅₅ to R₅₇" in the general formula (1-3) means the total number of carbon atoms in R₅₅, R₅₆, and R₅₇ in a case where R₅₆ is an alkylene group having 1 to 4 carbon atoms, and means the total number of carbon atoms in R₅₅ and R₅₇ in a case where R₅₆ is a phenylene group.

As R₅₆ in the general formula (1-3), a methylene group, an ethylene group, a trimethylene group, an o-phenylene group, and a p-phenylene group are preferable, and the ethylene group and the o-phenylene group are more preferable.

As Y₆ in the general formula (1-3), -NHCO- is preferable.

As Y₇ in the general formula (1-3), -COO- is preferable.

Specific examples preferred as the group represented by the general formula (1-3) include a group represented by the following general formula (1-7) or (1-8).

-(CH₂)ₕ₆-Y₆-(CH₂)ₕ₇-Y₇-(CH₂)ₕ₈- (1-7)

(In the formula, Y₆ and Y₇ are the same as described above, h₆ to h₈ each independently represent an integer of 1 to 4, and h₆ + h₇ + h₈ equals an integer of 3 to 6.)

-(CH₂)ₕ₉-Y₆-R₅₈-Y₇-(CH₂)ₕ₁₀- (1-8)

(In the formula, Y₆ and Y₇ are the same as described above, R₅₈ represents a phenylene group, h₉ and h₁₀ each independently represent an integer of 1 to 5, and h₉ + h₁₀ equals an integer of 2 to 6.)

As h₆ and h₇ in the general formula (1-7), 1 or 2 is preferable, and 2 is more preferable.

As h₈ in the general formula (1-7), 1 or 2 is preferable, and 1 is more preferable.

Examples of the preferred combination of Y₆, Y₇, and h₆ to h₈ in the general formula (1-7) include combinations 1 to 32 described in the following table. Among these, the combinations 1 to 4, 13 to 20, and 29 to 32 are preferable, the combinations 17 to 20 are more preferable, and the combination 18 is particularly preferable.

| **Combination** | **Y**₆ | **Y₇** | **h₆** | **h₇** | **h₈** |
|---|---|---|---|---|---|
| **1** | -OCO- | -COO- | 1 | 1 | 1 |
| **2** | -NHCO- | -COO- | 1 | 1 | 1 |
| **3** | -OCO- | -CONH- | 1 | 1 | 1 |
| **4** | -NHCO- | -CONH- | 1 | 1 | 1 |
| **5** | -OCO- | -COO- | 2 | 1 | 1 |
| **6** | -NHCO- | -COO- | 2 | 1 | 1 |
| **7** | -OCO- | -CONH- | 2 | 1 | 1 |
| **8** | -NHCO- | -CONH- | 2 | 1 | 1 |
| **9** | -OCO- | -COO- | 1 | 2 | 1 |
| **10** | -NHCO- | -COO- | 1 | 2 | 1 |
| **11** | -OCO- | -CONH- | 1 | 2 | 1 |
| **12** | -NHCO- | -CONH- | 1 | 2 | 1 |
| **13** | -OCO- | -COO- | 1 | 1 | 2 |
| **14** | -NHCO- | -COO- | 1 | 1 | 2 |
| **15** | -OCO- | -CONH- | 1 | 1 | 2 |
| **16** | -NHCO- | -CONH- | 1 | 1 | 2 |
| **17** | -OCO- | -COO- | 2 | 2 | 1 |
| **18** | -NHCO- | -COO- | 2 | 2 | 1 |
| **19** | -OCO- | -CONH- | 2 | 2 | 1 |
| **20** | -NHCO- | -CONH- | 2 | 2 | 1 |
| **21** | -OCO- | -COO- | 2 | 1 | 2 |
| **22** | -NHCO- | -COO- | 2 | 1 | 2 |
| **23** | -OCO- | -CONH- | 2 | 1 | 2 |
| **24** | -NHCO- | -CONH- | 2 | 1 | 2 |
| **25** | -OCO- | -COO- | 1 | 2 | 2 |
| **26** | -NHCO- | -COO- | 1 | 2 | 2 |
| **27** | -OCO- | -CONH- | 1 | 2 | 2 |
| **28** | -NHCO- | -CONH- | 1 | 2 | 2 |
| **29** | -OCO- | -COO- | 2 | 2 | 2 |
| **30** | -NHCO- | -COO- | 2 | 2 | 2 |
| **31** | -OCO- | -CONH- | 2 | 2 | 2 |
| **32** | -NHCO- | -CONH- | 2 | 2 | 2 |

Examples of the phenylene group represented by R₅₈ in the general formula (1-8) are the same as the examples of the phenylene group represented by Y₃ in the general formula (1-2), and preferred examples are also the same.

As h₉ in the general formula (1-8), 1 or 2 is preferable, and 2 is more preferable.

As h₁₀ in the general formula (1-8), 1 or 2 is preferable, and 1 is more preferable.

Examples of the preferred combination of R₅₈, Y₆, Y₇, h₉, and h₁₀ in the general formula (1-8) include combinations 1 to 32 described in the following table. Among these, the combinations 1 to 16 are preferable, the combinations 9 to 12 are more preferable, and the combination 11 is particularly preferable.

| **Combination** | **R₅₈** | **Y₆** | **Y₇** | **h₉** | **h₁₀** |
|---|---|---|---|---|---|
| **1** | o-Phenylene group | -OCO- | -COO- | 1 | 1 |
| **2** | o-Phenylene group | -NHCO- | -COO- | 1 | 1 |
| **3** | o-Phenylene group | -OCO- | -CONH- | 1 | 1 |
| **4** | o-Phenylene group | -NHCO- | -CONH- | 1 | 1 |
| **5** | o-Phenylene group | -OCO- | -COO- | 1 | 2 |
| **6** | o-Phenylene group | -NHCO- | -COO- | 1 | 2 |
| **7** | o-Phenylene group | -OCO- | -CONH- | 1 | 2 |
| **8** | o-Phenylene group | -NHCO- | -CONH- | 1 | 2 |
| **9** | o-Phenylene group | -OCO- | -COO- | 2 | 1 |
| **10** | o-Phenylene group | -NHCO- | -COO- | 2 | 1 |
| **11** | o-Phenylene group | -OCO- | -CONH- | 2 | 1 |
| **12** | o-Phenylene group | -NHCO- | -CONH- | 2 | 1 |
| **13** | o-Phenylene group | -OCO- | -COO- | 2 | 2 |
| **14** | o-Phenylene group | -NHCO- | -COO- | 2 | 2 |
| **15** | o-Phenylene group | -OCO- | -CONH- | 2 | 2 |
| **16** | o-Phenylene group | -NHCO- | -CONH- | 2 | 2 |
| **17** | p-Phenylene group | -OCO- | -COO- | 1 | 1 |
| **18** | p-Phenylene group | -NHCO- | -COO- | 1 | 1 |
| **19** | p-Phenylene group | -OCO- | -CONH- | 1 | 1 |
| **20** | p-Phenylene group | -NHCO- | -CONH- | 1 | 1 |
| **21** | p-Phenylene group | -OCO- | -COO- | 1 | 2 |
| **22** | p-Phenylene group | -NHCO- | -COO- | 1 | 2 |
| **23** | p-Phenylene group | -OCO- | -CONH- | 1 | 2 |
| **24** | p-Phenylene group | -NHCO- | -CONH- | 1 | 2 |
| **25** | p-Phenylene group | -OCO- | -COO- | 2 | 1 |
| **26** | p-Phenylene group | -NHCO- | -COO- | 2 | 1 |
| **27** | p-Phenylene group | -OCO- | -CONH- | 2 | 1 |
| **28** | p-Phenylene group | -NHCO- | -CONH- | 2 | 1 |
| **29** | p-Phenylene group | -OCO- | -COO- | 2 | 2 |
| **30** | p-Phenylene group | -NHCO- | -COO- | 2 | 2 |
| **31** | p-Phenylene group | -OCO- | -CONH- | 2 | 2 |
| **32** | p-Phenylene group | -NHCO- | -CONH- | 2 | 2 |

In a case where R₅₈, Y₆, Y₇, h₉, and h₁₀ in the general formula (1-8) are combined as the combinations 9 to 12, the general formula (1-8) is respectively represented by the following formulae (121) to (124).

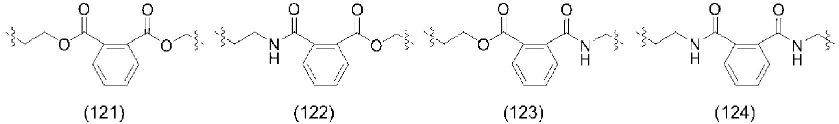

As A₁ in the general formula (1), an alkylene group having 1 to 6 carbon atoms, the groups represented by the general formulae (1-1) to (1-3), and a single bond are preferable, a linear alkylene group having 1 to 6 carbon atoms, the groups represented by the general formulae (1-5) to (1-8), and the single bond are more preferable, and a linear alkylene group having 1 to 3 carbon atoms, the group represented by the general formula (1-7) or (1-8), and the single bond are even more preferable. Specifically, a methylene group, an ethylene group, a trimethylene group, -(CH₂)₂-OCO-(CH₂)₂-COO-CH₂-, -(CH₂)₂-NHCO-(CH₂)₂-COO-CH₂-, -(CH₂)₂-OCO-(CH₂)₂-CONH-CH₂-, -(CH₂)₂-NHCO-(CH₂)₂-CONH-CH₂-, the groups represented by the formulae (121) to (124), and a single bond are preferable, the methylene group, -(CH₂)₂-NHCO-(CH₂)₂-COO-CH₂-, the group represented by the formula (122), and the single bond are more preferable, and the methylene group is particularly preferable.

In a case where A₂ in the general formula (1) represents "an alkylene group having 1 to 6 carbon atoms that may have -O- in a chain and has 1 to 6 groups represented by the general formula (2) in the chain or on the terminal of the chain", examples of the alkylene group having 1 to 6 carbon atoms are the same as the examples of the alkylene group having 1 to 6 carbon atoms represented by A₁ in the general formula (1), and preferred examples are also the same.

The alkyl group having 1 to 6 carbon atoms represented by R₃ in the general formula (2) is any one of linear, branched, and cyclic alkyl groups. Among these, linear and branched alkyl groups are preferable. In addition, among the alkyl group having 1 to 6 carbon atoms, an alkyl group having 1 to 4 carbon atoms is preferable. Specific examples thereof include a methyl group, an ethyl group, an n-propyl group, an isopropyl group, an n-butyl group, an isobutyl group, a sec-butyl group, a tert-butyl group, a cyclobutyl group, an n-pentyl group, an isopentyl group, a sec-pentyl group, a tert-pentyl group, a neopentyl group, a 2-methyl butyl group, a 1,2-dimethyl propyl group, a 1-ethyl propyl group, a cyclopentyl group, an n-hexyl group, an isohexyl group, a sec-hexyl group, a tert-hexyl group, a neohexyl group, a 2-methyl pentyl group, a 1,2-dimethyl butyl group, a 2,3-dimethyl butyl group, a 1-ethyl butyl group, a cyclohexyl group, and the like. Among these, the methyl group, the ethyl group, the n-propyl group, the n-butyl group, the n-pentyl group, and the n-hexyl group are preferable, and the methyl group, the ethyl group, the n-propyl group, and the n-butyl group are more preferable.

In a case where A₃ in the general formula (2) and the general formula (2-1) represents "an alkylene group having 1 to 6 carbon atoms that may have -O- in a chain", examples of the alkylene group having 1 to 6 carbon atoms are the same as the examples of the alkylene group having 1 to 6 carbon atoms represented by A₁ in the general formula (1), and preferred examples are also the same.

Specific examples of the alkylene group having 1 to 6 carbon atoms that has -O- in a chain, represented by A₃ in the general formula (2) and the general formula (2-1), include the group represented by the general formula (1-1). Among these, the group represented by the general formula (1-4) is preferable, and the group represented by the general formula (1-5) is more preferable. More specifically, -CH₂-O-CH₂-, -CH₂-O-(CH₂)₂-, -CH₂-O-(CH₂)₃-, -(CH₂)₂-O-CH₂-, -(CH₂)₂-O-(CH₂)₂-, -(CH₂)₂-O-(CH₂)₃-,-(CH₂)₃-O-CH₂-,-(CH₂)₃-O-(CH₂)₂-, and -(CH₂)₃-O-(CH₂)₃- are preferable, and -CH₂-O-(CH₂)₃- is more preferable.

As A₃ in the general formula (2), a linear alkylene group having 1 to 6 carbon atoms, the group represented by the general formula (1-4), and a single bond are preferable, and a linear alkylene group having 1 to 3 carbon atoms, the group represented by the general formula (1-5), and the single bond are more preferable. Specifically, a methylene group, an ethylene group, a trimethylene group, a tetramethylene group, a pentamethylene group, a hexamethylene group, -CH₂-O-CH₂-, -CH₂-O-(CH₂)₂-, -CH₂-O-(CH₂)₃-, -CH₂-O-(CH₂)₄-, -CH₂-O-(CH₂)₅-, -(CH₂)₂-O-CH₂-, -(CH₂)₂-O-(CH₂)₂-, -(CH₂)₂-O-(CH₂)₃-, -(CH₂)₂-O-(CH₂)₄-, -(CH₂)₃-O-CH₂-, -(CH₂)₃-O-(CH₂)₂-, -(CH₂)₃-O-(CH₂)₃-, -(CH₂)₄-O-CH₂-, -(CH₂)₄-O-(CH₂)₂-, -(CH₂)₅-O-CH₂-, and a single bond are preferable, the methylene group, the ethylene group, the trimethylene group, -CH₂-O-CH₂-, -CH₂-O-(CH₂)₂-, -CH₂-O-(CH₂)₃-, -(CH₂)₂-O-CH₂-, -(CH₂)₂-O-(CH₂)₂-, -(CH₂)₂-O-(CH₂)₃-, -(CH₂)₃-O-CH₂-, -(CH₂)₃-O-(CH₂)₂-, -(CH₂)₃-O-(CH₂)₃-, and the single bond are more preferable, the methylene group, -CH₂-O-(CH₂)₃-, and the single bond are even more preferable, and the methylene group is particularly preferable.

As A₃ in the general formula (2-1), a linear alkylene group having 1 to 6 carbon atoms and the group represented by the general formula (1-4) are preferable, and a linear alkylene group having 1 to 3 carbon atoms and the group represented by the general formula (1-5) are more preferable. Specifically, a methylene group, an ethylene group, a trimethylene group, a tetramethylene group, a pentamethylene group, a hexamethylene group, -CH₂-O-CH₂-, -CH₂-O-(CH₂)₂-, -CH₂-O-(CH₂)₃-, -CH₂-O-(CH₂)₄-, -CH₂-O-(CH₂)₅-, -(CH₂)₂-O-CH₂-, -(CH₂)₂-O-(CH₂)₂-, -(CH₂)₂-O-(CH₂)₃-, -(CH₂)₂-O-(CH₂)₄-, -(CH₂)₃-O-CH₂-, -(CH₂)₃-O-(CH₂)₂-, -(CH₂)₃-O-(CH₂)₃-, -(CH₂)₄-O-CH₂-, -(CH₂)₄-O-(CH₂)₂-, and -(CH₂)₅-O-CH₂- are preferable, the methylene group, the ethylene group, the trimethylene group, -CH₂-O-CH₂-, -CH₂-O-(CH₂)₂-, -CH₂-O-(CH₂)₃-, -(CH₂)₂-O-CH₂-, -(CH₂)₂-O-(CH₂)₂-, -(CH₂)₂-O-(CH₂)₃-, -(CH₂)₃-O-CH₂-, -(CH₂)₃-O-(CH₂)₂-, and -(CH₂)₃-O-(CH₂)₃- are more preferable, the methylene group and -CH₂-O-(CH₂)₃- are even more preferable, and the methylene group is particularly preferable.

Specific examples preferred as the group represented by the general formula (2-1) include a group represented by the following general formula (2-2).

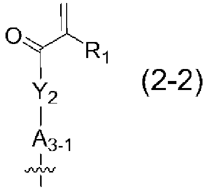

(In the formula, R₁ and Y₂ are the same as described above, and A₃₋₁ represents a linear alkylene group having 1 to 6 carbon atoms or the group represented by the general formula (1-4).)

As the linear alkylene group having 1 to 6 carbon atoms represented by A₃₋₁ in the general formula (2-2), a linear alkylene group having 1 to 3 carbon atoms is preferable. Specific examples thereof include a methylene group, an ethylene group, a trimethylene group, a tetramethylene group, a pentamethylene group, and a hexamethylene group. Among these, the methylene group, the ethylene group, and the trimethylene group are preferable, and the methylene group is more preferable.

As A₃₋₁ in the general formula (2-2), a linear alkylene group having 1 to 3 carbon atoms and the group represented by the general formula (1-5) are preferable. Specifically, a methylene group, an ethylene group, a trimethylene group, -CH₂-O-CH₂-, -CH₂-O-(CH₂)₂-, -CH₂-O-(CH₂)₃-, -(CH₂)₂-O-CH₂-, -(CH₂)₂-O-(CH₂)₂-, -(CH₂)₂-O-(CH₂)₃-, -(CH₂)₃-O-CH₂-, -(CH₂)₃-O-(CH₂)₂-, and -(CH₂)₃-O-(CH₂)₃- are preferable, and the methylene group and -CH₂-O-(CH₂)₃- are more preferable, and the methylene group is particularly preferable.

Specific examples preferred as the group represented by the general formula (2-2) include a group represented by the following general formula (2-3).

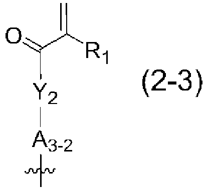

(In the formula, R₁ and Y₂ are the same as described above, and A₃₋₂ represents a linear alkylene group having 1 to 3 carbon atoms or the group represented by the general formula (1-5).)

Specific examples of the linear alkylene group having 1 to 3 carbon atoms represented by A₃₋₂ in the general formula (2-3) include a methylene group, an ethylene group, and a trimethylene group. Among these, the methylene group is preferable.

As A₃₋₂ in the general formula (2-3), a methylene group and -CH₂-O-(CH₂)₃- are preferable, and the methylene group is particularly preferable.

Specific examples of the group represented by the general formula (2-3) include groups shown below (hereinafter, simply described as a group (1) of functional groups in some cases).

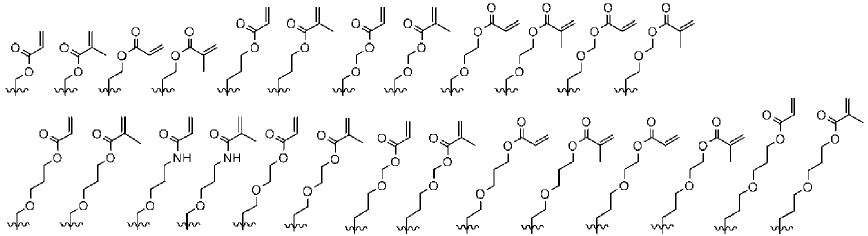

Among the above specific examples, groups represented by the following formulae (131) to (136) are preferable. Among these, the group represented by the formula (131), (133), or (135) is more preferable, and the group represented by the formula (131) is particularly preferable.

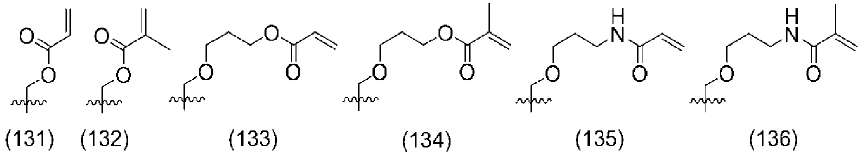

As R₃ in the general formula (2), a hydrogen atom, a linear alkyl group having 1 to 6 carbon atoms, and the group represented by the general formula (2-2) are preferable, and the hydrogen atom and the group represented by the general formula (2-3) are more preferable. Specifically, a hydrogen atom, a methyl group, an ethyl group, an n-propyl group, an n-butyl group, and the group (1) of functional groups are preferable, the hydrogen atom and the groups represented by the formulae (131) to (136) are more preferable, the hydrogen atom and the group represented by the formula (131), (133), or (135) are even more preferable, and the group represented by the formula (131) is particularly preferable.

Specific examples preferred as the group represented by the general formula (2) include a group represented by the following general formula (2').

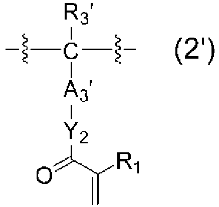

(In the formula, R₁ and Y₂ are the same as described above, R₃' represents a hydrogen atom, a linear alkyl group having 1 to 6 carbon atoms, or the group represented by the general formula (2-2), and A₃' represents a linear alkylene group having 1 to 6 carbon atoms, the group represented by the general formula (1-4), or a single bond.)

As the linear alkyl group having 1 to 6 carbon atoms represented by R₃' in the general formula (2'), a linear alkyl group having 1 to 4 carbon atoms is preferable. Specific examples thereof include a methyl group, an ethyl group, an n-propyl group, an n-butyl group, an n-pentyl group, and an n-hexyl group. Among these, the methyl group, the ethyl group, the n-propyl group, and the n-butyl group are preferable.

As R₃' in the general formula (2'), a hydrogen atom and the group represented by the general formula (2-3) are preferable. Specific examples thereof include a hydrogen atom, the group (1) of functional groups, and the like. Among these, the hydrogen atom and the groups represented by the formulae (131) to (136) are more preferable, the hydrogen atom and the group represented by the formula (131), (133), or (135) are even more preferable, and the group represented by the formula (131) is particularly preferable.

Examples of the linear alkylene group having 1 to 6 carbon atoms represented by A₃' in the general formula (2') are the same as the examples of the linear alkylene group having 1 to 6 carbon atoms represented by A₃₋₁ in the general formula (2-2), and preferred examples are also the same.

As A₃' in the general formula (2'), a linear alkylene group having 1 to 3 carbon atoms, the group represented by the general formula (1-5), and a single bond are preferable. Specifically, a methylene group, an ethylene group, a trimethylene group, -CH₂-O-CH₂-, -CH₂-O-(CH₂)₂-, -CH₂-O-(CH₂)₃-, -(CH₂)₂-O-CH₂-, -(CH₂)₂-O-(CH₂)₂-, -(CH₂)₂-O-(CH₂)₃-, -(CH₂)₃-O-CH₂-, -(CH₂)₃-O-(CH₂)₂-, -(CH₂)₃-O-(CH₂)₃-, and the single bond are preferable, and the methylene group, -CH₂-O-(CH₂)₃-, and the single bond are more preferable, and the methylene group is particularly preferable.

Specific examples preferred as the group represented by the general formula (2') include a group represented by the following general formula (2").

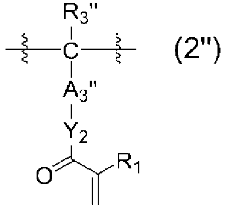

(In the formula, R₁ and Y₂ are the same as described above, R₃" represents a hydrogen atom or the group represented by the general formula (2-3), and A₃" represents a linear alkylene group having 1 to 3 carbon atoms, the group represented by the general formula (1-5), or a single bond.)

As R₃" in the general formula (2"), a hydrogen atom and the groups represented by the formulae (131) to (136) are preferable, the hydrogen atom and the group represented by the formula (131), (133), or (135) are more preferable, and the group represented by the formula (131) is particularly preferable.

Examples of the linear alkylene group having 1 to 3 carbon atoms represented by A₃" in the general formula (2") include a methylene group, an ethylene group, and a trimethylene group. Among these, the methylene group is preferable.

As A₃" in the general formula (2"), a methylene group, -CH₂-O-(CH₂)₃-, and a single bond are preferable, and the methylene group is more preferable.

Specific examples preferred as the group represented by the general formula (2") include groups represented by the following formulae (141) to (150). Among these, the group represented by the formula (141), (143), (145), (147), or (149) is preferable, and the group represented by the formula (145) is more preferable.

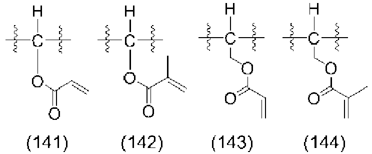

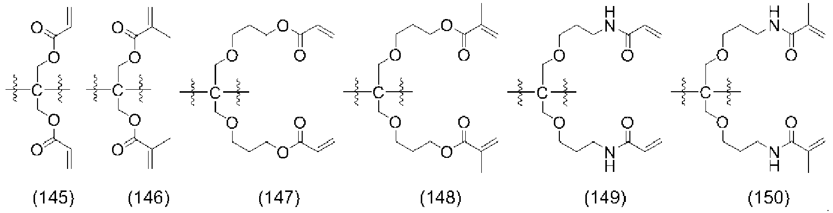

In a case where the number of the groups represented by the general formula (2) that A₂ in the general formula (1) has in a chain or on the terminal of the chain is 1, R₃ in the general formula (2) is the group represented by the general formula (2-1). In other words, in a case where R₃ in the general formula (2) is a hydrogen atom or an alkyl group having 1 to 6 carbon atoms, the number of the groups represented by the general formula (2) that A₂ in the general formula (1) has in a chain or on the terminal of the chain is 2 to 6, and in a case where R₃ in the general formula (2) is the group represented by the general formula (2-1), the number of the groups represented by the general formula (2) that A₂ in the general formula (1) has in a chain or on the terminal of the chain is 1 to 6.

In a case where R₃ is a hydrogen atom or an alkyl group having 1 to 6 carbon atoms, the number of the groups represented by the general formula (2) that A₂ in the general formula (1) has in a chain or on the terminal of the chain is preferably 2 to 6. In a case where R₃ is the group represented by the general formula (2-1), the number of the groups represented by the general formula (2) that A₂ in the general formula (1) has in a chain or on the terminal of the chain is preferably 1 to 3. In a case where R₃ is a hydrogen atom or an alkyl group having 1 to 6 carbon atoms, the number of the groups represented by the general formula (2) that A₂ in the general formula (1) has in a chain or on the terminal of the chain is more preferably 2 to 4. In a case where R₃ is the group represented by the general formula (2-1), the number of the groups represented by the general formula (2) that A₂ in the general formula (1) has in a chain or on the terminal of the chain is more preferably 1 or 2.

Specific examples of A₂ in the general formula (1) include a group represented by the following general formula (3).

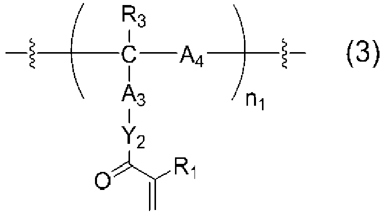

(In the formula, R₁, R₃, A₃, and Y₂ are the same as described above, A₄ represents an alkylene group having 1 to 6 carbon atoms that may have -O- in a chain, or represents a single bond, and n₁ represents an integer of 1 to 6. Here, a plurality of R₁'s, a plurality of R₃'s, a plurality of A₃'s, a plurality of A₄'s, and a plurality of Y₂'s are the same as or different from each other respectively, the total number of carbon atoms in n₁ pieces of A₄ is 1 to 6, and in a case where n₁ is 1, R₃ represents the group represented by the general formula (2-1).)

In a case where A₄ in the general formula (3) represents "an alkylene group having 1 to 6 carbon atoms that may have -O- in a chain", examples of the alkylene group having 1 to 6 carbon atoms are the same as the examples of the alkylene group having 1 to 6 carbon atoms represented by A₁ in the general formula (1), and preferred examples are also the same.

Specific examples of the alkylene group having 1 to 6 carbon atoms that may have -O- in a chain, represented by A₄ in the general formula (3), include the group represented by the general formula (1-1). Among these, the group represented by the general formula (1-4) is preferable, and the group represented by the general formula (1-5) is more preferable. More specifically, specific examples of the alkylene group are the same as the specific examples of the group represented by the general formula (1-5), and preferred examples are also the same.

As A₄ in the general formula (3), a linear alkylene group having 1 to 6 carbon atoms, the group represented by the general formula (1-4), and a single bond are preferable, and a linear alkylene group having 1 to 3 carbon atoms, the group represented by the general formula (1-5), and the single bond are more preferable. Specifically, a methylene group, an ethylene group, a trimethylene group, a tetramethylene group, a pentamethylene group, a hexamethylene group, -CH₂-O-CH₂-, -CH₂-O-(CH₂)₂-, -CH₂-O-(CH₂)₃-, -CH₂-O-(CH₂)₄-, -CH₂-O-(CH₂)₅-, -(CH₂)₂-O-CH₂-, -(CH₂)₂-O-(CH₂)₂-, -(CH₂)₂-O-(CH₂)₃-, -(CH₂)₂-O-(CH₂)₄-, -(CH₂)₃-O-CH₂-, -(CH₂)₃-O-(CH₂)₂-, -(CH₂)₃-O-(CH₂)₃-, -(CH₂)₄-O-CH₂-, -(CH₂)₄-O-(CH₂)₂-, -(CH₂)₅-O-CH₂-, and the single bond are preferable, the methylene group, the ethylene group, the trimethylene group, -CH₂-O-CH₂-, -CH₂-O-(CH₂)₂-, -CH₂-O-(CH₂)₃-, -(CH₂)₂-O-CH₂-, -(CH₂)₂-O-(CH₂)₂-, -(CH₂)₂-O-(CH₂)₃-, -(CH₂)₃-O-CH₂-, -(CH₂)₃-O-(CH₂)₂-, -(CH₂)₃-O-(CH₂)₃-, and the single bond are more preferable, the methylene group, -CH₂-O-CH₂-, -CH₂-O-(CH₂)₃-, and the single bond are even more preferable, and the methylene group and -CH₂-O-CH₂- are particularly preferable.

In a case where R₃ is a hydrogen atom or an alkyl group having 1 to 6 carbon atoms, n₁ in the general formula (3) is preferably an integer of 2 to 6, and more preferably an integer of 2 to 4. In addition, in a case where R₃ is the group represented by the general formula (2-1), n₁ is preferably an integer of 1 to 3, and more preferably 1 or 2.

Specific examples preferred as the group represented by the general formula (3) include a group represented by the following general formula (4-1) or (4-2). Among these, the group represented by the general formula (4-2) is preferable.

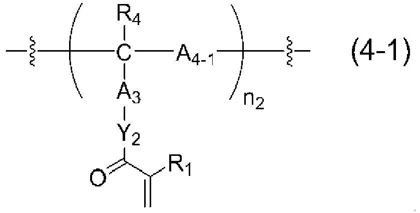

(In the formula, R₁, A₃, and Y₂ are the same as described above, R₄ represents a hydrogen atom or an alkyl group having 1 to 6 carbon atoms, A₄₋₁ represents an alkylene group having 1 to 6 carbon atoms or a single bond, and n₂ represents an integer of 2 to 6. Here, a plurality of R₁'s, a plurality of R₄'s, a plurality of A₃'s, a plurality of A₄₋₁'s, and a plurality of Y₂'s are the same as or different from each other respectively, and the total number of carbon atoms in n₂ pieces of A₄₋₁ is 1 to 6.)

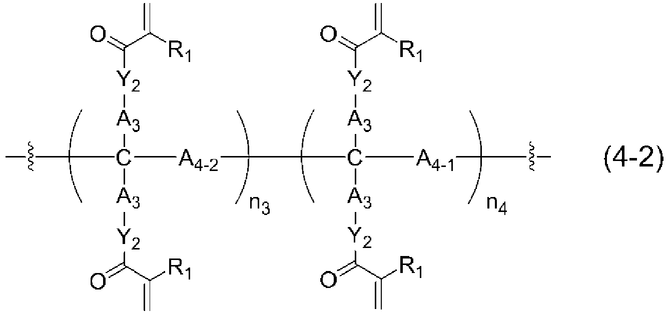

(In the formula, R₁, A₃, A₄₋₁, and Y₂ are the same as described above, A₄₋₂ represents an alkylene group having 1 to 6 carbon atoms that has -O- in a chain, n₃ and n₄ each independently represent an integer of 0 to 3, and n₃ + n₄ equals an integer of 1 to 3. Here, a plurality of R₁'s, a plurality of A₃'s, a plurality of A₄₋₁'s, a plurality of A₄₋₂'s, and a plurality of Y₂'s are the same as or different from each other respectively, and the total number of carbon atoms in n₄ pieces of A₄₋₁ and n₃ pieces of A₄₋₂ is 1 to 6.)

Examples of the alkyl group having 1 to 6 carbon atoms represented by R₄ in the general formula (4-1) are the same as the examples of the alkyl group having 1 to 6 carbon atoms represented by R₃ in the general formula (2), and preferred examples are also the same.

As R₄ in the general formula (4-1), a hydrogen atom and a linear alkyl group having 1 to 6 carbon atoms are preferable, and the hydrogen atom is more preferable. Specifically, a hydrogen atom, a methyl group, an ethyl group, an n-propyl group, and an n-butyl group are preferable, and the hydrogen atom is more preferable.

Examples of the alkylene group having 1 to 6 carbon atoms represented by A₄₋₁ in the general formula (4-1) are the same as the examples of the alkylene group having 1 to 6 carbon atoms represented by A₁ in the general formula (1), and preferred examples are also the same.

As A₄₋₁ in the general formula (4-1), a linear alkylene group having 1 to 6 carbon atoms and a single bond are preferable. Specifically, a methylene group, an ethylene group, a trimethylene group, a tetramethylene group, a pentamethylene group, a hexamethylene group, and a single bond are preferable, the methylene group, the ethylene group, the trimethylene group, and the single bond are more preferable, and the methylene group and the single bond are particularly preferable.

As n₂ in the general formula (4-1), an integer of 2 to 4 is preferable, and 4 is more preferable.

Examples of the alkylene group having 1 to 6 carbon atoms that has -O- in a chain, represented by A₄₋₂ in the general formula (4-2), are the same as the examples of the alkylene group having 1 to 6 carbon atoms that has -O- in a chain, represented by A₄ in the general formula (3), and preferred examples are also the same.

As the combination of n₃ and n₄ in the general formula (4-2), combinations 1 to 9 described in the following table can be considered. Among these, the combinations 1, 4, and 5 are preferable, and the combination 5 is more preferable.

| | **Combination 1** | **Combination 2** | **Combination** | **Combination 4** | **Combination 5** | **Combination 6** | **Combination 7** | **Combination 8** | **Combination 9** |
|---|---|---|---|---|---|---|---|---|---|
| **n₃** | 0 | 0 | 0 | 1 | 1 | 1 | 2 | 2 | 3 |
| **n₄** | 1 | 2 | 3 | 0 | 1 | 2 | 0 | 1 | 0 |

Specific examples preferred as the group represented by the general formula (4-1) include a group represented by the following general formula (4-1').

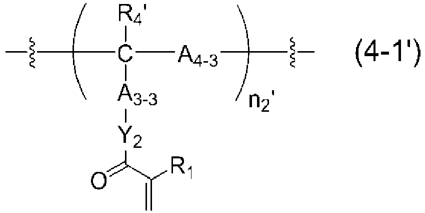

(In the formula, R₁ and Y₂ are the same as described above, R₄' represents a hydrogen atom or a linear alkyl group having 1 to 6 carbon atoms, A₃₋₃ and A₄₋₃ each independently represent a linear alkylene group having 1 to 6 carbon atoms or a single bond, and n₂' represents an integer of 2 to 4. Here, a plurality of R₁'s, a plurality of R₄"s, a plurality of A₃₋₃'s, a plurality of A₄₋₃'s, and a plurality of Y₂"s are the same as or different from each other respectively, and the total number of carbon atoms in n₂' pieces of A₄₋₃ is 1 to 6.)

Examples of the linear alkyl group having 1 to 6 carbon atoms represented by R₄' in the general formula (4-1') are the same as the examples of the linear alkyl group having 1 to 6 carbon atoms represented by R₃' in the general formula (2'), and preferred examples are also the same.

As R₄' in the general formula (4-1'), a hydrogen atom is preferable.

Examples of the linear alkylene group having 1 to 6 carbon atoms represented by A₃₋₃ and A₄₋₃ in the general formula (4-1') are the same as the examples of the linear alkylene group having 1 to 6 carbon atoms represented by A₃₋₁ in the general formula (2-2), and preferred examples are also the same.

As A₃₋₃ and A₄₋₃ in the general formula (4-1'), a linear alkylene group having 1 to 3 carbon atoms and a single bond are preferable. Specifically, a methylene group, an ethylene group, a trimethylene group, and a single bond are preferable, and the methylene group and the single bond are more preferable.

As n₂' in the general formula (4-1'), 4 is preferable.

Specific examples preferred as the group represented by the general formula (4-1') include a group represented by the following general formula (4-1").

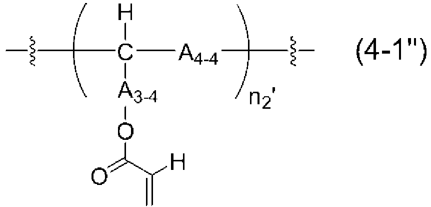

(In the formula, n₂' is the same as described above, A₃₋₄ and A₄₋₄ each independently represent a linear alkylene group having 1 to 3 carbon atoms or a single bond. Here, a plurality of A₃₋₄'s and a plurality of A₄₋₄'s are the same as or different from each other respectively, and the total number of carbon atoms in n₂' pieces of A₄₋₄ is 1 to 6.)

Examples of the linear alkylene group having 1 to 3 carbon atoms represented by A₃₋₄ and A₄₋₄ in the general formula (4-1") include a methylene group, an ethylene group, and a trimethylene group. Among these, the methylene group is preferable.

As A₃₋₄ and A₄₋₄ in the general formula (4-1"), a methylene group and a single bond are preferable.

Examples of the preferred combination of (n₂' pieces of) A₃₋₄, (n₂' pieces of) A₄₋₄, and n₂' in the general formula (4-1") include combinations 1 to 12 described in the following table. Among these, the combinations 1, 2, 4, 5, 8, and 9 are preferable, the combinations 8 and 9 are more preferable, and the combination 9 is particularly preferable.

| **Combination** | **(n₂' pieces of) A₃₋₄** | **(n₂' pieces of) A₄₋₄** | **n₂'** |
|---|---|---|---|
| **1** | Two single bonds | One single bond and one methylene group | 2 |
| **2** | One single bond and one methylene group | One single bond and one methylene group | 2 |
| **3** | Two methylene groups | One single bond and one methylene group | 2 |
| **4** | Three single bonds | Two single bonds and one methylene group | 3 |
| **5** | Two single bonds and one methylene group | Two single bonds and one methylene group | 3 |
| **6** | One single bond and two methylene groups | Two single bonds and one methylene group | 3 |
| **7** | Three methylene groups | Two single bonds and one methylene group | 3 |
| **8** | Four single bonds | Three single bonds and one methylene group | 4 |
| **9** | Three single bonds and one methylene group | Three single bonds and one methylene group | 4 |
| **10** | Two single bonds and two methylene groups | Three single bonds and one methylene group | 4 |
| **11** | One single bond and three methylene groups | Three single bonds and one methylene group | 4 |
| **12** | Four methylene groups | Three single bonds and one methylene group | 4 |

Specific examples preferred as the group represented by the general formula (4-1") include the following groups (hereinafter, simply described as a group (2-1) of functional groups in some cases).

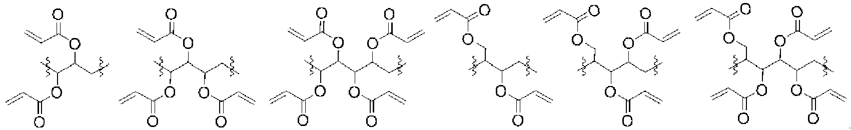

Specific examples preferred as the group represented by the general formula (4-2) include a group represented by the following general formula (4-2').

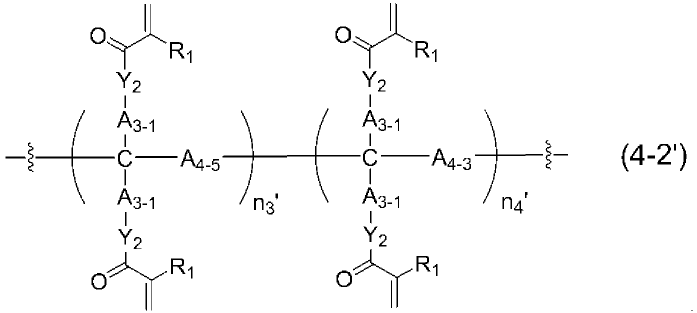

(In the formula, R₁, A₃₋₁, A₄₋₃, and Y₂ are the same as described above, A₄₋₅ represents the group represented by the general formula (1-4), and n₃' and n₄' each independently represent 0 or 1. Here, a plurality of R₁'s, a plurality of A₃₋₁'s, and a plurality of Y₂'s are the same as or different from each other respectively, and the total number of carbon atoms in n₃' pieces of A₄₋₃ and n₄' pieces of A₄₋₅ is 1 to 6.)

As A₄₋₅ in the general formula (4-2'), the group represented by the general formula (1-5) is preferable. Specific examples of A₄₋₅ are the same as the specific examples of the group represented by the general formula (1-5), and preferred examples are also the same.

Examples of n₃' and n₄' in the general formula (4-2') include n₃' = 1 and n₄' = 0, n₃' = 0 and n₄' = 1, and n₃' = n₄' = 1. Among these, n₃' = n₄' = 1 is preferable.

In the general formula (4-2'), a plurality of R₁'s, a plurality of A₃₋₁'s, and a plurality of Y₂'s are the same as or different from each other respectively. Among these, it is preferable that they are the same as each other.

Specific examples preferred as the group represented by the general formula (4-2') include a group represented by the following general formula (4-2").

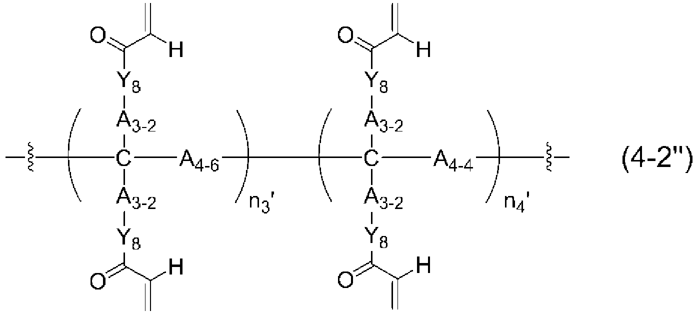

(In the formula, A₃₋₂, A₄₋₄, n₃', and n₄' are the same as described above, Y₈ represents -O- or -NH-, and A₄₋₆ represents the group represented by the general formula (1-5). Here, a plurality of A₃₋₂'s and a plurality of Y₈'s are the same as each other respectively, and the total number of carbon atoms in n₃' pieces of A₄₋₄ and n₄' pieces of A₄₋₆ is 1 to 6.)

As Y₈ in the general formula (4-2"), -O- is preferable.

Examples of A₄₋₆ in the general formula (4-2") are the same as the specific examples of the group represented by the general formula (1-5), and preferred examples are also the same.

Examples of the preferred combination of (a plurality of) A₃₋₂'s, A₄₋₄, A₄₋₆, (a plurality of) Y₈'s, n₃', and n₄' in the general formula (4-2") include combinations 1 to 15 described in the following table. Among these, the combinations 1, 4, and 9 are preferable, and the combination 4 is more preferable.

| **Combination** | **(a plurality of) A₃₋₂** | **A₄₋₄** | **A₄₋₆** | **(a plurality of) Y₈** | **n₃'** | **n₄'** |
|---|---|---|---|---|---|---|
| **1** | All represent methylene group | Methylene group | N/A | All represent -O- | 0 | 1 |
| **2** | All represent ethylene group | Methylene group | N/A | All represent -O- | 0 | 1 |
| **3** | All represent triethylene group | Methylene group | N/A | All represent -O- | 0 | 1 |
| **4** | All represent methylene group | Methylene group | -CH₂-O-CH₂- | All represent -O- | 1 | 1 |
| **5** | All represent ethylene group | Methylene group | -CH₂-O-CH₂- | All represent -O- | 1 | 1 |
| **6** | All represent trimethylene group | Methylene group | -CH₂-O-CH₂- | All represent -O- | 1 | 1 |
| **7** | All represent -CH₂-O-CH₂- | N/A | -CH₂-O-CH₂- | All represent -O- or all represent -NH- | 1 | 0 |
| **8** | All represent -CH₂-O-(CH2)₂- | N/A | -CH₂-O-(CH2)₂- | All represent -O- or all represent -NH- | 1 | 0 |
| **9** | All represent -CH₂-O-(CH₂)₃- | N/A | -CH₂-O-(CH₂)₃- | All represent -O- or all represent -NH- | 1 | 0 |
| **10** | All represent -(CH₂)₂-O-CH₂- | N/A | -(CH₂)₂-O-CH₂- | All represent -O- or all represent -NH- | 1 | 0 |
| **11** | All represent -(CH₂)₂-O-(CH₂)₂- | N/A | -(CH₂)₂-O-(CH₂)₂- | All represent -O- or all represent -NH- | 1 | 0 |
| **12** | All represent -(CH₂)₂-O-(CH₂)₃- | N/A | -(CH₂)₂-O-(CH₂)₃- | All represent -O- or all represent -NH- | 1 | 0 |
| **13** | All represent -(CH₂)₃-O-CH₂- | N/A | -(CH₂)₃-O-CH₂- | All represent -O- or all represent -NH- | 1 | 0 |
| **14** | All represent -(CH₂)₃-O-(CH₂)₂- | N/A | -(CH₂)₃-O-(CH₂)₂- | All represent -O- or all represent -NH- | 1 | 0 |
| **15** | All represent -(CH₂)₃-O-(CH₂)₃- | N/A | -(CH₂)₃-O-(CH₂)₃- | All represent -O- or all represent -NH- | 1 | 0 |

Specific examples preferred as the group represented by the general formula (4-2") include the following groups (hereinafter, simply described as a group (2-2) of functional groups in some cases).

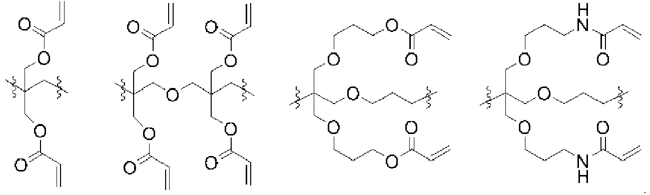

Examples of the combination of A₁ and A₂ in the general formula (1) include a combination in which A₁ is an alkylene group having 1 to 6 carbon atoms or a single bond and A₂ is the group represented by the general formula (4-1); and a combination in which A₁ is an alkylene group having 1 to 6 carbon atoms, the group represented by any one of general formulae (1-1) to (1-3), or a single bond and A₂ is the group represented by the general formula (4-2). Among these, a combination in which A₁ is a linear alkylene group having 1 to 6 carbon atoms or a single bond and A₂ is the group represented by the general formula (4-1'), and a combination in which A₁ is a linear alkylene group having 1 to 6 carbon atoms, a group represented by any one of the general formulae (1-5) to (1-8), or a single bond and A₂ is the group represented by the general formula (4-2') are preferable; a combination in which A₁ is a linear alkylene group having 1 to 3 carbon atoms or a single bond and A₂ is the group represented by the general formula (4-1"), and a combination in which A₁ is a linear alkylene group having 1 to 3 carbon atoms, a group represented by the general formula (1-7) or (1-8), or a single bond and A₂ is the group represented by the general formula (4-2") are more preferable; the combinations 1 to 16 described in the following table are even more preferable; and the combinations 1, 4, 5, 9, 13, and 16 are particularly preferable.

| **Combination** | **A₁** | **A₂** |
|---|---|---|
| **1** | Methylene group | Group (2-1) of functional groups |
| **2** | Ethylene group | Group (2-1) of functional groups |
| **3** | Trimethylene group | Group (2-1) of functional groups |
| **4** | Single bond | Group (2-1) of functional groups |
| **5** | Methylene group | Group (2-2) of functional groups |
| **6** | Ethylene group | Group (2-2) of functional groups |
| **7** | Trimethylene group | Group (2-2) of functional groups |
| **8** | -(CH₂)₂-OCO-(CH₂)₂-COO-CH₂- | Group (2-2) of functional groups |
| **9** | -(CH₂)₂-NHCO-(CH₂)₂-COO-CH₂- | Group (2-2) of functional groups |
| **10** | -(CH₂)₂-OCO-(CH₂)₂-CONH-CH₂- | Group (2-2) of functional groups |
| **11** | -(CH₂)₂-NHCO-(CH₂)₂-CONH-CH₂- | Group (2-2) of functional groups |
| **12** | Group represented by the formula (121) | Group (2-2) of functional groups |
| **13** | Group represented by the formula (122) | Group (2-2) of functional groups |
| **14** | Group represented by the formula (123) | Group (2-2) of functional groups |
| **15** | Group represented by the formula (124) | Group (2-2) of functional groups |
| **16** | Single bond | Group (2-2) of functional groups |

Specific examples preferred as -A₁-A₂- in the general formula (1) include groups represented by the following formulae (101) to (114). Among these, the groups represented by the formulae (103), (106), (107), (108), and (110) to (114) are preferable, the groups represented by the formulae (103), (106), (108), (113), and (114) are more preferable, the groups represented by the formulae (108), (113), and (114) are even more preferable, and the group represented by the formula (108) is particularly preferable.

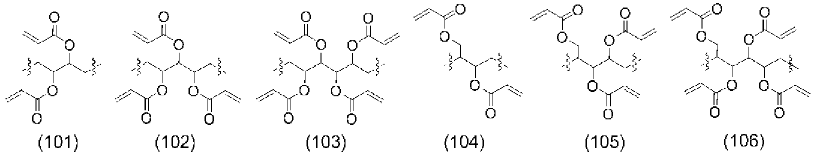

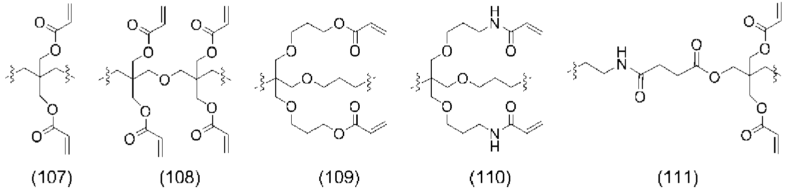

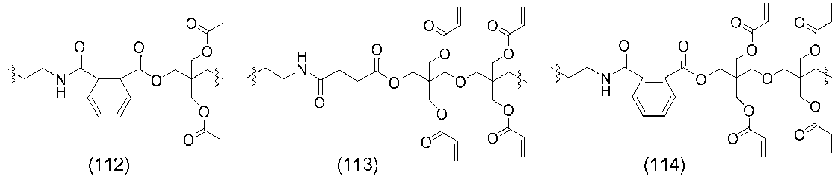

As n in the general formula (1), 1 is preferable.

The dye residue represented by Dye in the general formula (1) is a monovalent residue obtained by removing a group such as hydrogen from the molecule of a dye known in the related art, and may have any of dye skeletons. Examples of such a dye residue include residues derived from a dye such as a xanthene-based dye, a triarylmethane-based dye, a cyanine-based dye, a coumarin-based dye, a stilbene-based dye, a naphthalimide-based dye, a perylene-based dye, a pyridine-based dye, an oxazine-based dye, an olefin-based dye, an azole-based dye, an anthracene-based dye, a thiazine-based dye, a phthalocyanine-based dye, an anthraquinone-based dye, an acridone-based dye, a quinacridone-based dye, an isoindolinone-based dye, a thioflavin-based dye, a thioindigo-based dye, a fluorene-based dye, an azo-based dye, a diphenylmethane-based dye, a terphenyl-based dye, a chrysene-based dye, a pyrene-based dye, and the like. Among these, a residue derived from the xanthene-based dye, the triarylmethane-based dye, or the cyanine-based dye is preferable, and the residue derived from the xanthene-based dye or the cyanine-based dye is more preferable.

Examples of the xanthene-based dye include a rhodamine-based dye such as rhodamine B, rhodamine 6G, rhodamine 3B, rhodamine 101, rhodamine 110, sulforhodamine 101, basic violet 11, and basic red 2; an eosin-based dye such as eosin Y and eosin B; a fluorescein-based dye such as fluorescein and fluorescein isothiocyanate; and the like. Among these, the rhodamine-based dye is preferable.

Examples of the triarylmethane-based dye include a triarylmethane-based dye such as triphenylmethane, basic violet 3, phenol red, phenolphthalein, green S, malachite green, and thymol blue, and the like.

Examples of the cyanine-based dye include a cyanine-based dye such as 4-dicyanomethylene-2-methyl-6-(p-dimethylaminostyryl)-4H-pyran (DCM), basic yellow 11, basic yellow 13, basic red 12, basic red 13, basic red 14, basic red 15, basic red 37, basic violet 15, and basic violet 16, and the like.

Examples of the coumarin-based dye include a coumarin-based dye such as coumarin 6, coumarin 7, coumarin 153, coumarin 314, coumarin 334, coumarin 545, coumarin 545T, coumarin 545P, and 7-hydroxy-4-methyl coumarin, and the like.

Examples of the stilbene-based dye include a stilbene-based dye such as 1,4-bis(2-methylstyryl)benzene and trans-4,4'-diphenylstilbenzene, and the like.

Example of the naphthalimide-based dye include a naphthalimide-based dye such as basic yellow 51, solvent yellow 11, solvent yellow 98, solvent yellow 116, solvent yellow 43, and solvent yellow 44, and the like.

Example of the perylene-based dye include a perylene-based dye such as perylene, Lumogen yellow, Lumogen green, Lumogen orange, Lumogen pink, Lumogen red, solvent orange 5, and solvent green 5, and the like.

Examples of the pyridine-based dye include a pyridine-based dye such as 1-ethyl-2-[4-(p-dimethylaminophenyl)-1,3-butadienyl]-pyridinium-perchlorate (pyridine 1), an acridine-based dye, and the like.

Examples of the oxazine-based dye include an oxazine-based dye such as cresyl violet acetate, a dioxazine-based dye, and the like.

Examples of the olefin-based dye include an ethene-based dye, a butadiene-based dye, a hexatriene-based dye, and the like.

Examples of the azole-based dye include an oxazole-based dye, a thiazole-based dye, and the like.

The dyes described above can be classified into an acidic dye, a basic dye, or a nonionic dye. In the present invention, an acidic dye means an ionic dye in which an anionic portion becomes a chromophore, a basic dye means an ionic dye in which a cationic portion becomes a chromophore, and a nonionic dye means a dye that corresponds to none of the acidic dye and the basic dye (here, an ionic dye forming a salt in a molecule is included in the acidic dye or the basic dye). The dye residue represented by Dye in the general formula (1) is derived from any one of the acidic dye, the basic dye, and the nonionic dye. The dye residue is preferably derived from the acidic dye or the basic dye, and more preferably derived from the basic dye. Specifically, among the residues derived from the dyes described above, the residue derived from the basic dye is preferable. Among these, a residue derived from a xanthene-based basic dye, a triarylmethane-based basic dye, or a cyanine-based basic dye is preferable, the residue derived from the xanthene-based basic dye or the cyanine-based basic dye is more preferable, and the residue derived from a rhodamine-based dye or the cyanine-based basic dye are particularly preferable.

Specific examples of the dye residue represented by Dye in the general formula (1) include dye residues represented by the following general formula (I), the following general formula (III), and the following general formula (IV).

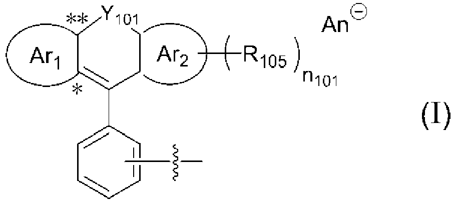

{In the formula, n₁₀₁ pieces of R₁₀₅ each independently represent a halogeno group, an alkyl group having 1 to 30 carbon atoms, an alkoxy group having 1 to 20 carbon atoms, an alkylthio group having 1 to 20 carbon atoms, an amino group which has a substituent or is unsubstituted, a hydroxy group, an aryl group having 6 to 14 carbon atoms, an aryloxy group having 6 to 14 carbon atoms, or an arylalkyl group having 7 to 20 carbon atoms, Y₁₀₁ represents an oxygen atom, a sulfur atom, or -NR₁₃₂-, R₁₃₂ represents an alkyl group having 1 to 6 carbon atoms, An⁻ represents an anion, and Ar₁ represents a ring structure represented by any one of the following general formulae (I-1-1) to (I-1-7);

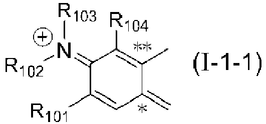

(In the formula, R₁₀₁ and R₁₀₄ each independently represent a hydrogen atom or a methyl group, R₁₀₂ and R₁₀₃ each independently represent an alkyl group having 1 to 30 carbon atoms or an aryl group having 6 to 14 carbon atoms that has a substituent or is unsubstituted, R₁₀₁ and R₁₀₂ may form an alkylene group having 2 to 4 carbon atoms together, and R₁₀₃ and R₁₀₄ may form an alkylene group having 2 to 4 carbon atoms together.),

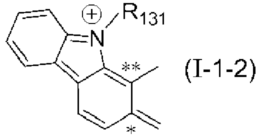

(In the formula, R₁₃₁ represents an alkyl group having 1 to 30 carbon atoms.),

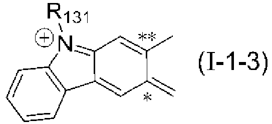

(In the formula, R₁₃₁ is the same as described above.),

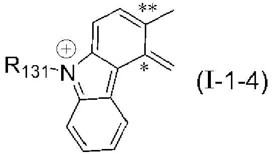

(In the formula, R₁₃₁ is the same as described above.),

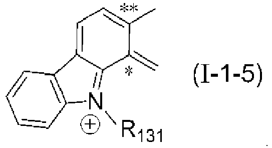

(In the formula, R₁₃₁ is the same as described above.),

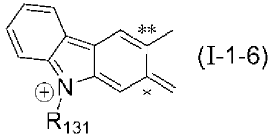

(In the formula, R₁₃₁ is the same as described above.),

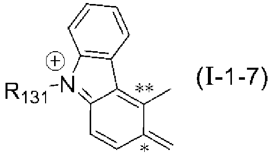

(In the formula, R₁₃₁ is the same as described above.),
* and ** represent each binding position, Ar₂ represents a benzene ring, a naphthalene ring, or an anthracene ring. In a case where Ar₂ is the benzene ring, n₁₀₁ represents an integer of 0 to 4. In a case where Ar₂ is the naphthalene ring, n₁₀₁ represents an integer of 0 to 6. In a case where Ar₂ is the anthracene ring, n₁₀₁ represents an integer of 0 to 8.}

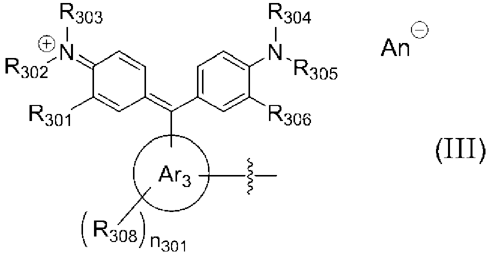

(In the formula, An⁻ is the same as described above, R₃₀₁ and R₃₀₆ each independently represent a hydrogen atom or a methyl group, R₃₀₂ to R₃₀₅ each independently represent an alkyl group having 1 to 30 carbon atoms or an aryl group having 6 to 14 carbon atoms that has a substituent or is unsubstituted, n₃₀₁ pieces of R₃₀₈ each independently represent an alkyl group having 1 to 30 carbon atoms, an alkoxy group having 1 to 6 carbon atoms, a phenyl group, a naphthyl group, a halogeno group, a hydroxy group, a nitro group, or a sulfo group, and Ar₃ represents a benzene ring or a naphthalene ring. In a case where n in the general formula (1) is 1, and Ar₃ is a benzene ring, n₃₀₁ represents an integer of 0 to 4. In a case where n in the general formula (1) is 2, and Ar₃ is a benzene ring, n₃₀₁ represents an integer of 0 to 3. In a case where n in the general formula (1) is 1, and Ar₃ is a naphthalene ring, n₃₀₁ represents an integer of 0 to 6. In a case where n in the general formula (1) is 2, and Ar₃ is a naphthalene ring, n₃₀₁ represents an integer of 0 to 5.)

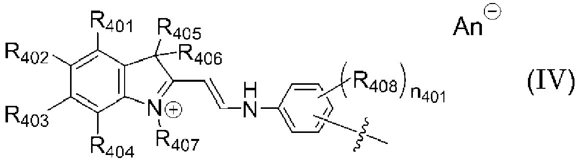

(In the formula, An⁻ is the same as described above, R₄₀₁ to R₄₀₄ each independently represent a hydrogen atom, an alkyl group having 1 to 6 carbon atoms, an alkoxy group having 1 to 6 carbon atoms, an alkyloxycarbonyl group having 2 to 4 carbon atoms, an alkylcarbonyloxy group having 2 to 4 carbon atoms, a phenylcarbonyl group, a naphthylcarbonyl group, a halogeno group, a carboxy group, a nitro group, a cyano group, or an amino group. R₄₀₅ and R₄₀₆ each independently represent a hydrogen atom; an alkyl group having 1 to 6 carbon atoms; a phenylalkyl group having 7 to 9 carbon atoms that has an alkyl group having 1 to 6 carbon atoms, a nitro group, a halogeno group, or a cyano group, or is unsubstituted; or a naphthylalkyl group having 11 to 13 carbon atoms. R₄₀₇ represents a hydrogen atom; an alkyl group having 1 to 6 carbon atoms that has an alkoxy group having 1 to 6 carbon atoms, an alkyloxycarbonyl group having 2 to 4 carbon atoms, a hydroxy group, a carboxy group, a halogeno group, a cyano group, or an amino group, or is unsubstituted; a phenylalkyl group having 7 to 9 carbon atoms that has an alkoxy group having 1 to 6 carbon atoms, a halogeno group, or an amino group, or is unsubstituted; or a naphthylalkyl group having 11 to 13 carbon atoms. n₄₀₁ pieces of R₄₀₈ each independently represent an alkyl group having 1 to 6 carbon atoms, an alkoxy group having 1 to 6 carbon atoms, an alkyloxycarbonyl group having 2 to 4 carbon atoms, an alkylcarbonyloxy group having 2 to 4 carbon atoms, a phenylcarbonyl group, a naphthylcarbonyl group, a halogeno group, a carboxy group, a nitro group, a cyano group, or an amino group. In a case where n in the general formula (1) is 1, n₄₀₁ represents an integer of 0 to 4, and in a case where n in the general formula (1) is 2, n₄₀₁ represents an integer of 0 to 3.)

### Anion represented by An⁻

An⁻ in the general formula (I), the general formula (III), and the general formula (IV) is not particularly limited as long as it is an anion generally used in the field of the related art. Specific examples thereof include an anion containing an aryl group having an electron-withdrawing substituent, a sulfonyl group having an electron-withdrawing substituent, a haloalkyl group, or a halogeno group, a halogen oxoacid anion, and a sulfonate anion (hereinafter, simply described as anions according to the present invention in some cases).

Examples of the anionic portion in the anion containing the aryl group having the electron-withdrawing substituent, the sulfonyl group having the electron-withdrawing substituent, or the haloalkyl group among the anions according to the present invention include a sulfonate anion, a nitrogen anion (N⁻), a quaternary boron anion, a nitrate ion, a phosphate ion, and the like. Among these, the sulfonate anion, the nitrogen anion, and the quaternary boron anion are preferable, and the quaternary boron anion is more preferable.

Examples of the anionic portion in the anion containing the halogeno group among the anions according to the present invention include a quaternary boron anion, a phosphorus anion, an antimony anion, and the like. Among these, the phosphorus anion and the antimony anion are preferable, and the phosphorus anion is more preferable.

In the anions according to the present invention, examples of the electron-withdrawing substituent in the aryl group having the electron-withdrawing substituent or the sulfonyl group having the electron-withdrawing substituent include a haloalkyl group having 1 to 3 carbon atoms, a halogeno group, a nitro group, and the like. Among these, the haloalkyl group having 1 to 3 carbon atoms and the halogeno group are preferable, and the halogeno group is particularly preferable.

Examples of the haloalkyl group having 1 to 3 carbon atoms as the electron-withdrawing substituent include a chloroalkyl group such as a chloromethyl group, a trichloromethyl group, a 2-chloroethyl group, a 2,2,2-trichloroethyl group, a pentachloroethyl group, a 2-chloropropyl group, a 3-chloropropyl group, a 2-chloro-2-propyl group, or a heptachloropropyl group; a bromoalkyl group such as a bromomethyl group, a tribromomethyl group, a 2-bromoethyl group, a 2,2,2-tribromoethyl group, a pentabromoethyl group, a 2-bromopropyl group, a 3-bromopropyl group, a 2-bromo-2-propyl group, or a heptabromopropyl group; an iodoalkyl group such as an iodomethyl group, a triiodomethyl group, a 2-iodoethyl group, a 2,2,2-triiodoethyl group, a pentaiodoethyl group, a 2-iodopropyl group, a 3-iodopropyl group, a 2-iodo-2-propyl group, or a heptaiodopropyl group; and a fluoroalkyl group such as a fluoromethyl group, a trifluoromethyl group, a 2-fluoroethyl group, a 2,2,2-trifluoroethyl group, a 1,1,2,2-tetrafluoroethyl group, a pentafluoroethyl group, a 3-fluoropropyl group, a 3,3,3-trifluoropropyl group, a 2,2,3,3-tetrafluoropropyl group, or a heptafluoropropyl group. Among these, a perhalogenoalkyl group such as the trichloromethyl group, the pentachloroethyl group, the heptachloropropyl group, the tribromomethyl group, the pentabromoethyl group, the heptabromopropyl group, the triiodomethyl group, the pentaiodoethyl group, the heptaiodopropyl group, the trifluoromethyl group, the pentafluoroethyl group, or the heptafluoropropyl group is preferable, a perfluoroalkyl group such as the trifluoromethyl group, the pentafluoroethyl group, or the heptafluoropropyl group is more preferable, and the trifluoromethyl group is particularly preferable.

Examples of the halogeno group as the electron-withdrawing substituent include a fluoro group, a chloro group, a bromo group, and an iodo group. Among these, the fluoro group is preferable.

In the anions according to the present invention, as the electron-withdrawing substituent in the aryl group having the electron-withdrawing substituent, among the above specific examples, those having a strong electron-withdrawing force are preferable. Among these, a trifluoromethyl group, a fluoro group, and a nitro group are preferable, and the fluoro group is more preferable.

In the anions according to the present invention, as the electron-withdrawing substituent in the sulfonyl group having the electron-withdrawing substituent, among the above specific examples, the trifluoromethyl group, the pentafluoroethyl group, the heptafluoropropyl group, and the fluoro group are preferable.

Examples of the aryl group in the aryl group having the electron-withdrawing substituent in the anions according to the present invention include a phenyl group, a naphthyl group, and the like. Among these, the phenyl group is preferable.

Specific examples of the aryl group having the electron-withdrawing substituent in the anions according to the present invention include aryl groups represented by the following general formulae (20-1) and (20-2).

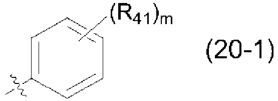

(In the formula, m represents an integer of 1 to 5, and m pieces of R₄₁ each independently represent a haloalkyl group having 1 to 3 carbon atoms, a halogeno group, or a nitro group.)

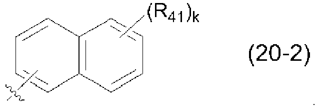

(In the formula, k represents an integer of 1 to 7, and R₄₁ is the same as described above. Here, k pieces of R₄₁ are the same as or different from each other.)

In the general formula (20-1), m is generally an integer of 1 to 5. In a case where R₄₁ is a halogeno group, m is preferably 2 to 5, more preferably 3 to 5, and even more preferably 5. In a case where R₄₁ is a nitro group, m is preferably 1 to 3, and more preferably 1. In a case where R₄₁ is a haloalkyl group, m is preferably 1 to 5, and more preferably 1 to 3.

In the general formula (20-1), k is generally an integer of 1 to 7. In a case where R₄₁ is a halogeno group, k is preferably 2 to 7. In a case where R₄₁ is a nitro group, k is preferably 1 to 3, and more preferably 1. In a case where R₄₁ is a haloalkyl group, k is preferably 1 to 7, and more preferably 1 to 3.

Examples of the haloalkyl group having 1 to 3 carbon atoms represented by R₄₁ in the general formulae (20-1) and (20-2) are the same as the examples of the haloalkyl group having 1 to 3 carbon atoms as the electron-withdrawing substituent in the anions according to the present invention, and preferred examples are also the same.

Examples of the halogeno group represented by R₄₁ in the general formulae (20-1) and (20-2) include a fluoro group, a chloro group, a bromo group, and an iodo group. Among these, the fluoro group is preferable.

Specific examples preferred as R₄₁ in the general formulae (20-1) and (20-2) are the same as the specific examples preferred as the electron-withdrawing substituent in the aryl group having the electron-withdrawing substituent.

Specific examples of the group represented by the general formula (20-1) include a trifluoromethylphenyl group, a di(trifluoromethyl)phenyl group, a tri(trifluoromethyl)phenyl group, a pentafluoroethylphenyl group, a di(pentafluoroethyl)phenyl group, a tri(pentafluoroethyl)phenyl group, a heptafluoropropylphenyl group, a di(heptafluoropropyl)phenyl group, a tri(heptafluoropropyl)phenyl group, a monofluorophenyl group, a difluorophenyl group, a trifluorophenyl group, a perfluorophenyl group, a monochlorophenyl group, a dichlorophenyl group, a trichlorophenyl group, a perchlorophenyl group, a monobromophenyl group, a dibromophenyl group, a tribromophenyl group, a perbromophenyl group, a monoiodophenyl group, a diiodophenyl group, a triiodophenyl group, a periodophenyl group, a nitrophenyl group, a dinitrophenyl group, a trinitrophenyl group, and the like. Among these, the difluorophenyl group, the trifluorophenyl group, the perfluorophenyl group, and the like are preferable, and the perfluorophenyl group is more preferable.

Specific examples of the group represented by the general formula (20-2) include a trifluoromethylnaphthyl group, a di(trifluoromethyl)naphthyl group, a tri(trifluoromethyl)naphthyl group, a pentafluoroethylnaphthyl group, a di(pentafluoroethyl)naphthyl group, a tri(pentafluoroethyl)naphthyl group, a heptafluoropropylnaphthyl group, a di(heptafluoropropyl)naphthyl group, a tri(heptafluoropropyl)naphthyl group, a monofluoronaphthyl group, a difluoronaphthyl group, a trifluoronaphthyl group, a perfluoronaphthyl group, a monochloronaphthyl group, a dichloronaphthyl group, a trichloronaphthyl group, a perchloronaphthyl group, a monobromonaphthyl group, a dibromonaphthyl group, a tribromonaphthyl group, a perbromonaphthyl group, a monoiodonaphthyl group, a diiodonaphthyl group, a triiodonaphthyl group, a periodonaphthyl group, a nitronaphthyl group, a dinitronaphthyl group, a trinitronaphthyl group, and the like.

As the aryl group having the electron-withdrawing substituent in the anions according to the present invention, among the above specific examples, the group represented by the general formula (20-1) is preferable. Specifically, a trifluoromethylphenyl group, a pentafluoroethylphenyl group, a heptafluoropropylphenyl group, a nitrophenyl group, a dinitrophenyl group, a trinitrophenyl group, a monofluorophenyl group, a difluorophenyl group, a trifluorophenyl group, and a perfluorophenyl group are preferable, the difluorophenyl group, the trifluorophenyl group, and the perfluorophenyl group are more preferable, and the perfluorophenyl group is particularly preferable.

Examples of the sulfonyl group having the electron-withdrawing substituent in the anions according to the present invention include -SO₂-CF₃, -SO₂-C₂F₅, -SO₂-C₃F₇, -SO₂-F, -SO₂-Cl, -SO₂-Br, -SO₂-I, and the like.

Examples of the haloalkyl group in the anions according to the present invention are the same as the examples of the haloalkyl group having 1 to 3 carbon atoms as an electron-withdrawing substituent in the anions according to the present invention, and preferred examples are also the same.

Examples of the halogeno group in the anions according to the present invention include a fluoro group, a chloro group, a bromo group, and an iodo group. Among these, the fluoro group is preferable.

Specific examples of the anion containing the aryl group having the electron-withdrawing substituent, the sulfonyl group having the electron-withdrawing substituent, the haloalkyl group, or the halogeno group according to the present invention include groups represented by the following general formulae (13) to (19).

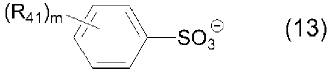

(In the formula, R₄₁ and m are the same as described above. Here, m pieces of R₄₁ are the same as or different from each other.)

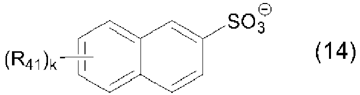

(In the formula, R₄₁ and k are the same as described above. Here, k pieces of R₄₁ are the same as or different from each other.)

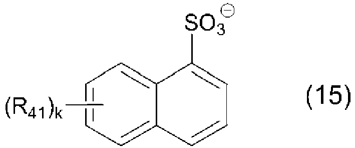

(In the formula, R₄₁ and k are the same as described above. Here, k pieces of R₄₁ are the same as or different from each other.)

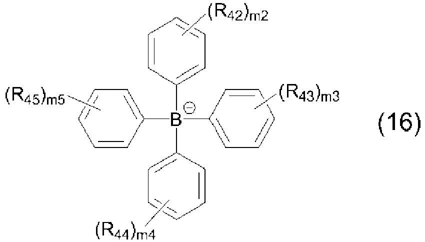

(In the formula, R₄₂ to R₄₅ each independently represent a haloalkyl group having 1 to 3 carbon atoms, a halogeno group, or a nitro group, and m₂ to m₅ each independently represent an integer of 1 to 5. Here, m₂ pieces of R₄₂, m₃ pieces of R₄₃, m₄ pieces of R₄₄, and m₅ pieces of R₄₅ are the same as or different from each other respectively.)

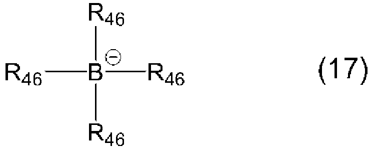

(In the formula, four pieces of R₄₆ each independently represent a haloalkyl group having 1 to 3 carbon atoms or a halogeno group.)

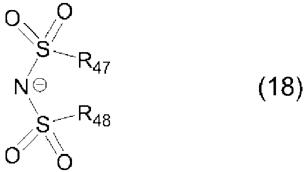

(In the formula, R₄₇ and R₄₈ each independently represent a haloalkyl group having 1 to 3 carbon atoms or a halogeno group. R₄₇ and R₄₈ may form a haloalkylene group having 2 or 3 carbon atoms together.)

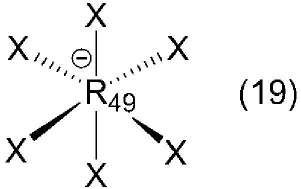

(In the formula, R₄₉ represents a phosphorus atom or an antimony atom, and all of six pieces of X represent the same halogeno group.)

Examples of the haloalkyl group having 1 to 3 carbon atoms that is represented by R₄₂ to R₄₅ in the general formula (16), R₄₆ in the general formula (17), and R₄₇ and R₄₈ in the general formula (18) are the same as the examples of the haloalkyl group having 1 to 3 carbon atoms as the electron-withdrawing substituent in the anions according to the present invention, and preferred examples are also the same.

Examples of the halogeno group represented by R₄₂ to R₄₅ in the general formula (16), R₄₆ in the general formula (17), R₄₇ and R₄₈ in the general formula (18), and X in the general formula (19) include a fluoro group, a chloro group, a bromo group, and an iodo group. Among these, the fluoro group is preferable.

Examples of the haloalkylene group having 2 or 3 carbon atoms formed by R₄₇ and R₄₈ in the general formula (18) include a tetrafluoroethylene group, a hexafluorotrimethylene group, and the like. Among these, the hexafluorotrimethylene group is preferable.

As R₄₉ in the general formula (19), a phosphorus atom is preferable.

Examples of the combination of R₄₁ and m in the general formula (13) include combinations describe in the following table. It should be noted that a case where all of m pieces of R₄₁ represent the same group is preferable.

| R₄₁ | m |
|---|---|
| Trifluoromethyl group (-CF₃) | 1 to 3 |
| Pentafluoroethyl group (-C₂F₅) | 1 to 3 |
| Heptafluoropropyl group (-C₃F₇) | 1 to 3 |
| Nitro group | 1 to 3 |
| Fluorine atom | 1 to 5 |
| Chlorine atom | 1 to 5 |
| Bromine atom | 1 to 5 |
| Iodine atom | 1 to 5 |

Specific examples preferred as the anion represented by the general formula (13) include the following anions.

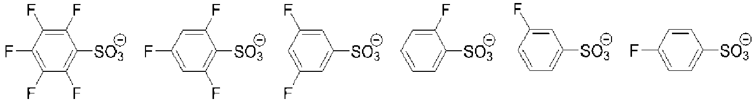

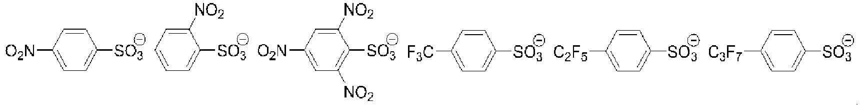

Examples of the combination of R₄₁ and k in the general formulae (14) and (15) include combinations described in the following table. It should be noted that a case where all of k pieces of R₄₁ represent the same group is preferable.

| R₄₁ | k |
|---|---|
| Trifluoromethyl group (-CF₃) | 1 to 3 |
| Pentafluoroethyl group (-C₂F₅) | 1 to 3 |
| Heptafluoropropyl group (-C₃F₇) | 1 to 3 |
| Nitro group | 1 to 3 |
| Fluorine atom | 1 to 7 |
| Chlorine atom | 1 to 7 |
| Bromine atom | 1 to 7 |
| Iodine atom | 1 to 7 |

Specific examples preferred as the anions represented by the general formulae (14) and (15) include the following anions.

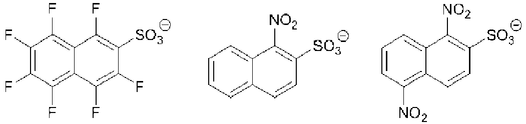

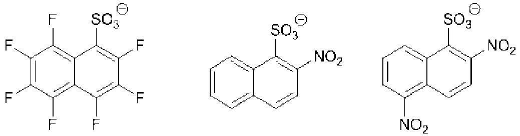

A case where all of m₂ pieces of R₄₂, m₃ pieces of R₄₃, m₄ pieces of R₄₄, and m₅ pieces of R₄₅ in the general formula (16) represent the same group respectively is preferable. Examples of the combination of R₄₂ to R₄₅ and m₂ to m₅ in the general formula (16) include combinations described in the following table.

| R₄₂ | m₂ | R₄₃ | m₃ | R₄₄ | m₄ | R₄₅ | m₅ |
|---|---|---|---|---|---|---|---|
| -CF ₃ | 1 to 3 | -CF ₃ | 1 to 3 | -CF ₃ | 1 to 3 | -CF ₃ | 1 to 3 |
| -C₂F₅ | 1 to 3 | -C₂F₅ | 1 to 3 | -C₂F₅ | 1 to 3 | -C₂F₅ | 1 to 3 |
| -C₃F₇ | 1 to 3 | -C₃F₇ | 1 to 3 | -C₃F₇ | 1 to 3 | -C₃F₇ | 1 to 3 |
| Nitro group | 1 to 3 | Nitro group | 1 to 3 | Nitro group | 1 to 3 | Nitro group | 1 to 3 |
| Fluorine | 1 to 5 | Fluorine | 1 to 5 | Fluorine | 1 to 5 | Fluorine | 1 to 5 |
| Chlorine | 1 to 5 | Chlorine | 1 to 5 | Chlorine | 1 to 5 | Chlorine | 1 to 5 |
| Bromine | 1 to 5 | Bromine | 1 to 5 | Bromine | 1 to 5 | Bromine | 1 to 5 |
| Iodine | 1 to 5 | Iodine | 1 to 5 | Iodine | 1 to 5 | Iodine | 1 to 5 |
| Nitro group | 1 to 3 | Fluorine | 1 to 5 | Fluorine | 1 to 5 | Fluorine | 1 to 5 |
| Nitro group | 1 to 3 | Nitro group | 1 to 3 | Fluorine | 1 to 5 | Fluorine | 1 to 5 |
| Nitro group | 1 to 3 | Nitro group | 1 to 3 | Nitro group | 1 to 3 | Fluorine | 1 to 5 |

Specific examples preferred as the anion represented by the general formula (16) include the following anions.

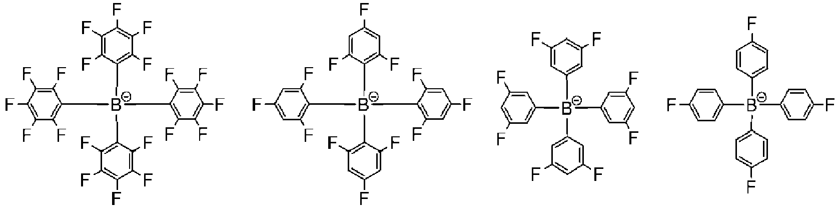

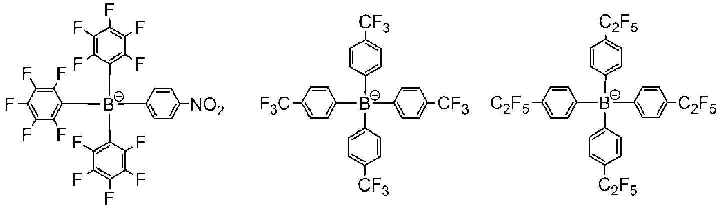

Among the above specific examples, the following anions are preferable, and a tetrakis(pentafluorophenyl)boron (IV) anion is more preferable.

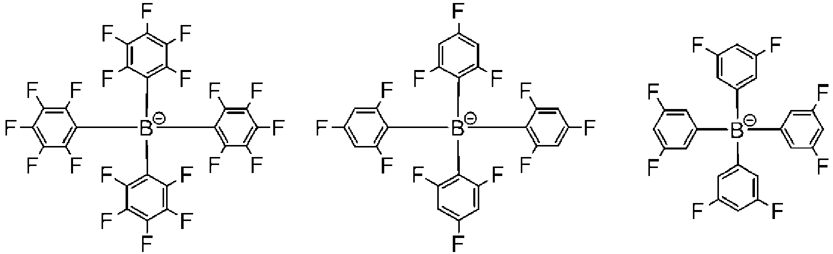

Specific examples preferred as the anion represented by the general formula (17) include BF₄⁻, CF₃BF₃⁻, C₂F₅BF₃⁻, C₃F₇BF₃⁻, (CF₃)₄B⁻, (C₂F₅)₄B⁻, (C₃F₇)₄B⁻, and the like.

Specific examples preferred as the anion represented by the general formula (18) include the following anions.

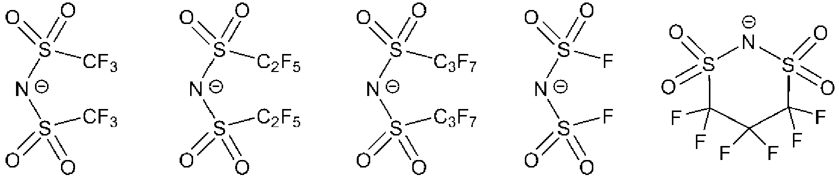

Specific examples preferred as the anion represented by the general formula (19) include PF₆⁻, SbF₆⁻, and the like. Among these, PF₆⁻ is preferable.

Specific examples of the halogen oxoacid anion in the anions according to the present invention include a hypochlorite anion, a chlorite anion, a chlorate anion, a perchlorate anion, and the like. Among these, the perchlorate anion is preferable.

Specific examples of the sulfonate anion in the anions according to the present invention include an alkylsulfonate anion having 1 to 20 carbon atoms such as a methanesulfonate anion; a haloalkylsulfonate anion having 1 to 20 carbon atoms such as a trifluoromethanesulfonate anion; a benzenesulfonate anion which has a substituent or is unsubstituted, such as a benzenesulfonate anion or a toluenesulfoante anion; and the like.

As An⁻ in the general formula (I), the general formula (III), and the general formula (IV), the anions according to the present invention are preferable. Among these, the anion containing the aryl group having the electron-withdrawing substituent, the sulfonyl group having the electron-withdrawing substituent, the haloalkyl group, or a halogeno group are more preferable. Specifically, the anions represented by the general formulae (16) to (19) are preferable, the anions represented by the general formula (16), the general formula (18), and the general formula (19) are more preferable, the anions represented by the general formula (16) and the general formula (19) are even more preferable, and the anion represented by the general formula (16) is particularly preferable.

As An⁻ in the general formula (I), the general formula (III), and the general formula (IV), among the above specific examples, the following anions are preferable. Among these, a tetrakis(pentafluorophenyl)boron (IV) anion, a bis(trifluoromethanesulfonyl)imide anion, and PF₆⁻ are more preferable, the tetrakis(pentafluorophenyl)boron (IV) anion and PF₆⁻ are even more preferable, and the tetrakis(pentafluorophenyl)boron (IV) anion is particularly preferable.

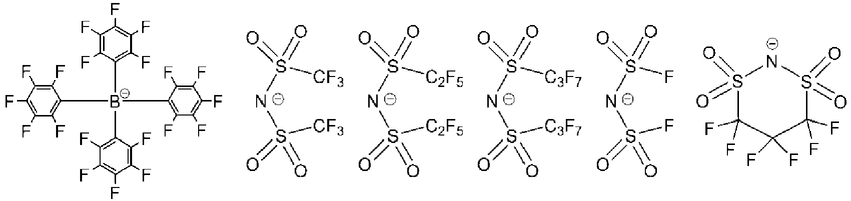

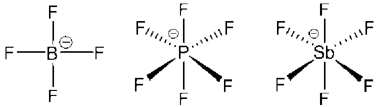

### Dye residue represented by general formula (I)

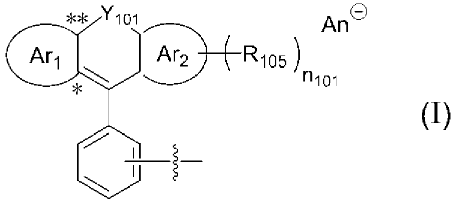

(In the formula, R₁₀₅, Y₁₀₁, Ar₁, Ar₂, An⁻, n₁₀₁, *, and ** are the same as described above.)

Examples of the halogeno group represented by R₁₀₅ in the general formula (I) include a fluoro group, a chloro group, a bromo group, and an iodo group. Among these, the fluoro group is preferable.

The alkyl group having 1 to 30 carbon atoms represented by R₁₀₅ in the general formula (I) is any one of linear, branched, and cyclic alkyl groups. Among these, linear and branched alkyl groups are preferable. In addition, among the alkyl group having 1 to 30 carbon atoms, an alkyl group having 1 to 12 carbon atoms is preferable, an alkyl group having 1 to 6 carbon atoms is more preferable, an alkyl group having 1 to 4 carbon atoms is even more preferable, and an alkyl group having 1 or 2 carbon atoms is particularly preferable. Specific examples thereof include a methyl group, an ethyl group, an n-propyl group, an isopropyl group, an n-butyl group, an isobutyl group, a sec-butyl group, a tert-butyl group, a cyclobutyl group, an n-pentyl group, an isopentyl group, a sec-pentyl group, a tert-pentyl group, a neopentyl group, a 2-methylbutyl group, a 1,2-dimethylpropyl group, a 1-ethylpropyl group, a cyclopentyl group, an n-hexyl group, an isohexyl group, a sec-hexyl group, a tert-hexyl group, a neohexyl group, a 2-methylpentyl group, a 1,2-dimethylbutyl group, a 2,3-dimethylbutyl group, a 1-ethylbutyl group, a cyclohexyl group, an n-heptyl group, an isoheptyl group, a sec-heptyl group, a tert-heptyl group, a neoheptyl group, a cycloheptyl group, an n-octyl group, an isooctyl group, a sec-octyl group, a tert-octyl group, a neooctyl group, a 2-ethylhexyl group, a cyclooctyl group, an n-nonyl group, an isononyl group, a sec-nonyl group, a tert-nonyl group, a neononyl group, a cyclononyl group, an n-decyl group, an isodecyl group, a sec-decyl group, a tert-decyl group, a neodecyl group, a cyclodecyl group, an n-undecyl group, a cycloundecyl group, an n-dodecyl group, a cyclododecyl group, an n-tridecyl group, an isotridecyl group, an n-tetradecyl group, an isotetradecyl group, an n-pentadecyl group, an isopentadecyl group, an n-hexadecyl group, an isohexadecyl group, an n-heptadecyl group, an isoheptadecyl group, an n-octadecyl group, an isooctadecyl group, an n-nonadecyl group, an isononadecyl group, an n-icosyl group, an isoicosyl group, an n-henicosyl group, an isohenicosyl group, an n-docosyl group, an isodocosyl group, an n-tricosyl group, an isotricosyl group, an n-tetracosyl group, an isotetracosyl group, an n-pentacosyl group, an isopentacosyl group, an n-hexacosyl group, an isohexacosyl group, an n-heptacosyl group, an isoheptacosyl group, an n-octacosyl group, an isooctacosyl group, an n-nonacosyl group, an isononacosyl group, an n-triacontyl group, an isotriacontyl group, a cyclohexylmethyl group, a 1-cyclohexylethyl group, a 2-methylcyclohexyl group, a 3-methylcyclohexyl group, a 4-methylcyclohexyl group, a 2,6-dimethylcyclohexyl group, a 2,4-dimethylcyclohexyl group, a 3,5-dimethylcyclohexyl group, a 2,5-dimethylcyclohexyl group, a 2,3-dimethylcyclohexyl group, a 3,3,5-trimethylcyclohexyl group, a 4-tert-butylcyclohexyl group, a 1-adamantyl group, a 2-adamantyl group, an isobornyl group, a dicyclopentanyl group, a 1-methylcyclopentyl group, a 1-ethylcyclopentyl group, a 1-n-propylcyclopentyl group, a 1-methylcyclohexyl group, a 1-ethylcyclohexyl group, a 1-n-propylcyclohexyl group, a 1-n-butylcyclohexyl group, a 1-methylcycloheptyl group, a 1-ethylcycloheptyl group, a 1-n-propylcycloheptyl group, a 1-methylcyclooctyl group, a 1-ethylcyclooctyl group, and the like. Among these, the methyl group, the ethyl group, the n-propyl group, the isopropyl group, the n-butyl group, the isobutyl group, the sec-butyl group, the tert-butyl group, the n-pentyl group, the isopentyl group, the sec-pentyl group, the tert-pentyl group, the neopentyl group, the 2-methylbutyl group, the 1,2-dimethylpropyl group, the 1-ethylpropyl group, the n-hexyl group, the isohexyl group, the sec-hexyl group, the tert-hexyl group, the neohexyl group, the 2-methylpentyl group, the 1,2-dimethylbutyl group, the 2,3-dimethylbutyl group, the 1-ethylbutyl group, the n-heptyl group, the n-octyl group, the n-nonyl group, the n-decyl group, the n-undecyl group, and the n-dodecyl group are preferable, the methyl group, the ethyl group, the n-propyl group, the isopropyl group, the n-butyl group, the isobutyl group, the sec-butyl group, the tert-butyl group, the n-pentyl group, the isopentyl group, and the n-hexyl group are more preferable, the methyl group, the ethyl group, the n-propyl group, the isopropyl group, the n-butyl group, the isobutyl group, the sec-butyl group, and the tert-butyl group are even more preferable, and the methyl group and the ethyl group are particularly preferable.

The alkoxy group having 1 to 20 carbon atoms represented by R₁₀₅ in the general formula (I) is any one of linear, branched, and cyclic alkoxy groups. Among these, linear and branched alkoxy groups are preferable. In addition, among the alkoxy group having 1 to 20 carbon atoms, an alkoxy group having 1 to 12 carbon atoms is preferable, an alkoxy group having 1 to 6 carbon atoms is more preferable, and an alkoxy group having 1 to 4 carbon atoms is particularly preferable. Specific examples thereof include a methoxy group, an ethoxy group, an n-propoxy group, an isopropoxy group, an n-butoxy group, an isobutoxy group, a sec-butoxy group, a tert-butoxy group, a cyclobutoxy group, an n-pentyloxy group, an isopentyloxy group, a sec-pentyloxy group, a tert-pentyloxy group, a neopentyloxy group, a 2-methylbutoxy group, a 1,2-dimethylpropoxy group, a 1-ethylpropoxy group, a cyclopentyloxy group, an n-hexyloxy group, an isohexyloxy group, a sec-hexyloxy group, a tert-hexyloxy group, a neohexyloxy group, a 2-methylpentyloxy group, a 1,2-dimethylbutoxy group, a 2,3-dimethylbutoxy group, a 1-ethylbutoxy group, a cyclohexyloxy group, an n-heptyloxy group, an isoheptyloxy group, a sec-heptyloxy group, a tert-heptyloxy group, a neoheptyloxy group, a cycloheptyloxy group, an n-octyloxy group, an isooctyloxy group, a sec-octyloxy group, a tert-octyloxy group, a neooctyloxy group, a 2-ethylhexyloxy group, a cyclooctyloxy group, an n-nonyloxy group, an isononyloxy group, a sec-nonyloxy group, a tert-nonyloxy group, a neononyloxy group, a cyclononyloxy group, an n-decyloxy group, an isodecyloxy group, a sec-decyloxy group, a tert-decyloxy group, a neodecyloxy group, a cyclodecyloxy group, an n-undecyloxy group, a cycloundecyloxy group, an n-dodecyloxy group, a cyclododecyloxy group, an n-tridecyloxy group, an isotridecyloxy group, an n-tetradecyloxy group, an isotetradecyloxy group, an n-pentadecyloxy group, an isopentadecyloxy group, an n-hexadecyloxy group, an isohexadecyloxy group, an n-heptadecyloxy group, an isoheptadecyloxy group, an n-octadecyloxy group, an isooctadecyloxy group, an n-nonadecyloxy group, an isononadecyloxy group, an n-icosyloxy group, an isoicosyloxy group, and the like. Among these, the methoxy group, the ethoxy group, the n-propoxy group, the isopropoxy group, the n-butoxy group, the isobutoxy group, the sec-butoxy group, the tert-butoxy group, the n-pentyloxy group, the isopentyloxy group, the sec-pentyloxy group, the tert-pentyloxy group, the neopentyloxy group, the 2-methylbutoxy group, the 1,2-dimethylpropoxy group, the 1-ethylpropoxy group, the n-hexyloxy group, the isohexyloxy group, the sec-hexyloxy group, the tert-hexyloxy group, the neohexyloxy group, the 2-methylpentyloxy group, the 1,2-dimethylbutoxy group, the 2,3-dimethylbutoxy group, and the 1-ethylbutoxy group are preferable, the methoxy group, the ethoxy group, the n-propoxy group, the isopropoxy group, the n-butoxy group, the isobutoxy group, the sec-butoxy group, and the tert-butoxy group are more preferable, and the methoxy group and the ethoxy group are even more preferable.

The alkylthio group having 1 to 20 carbon atoms represented by R₁₀₅ in the general formula (I) is any one of linear, branched, and cyclic alkylthio groups. Among these, linear and branched alkylthio groups are preferable. In addition, among the alkylthio group having 1 to 20 carbon atoms, an alkylthio group having 1 to 12 carbon atoms is preferable, an alkylthio group having 1 to 6 carbon atoms is more preferable, and an alkylthio group having 1 to 4 carbon atoms is particularly preferable. Specific examples thereof include a methylthio group, an ethylthio group, an n-propylthio group, an isopropylthio group, an n-butylthio group, an isobutylthio group, a sec-butylthio group, a tert-butylthio group, a cyclobutylthio group, an n-pentylthio group, an isopentylthio group, a sec-pentylthio group, a tert-pentylthio group, a neopentylthio group, a 2-methylbutylthio group, a 1,2-dimethylpropylthio group, a 1-ethylpropylthio group, a cyclopentylthio group, an n-hexylthio group, an isohexylthio group, a sec-hexylthio group, a tert-hexylthio group, a neohexylthio group, a 2-methylpentylthio group, a 1,2-dimethylbutylthio group, a 2,3-dimethylbutylthio group, a 1-ethylbutylthio group, a cyclohexylthio group, an n-heptylthio group, an isoheptylthio group, a sec-heptylthio group, a tert-heptylthio group, a neoheptylthio group, a cycloheptylthio group, an n-octylthio group, an isooctylthio group, a sec-octylthio group, a tert-octylthio group, a neooctylthio group, a 2-ethylhexylthio group, a cyclooctylthio group, an n-nonylthio group, an isononylthio group, a sec-nonylthio group, a tert-nonylthio group, a neononylthio group, a cyclononylthio group, an n-decylthio group, an isodecylthio group, a sec-decylthio group, a tert-decylthio group, a neodecylthio group, a cyclodecylthio group, an n-undecylthio group, a cycloundecylthio group, an n-dodecylthio group, a cyclododecylthio group, an n-tridecylthio group, an isotridecylthio group, an n-tetradecylthio group, an isotetradecylthio group, an n-pentadecylthio group, an isopentadecylthio group, an n-hexadecylthio group, an isohexadecylthio group, an n-heptadecylthio group, an isoheptadecylthio group, an n-octadecylthio group, an isooctadecylthio group, an n-nonadecylthio group, an isononadecylthio group, an n-icosylthio group, an isoicosylthio group, and the like. Among these, the methylthio group, the ethylthio group, the n-propylthio group, the isopropylthio group, the n-butylthio group, the isobutylthio group, the sec-butylthio group, the tert-butylthio group, the n-pentylthio group, the isopentylthio group, the sec-pentylthio group, the tert-pentylthio group, the neopentylthio group, the 2-methylbutylthio group, the 1,2-dimethylpropylthio group, the 1-ethylpropylthio group, the n-hexylthio group, the isohexylthio group, the sec-hexylthio group, the tert-hexylthio group, the neohexylthio group, the 2-methylpentylthio group, the 1,2-dimethylbutylthio group, the 2,3-dimethylbutylthio group, and the 1-ethylbutylthio group are preferable, the methylthio group, the ethylthio group, the n-propylthio group, the isopropylthio group, the n-butylthio group, the isobutylthio group, the sec-butylthio group, and the tert-butylthio group are more preferable, and the methylthio group and the ethylthio group are even more preferable.

The amino group having a substituent represented by R₁₀₅ in the general formula (I) has one substituent or two substituents. Examples of the substituent include an alkyl group having 1 to 30 carbon atoms, a haloalkyl group having 1 to 20 carbon atoms, an aryl group having 6 to 10 carbon atoms, an arylalkyl group having 7 to 13 carbon atoms, and the like.

Examples of the alkyl group having 1 to 30 carbon atoms that is the substituent of the amino group having a substituent represented by R₁₀₅ in the general formula (I) are the same as the examples of the alkyl group having 1 to 30 carbon atoms represented by R₁₀₅ in the general formula (I), and preferred examples are also the same.

The haloalkyl group having 1 to 20 carbon atoms that is the substituent of the amino group having a substituent represented by R₁₀₅ in the general formula (I) is any one of linear, branched, and cyclic haloalkyl groups. Among these, linear and branched haloalkyl groups are preferable. In addition, among the haloalkyl group having 1 to 20 carbon atoms, a haloalkyl group having 1 to 12 carbon atoms is preferable, a haloalkyl group having 1 to 6 carbon atoms is more preferable, and a haloalkyl group having 1 to 3 carbon atoms is particularly preferable. Specific examples thereof include a chloroalkyl group such as a chloromethyl group, a trichloromethyl group, a 2-chloroethyl group, a 2,2,2-trichloroethyl group, a pentachloroethyl group, a 2-chloropropyl group, a 3-chloropropyl group, a 2-chloro-2-propyl group, or a heptachloropropyl group; a bromoalkyl group such as a bromomethyl group, a tribromomethyl group, a 2-bromoethyl group, a 2,2,2-tribromoethyl group, a pentabromoethyl group, a 2-bromopropyl group, a 3-bromopropyl group, a 2-bromo-2-propyl group, or a heptabromopropyl group; an iodoalkyl group such as an iodomethyl group, a triiodomethyl group, a 2-iodoethyl group, a 2,2,2-triiodoethyl group, a pentaiodoethyl group, a 2-iodopropyl group, a 3-iodopropyl group, a 2-iodo-2-propyl group, or a heptaiodopropyl group; and a fluoroalkyl group such as a fluoromethyl group, a trifluoromethyl group, a 2-fluoroethyl group, a 2,2,2-trifluoroethyl group, a 1,1,2,2-tetrafluoroethyl group, a pentafluoroethyl group, a 3-fluoropropyl group, a 3,3,3-trifluoropropyl group, a 2,2,3,3-tetrafluoropropyl group, or a heptafluoropropyl group. Among these, a perhalogenoalkyl group such as the trichloromethyl group, the pentachloroethyl group, the heptachloropropyl group, the tribromomethyl group, the pentabromoethyl group, the heptabromopropyl group, the triiodomethyl group, the pentaiodoethyl group, the heptaiodopropyl group, the trifluoromethyl group, the pentafluoroethyl group, or the heptafluoropropyl group is preferable, a perfluoroalkyl group such as the trifluoromethyl group, the pentafluoroethyl group, or the heptafluoropropyl group is more preferable, and the trifluoromethyl group is particularly preferable.

Examples of the aryl group having 6 to 10 carbon atoms that is the substituent of the amino group having a substituent represented by R₁₀₅ in the general formula (I) include a phenyl group, a naphthyl group, and the like. Among these, the phenyl group is preferable.

Examples of the arylalkyl group having 7 to 13 carbon atoms that is the substituent of the amino group having a substituent represented by R₁₀₅ in the general formula (I) include a phenylalkyl group having 7 to 9 carbon atoms, a naphthylalkyl group having 11 to 13 carbon atoms, and the like. Among these, the phenylalkyl group having 7 to 9 carbon atoms is preferable. Specific examples thereof include a benzyl group, a phenethyl group (a 2-phenylethyl group), a 1-phenylethyl group, a hydrocinnamyl group (a 3-phenylpropyl group), a 2-phenylpropyl group, a 1-phenylpropyl group, a cumyl group (a 2-phenylpropane-2-yl group), a naphthylmethyl group, a 2-naphthylethyl group, a 3-naphthylpropyl group, and the like. Among these, the benzyl group, the phenethyl group, the 1-phenylethyl group, the hydrocinnamyl group, the 2-phenylpropyl group, the 1-phenylpropyl group, and the cumyl group are preferable, the benzyl group, the phenethyl group, and the hydrocinnamyl group are more preferable, and the benzyl group is even more preferable.

As the amino group which has a substituent or is unsubstituted, represented by R₁₀₅ in the general formula (I), an amino group having a substituent is preferable, an amino group having an alkyl group having 1 to 30 carbon atoms, a haloalkyl group having 1 to 20 carbon atoms, an aryl group having 6 to 10 carbon atoms, or an arylalkyl group having 7 to 13 carbon atoms is more preferable, and an amino group having an alkyl group having 1 to 12 carbon atoms, a phenyl group, or a phenylalkyl group having 7 to 9 carbon atoms is even more preferable. Specific examples thereof include a methylamino group, an ethylamino group, an n-propylamino group, an isopropylamino group, an n-butylamino group, an isobutylamino group, a sec-butylamino group, a tert-butylamino group, an n-pentylamino group, an isopentylamino group, an n-hexylamino group, a phenylamino group, a benzylamino group, a phenethylamino group, a hydrocinnamylamino group, a dimethylamino group, a diethylamino group, a di-n-propylamino group, a diisopropylamino group, a di-n-butylamino group, a diisobutylamino group, a di-sec-butylamino group, a di-tert-butylamino group, a di-n-pentylamino group, a diisopentylamino group, a di-n-hexylamino group, a diphenylamino group, a dibenzylamino group, a diphenethylamino group, a bis(hydrocinnamyl)amino group, and the like. Among these, the methylamino group, the ethylamino group, the n-propylamino group, the isopropylamino group, the n-butylamino group, the isobutylamino group, the sec-butylamino group, the tert-butylamino group, the phenylamino group, the benzylamino group, the dimethylamino group, the diethylamino group, the di-n-propylamino group, the diisopropylamino group, the di-n-butylamino group, the diisobutylamino group, the di-sec-butylamino group, the di-tert-butylamino group, the diphenylamino group, and the dibenzylamino group are preferable, and the methylamino group, the ethylamino group, the phenylamino group, the benzylamino group, the dimethylamino group, the diethylamino group, the diphenylamino group, and the dibenzylamino group are more preferable.

Specific examples of the aryl group having 6 to 14 carbon atoms represented by R₁₀₅ in the general formula (I) include a phenyl group, a naphthyl group, an anthracenyl group, and the like. Among these, the phenyl group is preferable.

Specific examples of the aryloxy group having 6 to 14 carbon atoms represented by R₁₀₅ in the general formula (I) include a phenoxy group, a naphthyloxy group, an anthracenyloxy group, and the like. Among these, the phenoxy group is preferable.

Examples of the arylalkyl group having 7 to 20 carbon atoms represented by R₁₀₅ in the general formula (I) include a phenylalkyl group having 7 to 12 carbon atoms, a naphthylalkyl group having 11 to 16 carbon atoms, an anthracenylalkyl group having 15 to 20 carbon atoms, and the like. Among these, the phenylalkyl group having 7 to 12 carbon atoms is preferable, and a phenylalkyl group having 7 to 9 carbon atoms is more preferable. Specific examples thereof include a benzyl group, a phenethyl group (a 2-phenylethyl group), a 1-phenylethyl group, a hydrocinnamyl group (a 3-phenylpropyl group), a 2-phenylpropyl group, a 1-phenylpropyl group, a cumyl group (a 2-phenylpropane-2-yl group), a 4-phenylbutyl group, a 3-phenylbutyl group, a 2-phenylbutyl group, a 1-phenylbutyl group, a 5-phenylpentyl group, a 4-phenylpentyl group, a 3-phenylpentyl group, a 2-phenylpentyl group, a 1-phenylpentyl group, a 6-phenylhexyl group, a 5-phenylhexyl group, a 4-phenylhexyl group, a 3-phenylhexyl group, a 2-phenylhexyl group, a 1-phenylhexyl group, a naphthylmethyl group, a 2-naphthylethyl group, a 3-naphthylpropyl group, a 4-naphthylbutyl group, a 5-naphthylpentyl group, a 6-naphthylhexyl group, an anthracenylmethyl group, a 2-anthracenylethyl group, a 3-anthracenylpropyl group, a 4-anthracenylbutyl group, a 5-anthracenylpentyl group, a 6-anthracenylhexyl group, and the like. Among these, the benzyl group, the phenethyl group, the 1-phenylethyl group, the hydrocinnamyl group, the 2-phenylpropyl group, the 1-phenylpropyl group, the cumyl group, the 4-phenylbutyl group, the 3-phenylbutyl group, the 2-phenylbutyl group, the 1-phenylbutyl group, the 5-phenylpentyl group, the 4-phenylpentyl group, the 3-phenylpentyl group, the 2-phenylpentyl group, the 1-phenylpentyl group, the 6-phenylhexyl group, the 5-phenylhexyl group, the 4-phenylhexyl group, the 3-phenylhexyl group, the 2-phenylhexyl group, and the 1-phenylhexyl group are preferable, the benzyl group, the phenethyl group, the 1-phenylethyl group, the hydrocinnamyl group, the 2-phenylpropyl group, the 1-phenylpropyl group, and the cumyl group are more preferable, and the benzyl group, the phenethyl group, the hydrocinnamyl group, and the cumyl group are even more preferable.

As R₁₀₅ in the general formula (I), a halogeno group; an alkyl group having 1 to 12 carbon atoms; an alkoxy group having 1 to 12 carbon atoms; an alkylthio group having 1 to 12 carbon atoms; an amino group having an alkyl group having 1 to 30 carbon atoms, a haloalkyl group having 1 to 20 carbon atoms, an aryl group having 6 to 10 carbon atoms, or an arylalkyl group having 7 to 13 carbon atoms; a hydroxy group; an aryl group having 6 to 14 carbon atoms; an aryloxy group having 6 to 14 carbon atoms; and an arylalkyl group having 7 to 20 carbon atoms are preferable, the halogeno group; the alkyl group having 1 to 12 carbon atoms; the alkoxy group having 1 to 12 carbon atoms; the alkylthio group having 1 to 12 carbon atoms; an amino group having an alkyl group having 1 to 12 carbon atoms, a phenyl group, or a phenylalkyl group having 7 to 9 carbon atoms; the hydroxy group; a phenyl group; a phenoxy group; and a phenylalkyl group having 7 to 12 carbon atoms are more preferable; and the halogeno group; an alkyl group having 1 to 6 carbon atoms; an alkoxy group having 1 to 6 carbon atoms; an alkylthio group having 1 to 6 carbon atoms; the amino group having the alkyl group having 1 to 12 carbon atoms, the phenyl group, or the phenylalkyl group having 7 to 9 carbon atoms; the hydroxy group; the phenyl group; the phenoxy group; and the phenylalkyl group having 7 to 9 carbon atoms are even more preferable. Specifically, a fluorine atom, a methyl group, an ethyl group, an n-propyl group, an isopropyl group, an n-butyl group, an isobutyl group, a sec-butyl group, a tert-butyl group, an n-pentyl group, an isopentyl group, an n-hexyl group, a methoxy group, an ethoxy group, an n-propoxy group, an isopropoxy group, an n-butoxy group, an isobutoxy group, a sec-butoxy group, a tert-butoxy group, a methylthio group, an ethylthio group, an n-propylthio group, an isopropylthio group, an n-butylthio group, an isobutylthio group, a sec-butylthio group, a tert-butylthio group, a methylamino group, an ethylamino group, an n-propylamino group, an isopropylamino group, an n-butylamino group, an isobutylamino group, a sec-butylamino group, a tert-butylamino group, a phenylamino group, a benzylamino group, a dimethylamino group, a diethylamino group, a di-n-propylamino group, a diisopropylamino group, a di-n-butylamino group, a diisobutylamino group, a di-sec-butylamino group, a di-tert-butylamino group, a diphenylamino group, a dibenzylamino group, a hydroxy group, a phenyl group, a phenoxy group, a benzyl group, a phenethyl group, a 1-phenylethyl group, a hydrocinnamyl group, a 2-phenylpropyl group, a 1-phenylpropyl group, and a cumyl group are preferable, and the fluorine atom, the methyl group, the ethyl group, the n-propyl group, the isopropyl group, the n-butyl group, the isobutyl group, the sec-butyl group, the tert-butyl group, the methoxy group, the ethoxy group, the methylthio group, the ethylthio group, the methylamino group, the ethylamino group, the phenylamino group, the benzylamino group, the dimethylamino group, the diethylamino group, the diphenylamino group, the dibenzylamino group, the hydroxy group, the phenyl group, the phenoxy group, the benzyl group, the phenethyl group, the hydrocinnamyl group, and the cumyl group are more preferable.

Examples of the alkyl group having 1 to 6 carbon atoms represented by R₁₃₂ in Y₁₀₁ in the general formula (I) are the same as the examples of the alkyl group having 1 to 6 carbon atoms represented by R₃ in the general formula (2), and preferred examples are also the same.

As Y₁₀₁ in the general formula (I), an oxygen atom and -NR₁₃₂- are preferable, and the oxygen atom is more preferable. Specific examples of Y₁₀₁ include an oxygen atom, a sulfur atom, -NCH₃-, -NC₂H₅-, -NC₃H₇-, -NC₄H₉-, and the like. Among these, the oxygen atom, the sulfur atom, and -NCH₃- are preferable, the oxygen atom and -NCH₃- are more preferable, and the oxygen atom is particularly preferable.

Examples of the alkyl group having 1 to 30 carbon atoms represented by R₁₀₂ and R₁₀₃ in the general formula (I-1-1) are the same as the examples of the alkyl group having 1 to 30 carbon atoms represented by R₁₀₅ in the general formula (I), and preferred examples are also the same.

In a case where R₁₀₂ and R₁₀₃ in the general formula (I-1-1) represent "an aryl group having 6 to 14 carbon atoms that has a substituent or is unsubstituted", examples of the aryl group having 6 to 14 carbon atoms include a phenyl group, a naphthyl group, an anthracenyl group, and the like. Among these, the phenyl group is preferable.

The aryl group having 6 to 14 carbon atoms that has a substituent represented by R₁₀₂ and R₁₀₃ in the general formula (I-1-1) generally has 1 to 5 substituents, preferably has 1 to 3 substituents, and more preferably has one substituent. Examples of the substituent include an alkyl group having 1 to 30 carbon atoms, and the like. Examples of the alkyl group having 1 to 30 carbon atoms are the same as the examples of the alkyl group having 1 to 30 carbon atoms represented by R₁₀₅ in the general formula (I), and preferred examples are also the same.

Examples of the aryl group having 6 to 14 carbon atoms that has a substituent represented by R₁₀₂ and R₁₀₃ in the general formula (1-1-1) include an aryl group having 6 to 14 carbon atoms that has an alkyl group having 1 to 30 carbon atoms, and the like. Among these, a phenyl group, a naphthyl group, and an anthracenyl group that has an alkyl group having 1 to 30 carbon atoms are preferable, a phenyl group having an alkyl group having 1 to 12 carbon atoms is more preferable, a phenyl group having an alkyl group having 1 to 6 carbon atoms is even more preferable, and a phenyl group having an alkyl group having 1 to 3 carbon atoms is particularly preferable. Specific examples thereof include an o-tolyl group (a methylphenyl group), a m-tolyl group, a p-tolyl group, an o-ethylphenyl group, a m-ethylphenyl group, a p-ethylphenyl group, an o-propylphenyl group, a m-propylphenyl group, a p-propylphenyl group, an o-butylphenyl group, a m-butylphenyl group, a p-butylphenyl group, an o-pentylphenyl group, a m-pentylphenyl group, a p-pentylphenyl group, an o-hexylphenyl group, a m-hexylphenyl group, a p-hexylphenyl group, a 2,3-xylyl group (a 2,3-dimethylphenyl group), a 2,4-xylyl group, a 2,5-xylyl group, a 2,6-xylyl group, a 3,4-xylyl group, a 3,5-xylyl group, a mesityl group (a 2,4,6-trimethylphenyl group), and the like. Among these, the p-tolyl group, the p-ethylphenyl group, the p-propylphenyl group, the p-butylphenyl group, the p-pentylphenyl group, the p-hexylphenyl group, the 2,4-xylyl group, the 2,6-xylyl group, the 3,5-xylyl group, and the mesityl group are preferable, and the p-tolyl group, the p-ethylphenyl group, and the p-propylphenyl group are more preferable. It should be noted that the alkyl group in the above specific examples is not limited to a normal-isomer and includes all of the branched isomers such as a sec-isomer, a tert-isomer, an iso-isomer, and a neo-isomer.

In a case where R₁₀₁ and R₁₀₂ in the general formula (I-1-1) form an alkylene group having 2 to 4 carbon atoms together and in a case where R₁₀₃ and R₁₀₄ in the general formula (I-1-1) form an alkylene group having 2 to 4 carbon atoms together, the alkylene group having 2 to 4 carbon atoms is any one of linear and branched alkylene groups. Among these, a linear alkylene group is preferable. Specific examples thereof include an ethylene group, a trimethylene group, a propylene group, a 1,1-dimethylmethylene group, a tetramethylene group, a 1-methyltrimethylene group, a 2-methyltrimethylene group, a 1,2-dimethylethylene group, a 1,1-dimethylethylene group, an ethylethylene group, and the like. Among these, the ethylene group, the trimethylene group, and the tetramethylene group are preferable, and the trimethylene group is more preferable.

In a case where R₁₀₁ and R₁₀₂ in the general formula (I-1-1) form an alkylene group having 2 to 4 carbon atoms together and/or in a case where R₁₀₃ and R₁₀₄ in the general formula (I-1-1) form an alkylene group having 2 to 4 carbon atoms together, specific examples of the ring structure represented by the general formula (I-1-1) include ring structures represented by the following general formulae (1-1-1-1) to (1-1-1-9). Among these, the ring structure represented by the general formula (1-1-1-2), (1-1-1-5), or (1-1-1-8) is preferable, and the ring structure represented by the general formula (1-1-1-8) is more preferable.

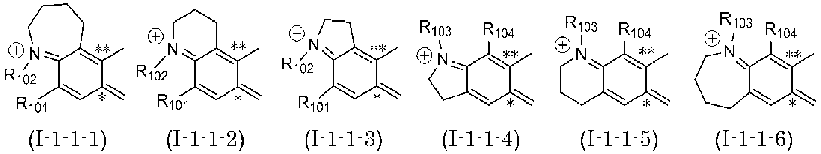

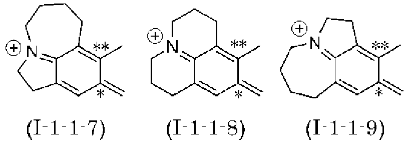

(In the formulae, R₁₀₁ to R₁₀₄, *, and ** are the same as described above.)

As R₁₀₁ in the general formula (I-1-1), a hydrogen atom, a methyl group, and a group which forms a linear alkylene group having 2 to 4 carbon atoms together with R₁₀₂ are preferable. Specific examples thereof include a hydrogen atom, a methyl group, a group which forms an ethylene group together with R₁₀₂, a group which forms a trimethylene group together with R₁₀₂, a group which forms a tetramethylene group together with R₁₀₂, and the like. Among these, the hydrogen atom, the methyl group, and the group which forms a trimethylene group together with R₁₀₂ are preferable.

As R₁₀₂ in the general formula (1-1-1), an alkyl group having 1 to 12 carbon atoms, a phenyl group which has an alkyl group having 1 to 6 carbon atoms or is unsubstituted, and a group which forms an alkylene group having 2 to 4 carbon atoms together with R₁₀₁ are preferable, and an alkyl group having 1 to 6 carbon atoms, a phenyl group which has an alkyl group having 1 to 3 carbon atoms or is unsubstituted, and a group which forms a linear alkylene group having 2 to 4 carbon atoms together with R₁₀₁ are more preferable. Specific examples thereof include a methyl group, an ethyl group, a propyl group, a butyl group, a pentyl group, a hexyl group, a phenyl group, a p-tolyl group, a p-ethylphenyl group, a p-propylphenyl group, a p-butylphenyl group, a p-pentylphenyl group, a p-hexylphenyl group, a 2,4-xylyl group, a 2,6-xylyl group, a 3,5-xylyl group, a mesityl group, a group which forms an ethylene group together with R₁₀₁, a group which forms a trimethylene group together with R₁₀₁, a group which forms a tetramethylene group together with R₁₀₁, and the like. Among these, the methyl group, the ethyl group, the propyl group, the butyl group, the pentyl group, the hexyl group, the phenyl group, the p-tolyl group, the p-ethylphenyl group, the p-propylphenyl group, and the group which forms a trimethylene group together with R₁₀₁ are preferable. It should be noted that the alkyl group in the above specific examples is not limited to a normal-isomer, and includes all of the branched isomers such as a sec-isomer, a tert-isomer, an iso-isomer, and a neo-isomer.

As R₁₀₃ in the general formula (1-1-1), an alkyl group having 1 to 12 carbon atoms, a phenyl group which has an alkyl group having 1 to 6 carbon atoms or is unsubstituted, and a group which forms an alkylene group having 2 to 4 carbon atoms together with R₁₀₄ are preferable, and an alkyl group having 1 to 6 carbon atoms, a phenyl group which has an alkyl group having 1 to 3 carbon atoms or is unsubstituted, and a group which forms a linear alkylene group having 2 to 4 carbon atoms together with R₁₀₄ are more preferable. Specific examples thereof include a methyl group, an ethyl group, a propyl group, a butyl group, a pentyl group, a hexyl group, a phenyl group, a p-tolyl group, a p-ethylphenyl group, a p-propylphenyl group, a p-butylphenyl group, a p-pentylphenyl group, a p-hexylphenyl group, a 2,4-xylyl group, a 2,6-xylyl group, a 3,5-xylyl group, a mesityl group, a group which forms an ethylene group together with R₁₀₄, a group which forms a trimethylene group together with R₁₀₄, a group which forms a tetramethylene group together with R₁₀₄, and the like. Among these, the methyl group, the ethyl group, the propyl group, the butyl group, the pentyl group, the hexyl group, the phenyl group, the p-tolyl group, the p-ethylphenyl group, the p-propylphenyl group, and the group which forms a trimethylene group together with R₁₀₄ are preferable. It should be noted that the alkyl group in the above specific examples is not limited to a normal-isomer, and includes all of the branched isomers such as a sec-isomer, a tert-isomer, an iso-isomer, and a neo-isomer.

As R₁₀₄ in the general formula (I-1-1), a hydrogen atom, a methyl group, and a group which forms a linear alkylene group having 2 to 4 carbon atoms together with R₁₀₃ are preferable. Specific examples thereof include a hydrogen atom, a methyl group, a group which forms an ethylene group together with R₁₀₃, a group which forms a trimethylene group together with R₁₀₃, a group which forms a tetramethylene group together with R₁₀₃, and the like. Among these, the hydrogen atom and the group which forms a trimethylene group together with R₁₀₃ are preferable.

Examples of the preferred combination of R₁₀₁ to R₁₀₄ in the general formula (I-1-1) include combinations described in the following table.

| **R₁₀₁** | **R₁₀₂** | **R₁₀₃** | **R₁₀₄** |
|---|---|---|---|
| Hydrogen atom | Methyl group | Methyl group, ethyl group, n-propyl group, isopropyl group, n-butyl group, isobutyl group, n-pentyl group, isopentyl group, n-hexyl group, isohexyl group, phenyl group, p-tolyl group, or p-ethylphenyl group | Hydrogen atom |
| Hydrogen atom | Ethyl group | | Hydrogen atom |
| Hydrogen atom | n-Propyl group | | Hydrogen atom |
| Hydrogen atom | n-Butyl group | | Hydrogen atom |
| Hydrogen atom | n-Pentyl group | | Hydrogen atom |
| Hydrogen atom | n-Hexyl group | | Hydrogen atom |
| Hydrogen atom | Isopropyl group | Methyl group, ethyl group, n-propyl group, n-butyl group, n-pentyl group, or n-hexyl group | Hydrogen atom |
| Hydrogen atom | Isobutyl group | | Hydrogen atom |
| Hydrogen atom | Isopentyl group | | Hydrogen atom |
| Hydrogen atom | Isohexyl group | | Hydrogen atom |
| Hydrogen atom | Phenyl group | | Hydrogen atom |
| Hydrogen atom | p-Tolyl group | | Hydrogen atom |
| Hydrogen atom | p-ethylphenyl group | | Hydrogen atom |
| Hydrogen atom | Methyl group | Methyl group, ethyl group, n-propyl group, n-butyl group, n-pentyl group, or n-hexyl group | Methyl group |
| Hydrogen atom | Ethyl group | | Methyl group |
| Hydrogen atom | n-Propyl group | | Methyl group |
| Hydrogen atom | n-Butyl group | | Methyl group |
| Hydrogen atom | n-Pentyl group | | Methyl group |
| Hydrogen atom | n-Hexyl group | | Methyl group |
| Methyl group | Methyl group | Methyl group, ethyl group, n-propyl group, n-butyl group, n-pentyl group, or n-hexyl group | Hydrogen atom |
| Methyl group | Ethyl group | | Hydrogen atom |
| Methyl group | n-Propyl group | | Hydrogen atom |
| Methyl group | n-Butyl group | | Hydrogen atom |
| Methyl group | n-Pentyl group | | Hydrogen atom |
| Methyl group | n-Hexyl group | | Hydrogen atom |
| Trimethylene group | | Trimethylene group | |

Examples of the alkyl group having 1 to 30 carbon atoms represented by R₁₃₁ in the general formulae (1-1-2) to (1-1-7) are the same as the examples of the alkyl group having 1 to 30 carbon atoms represented by R₁₀₅ in the general formula (I), and preferred examples are also the same.

As Ar₁ in the general formula (I), the ring structure represented by the general formula (I-1-1) is preferable.

In the general formula (I), * and ** correspond to * and ** in the general formulae (I-1-1) to (1-1-7), and represent that the ring structures represented by the general formulae (1-1-1) to (I-1-7) are bonded to the positions represented by * and ** in the dye residue represented by the general formula (I). Specific examples represent the following structures.

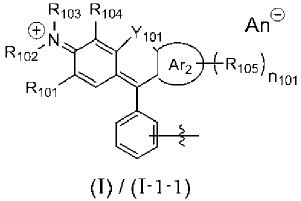

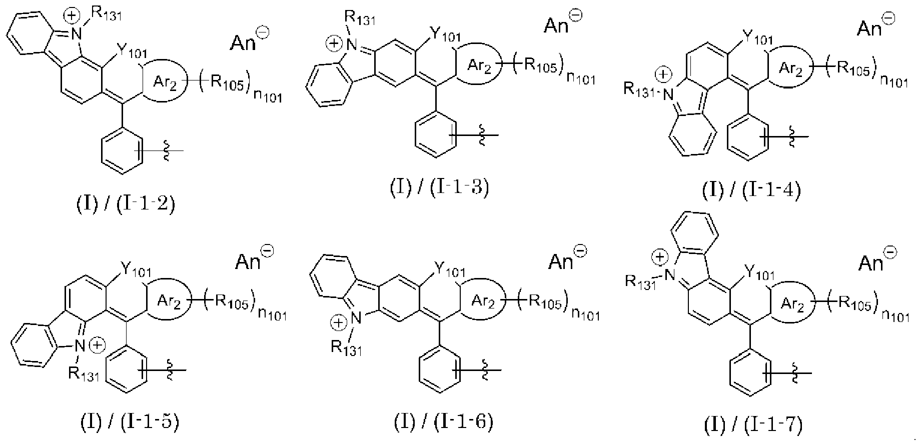

(In the formulae, R₁₀₁ to R₁₀₅, R₁₃₁, Y₁₀₁, An⁻, Ar₂, and n₁₀₁ are the same as described above.)

As Ar₂ in the general formula (I), a benzene ring and a naphthalene ring are preferable, and the benzene ring is more preferable.

In a case where Ar₂ is a benzene ring, n₁₀₁ in the general formula (1) is preferably an integer of 0 to 3 and more preferably 1 or 2, and in a case where Ar₂ is a naphthalene ring and an anthracene ring, n₁₀₁ is preferably 0 or 1 and more preferably 0.

In a case where Ar₂ in the general formula (I) is a benzene ring, the general formula (I) is represented by the following general formula (I-2-1). In a case where Ar₂ is a naphthalene ring, the general formula (I) is represented by any one of the following general formulae (1-2-2) to (1-2-4). In a case where Ar₂ is an anthracene ring, the general formula (I) is represented by any one of the following general formulae (1-2-5) to (1-2-7). Among these, the general formulae (1-2-1), (1-2-2), and (1-2-5) are preferable, and the general formula (1-2-1) is more preferable.

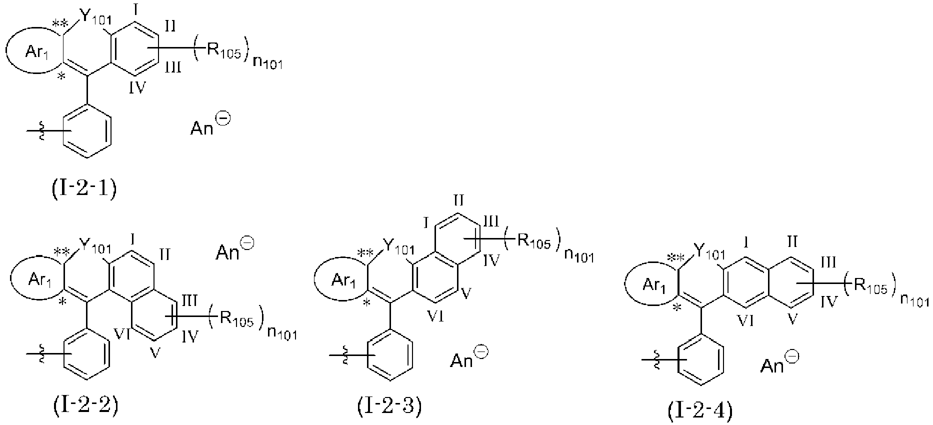

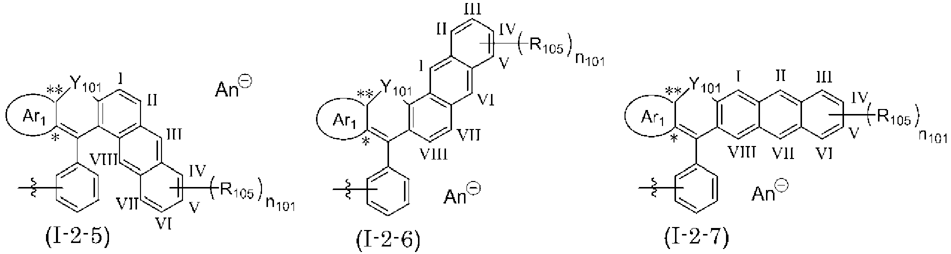

(In the formulae, I to VIII represent positions that can be substituted with R₁₀₅, and R₁₀₅, Y₁₀₁, An⁻, Ar₁, n₁₀₁, *, and ** are the same as described above.)

In the general formula (1-2-1), n₁₀₁ pieces of R₁₀₅ are located in any of positions I to IV in a benzene ring. In a case where n₁₀₁ is 1, R₁₀₅ is preferably located in position II or position III. In a case where n₁₀₁ is 2, R₁₀₅'s are preferably located in position II and position III or in position II and position IV. In a case where n₁₀₁ is 3, R₁₀₅'s are preferably located in position I, position II, and position III or in position II, position III, and position IV.

In the general formula (1-2-2), n₁₀₁ pieces of R₁₀₅ are located in any of positions I to VI in a naphthalene ring, and are preferably located in position IV or position VI.

In the general formula (1-2-3), n₁₀₁ pieces of R₁₀₅ are located in any of positions I to VI in a naphthalene ring, and are preferably located in position III.

In the general formula (1-2-4), n₁₀₁ pieces of R₁₀₅ are located in any of positions I to VI in a naphthalene ring, and are preferably located in position II or position IV.

In the general formula (1-2-5), n₁₀₁ pieces of R₁₀₅ are located in any of positions I to VIII in an anthracene ring, and are preferably located in position I, position V, or position VI.

In the general formula (1-2-6), n₁₀₁ pieces of R₁₀₅ are located in any of positions I to VIII in an anthracene ring, and are preferably located in position II, position V, or position VII.

In the general formula (1-2-7), n₁₀₁ pieces of R₁₀₅ are located in any of positions I to VIII in an anthracene ring, and are preferably located in position IV or position V.

In a case where Dye in the general formula (1) is the dye residue represented by the general formula (I), the portion to which Y₀ is bonded in the dye residue is any of the ortho-position, the meta-position, and the para-position of the phenyl group in the general formula (I). In a case where n in the general formula (1) is 1, the ortho-position or the para-position is preferable, and the ortho-position is more preferable. In a case where n in the general formula (1) is 2, a combination of the ortho-position and the para-position is preferable. Specifically, in a case where n in the general formula (1) is 1, it is preferable that Y₀ is bonded as shown in a compound represented by the following general formula (I'-1); and in a case where n in the general formula (1) is 2, it is preferable that Y₀ is bonded as shown in a compound represented by the following general formula (I'-2).

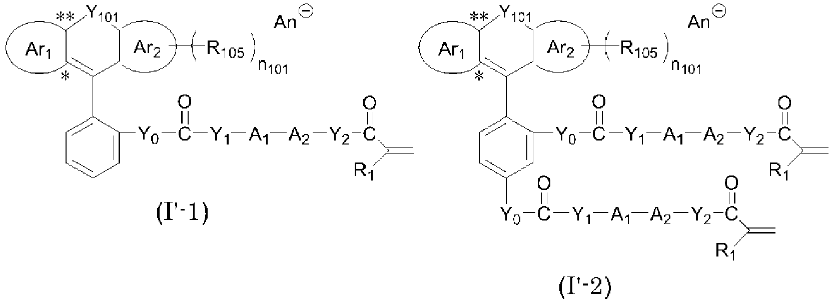

(In the formulae, R₁, R₁₀₅, Y₀, Y₁, Y₂, Y₁₀₁, A₁, A₂, Ar₁, Ar₂, An⁻, n₁₀₁, *, and ** are the same as described above. Here, a plurality of R₁'s, a plurality of Y₀'s, a plurality of Y₁'s, a plurality of Y₂'s, a plurality of A₁'s, and a plurality of A₂'s are the same as or different from each other respectively.)

Specific examples preferred as the general formula (I) include a dye residue represented by the following general formula (1-3).

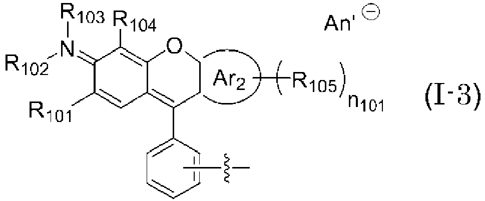

(In the formula, An'⁻ represents an anion containing an aryl group having an electron-withdrawing substituent, a sulfonyl group having an electron-withdrawing substituent, a haloalkyl group, or a halogeno group, and R₁₀₁ to R₁₀₅, Ar₂, and n₁₀₁ are the same as described above.)

Examples of the anion, which contains an aryl group having an electron-withdrawing substituent, a sulfonyl group having an electron-withdrawing substituent, a haloalkyl group, or a halogeno group, represented by An'⁻ in the general formula (1-3) are the same as the examples of the anions according to the present invention, and preferred examples are also the same.

Specific examples preferred as the general formula (1-3) include a dye residue represented by the following general formula (1-4).

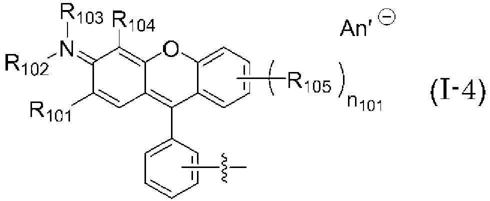

(In the formula, R₁₀₁ to R₁₀₅, An'⁻, and n₁₀₁ are the same as described above.)

Specific examples preferred as the general formula (1-4) include a dye residue represented by the following general formula (1-5).

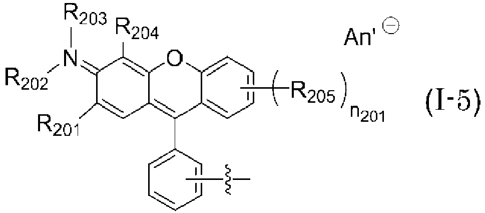

(In the formula, R₂₀₁ and R₂₀₄ each independently represent a hydrogen atom or a methyl group, R₂₀₂ and R₂₀₃ each independently represent an alkyl group having 1 to 12 carbon atoms or a phenyl group which has an alkyl group having 1 to 6 carbon atoms or is unsubstituted, n₂₀₁ pieces of R₂₀₅ represents a halogeno group; an alkyl group having 1 to 12 carbon atoms; an alkoxy group having 1 to 12 carbon atoms; an alkylthio group having 1 to 12 carbon atoms; an amino group having an alkyl group having 1 to 30 carbon atoms, a haloalkyl group having 1 to 20 carbon atoms, an aryl group having 6 to 10 carbon atoms, or an arylalkyl group having 7 to 13 carbon atoms; a hydroxy group; an aryl group having 6 to 14 carbon atoms; an aryloxy group having 6 to 14 carbon atoms; or an arylalkyl group having 7 to 20 carbon atoms, n₂₀₁ represents an integer of 0 to 4, R₂₀₁ and R₂₀₂ may form an alkylene group having 2 to 4 carbon atoms together, R₂₀₃ and R₂₀₄ may form an alkylene group having 2 to 4 carbon atoms together, and An'⁻ is the same as described above.)

The alkyl group having 1 to 12 carbon atoms represented by R₂₀₂ and R₂₀₃ in the general formula (1-5) is any one of linear, branched, and cyclic alkyl groups. Among these, linear and branched alkyl groups are preferable. In addition, among the alkyl group having 1 to 12 carbon atoms, an alkyl group having 1 to 6 carbon atoms is more preferable, an alkyl group having 1 to 4 carbon atoms is even more preferable, and an alkyl group having 1 or 2 carbon atoms is particularly preferable. Specific examples thereof include a methyl group, an ethyl group, an n-propyl group, an isopropyl group, an n-butyl group, an isobutyl group, a sec-butyl group, a tert-butyl group, a cyclobutyl group, an n-pentyl group, an isopentyl group, a sec-pentyl group, a tert-pentyl group, a neopentyl group, a 2-methylbutyl group, a 1,2-dimethylpropyl group, a 1-ethylpropyl group, a cyclopentyl group, an n-hexyl group, an isohexyl group, a sec-hexyl group, a tert-hexyl group, a neohexyl group, a 2-methylpentyl group, a 1,2-dimethylbutyl group, a 2,3-dimethylbutyl group, a 1-ethylbutyl group, a cyclohexyl group, an n-heptyl group, an isoheptyl group, a sec-heptyl group, a tert-heptyl group, a neoheptyl group, a cycloheptyl group, an n-octyl group, an isooctyl group, a sec-octyl group, a tert-octyl group, a neooctyl group, a 2-ethylhexyl group, a cyclooctyl group, an n-nonyl group, an isononyl group, a sec-nonyl group, a tert-nonyl group, a neononyl group, a cyclononyl group, an n-decyl group, an isodecyl group, a sec-decyl group, a tert-decyl group, a neodecyl group, a cyclodecyl group, an n-undecyl group, a cycloundecyl group, an n-dodecyl group, a cyclododecyl group, a cyclohexylmethyl group, a 1-cyclohexylethyl group, a 2-methylcyclohexyl group, a 3-methylcyclohexyl group, a 4-methylcyclohexyl group, a 2,6-dimethylcyclohexyl group, a 2,4-dimethylcyclohexyl group, a 3,5-dimethylcyclohexyl group, a 2,5-dimethylcyclohexyl group, a 2,3-dimethylcyclohexyl group, a 3,3,5-trimethylcyclohexyl group, a 4-tert-butylcyclohexyl group, a 1-adamantyl group, a 2-adamantyl group, and the like. Among these, the methyl group, the ethyl group, the n-propyl group, the isopropyl group, the n-butyl group, the isobutyl group, the sec-butyl group, the tert-butyl group, the n-pentyl group, the isopentyl group, the sec-pentyl group, the tert-pentyl group, the neopentyl group, the 2-methylbutyl group, the 1,2-dimethylpropyl group, the 1-ethylpropyl group, the n-hexyl group, the isohexyl group, the sec-hexyl group, the tert-hexyl group, the neohexyl group, the 2-methylpentyl group, the 1,2-dimethylbutyl group, the 2,3-dimethylbutyl group, the 1-ethylbutyl group, the n-heptyl group, the n-octyl group, the n-nonyl group, the n-decyl group, the n-undecyl group, and the n-dodecyl group are preferable, the methyl group, the ethyl group, the n-propyl group, the isopropyl group, the n-butyl group, the isobutyl group, the sec-butyl group, the tert-butyl group, the n-pentyl group, the isopentyl group, and the n-hexyl group are more preferable, the methyl group, the ethyl group, the n-propyl group, the isopropyl group, the n-butyl group, the isobutyl group, the sec-butyl group, and the tert-butyl group are even more preferable, and the methyl group and the ethyl group are particularly preferable.

The phenyl group having an alkyl group having 1 to 6 carbon atoms represented by R₂₀₂ and R₂₀₃ in the general formula (1-5) generally has 1 to 5 alkyl groups, preferably has 1 to 3 alkyl groups, and more preferably has 1 alkyl group. Examples of the alkyl group are the same as the examples of the alkyl group having 1 to 6 carbon atoms represented by R₃ in the general formula (2), and preferred examples are also the same.

As the phenyl group having an alkyl group having 1 to 6 carbon atoms represented by R₂₀₂ and R₂₀₃ in the general formula (1-5), a phenyl group having an alkyl group having 1 to 3 carbon atoms is particularly preferable. Specific examples thereof include, an o-tolyl group, a m-tolyl group, a p-tolyl group, an o-ethylphenyl group, a m-ethylphenyl group, a p-ethylphenyl group, an o-propylphenyl group, a m-propylphenyl group, a p-propylphenyl group, an o-butylphenyl group, a m-butylphenyl group, a p-butylphenyl group, an o-pentylphenyl group, a m-pentylphenyl group, a p-pentylphenyl group, an o-hexylphenyl group, a m-hexylphenyl group, a p-hexylphenyl group, a 2,3-xylyl group, a 2,4-xylyl group, a 2,5-xylyl group, a 2,6-xylyl group, a 3,4-xylyl group, a 3,5-xylyl group, a mesityl group, and the like. Among these, the p-tolyl group, the p-ethylphenyl group, the p-propylphenyl group, the p-butylphenyl group, the p-pentylphenyl group, the p-hexylphenyl group, the 2,4-xylyl group, the 2,6-xylyl group, the 3,5-xylyl group, and the mesityl group are preferable, and the p-tolyl group, the p-ethylphenyl group, and the p-propylphenyl group are more preferable. It should be noted that the alkyl group in the above specific examples is not limited to a normal-isomer and includes all of the branched isomers such as a sec-isomer, a tert-isomer, an iso-isomer, and a neo-isomer.

In a case where R₂₀₁ and R₂₀₂ in the general formula (I-5) form an alkylene group having 2 to 4 carbon atoms together, and in a case where R₂₀₃ and R₂₀₄ in the general formula (I-5) form an alkylene group having 2 to 4 carbon atoms together, examples of the alkylene group having 2 to 4 carbon atoms are the same as the examples of the alkylene group having 2 to 4 carbon atoms formed in a case where R₁₀₁ and R₁₀₂ in the general formula (I-1-1) form an alkylene group having 2 to 4 carbon atoms together and in a case where R₁₀₃ and R₁₀₄ in the general formula (I-1-1) form an alkylene group having 2 to 4 carbon atoms together, and preferred examples are also the same.

In a case where R₂₀₁ and R₂₀₂ in the general formula (1-5) form an alkylene group having 2 to 4 carbon atoms together and/or in a case where R₂₀₃ and R₂₀₄ in the general formula (1-5) form an alkylene group having 2 to 4 carbon atoms together, specific examples of the formed compounds include compounds represented by the following general formulae (I-5-1) to (I-5-9). Among these, the compound represented by the general formula (I-5-2), (I-5-5), or (I-5-8) is preferable, and the compound represented by the general formula (I-5-8) is more preferable.

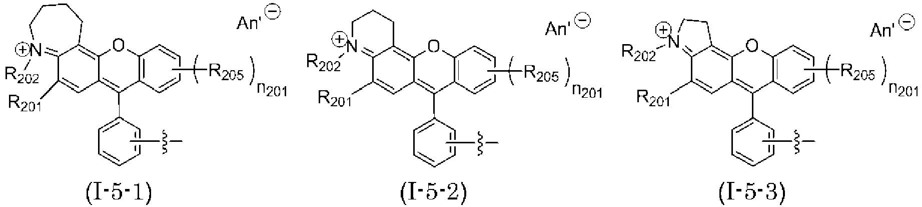

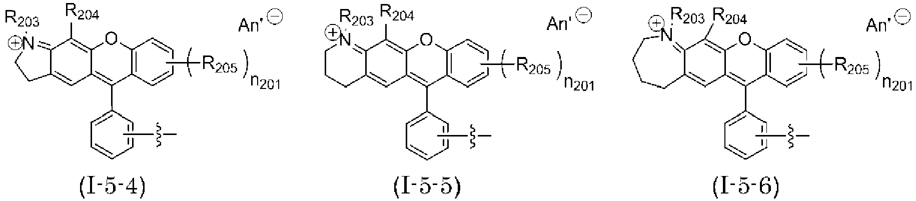

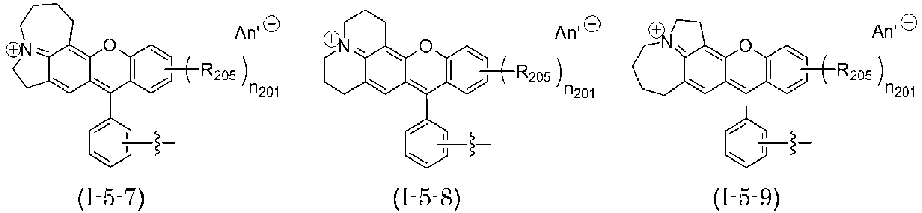

(In the formulae, R₂₀₁ to R₂₀₅, An'⁻, and n₂₀₁ are the same as described above.)

As R₂₀₁ in the general formula (I-5), a hydrogen atom, a methyl group, and a group which forms a linear alkylene group having 2 to 4 carbon atoms together with R₂₀₂ are preferable. Specific examples thereof include a hydrogen atom, a methyl group, a group which forms an ethylene group together with R₂₀₂, a group which forms a trimethylene group together with R₂₀₂, a group which forms a tetramethylene group together with R₂₀₂, and the like. Among these, the hydrogen atom, the methyl group, and the group which forms a trimethylene group together with R₂₀₂ are preferable.

As R₂₀₂ in the general formula (1-5), an alkyl group having 1 to 6 carbon atoms, a phenyl group which has an alkyl group having 1 to 3 carbon atoms or is unsubstituted, and a group which forms a linear alkylene group having 2 to 4 carbon atoms together with R₂₀₁ are preferable. Specific examples thereof include a methyl group, an ethyl group, a propyl group, a butyl group, a pentyl group, a hexyl group, a phenyl group, a p-tolyl group, a p-ethylphenyl group, a p-propylphenyl group, a p-butylphenyl group, a p-pentylphenyl group, a p-hexylphenyl group, a 2,4-xylyl group, a 2,6-xylyl group, a 3,5-xylyl group, a mesityl group, a group which forms an ethylene group together with R₂₀₁, a group which forms a trimethylene group together with R₂₀₁, a group which forms a tetramethylene group together with R₂₀₁, and the like. Among these, the methyl group, the ethyl group, the propyl group, the butyl group, the pentyl group, the hexyl group, the phenyl group, the p-tolyl group, the p-ethylphenyl group, the p-propylphenyl group, and the group which forms a trimethylene group together with R₂₀₁ are preferable. It should be noted that the alkyl group in the above specific examples is not limited to a normal-isomer, and includes all of the branched isomers such as a sec-isomer, a tert-isomer, an iso-isomer, and a neo-isomer.

As R₂₀₃ in the general formula (1-5), an alkyl group having 1 to 6 carbon atoms, a phenyl group which has an alkyl group having 1 to 3 carbon atoms or is unsubstituted, and a group which forms a linear alkylene group having 2 to 4 carbon atoms together with R₂₀₄ are preferable. Specific examples thereof include a methyl group, an ethyl group, a propyl group, a butyl group, a pentyl group, a hexyl group, a phenyl group, a p-tolyl group, a p-ethylphenyl group, a p-propylphenyl group, a p-butylphenyl group, a p-pentylphenyl group, a p-hexylphenyl group, a 2,4-xylyl group, a 2,6-xylyl group, a 3,5-xylyl group, a mesityl group, a group which forms an ethylene group together with R₂₀₄, a group which forms a trimethylene group together with R₂₀₄, a group which forms a tetramethylene group together with R₂₀₄, and the like. Among these, the methyl group, the ethyl group, the propyl group, the butyl group, the pentyl group, the hexyl group, the phenyl group, the p-tolyl group, the p-ethylphenyl group, the p-propylphenyl group, and the group which forms a trimethylene group together with R₂₀₄ are preferable. It should be noted that the alkyl group in the above specific examples is not limited to a normal-isomer, and includes all of the branched isomers such as a sec-isomer, a tert-isomer, an iso-isomer, and a neo-isomer.

As R₂₀₄ in the general formula (1-5), a hydrogen atom, a methyl group, and a group which forms a linear alkylene group having 2 to 4 carbon atoms together with R₂₀₃ are preferable. Specific examples thereof include a hydrogen atom, a methyl group, a group which forms an ethylene group together with R₂₀₃, a group which forms a trimethylene group together with R₂₀₃, a group which forms a tetramethylene group together with R₂₀₃, and the like. Among these, the hydrogen atom and the group which forms a trimethylene group together with R₂₀₃ are preferable.

Examples of the halogeno group represented by R₂₀₅ in the general formula (1-5) include a fluoro group, a chloro group, a bromo group, and an iodo group. Among these, the fluoro group is preferable.

Examples of the alkyl group having 1 to 12 carbon atoms represented by R₂₀₅ in the general formula (1-5) are the same as the examples of the alkyl group having 1 to 12 carbon atoms represented by R₂₀₂ and R₂₀₃ in the general formula (1-5), and preferred examples are also the same.

The alkoxy group having 1 to 12 carbon atoms represented by R₂₀₅ in the general formula (1-5) is any one of linear, branched, and cyclic alkoxy groups. Among these, linear and branched alkoxy groups are preferable. In addition, among the alkoxy group having 1 to 12 carbon atoms, an alkoxy group having 1 to 6 carbon atoms is preferable, and an alkoxy group having 1 to 4 carbon atoms is more preferable. Specific examples thereof include a methoxy group, an ethoxy group, an n-propoxy group, an isopropoxy group, an n-butoxy group, an isobutoxy group, a sec-butoxy group, a tert-butoxy group, a cyclobutoxy group, an n-pentyloxy group, an isopentyloxy group, a sec-pentyloxy group, a tert-pentyloxy group, a neopentyloxy group, a 2-methylbutoxy group, a 1,2-dimethylpropoxy group, a 1-ethylpropoxy group, a cyclopentyloxy group, an n-hexyloxy group, an isohexyloxy group, a sec-hexyloxy group, a tert-hexyloxy group, a neohexyloxy group, a 2-methylpentyloxy group, a 1,2-dimethylbutoxy group, a 2,3-dimethylbutoxy group, a 1-ethylbutoxy group, a cyclohexyloxy group, an n-heptyloxy group, an isoheptyloxy group, a sec-heptyloxy group, a tert-heptyloxy group, a neoheptyloxy group, a cycloheptyloxy group, an n-octyloxy group, an isooctyloxy group, a sec-octyloxy group, a tert-octyloxy group, a neooctyloxy group, a 2-ethylhexyloxy group, a cyclooctyloxy group, an n-nonyloxy group, an isononyloxy group, a sec-nonyloxy group, a tert-nonyloxy group, a neononyloxy group, a cyclononyloxy group, an n-decyloxy group, an isodecyloxy group, a sec-decyloxy group, a tert-decyloxy group, a neodecyloxy group, a cyclodecyloxy group, an n-undecyloxy group, a cycloundecyloxy group, an n-dodecyloxy group, a cyclododecyloxy group, and the like. Among these, the methoxy group, the ethoxy group, the n-propoxy group, the isopropoxy group, the n-butoxy group, the isobutoxy group, the sec-butoxy group, the tert-butoxy group, the n-pentyloxy group, the isopentyloxy group, the sec-pentyloxy group, the tert-pentyloxy group, the neopentyloxy group, the 2-methylbutoxy group, the 1,2-dimethylpropoxy group, the 1-ethylpropoxy group, the n-hexyloxy group, the isohexyloxy group, the sec-hexyloxy group, the tert-hexyloxy group, the neohexyloxy group, the 2-methylpentyloxy group, the 1,2-dimethylbutoxy group, the 2,3-dimethylbutoxy group, and the 1-ethylbutoxy group are preferable, the methoxy group, the ethoxy group, the n-propoxy group, the isopropoxy group, the n-butoxy group, the isobutoxy group, the sec-butoxy group, and the tert-butoxy group are more preferable, and the methoxy group and the ethoxy group are even more preferable.

The alkylthio group having 1 to 12 carbon atoms represented by R₂₀₅ in the general formula (1-5) is any one of linear, branched, and cyclic alkylthio groups. Among these, linear and branched alkylthio groups are preferable. In addition, among the alkylthio group having 1 to 12 carbon atoms, an alkylthio group having 1 to 6 carbon atoms is preferable, and an alkylthio group having 1 to 4 carbon atoms is more preferable. Specific examples thereof include a methylthio group, an ethylthio group, an n-propylthio group, an isopropylthio group, an n-butylthio group, an isobutylthio group, a sec-butylthio group, a tert-butylthio group, a cyclobutylthio group, an n-pentylthio group, an isopentylthio group, a sec-pentylthio group, a tert-pentylthio group, a neopentylthio group, a 2-methylbutylthio group, a 1,2-dimethylpropylthio group, a 1-ethylpropylthio group, a cyclopentylthio group, an n-hexylthio group, an isohexylthio group, a sec-hexylthio group, a tert-hexylthio group, a neohexylthio group, a 2-methylpentylthio group, a 1,2-dimethylbutylthio group, a 2,3-dimethylbutylthio group, a 1-ethylbutylthio group, a cyclohexylthio group, an n-heptylthio group, an isoheptylthio group, a sec-heptylthio group, a tert-heptylthio group, a neoheptylthio group, a cycloheptylthio group, an n-octylthio group, an isooctylthio group, a sec-octylthio group, a tert-octylthio group, a neooctylthio group, a 2-ethylhexylthio group, a cyclooctylthio group, an n-nonylthio group, an isononylthio group, a sec-nonylthio group, a tert-nonylthio group, a neononylthio group, a cyclononylthio group, an n-decylthio group, an isodecylthio group, a sec-decylthio group, a tert-decylthio group, a neodecylthio group, a cyclodecylthio group, an n-undecylthio group, a cycloundecylthio group, an n-dodecylthio group, a cyclododecylthio group, and the like. Among these, the methylthio group, the ethylthio group, the n-propylthio group, the isopropylthio group, the n-butylthio group, the isobutylthio group, the sec-butylthio group, the tert-butylthio group, the n-pentylthio group, the isopentylthio group, the sec-pentylthio group, the tert-pentylthio group, the neopentylthio group, the 2-methylbutylthio group, the 1,2-dimethylpropylthio group, the 1-ethylpropylthio group, the n-hexylthio group, the isohexylthio group, the sec-hexylthio group, the tert-hexylthio group, the neohexylthio group, the 2-methylpentylthio group, the 1,2-dimethylbutylthio group, the 2,3-dimethylbutylthio group, and the 1-ethylbutylthio group are preferable, the methylthio group, the ethylthio group, the n-propylthio group, the isopropylthio group, the n-butylthio group, the isobutylthio group, the sec-butylthio group, and the tert-butylthio group are more preferable, and the methylthio group and the ethylthio group are even more preferable.

In a case where R₂₀₅ in the general formula (1-5) represents "an amino group having an alkyl group having 1 to 30 carbon atoms, a haloalkyl group having 1 to 20 carbon atoms, an aryl group having 6 to 10 carbon atoms, or an arylalkyl group having 7 to 13 carbon atoms", specific examples of the alkyl group having 1 to 30 carbon atoms, the haloalkyl group having 1 to 20 carbon atoms, the aryl group having 6 to 10 carbon atoms, and the arylalkyl group having 7 to 13 carbon atoms are the same as the specific examples of "the amino group having a substituent" represented by R₁₀₅ in the general formula (I), and preferred examples are also the same.

As "an amino group having an alkyl group having 1 to 30 carbon atoms, a haloalkyl group having 1 to 20 carbon atoms, an aryl group having 6 to 10 carbon atoms, or an arylalkyl group having 7 to 13 carbon atoms" represented by R₂₀₅ in the general formula (1-5), an amino group having an alkyl group having 1 to 12 carbon atoms, a phenyl group, or a phenylalkyl group having 7 to 9 carbon atoms is preferable. Specific examples thereof include a methylamino group, an ethylamino group, an n-propylamino group, an isopropylamino group, an n-butylamino group, an isobutylamino group, a sec-butylamino group, a tert-butylamino group, an n-pentylamino group, an isopentylamino group, an n-hexylamino group, a phenylamino group, a benzylamino group, a phenethylamino group, a hydrocinnamylamino group, a dimethylamino group, a diethylamino group, a di-n-propylamino group, a diisopropylamino group, a di-n-butylamino group, a diisobutylamino group, a di-sec-butylamino group, a di-tert-butylamino group, a di-n-pentylamino group, a diisopentylamino group, a di-n-hexylamino group, a diphenylamino group, a dibenzylamino group, a diphenethylamino group, a bis(hydrocinnamyl)amino group, and the like. Among these, the methylamino group, the ethylamino group, the n-propylamino group, the isopropylamino group, the n-butylamino group, the isobutylamino group, the sec-butylamino group, the tert-butylamino group, the phenylamino group, the benzylamino group, the dimethylamino group, the diethylamino group, the di-n-propylamino group, the diisopropylamino group, the di-n-butylamino group, the diisobutylamino group, the di-sec-butylamino group, the di-tert-butylamino group, the diphenylamino group, and the dibenzylamino group are preferable, and the methylamino group, the ethylamino group, the phenylamino group, the benzylamino group, the dimethylamino group, the diethylamino group, the diphenylamino group, and the dibenzylamino group are more preferable.

Specific examples of the aryl group having 6 to 14 carbon atoms, the aryloxy group having 6 to 14 carbon atoms, and the arylalkyl group having 7 to 20 carbon atoms that are represented by R₂₀₅ in the general formula (1-5) are the same as the specific examples of those represented by R₁₀₅ in the general formula (I), and preferred examples are also the same.

As R₂₀₅ in the general formula (1-5), a halogeno group; an alkyl group having 1 to 12 carbon atoms; an alkoxy group having 1 to 12 carbon atoms; an alkylthio group having 1 to 12 carbon atoms; an amino group having an alkyl group having 1 to 12 carbon atoms, a phenyl group, or a phenylalkyl group having 7 to 9 carbon atoms; a hydroxy group; a phenyl group; a phenoxy group; and a phenylalkyl group having 7 to 12 carbon atoms are more preferable, the halogeno group; an alkyl group having 1 to 6 carbon atoms; an alkoxy group having 1 to 6 carbon atoms; an alkylthio group having 1 to 6 carbon atoms; the amino group having the alkyl group having 1 to 12 carbon atoms, the phenyl group, or the phenylalkyl group having 7 to 9 carbon atoms; the hydroxy group; the phenyl group; the phenoxy group; and the phenylalkyl group having 7 to 9 carbon atoms are even more preferable. Specifically, a fluorine atom, a methyl group, an ethyl group, an n-propyl group, an isopropyl group, an n-butyl group, an isobutyl group, a sec-butyl group, a tert-butyl group, an n-pentyl group, an isopentyl group, an n-hexyl group, a methoxy group, an ethoxy group, an n-propoxy group, an isopropoxy group, an n-butoxy group, an isobutoxy group, a sec-butoxy group, a tert-butoxy group, a methylthio group, an ethylthio group, an n-propylthio group, an isopropylthio group, an n-butylthio group, an isobutylthio group, a sec-butylthio group, a tert-butylthio group, a methylamino group, an ethylamino group, an n-propylamino group, an isopropylamino group, an n-butylamino group, an isobutylamino group, a sec-butylamino group, a tert-butylamino group, a phenylamino group, a benzylamino group, a dimethylamino group, a diethylamino group, a di-n-propylamino group, a diisopropylamino group, a di-n-butylamino group, a diisobutylamino group, a di-sec-butylamino group, a di-tert-butylamino group, a diphenylamino group, a dibenzylamino group, a hydroxy group, a phenyl group, a phenoxy group, a benzyl group, a phenethyl group, a 1-phenylethyl group, a hydrocinnamyl group, a 2-phenylpropyl group, a 1-phenylpropyl group, and a cumyl group are preferable, and the fluorine atom, the methyl group, the ethyl group, the n-propyl group, the isopropyl group, the n-butyl group, the isobutyl group, the sec-butyl group, the tert-butyl group, the methoxy group, the ethoxy group, the methylthio group, the ethylthio group, the methylamino group, the ethylamino group, the phenylamino group, the benzylamino group, the dimethylamino group, the diethylamino group, the diphenylamino group, the dibenzylamino group, the hydroxy group, the phenyl group, the phenoxy group, the benzyl group, the phenethyl group, the hydrocinnamyl group, and the cumyl group are more preferable.

As n₂₀₁ in the general formula (I-5), an integer of 0 to 3 is preferable, and 1 or 2 is more preferable.

In the general formula (1-5), n₂₀₁ pieces of R₂₀₅ are located in any of positions I to IV in a benzene ring in the following general formula (I-6). In a case where n₂₀₁ is 1, R₂₀₅ is preferably located in position II or position III. In a case where n₂₀₁ is 2, R₂₀₅'s are preferably located in position II and position III or in position II and position IV. In a case where n₂₀₁ is 3, R₂₀₅'s are preferably located in position I, position II, and position III or in position II, position III, and position IV.

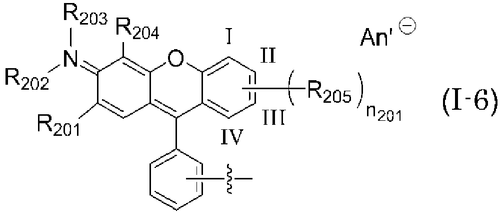

(In the formula, I to IV represent positions that can be substituted with R₂₀₅, and R₂₀₁ to R₂₀₅, An'⁻, and n₂₀₁ are the same as described above.)

Specific examples preferred as the general formula (I-5) include a dye residue represented by the following general formula (II).

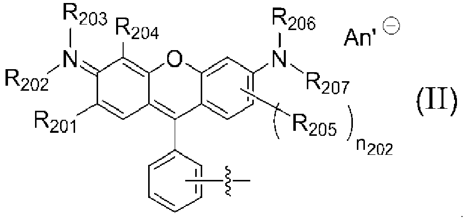

(In the formula, R₂₀₆ and R₂₀₇ each independently represent an alkyl group having 1 to 12 carbon atoms, a phenyl group, or a phenylalkyl group having 7 to 9 carbon atoms, n₂₀₂ represents an integer of 0 to 3, and R₂₀₁ to R₂₀₅ and An'⁻ are the same as described above.)

Examples of the alkyl group having 1 to 12 carbon atoms represented by R₂₀₆ and R₂₀₇ in the general formula (II) are the same as the examples of the alkyl group having 1 to 12 carbon atoms represented by R₂₀₂ and R₂₀₃ in the general formula (1-5), and preferred examples are also the same.

Examples of the phenylalkyl group having 7 to 9 carbon atoms represented by R₂₀₆ and R₂₀₇ in the general formula (II) include a benzyl group, a phenethyl group, a 1-phenylethyl group, a hydrocinnamyl group, a 2-phenylpropyl group, a 1-phenylpropyl group, and a cumyl group. Among these, the benzyl group, the phenethyl group, the hydrocinnamyl group, and the cumyl group are preferable.

As R₂₀₆ and R₂₀₇ in the general formula (II), an alkyl group having 1 to 6 carbon atoms, a phenyl group, and a phenylalkyl group having 7 to 9 carbon atoms are preferable, and the alkyl group having 1 to 6 carbon atoms is more preferable. Specific examples thereof include a methyl group, an ethyl group, a propyl group, a butyl group, a pentyl group, a hexyl group, a phenyl group, a benzyl group, a phenethyl group, a hydrocinnamyl group, a cumyl group, and the like. Among these, the methyl group, the ethyl group, the propyl group, the butyl group, the pentyl group, and the hexyl group are preferable. It should be noted that the alkyl group in the above specific examples is not limited to a normal-isomer and includes all of the branched isomers such as a sec-isomer, a tert-isomer, an iso-isomer, and a neo-isomer.

In addition, R₂₀₆ in the general formula (II) is preferably the same functional group as R₂₀₃ in the general formula (II), and R₂₀₇ in the general formula (II) is preferably the same functional group as R₂₀₂ in the general formula (II).

As n₂₀₂ in the general formula (II), 0 or 1 is preferable.

In the general formula (II), n₂₀₂ pieces of R₂₀₅ are located in any of position I, position III, and position IV in a benzene ring in the following general formula (II-1). In a case where n₂₀₂ is 1, R₂₀₅ is preferably located in position III or position IV, and more preferably located in position III. In a case where n₂₀₂ is 2, R₂₀₅'s are preferably located in position I and position III or in position III and position IV, and more preferably located in position I and position III.

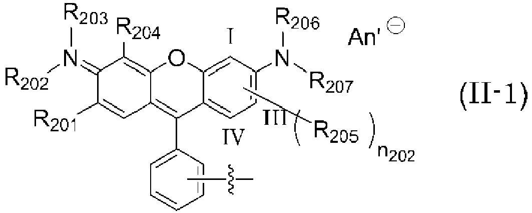

(In the formula, I, III, and IV represent positions that can be substituted with R₂₀₅, and R₂₀₁ to R₂₀₇, An'⁻, and n₂₀₂ are the same as described above.)

Examples of the preferred combination of R₂₀₁ to R₂₀₇ and n₂₀₂ in the general formula (II) include combinations described in the following table.

| **R₂₀₁** | **R₂₀₂ and R₂₀₇** | **R₂₀₃ and R₂₀₆** | **R₂₀₄** | (**n₂₀₂ pieces of) R₂₀₅** | **n₂₀₂** |
|---|---|---|---|---|---|
| Hydrogen atom | Methyl group | Methyl group, ethyl group, n-propyl group, isopropyl group, n-butyl group, isobutyl group, n-pentyl group, isopentyl group, n-hexyl group, isohexyl group, or phenyl group | Hydrogen atom | N/A | 0 |
| Hydrogen atom | Ethyl group | | Hydrogen atom | N/A | 0 |
| Hydrogen atom | n-Propyl group | | Hydrogen atom | N/A | 0 |
| Hydrogen atom | n-Butyl group | | Hydrogen atom | N/A | 0 |
| Hydrogen atom | n-Pentyl group | | Hydrogen atom | N/A | 0 |
| Hydrogen atom | n-Hexyl group | | Hydrogen atom | N/A | 0 |
| Hydrogen atom | Isopropyl group | Methyl group, ethyl group, n-propyl group, n-butyl group, n-pentyl group, or n-hexyl group | Hydrogen atom | N/A | 0 |
| Hydrogen atom | Isobutyl group | | Hydrogen atom | N/A | 0 |
| Hydrogen atom | Isopentyl group | | Hydrogen atom | N/A | 0 |
| Hydrogen atom | Isohexyl group | | Hydrogen atom | N/A | 0 |
| Hydrogen atom | Phenyl group | | Hydrogen atom | N/A | 0 |
| Hydrogen atom | Methyl group | Methyl group, ethyl group, n-propyl group, n-butyl group, n-pentyl group, or n-hexyl group | Methyl group | Methyl group | 1 |
| Hydrogen atom | Ethyl group | | Methyl group | Methyl group | 1 |
| Hydrogen atom | n-Propyl group | | Methyl group | Methyl group | 1 |
| Hydrogen atom | n-Butyl group | | Methyl group | Methyl group | 1 |
| Hydrogen atom | n-Pentyl group | | Methyl group | Methyl group | 1 |
| Hydrogen atom | n-Hexyl group | | Methyl group | Methyl group | 1 |
| Methyl group | Methyl group | Methyl group, ethyl group, n-propyl group, n-butyl group, n-pentyl group, or n-hexyl group | Hydrogen atom | Methyl group | 1 |
| Methyl group | Ethyl group | | Hydrogen atom | Methyl group | 1 |
| Methyl group | n-Propyl group | | Hydrogen atom | Methyl group | 1 |
| Methyl group | n-Butyl group | | Hydrogen atom | Methyl group | 1 |
| Methyl group | n-Pentyl group | | Hydrogen atom | Methyl group | 1 |
| Methyl group | n-Hexyl group | | Hydrogen atom | Methyl group | 1 |
| Methyl group | Methyl group | Methyl group, ethyl group, n-propyl group, n-butyl group, n-pentyl group, or n-hexyl group | Methyl group | Two methyl groups | 2 |
| Methyl group | Ethyl group | | Methyl group | Two methyl groups | 2 |
| Methyl group | n-Propyl group | | Methyl group | Two methyl groups | 2 |
| Methyl group | n-Butyl group | | Methyl group | Two methyl groups | 2 |
| Methyl group | n-Pentyl group | | Methyl group | Two methyl groups | 2 |
| Methyl group | n-Hexyl group | | Methyl group | Two methyl groups | 2 |

Examples of An'⁻ used by being combined with the combinations described in the table include the following anions. Among these, a tetrakis(pentafluorophenyl)boron (IV) anion, a bis(trifluoromethanesulfonyl)imide anion, and PF₆⁻ are preferable, the tetrakis(pentafluorophenyl)boron (IV) anion and PF₆⁻ are more preferable, and the tetrakis(pentafluorophenyl)boron (IV) anion is particularly preferable.

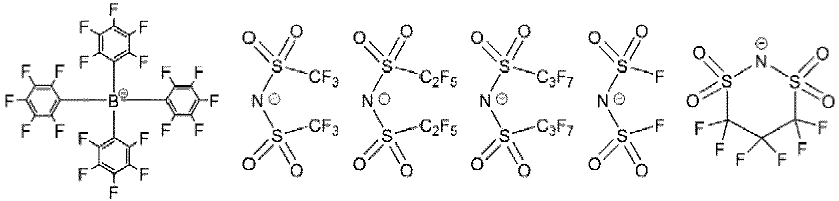

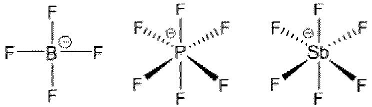

### Dye residue represented by general formula (III)

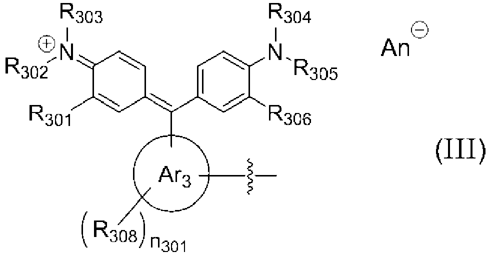

(In the formula, R₃₀₁ to R₃₀₆, R₃₀₈, Ar₃, An⁻, and n₃₀₁ are the same as described above.)

R₃₀₁ and R₃₀₆ in the general formula (III) each independently represent a hydrogen atom or a methyl group, and it is preferable that R₃₀₁ and R₃₀₆ are the same as each other.

Examples of the alkyl group having 1 to 30 carbon atoms represented by R₃₀₂ to R₃₀₅ and R₃₀₈ in the general formula (III) are the same as the examples of the alkyl group having 1 to 30 carbon atoms represented by R₁₀₅ in the general formula (I), and preferred examples are also the same.

Examples of the aryl group having 6 to 14 carbon atoms that is unsubstituted represented by R₃₀₂ to R₃₀₅ in the general formula (III) include a phenyl group, a naphthyl group, an anthracenyl group, and the like. Among these, the phenyl group is preferable.

Examples of the aryl group having 6 to 14 carbon atoms that has a substituent represented by R₃₀₂ to R₃₀₅ in the general formula (III) are the same as the examples of the aryl group having 6 to 14 carbon atoms that has a substituent represented by R₁₀₂ and R₁₀₃ in the general formula (I-1-1), and preferred examples are also the same.

As R₃₀₂ to R₃₀₅ in the general formula (III), a hydrogen atom and an alkyl group having 1 to 30 carbon atoms is preferable, the hydrogen atom and an alkyl group having 1 to 12 carbon atoms are more preferable, and the hydrogen atom and an alkyl group having 1 to 6 carbon atoms are particularly preferable. Specifically, a hydrogen atom, a methyl group, an ethyl group, an n-propyl group, an isopropyl group, an n-butyl group, an isobutyl group, a sec-butyl group, a tert-butyl group, an n-pentyl group, and an n-hexyl group are preferable, the hydrogen atom, the methyl group, the ethyl group, the n-propyl group, the isopropyl group, the n-butyl group, the isobutyl group, the sec-butyl group, and the tert-butyl group are more preferable, and the hydrogen atom, the methyl group, and the ethyl group are particularly preferable.

Examples of the alkoxy group having 1 to 6 carbon atoms represented by R₃₀₈ in the general formula (III) include a methoxy group, an ethoxy group, an n-propoxy group, an isopropoxy group, an n-butoxy group, an isobutoxy group, a sec-butoxy group, a tert-butoxy group, an n-pentyloxy group, an n-hexyloxy group, and the like. Among these, the methoxy group, the ethoxy group, the n-propoxy group, the isopropoxy group, the n-butoxy group, the isobutoxy group, the sec-butoxy group, and the tert-butoxy group are preferable, and the methoxy group, the ethoxy group, the n-propoxy group, and the n-butoxy group are more preferable.

Examples of the halogeno group represented by R₃₀₈ in the general formula (III) include a fluoro group, a chloro group, a bromo group, and an iodo group.

As R₃₀₈ in the general formula (III), an alkoxy group having 1 to 6 carbon atoms, a halogeno group, a hydroxy group, a nitro group, and a sulfo group are preferable. Among these, an alkoxy group having 1 to 4 carbon atoms, the halogeno group, the hydroxy group, the nitro group, and the sulfo group are more preferable. Specifically, for example, a methoxy group, an ethoxy group, an n-propoxy group, an isopropoxy group, an n-butoxy group, an isobutoxy group, a sec-butoxy group, a tert-butoxy group, a fluoro group, a chloro group, a bromo group, an iodo group, a hydroxy group, a nitro group, and a sulfo group are preferable, and the methoxy group, the ethoxy group, the fluoro group, the hydroxy group, the nitro group, and the sulfo group are more preferable.

In a case where Ar₃ in the general formula (III) is a benzene ring, the general formula (III) is represented by the following general formula (III-1-1), and in a case where Ar₃ is a naphthalene ring, the general formula (III) is represented by the following general formula (III-1-2).

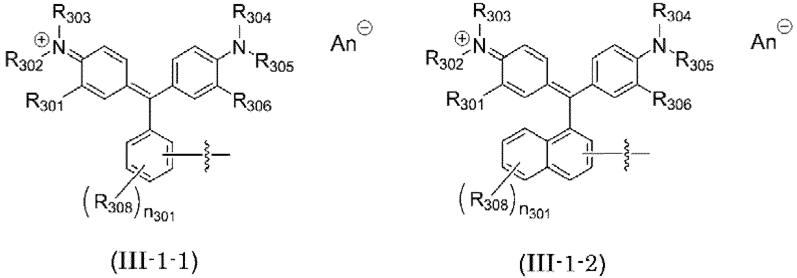

(In the formulae, R₃₀₁ to R₃₀₆, R₃₀₈, An⁻, and n₃₀₁ are the same as described above.)

As n₃₀₁ in the general formula (III), 0 or 1 is preferable, and 0 is more preferable.

In a case where Dye in the general formula (1) is the dye residue represented by the general formula (III), and Ar₃ in the general formula (III) is a benzene ring, the portion to which Y₀ is bonded in the dye residue is located in any of the ortho-position, the meta-position, and the para-position. In a case where n in the general formula (1) is 1, the para-position is preferable. In a case where n in the general formula (1) is 2, a combination of the ortho-position and the para-position and a combination of the meta-position and the para-position are preferable. In addition, in a case where Ar₃ in the general formula (III) is a naphthalene ring, the portion to which Y₀ is bonded is located in any of 2- to 8-positions. In a case where n in the general formula (1) is 1, 4-position is preferable. In a case where n in the general formula (1) is 2, a combination of 2-position and 4-position, a combination of 3-position and 4-position, and a combination of 4-position and 7-position are preferable. Specifically, in a case where Ar₃ in the general formula (III) is a benzene ring, and n in the general formula (1) is 1, Y₀ is preferably bonded as shown in a compound represented by the following general formula (III'-1-1). In a case where n in the general formula (1) is 2, Y₀ is preferably bonded as shown in a compound represented by the following general formula (III'-1-2) or (III'-1-3). In addition, in a case where Ar₃ in the general formula (III) is a naphthalene ring, and n in the general formula (1) is 1, Y₀ is preferably bonded as shown in a compound represented by the following general formula (III'-2-1). In a case where n in the general formula (1) is 2, Y₀ is preferably bonded as shown in compounds represented by the following general formulae (III'-2-2) to (III'-2-4).

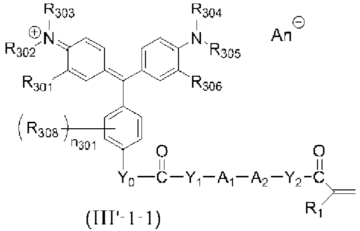

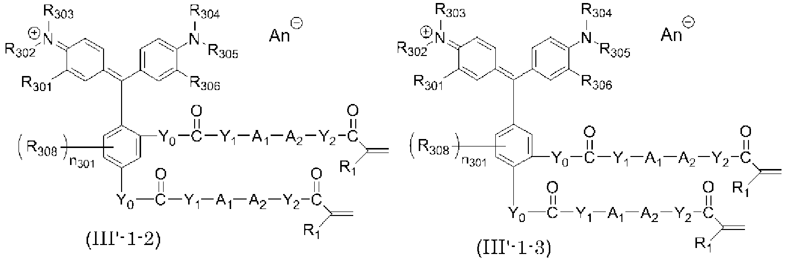

(In the formulae, R₁, R₃₀₁ to R₃₀₆, R₃₀₈, Y₀, Y₁, Y₂, A_{1,} A₂, An⁻, and n₃₀₁ are the same as described above. Here, a plurality of R₁'s, a plurality of Y₀'s, a plurality of Y₁'s, a plurality of Y₂'s, a plurality of A₁'s, and a plurality of A₂'s are the same as or different from each other respectively.)

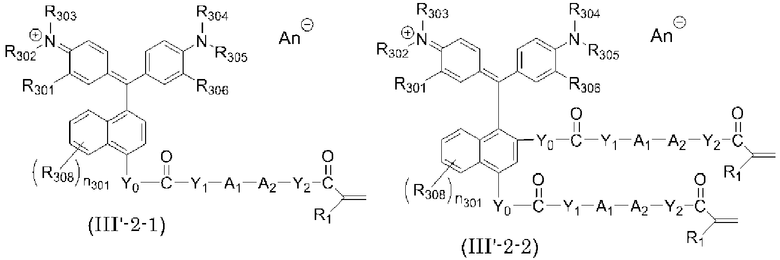

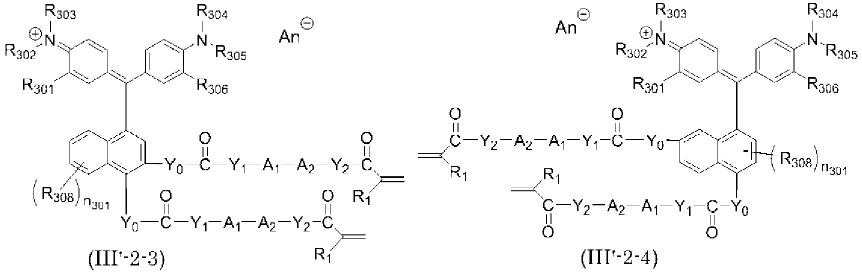

(In the formulae, R₁, R₃₀₁ to R₃₀₆, R₃₀₈, Y₀, Y₁, Y₂, A_{1,} A₂, An⁻, and n₃₀₁ are the same as described above. Here, a plurality of R₁'s, a plurality of Y₀'s, a plurality of Y₁'s, a plurality of Y₂'s, a plurality of A₁'s, and a plurality of A₂'s are the same as or different from each other respectively.)

Examples of the preferred combination of R₃₀₁ to R₃₀₆, R₃₀₈, Ar₃, and n₃₀₁ in the general formula (III) include combinations described in the following table.

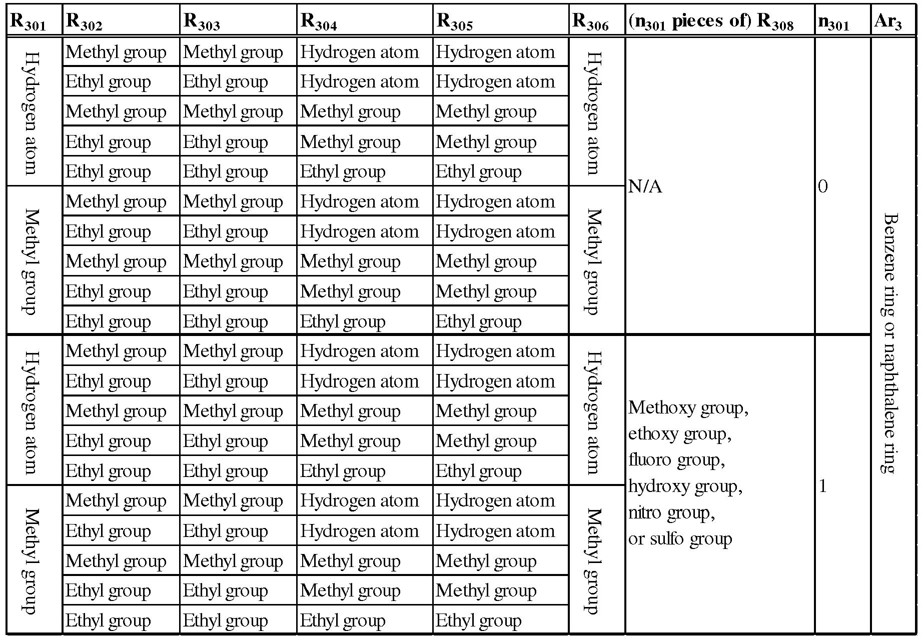

Examples of An⁻ used by being combined with the combinations described in the table include the following anions. Among these, a tetrakis(pentafluorophenyl)boron (IV) anion, a bis(trifluoromethanesulfonyl)imide anion, and PF₆⁻ are preferable, the tetrakis(pentafluorophenyl)boron (IV) anion and PF₆⁻ are more preferable, and the tetrakis(pentafluorophenyl)boron (IV) anion is particularly preferable.

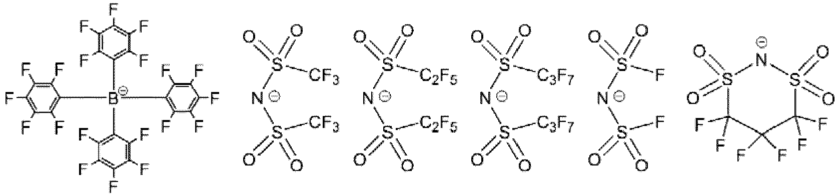

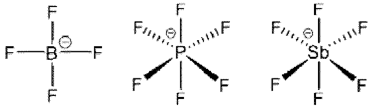

### Dye residue represented by general formula (IV)

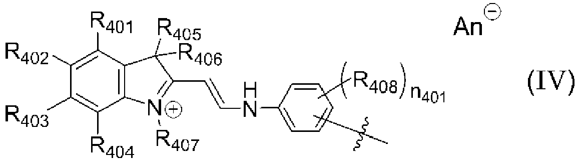

### (In the formula, R₄₀₁ to R₄₀₈, An⁻, and n₄₀₁ are the same as described above.)

Examples of the alkyl group having 1 to 6 carbon atoms represented by R₄₀₁ to R₄₀₆ and R₄₀₈ in the general formula (IV) are the same as the examples of the alkyl group having 1 to 6 carbon atoms represented by R₃ in the general formula (2), and preferred examples are also the same.

Examples of the alkoxy group having 1 to 6 carbon atoms represented by R₄₀₁ to R₄₀₄ and R₄₀₈ in the general formula (IV) are the same as the examples of the alkoxy group having 1 to 6 carbon atoms represented by R₃₀₈ in the general formula (III), and preferred examples are also the same.

Examples of the alkyloxycarbonyl group having 2 to 4 carbon atoms represented by R₄₀₁ to R₄₀₄ and R₄₀₈ in the general formula (IV) include a methoxycarbonyl group, an ethoxycarbonyl group, an n-propoxycarbonyl group, an isopropoxycarbonyl group, and the like.

Examples of the alkylcarbonyloxy group having 2 to 4 carbon atoms represented by R₄₀₁ to R₄₀₄ and R₄₀₈ in the general formula (IV) include a methylcarbonyloxy group, an ethylcarbonyloxy group, an n-propylcarbonyloxy group, an isopropylcarbonyloxy group, and the like.

Examples of the halogeno group represented by R₄₀₁ to R₄₀₄ and R₄₀₈ in the general formula (IV) include a fluoro group, a chloro group, a bromo group, and an iodo group.

As R₄₀₁ to R₄₀₄ in the general formula (IV), a hydrogen atom and an alkyl group having 1 to 6 carbon atoms are preferable, and the hydrogen atom is more preferable.

As R₄₀₈ in the general formula (IV), an alkyl group having 1 to 6 carbon atoms and an alkoxy group having 1 to 6 carbon atoms are preferable, the alkoxy group having 1 to 6 carbon atoms is more preferable, and an alkoxy group having 1 to 4 carbon atoms is particularly preferable. Specifically, for example, a methoxy group, an ethoxy group, an n-propoxy group, an isopropoxy group, an n-butoxy group, an isobutoxy group, a sec-butoxy group, and a tert-butoxy group are preferable, and the methoxy group and the ethoxy group are more preferable.

In a case where R₄₀₈ and R₄₀₆ in the general formula (IV) represent "a phenylalkyl group having 7 to 9 carbon atoms that has an alkyl group having 1 to 6 carbon atoms, a nitro group, a halogeno group, or a cyano group, or is unsubstituted", examples of the phenylalkyl group having 7 to 9 carbon atoms include a benzyl group, a phenethyl group, a 3-phenylpropyl group, and the like.

The phenylalkyl group having 7 to 9 carbon atoms that has an alkyl group having 1 to 6 carbon atoms, a nitro group, a halogeno group, or a cyano group represented by R₄₀₈ and R₄₀₆ in the general formula (IV) has any one or more kinds of 1 to 5 substituents on a benzene ring of the phenylalkyl group. The phenylalkyl group preferably has any one kind of 1 to 3 substituents, and more preferably has any one kind of one substituent. In addition, the substituent is located in any of the ortho-position, the meta-position, and the para-position, and preferably located in the para-position.

In a case where R₄₀₈ and R₄₀₆ in the general formula (IV) represent "a phenylalkyl group having 7 to 9 carbon atoms that has an alkyl group having 1 to 6 carbon atoms, a nitro group, a halogeno group, or a cyano group, or is unsubstituted", examples of the alkyl group having 1 to 6 carbon atoms are the same as the examples of the alkyl group having 1 to 6 carbon atoms represented by R₃ in the general formula (2), and preferred examples are also the same.

In a case where R₄₀₅ and R₄₀₆ in the general formula (IV) represent "a phenylalkyl group having 7 to 9 carbon atoms that has an alkyl group having 1 to 6 carbon atoms, a nitro group, a halogeno group, or a cyano group, or is unsubstituted", examples of the halogeno group include a fluoro group, a chloro group, a bromo group, and an iodo group.

Specific examples of the phenylalkyl group having 7 to 9 carbon atoms that has an alkyl group having 1 to 6 carbon atoms, a nitro group, a halogeno group, or a cyano group represented by R₄₀₅ and R₄₀₆ in the general formula (IV) include a methylbenzyl group, a methyphenethyl group, a 3-(methylphenyl)propyl group, an ethylbenzyl group, an ethylphenethyl group, a 3-(ethylphenyl)propyl group, an n-propylbenzyl group, an n-propylphenethyl group, a 3-(n-propylphenyl)propyl group, an isopropylbenzyl group, an isopropylphenethyl group, a 3-(isopropylphenyl)propyl group, an n-butylbenzyl group, an n-butylphenethyl group, a 3-(n-butylphenyl)propyl group, an isobutylbenzyl group, an isobutylphenethyl group, a 3-(isobutylphenyl)propyl group, a sec-butylbenzyl group, a sec-butylphenethyl group, a 3-(sec-butylphenyl)propyl group, a tert-butylbenzyl group, a tert-butylphenethyl group, a 3-(tert-butylphenyl)propyl group, an n-pentylbenzyl group, an n-pentylphenethyl group, a 3-(n-pentylphenyl)propyl group, an n-hexylbenzyl group, an n-hexylphenethyl group, a 3-(n-hexylphenyl)propyl group, a fluorobenzyl group, a fluorophenethyl group, a 3-(fluorophenyl)propyl group, a chlorobenzyl group, a chlorophenethyl group, a 3-(chlorophenyl)propyl group, a bromobenzyl group, a bromophenethyl group, a 3-(bromophenyl)propyl group, an iodobenzyl group, an iodophenethyl group, a 3-(iodophenyl)propyl group, a nitrobenzyl group, a nitrophenethyl group, a 3-(nitrophenyl)propyl group, a cyanobenzyl group, a cyanophenethyl group, a 3-(cyanophenyl)propyl group, and the like. It should be noted that the above specific examples include all of the ortho-isomer, the meta-isomer, and the para-isomer.

As the phenylalkyl group having 7 to 9 carbon atoms that has an alkyl group having 1 to 6 carbon atoms, a nitro group, a halogeno group, or a cyano group, or is unsubstituted, represented by R₄₀₅ and R₄₀₆ in the general formula (IV), an unsubstituted phenylalkyl group having 7 to 9 carbon atoms is preferable.

Examples of the naphthylalkyl group having 11 to 13 carbon atoms represented by R₄₀₅ and R₄₀₆ in the general formula (IV) include a 1-naphthylmethyl group, a 2-naphthylmethyl group, a 2-(1-naphthyl)ethyl group, a 2-(2-naphthyl)ethyl group, a 3-(1-naphthyl)propyl group, a 3-(2-naphthyl)ethyl group, and the like.

As R₄₀₅ and R₄₀₆ in the general formula (IV), a hydrogen atom and an alkyl group having 1 to 6 carbon atoms are preferable, the alkyl group having 1 to 6 carbon atoms is more preferable, and an alkyl group having 1 to 4 carbon atoms is particularly preferable. Specifically, for example, a methyl group, an ethyl group, an n-propyl group, an isopropyl group, an n-butyl group, an isobutyl group, a sec-butyl group, and a tert-butyl group are preferable, and the methyl group and the ethyl group are more preferable.

In a case where R₄₀₇ in the general formula (IV) represents "an alkyl group having 1 to 6 carbon atoms that has an alkoxy group having 1 to 6 carbon atoms, an alkyloxycarbonyl group having 2 to 4 carbon atoms, a hydroxy group, a carboxy group, a halogeno group, a cyano group, or an amino group, or is unsubstituted", examples of the alkyl group having 1 to 6 carbon atoms are the same as the examples of the alkyl group having 1 to 6 carbon atoms represented by R₃ in the general formula (2), and preferred examples are also the same.

The alkyl group having 1 to 6 carbon atoms that has an alkoxy group having 1 to 6 carbon atoms, an alkyloxycarbonyl group having 2 to 4 carbon atoms, a hydroxy group, a carboxy group, a halogeno group, a cyano group, or an amino group represented by R₄₀₇ in the general formula (IV) has any one or more kinds of one or more substituents on the alkyl group, and preferably has any one kind of one substituent.

In a case where R₄₀₇ in the general formula (IV) represents "a phenylalkyl group having 7 to 9 carbon atoms that has an alkoxy group having 1 to 6 carbon atoms, a halogeno group, or an amino group, or is unsubstituted", examples of the phenylalkyl group having 7 to 9 carbon atoms include a benzyl group, a phenethyl group, a 3-phenylpropyl group, and the like.

The phenylalkyl group having 7 to 9 carbon atoms that has an alkoxy group having 1 to 6 carbon atoms, a halogeno group, or an amino group represented by R₄₀₇ in the general formula (IV) has any one or more kinds of 1 to 5 substituents on a benzene ring of the phenylalkyl group, preferably has any one kind of 1 to 3 substituents, and more preferably has any one kind of one substituent. The substituent is located in any of the ortho-position, the meta-position, and the para-position, and is preferably located in the para-position.

In a case where R₄₀₇ in the general formula (IV) represents "an alkyl group having 1 to 6 carbon atoms that has an alkoxy group having 1 to 6 carbon atoms, an alkyloxycarbonyl group having 2 to 4 carbon atoms, a hydroxy group, a carboxy group, a halogeno group, a cyano group, or an amino group, or is unsubstituted" or represents "a phenylalkyl group having 7 to 9 carbon atoms that has an alkoxy group having 1 to 6 carbon atoms, a halogeno group, or an amino group, or is unsubstituted", examples of the alkoxy group having 1 to 6 carbon atoms are the same as the examples of the alkoxy group having 1 to 6 carbon atoms represented by R₃₀₈ in the general formula (III), and preferred examples are also the same.

In a case where R₄₀₇ in the general formula (IV) represents "an alkyl group having 1 to 6 carbon atoms that has an alkoxy group having 1 to 6 carbon atoms, an alkyloxycarbonyl group having 2 to 4 carbon atoms, a hydroxy group, a carboxy group, a halogeno group, a cyano group, or an amino group, or is unsubstituted", examples of the alkyloxycarbonyl group having 2 to 4 carbon atoms include a methoxycarbonyl group, an ethoxycarbonyl group, an n-propoxycarbonyl group, an isopropoxycarbonyl group, and the like.

In a case where R₄₀₇ in the general formula (IV) represents "an alkyl group having 1 to 6 carbon atoms that has an alkoxy group having 1 to 6 carbon atoms, an alkyloxycarbonyl group having 2 to 4 carbon atoms, a hydroxy group, a carboxy group, a halogeno group, a cyano group, or an amino group, or is unsubstituted" or represents "a phenylalkyl group having 7 to 9 carbon atoms that has an alkoxy group having 1 to 6 carbon atoms, a halogeno group, or an amino group, or is unsubstituted", examples of the halogeno group include a fluoro group, a chloro group, a bromo group, and an iodo group.

Specific examples of the alkyl group having 1 to 6 carbon atoms that has an alkoxy group having 1 to 6 carbon atoms, an alkyloxycarbonyl group having 2 to 4 carbon atoms, a hydroxy group, a carboxy group, a halogeno group, a cyano group, or an amino group represented by R₄₀₇ in the general formula (IV) include a methoxymethyl group, a methoxyethyl group, a 3-methoxy-n-propyl group, a 4-methoxy-n-butyl group, an ethoxymethyl group, an ethoxyethyl group, a 3-ethoxy-n-propyl group, a 4-ethoxy-n-butyl group, an n-propoxymethyl group, an n-propoxyethyl group, a 3-n-propoxy-n-propyl group, a 4-n-propoxy-n-butyl group, an n-butoxymethyl group, an n-butoxyethyl group, a 3-n-butoxy-n-propyl group, a 4-n-butoxy-n-butyl group, a methoxycarbonylmethyl group, a methoxycarbonylethyl group, a 3-methoxycarbonyl-n-propyl group, a 4-methoxycarbonyl-n-butyl group, an ethoxycarbonylmethyl group, an ethoxycarbonylethyl group, a 3-ethoxycarbonyl-n-propyl group, a 4-ethoxycarbonyl-n-butyl group, an n-propoxycarbonylmethyl group, an n-propoxycarbonylethyl group, a 3-n-propoxycarbonyl-n-npropyl group, a 4-n-propoxycarbonyl-n-butyl group, an isopropoxycarbonylmethyl group, an isopropoxycarbonylethyl group, a 3-isopropoxycarbonyl-n-propyl group, a 4-isopropoxycarbonyl-n-butyl group, a hydroxymethyl group, a hydroxyethyl group, a 3-hydroxy-n-propyl group, a 4-hydroxy-n-butyl group, a carboxymethyl group, a carboxyethyl group, a 3-carboxy-n-propyl group, a 4-carboxy-n-butyl group, a fluoromethyl group, a fluoroethyl group, a 3-fluoro-n-propyl group, a 4-fluoro-n-butyl group, a chloromethyl group, a chloroethyl group, a 3-chloro-n-propyl group, a 4-chloro-n-butyl group, a bromomethyl group, a bromoethyl group, a 3-bromo-n-propyl group, a 4-bromo-n-butyl group, an iodomethyl group, an iodoethyl group, a 3-iodo-n-propyl group, a 4-iodo-n-butyl group, a cyanomethyl group, a cyanoethyl group, a 3-cyano-n-propyl group, a 4-cyano-n-butyl group, an aminomethyl group, an aminoethyl group, a 3-amino-n-propyl group, a 4-amino-n-butyl group, and the like.

As the alkyl group having 1 to 6 carbon atoms that has an alkoxy group having 1 to 6 carbon atoms, an alkyloxycarbonyl group having 2 to 4 carbon atoms, a hydroxy group, a carboxy group, a halogeno group, a cyano group, or an amino group, or is unsubstituted, represented by R₄₀₇ in the general formula (IV), an unsubstituted alkyl group having 1 to 6 carbon atoms is preferable.

Specific examples of the phenylalkyl group having 7 to 9 carbon atoms that has an alkoxy group having 1 to 6 carbon atoms, a halogeno group, or an amino group represented by R₄₀₇ in the general formula (IV) include a methoxybenzyl group, an methoxyphenethyl group, a 3-(methoxyphenyl)propyl group, an ethoxybenzyl group, an ethoxyphenethyl group, a 3-(ethoxyphenyl)propyl group, an n-propoxybenzyl group, an n-propoxyphenethyl group, a 3-(n-propoxyphenyl)propyl group, an isopropoxybenzyl group, an isopropoxyphenethyl group, a 3-(isopropoxyphenyl)propyl group, an n-butoxybenzyl group, an n-butoxyphenethyl group, a 3-(n-butoxyphenyl)propyl group, an isobutoxybenzyl group, an isobutoxyphenethyl group, a 3-(isobutoxyphenyl)propyl group, a sec-butoxybenzyl group, a sec-butoxyphenethyl group, a 3-(sec-butoxyphenyl)propyl group, a tert-butoxybenzyl group, a tert-butoxyphenethyl group, a 3-(tert-butoxyphenyl)propyl group, an n-pentyloxybenzyl group, an n-pentyloxyphenethyl group, a 3-(n-pentyloxyphenyl)propyl group, an n-hexyloxybenzyl group, an n-hexyloxyphenethyl group, a 3-(n-hexyloxyphenyl)propyl group, a fluorobenzyl group, a fluorophenethyl group, a 3-(fluorophenyl)propyl group, a chlorobenzyl group, a chlorophenethyl group, a 3-(chlorophenyl)propyl group, a bromobenzyl group, a bromophenethyl group, a 3-(bromophenyl)propyl group, an iodobenzyl group, an iodophenethyl group, a 3-(iodophenyl)propyl group, an aminobenzyl group, an aminophenethyl group, a 3-(aminophenyl)propyl group, and the like. It should be noted that the above specific examples include all of the ortho-isomer, the meta-isomer, and the para-isomer.

As the phenylalkyl group having 7 to 9 carbon atoms that has an alkoxy group having 1 to 6 carbon atoms, a halogeno group, or an amino group, or is unsubstituted, represented by R₄₀₇ in the general formula (IV), an unsubstituted phenylalkyl group having 7 to 9 carbon atoms is preferable.

Examples of the naphthylalkyl group having 11 to 13 carbon atoms represented by R₄₀₇ in the general formula (IV) are the same as the examples of the naphthylalkyl group having 11 to 13 carbon atoms represented by R₄₀₅ and R₄₀₆ in the general formula (IV), and preferred examples are also the same.

As R₄₀₇ in the general formula (IV), the alkyl group having 1 to 6 carbon atoms that has an alkoxy group having 1 to 6 carbon atoms, an alkyloxycarbonyl group having 2 to 4 carbon atoms, a hydroxy group, a carboxy group, a halogeno group, a cyano group, or an amino group, or is unsubstituted is preferable, an unsubstituted alkyl group having 1 to 6 carbon atoms is more preferable, and an alkyl group having 1 to 4 carbon atoms is particularly preferable. Specifically, for example, a methyl group, an ethyl group, an n-propyl group, an isopropyl group, an n-butyl group, an isobutyl group, a sec-butyl group, and a tert-butyl group are preferable, and the methyl group and the ethyl group are more preferable.

As n₄₀₁ in the general formula (IV), 0 to 2 are preferable, and 0 is more preferable.

In a case where Dye in the general formula (1) is the dye residue represented by the general formula (IV), the portion to which Y₀ is bonded in the dye residue is located in any of the ortho-position, the meta-position, and the para-position of a phenyl group in the general formula (IV). In a case where n in the general formula (1) is 1, the para-position is preferable. In a case where n in the general formula (1) is 2, a combination of the ortho-position and the para-position, a combination of the meta-position and the para-position, and a combination of the two meta-positions are preferable. Specifically, in a case where n in the general formula (1) is 1, it is preferable that Y₀ is bonded as shown in a compound represented by the following general formula (IV'-1), and in a case where n in the general formula (1) is 2, it is preferable that Y₀ is bonded as shown in compounds represented by the following general formulae (IV'-2) to (IV'-4).

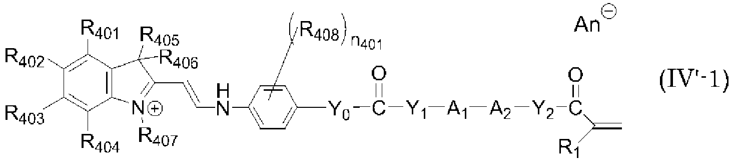

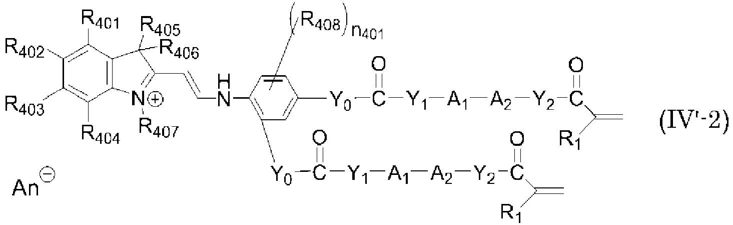

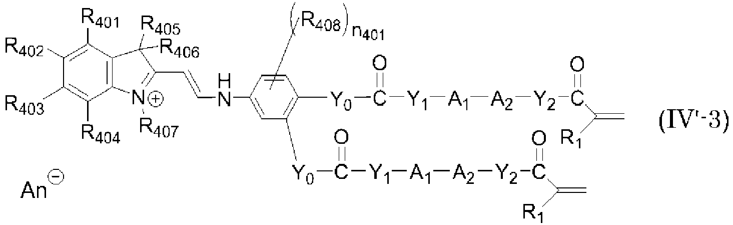

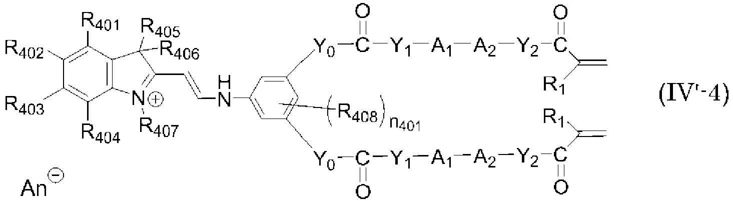

(In the formulae, R₁, R₄₀₁ to R₄₀₈, Y₀, Y₁, Y₂, A_{1,} A₂, An⁻, and n₄₀₁ are the same as described above. Here, a plurality of R₁'s, a plurality of Y₀'s, a plurality of Y₁'s, a plurality of Y₂'s, a plurality of A₁'s, and a plurality of A₂'s are the same as or different from each other respectively.)

Specific examples preferred as the general formula (IV) include a dye residue represented by the following general formula (V).

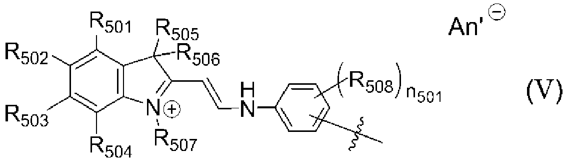

(In the formula, An⁻ is the same as described above, R₅₀₁ to R₅₀₆ each independently represent a hydrogen atom or an alkyl group having 1 to 6 carbon atoms, R₅₀₇ represents an alkyl group having 1 to 6 carbon atoms that has an alkoxy group having 1 to 6 carbon atoms, an alkyloxycarbonyl group having 2 to 4 carbon atoms, a hydroxy group, a carboxy group, a halogeno group, a cyano group, or an amino group, or is unsubstituted, n₅₀₁ pieces of R₅₀₈ each independently represent an alkyl group having 1 to 6 carbon atoms or an alkoxy group having 1 to 6 carbon atoms, in a case where n in the general formula (1) is 1, n₅₀₁ represents an integer of 0 to 2, and in a case where n in the general formula (1) is 2, n₅₀₁ represents an integer of 0 to 3.)

Examples of the alkyl group having 1 to 6 carbon atoms represented by R₅₀₁ to R₅₀₆ and R₅₀₈ in the general formula (V); examples of the alkyl group having 1 to 6 carbon atoms that has an alkoxy group having 1 to 6 carbon atoms, an alkyloxycarbonyl group having 2 to 4 carbon atoms, a hydroxy group, a carboxy group, a halogeno group, a cyano group, or an amino group, or is unsubstituted, represented by R₅₀₇; and the alkoxy group having 1 to 6 carbon atoms represented by R₅₀₈ are the same as the examples of those represented by R₄₀₁ to R₄₀₈ in the general formula (IV), and preferred examples are also the same.

As R₅₀₁ to R₅₀₄ in the general formula (V), a hydrogen atom is preferable.

As R₅₀₅ and R₅₀₆ in the general formula (V), an alkyl group having 1 to 6 carbon atoms is preferable, and an alkyl group having 1 to 4 carbon atoms is more preferable. Specifically, for example, a methyl group, an ethyl group, an n-propyl group, an isopropyl group, an n-butyl group, an isobutyl group, a sec-butyl group, and a tert-butyl group are preferable, and the methyl group and the ethyl group are more preferable.

As R₅₀₇ in the general formula (V), an unsubstituted alkyl group having 1 to 6 carbon atoms is preferable, and an alkyl group having 1 to 4 carbon atoms is more preferable. Specifically, for example, a methyl group, an ethyl group, an n-propyl group, an isopropyl group, an n-butyl group, an isobutyl group, a sec-butyl group, and a tert-butyl group are preferable, and the methyl group and the ethyl group are more preferable.

As R₅₀₈ in the general formula (V), an alkoxy group having 1 to 6 carbon atoms is preferable, and an alkoxy group having 1 to 4 carbon atoms is more preferable. Specifically, for example, a methoxy group, an ethoxy group, an n-propoxy group, an isopropoxy group, an n-butoxy group, an isobutoxy group, a sec-butoxy group, and a tert-butoxy group are preferable, and the methoxy group and the ethoxy group are more preferable.

As n₅₀₁ in the general formula (V), 0 is preferable.

Examples of the preferred combination of R₅₀₁ to R₅₀₈ and n₅₀₁ in the general formula (V) include combinations described in the following table.

| **R₅₀₁ to R₅₀₄** | **R₅₀₅** | **R₅₀₆** | **R₅₀₇** | **(n₅₀₁ pieces of) R₅₀₈** | **n₅₀₁** |
|---|---|---|---|---|---|
| Hydrogen atom | Methyl group | Methyl group | Methyl group | N/A | 0 |
| | Methyl group | Methyl group | Methyl group | Methoxy group or ethoxy group | 1 |
| | Methyl group | Methyl group | Methyl group | Methoxy group or ethoxy group | 2 |
| | Methyl group | Methyl group | Ethyl group | N/A | 0 |
| | Methyl group | Methyl group | Ethyl group | Methoxy group or ethoxy group | 1 |
| | Methyl group | Methyl group | Ethyl group | Methoxy group or ethoxy group | 2 |
| | Ethyl group | Ethyl group | Methyl group | N/A | 0 |
| | Ethyl group | Ethyl group | Methyl group | Methoxy group or ethoxy group | 1 |
| | Ethyl group | Ethyl group | Methyl group | Methoxy group or ethoxy group | 2 |
| | Ethyl group | Ethyl group | Ethyl group | N/A | 0 |
| | Ethyl group | Ethyl group | Ethyl group | Methoxy group or ethoxy group | 1 |
| | Ethyl group | Ethyl group | Ethyl group | Methoxy group or ethoxy group | 2 |

Examples of An⁻ used by being combined with the combinations described in the table include the following anions. Among these, a tetrakis(pentafluorophenyl)boron (IV) anion, a bis(trifluoromethanesulfonyl)imide anion, and PF₆⁻ are preferable, the tetrakis(pentafluorophenyl)boron (IV) anion and PF₆⁻ are more preferable, and the tetrakis(pentafluorophenyl)boron (IV) anion is particularly preferable.

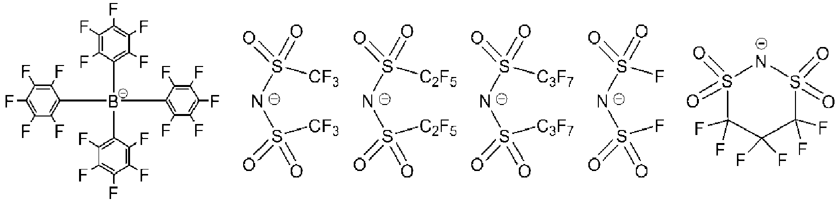

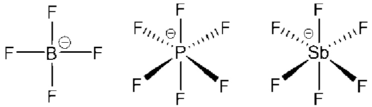

As the dye residue represented by Dye in the general formula (1), the dye residue represented by the general formula (I), (III), or (IV) is preferable, the dye residue represented by the general formula (I) or (IV) is more preferable, and the dye residue represented by the general formula (II) or (V) is even more preferable.

Specific examples preferred as the general formula (1) include the following general formula (1-10).

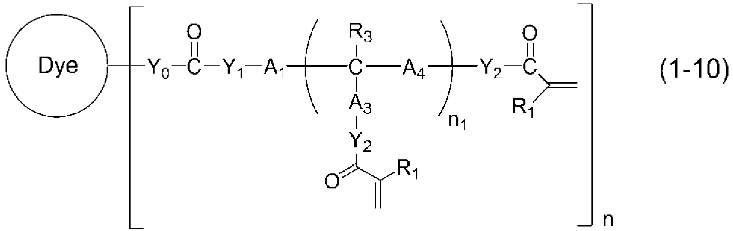

(In the formula, Dye, R₁, R₃, A₁, A₃, A₄, Y₀, Y₁, Y₂, n, and n₁ are the same as described above. Here, a plurality of R₁'s, a plurality of R₃'s, a plurality of A₁'s, a plurality of A₃'s, a plurality of A₄'s, a plurality of Y₀'s, a plurality of Y₁'s, a plurality of Y₂'s, and a plurality of n₁'s are the same as or different from each other respectively, and the total number of carbon atoms in n₁ pieces of A₄ is 1 to 6. In addition, in a case where n₁ is 1, R₃ represents the group represented by the general formula (2-1).)

Specific examples preferred as the general formula (1-10) include the following general formulae (1-11-1) and (1-11-2).

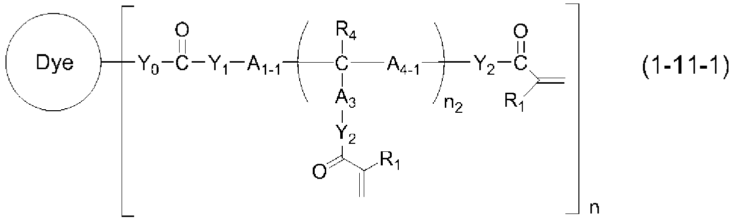

(In the formula, Dye R₁, R₄, A₃, A₄₋₁, Y₀, Y₁, Y₂, n, and n₂ are the same as described above, and A₁₋₁ represents an alkylene group having 1 to 6 carbon atoms or a single bond. Here, a plurality of R₁'s, a plurality of R₄'s, a plurality of A₁₋₁'s, a plurality of A₃'s, a plurality of A₄₋₁'s, a plurality of Y₀'s, a plurality of Y₁'s, a plurality of Y₂'s, and a plurality of n₂'s are the same as or different from each other respectively, and the total number of carbon atoms in n₂ pieces of A₄₋₁ is 1 to 6.)

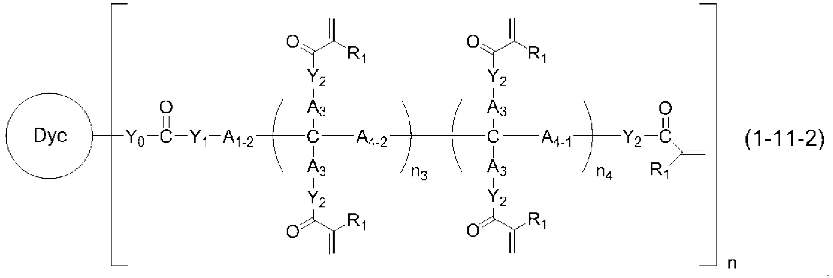

(In the formula, Dye, R₁, A₃, A₄₋₁, A₄₋₂, Y₀, Y₁, Y₂, n, n₃, and n₄ are the same as described above, and A₁₋₂ represents an alkylene group having 1 to 6 carbon atoms, the group represented by any one of the general formulae (1-1) to (1-3), or a single bond. Here, a plurality of R₁'s, a plurality of A₁₋₂'s, a plurality of A₃'s, a plurality of A₄₋₁'s, a plurality of A₄₋₂'s, a plurality of Y₀'s, a plurality of Y₁'s, a plurality of Y₂'s, a plurality of n₃'s, and a plurality of n₄'s are the same as or different from each other respectively, and the total number of carbon atoms in n₄ pieces of A₄₋₁ and n₃ pieces of A₄₋₂ is 1 to 6.)

Examples of the alkylene group having 1 to 6 carbon atoms represented by A₁₋₁ in the general formula (1-11-1) and A₁₋₂ in the general formula (1-11-2) are the same as the examples of the alkylene group having 1 to 6 carbon atoms represented by A₁ in the general formula (1), and preferred examples are also the same.

The group represented by any one of the general formulae (1-1) to (1-3) in A₁₋₂ in the general formula (1-11-2) is the same as the group represented by any one of the general formulae (1-1) to (1-3) in A₁ in the general formula (1).

As A₁₋₁ in the general formula (1-11-1), a linear alkylene group having 1 to 6 carbon atoms and a single bond are preferable, and a linear alkylene group having 1 to 3 carbon atoms and the single bond are more preferable. Specifically, a methylene group, an ethylene group, a trimethylene group, and a single bond are preferable, and the methylene group and the single bond are more preferable.

As A₁₋₂ in the general formula (1-11-2), a linear alkylene group having 1 to 6 carbon atoms, the groups represented by any of the general formulae (1-5) to (1-8), and a single bond are preferable, and a linear alkylene group having 1 to 3 carbon atoms, the group represented by the general formula (1-7) or (1-8), and the single bond are more preferable. Specific examples thereof are the same as the specific examples of A₁ in the general formula (1), and preferred examples are also the same.

Specific examples preferred as the general formula (1-11-1) include the following general formula (1-12-1), and specific examples preferred as the general formula (1-11-2) include the following general formula (1-12-2).

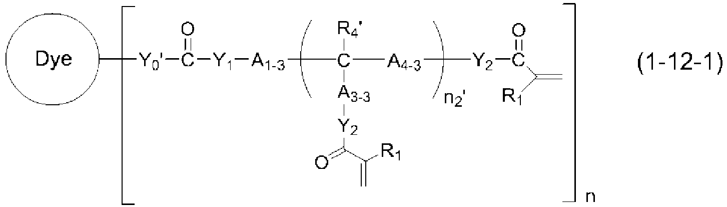

(In the formula, Dye R₁, R₄', A₃₋₃, A₄₋₃, Y₁, Y₂, n, and n₂' are the same as described above, Y₀' represents a methylene group, -NH-, or a single bond, and A₁₋₃ represents a linear alkylene group having 1 to 6 carbon atoms or a single bond. Here, a plurality of R₁'s, a plurality of R₄"s, a plurality of A₁₋₃'s, a plurality of A₃₋₃'s, a plurality of A₄₋₃'s, a plurality of Y₀"s, a plurality of Y₁'s, a plurality of Y₂'s, and a plurality of n₂"s are the same as or different from each other respectively, and the total number of carbon atoms in n₂' pieces of A₄₋₃ is 1 to 6.)

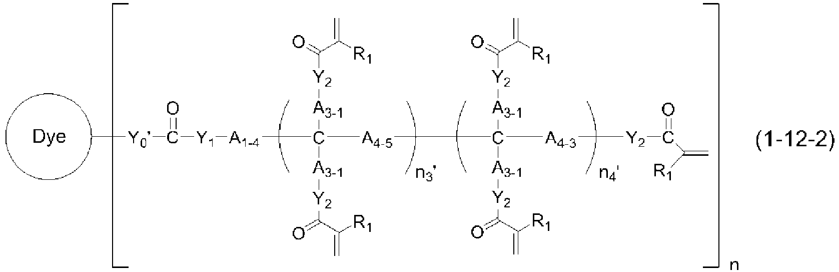

(In the formula, Dye, R₁, A₃₋₁, A₄₋₃, A₄₋₅, Y₀', Y₁, Y₂, n, n₃', and n₄' are the same as described above, and A₁₋₄ represents a linear alkylene group having 1 to 6 carbon atoms, the group represented by any one of the general formulae (1-5) to (1-8), or a single bond. Here, a plurality of R₁'s, a plurality of A₁₋₄'s, a plurality of A₃₋₁'s, a plurality of A₄₋₃'s, a plurality of A₄₋₅'s, a plurality of Y₀"s, a plurality of Y₁'s, a plurality of Y₂'s, a plurality of n₃"s, and a plurality of n₄"s are the same as or different from each other respectively, and the total number of carbon atoms in n₄' pieces of A₄₋₃ and n₃' pieces of A₄₋₅ is 1 to 6.)

Those preferred as Y₀' in the general formulae (1-12-1) and (1-12-2) vary with the structure of Dye. For example, in a case where Dye is a residue derived from a xanthene-based dye, a single bond is preferable. In a case where Dye is a residue derived from a triarylmethane-based dye, -NH- and a single bond are preferable. In a case where Dye is a residue derived from a cyanine-based dye, a methylene group and a single bond are preferable. More specifically, in a case where Dye is the dye residue represented by the general formula (I), a single bond is preferable. In a case where Dye is the dye residue represented by the general formula (III-1-1), a single bond is preferable. In a case where Dye is the dye residue represented by the general formula (111-1-2), -NH- is preferable. In a case where Dye is the dye residue represented by the general formula (IV), a methylene group and a single bond are preferable.

Examples of the linear alkylene group having 1 to 6 carbon atoms represented by A₁₋₃ in the general formula (1-12-1) and A₁₋₄ in the general formula (1-12-2) are the same as the examples of the linear alkylene group having 1 to 6 carbon atoms represented by A₃₋₁ in the general formula (2-2), and preferred examples are also the same.

The group represented by any one of the general formulae (1-5) to (1-8) in A₁₋₄ in the general formula (1-12-2) is the same as the group represented by any one of the general formulae (1-5) to (1-8) in A₁ in the general formula (1).

As A₁₋₃ in the general formula (1-12-1), a linear alkylene group having 1 to 3 carbon atoms and a single bond are preferable. Specific examples thereof include a methylene group, an ethylene group, a trimethylene group, and a single bond. Among these, the methylene group and the single bond are preferable.

As A₁₋₄ in the general formula (1-12-2), a linear alkylene group having 1 to 3 carbon atoms, the group represented by the general formula (1-7) or (1-8), and a single bond are preferable. Specific examples thereof are the same as the specific examples of A₁ in the general formula (1), and preferred examples are also the same.

Specific examples preferred as the general formula (1-12-1) include the following general formula (1-13-1), and specific examples preferred as the general formula (1-12-2) include the following general formula (1-13-2).

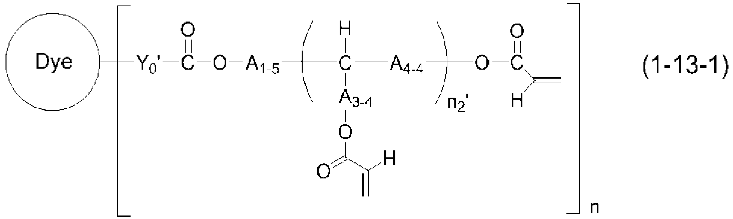

(In the formula, Dye, A₃₋₄, A₄₋₄, Y₀', n, and n₂' are the same as described above, and A₁₋₅ represents a linear alkylene group having 1 to 3 carbon atoms or a single bond. Here, a plurality of A₁₋₅'s and a plurality of n₂"s are the same as each other respectively, a plurality of A₃₋₄'s, a plurality of A₄₋₄'s, and a plurality of Y₀"s are the same as or different from each other respectively, and the total number of carbon atoms in n₂' pieces of A₄₋₄ is 1 to 6.)

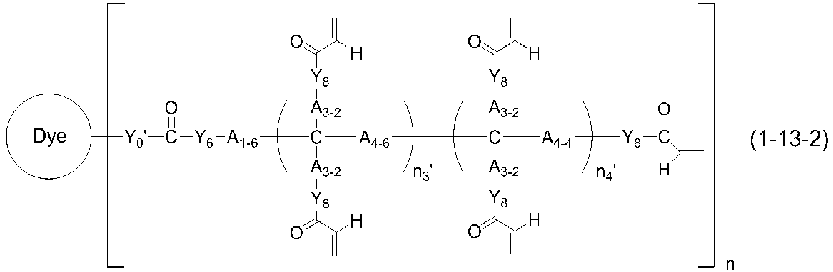

(In the formula, Dye, A₃₋₂, A₄₋₄, A₄₋₆, Y₀', Y₈, n, n₃', and n₄' are the same as described above, and A₁₋₆ represents a linear alkylene group having 1 to 3 carbon atoms, the group represented by the general formula (1-7) or (1-8), or a single bond. Here, a plurality of A₁₋₆'s, a plurality of A₃₋₂'s, a plurality of A₄₋₄'s, a plurality of A₄₋₆'s, a plurality of Y₈'s, a plurality of n₃"s, and a plurality of n₄"s are the same as each other respectively, a plurality of Y₀"s are the same as or different from each other, and the total number of carbon atoms in n₄' pieces of A₄₋₄ and n₃' pieces of A₄₋₆ is 1 to 6.)

Examples of the linear alkylene group having 1 to 3 carbon atoms represented by A₁₋₅ in the general formula (1-13-1) and A₁₋₆ in the general formula (1-13-2) include a methylene group, an ethylene group, and a trimethylene group. Among these, the methylene group is preferable.

The group represented by the general formula (1-7) or (1-8) in A₁₋₆ in the general formula (1-13-2) is the same as the group represented by the general formula (1-7) or (1-8) in A₁ in the general formula (1).

As Aₗ-_{S} in the general formula (1-13-1), a methylene group and a single bond are preferable.

As A₁₋₆ in the general formula (1-13-2), a methylene group, -(CH₂)₂-NHCO-(CH₂)₂-COO-CH₂-, the group represented by the formula (122), and a single bond are preferable, and the methylene group is more preferable.

Examples of the preferred combination of A₁₋₅, A₃₋₄, A₄₋₄, n, and n₂' in the general formula (1-13-1) include combinations 1 to 24 described in the following table. Among these, the combinations 1 to 4, 7 to 10, and 15 to 18 are preferable, the combinations 1, 4, 7, 10, 15, and 18 are more preferable, the combinations 15 and 18 are even more preferable, and the combination 18 is particularly preferable.

| **Combination** | **A₁₋₅** | **A₃₋₄** | **A₄₋₄** | **n₂'** | **n** |
|---|---|---|---|---|---|
| **1** | Methylene group | Two single bonds | One single bond and one methylene group | 2 | 1 |
| **2** | Single bond | Two single bonds | One single bond and one methylene group | 2 | 1 |
| **3** | Methylene group | One single bond and one methylene group | One single bond and one methylene group | 2 | 1 |
| **4** | Single bond | One single bond and one methylene group | One single bond and one methylene group | 2 | 1 |
| **5** | Methylene group | Two methylene groups | One single bond and one methylene group | 2 | 1 |
| **6** | Single bond | Two methylene groups | One single bond and one methylene group | 2 | 1 |
| **7** | Methylene group | Three single bonds | Two single bonds and one methylene group | 3 | 1 |
| **8** | Single bond | Three single bonds | Two single bonds and one methylene group | 3 | 1 |
| **9** | Methylene group | Two single bonds and one methylene group | Two single bonds and one methylene group | 3 | 1 |
| **10** | Single bond | Two single bonds and one methylene group | Two single bonds and one methylene group | 3 | 1 |
| **11** | Methylene group | One single bond and two methylene groups | Two single bonds and one methylene group | 3 | 1 |
| **12** | Single bond | One single bond and two methylene groups | Two single bonds and one methylene group | 3 | 1 |
| **13** | Methylene group | Three methylene groups | Two single bonds and one methylene group | 3 | 1 |
| **14** | Single bond | Three methylene groups | Two single bonds and one methylene group | 3 | 1 |
| **15** | Methylene group | Four single bonds | Three single bonds and one methylene group | 4 | 1 |
| **16** | Single bond | Four single bonds | Three single bonds and one methylene group | 4 | 1 |
| **17** | Methylene group | Three single bonds and one methylene group | Three single bonds and one methylene group | 4 | 1 |
| **18** | Single bond | Three single bonds and one methylene group | Three single bonds and one methylene group | 4 | 1 |
| **19** | Methylene group | Two single bonds and two methylene groups | Three single bonds and one methylene group | 4 | 1 |
| **20** | Single bond | Two single bonds and two methylene groups | Three single bonds and one methylene group | 4 | 1 |
| **21** | Methylene group | One single bond and three methylene groups | Three single bonds and one methylene group | 4 | 1 |
| **22** | Single bond | One single bond and three methylene groups | Three single bonds and one methylene group | 4 | 1 |
| **23** | Methylene group | Four methylene groups | Three single bonds and one methylene group | 4 | 1 |
| **24** | Single bond | Four methylene groups | Three single bonds and one methylene group | 4 | 1 |

Specific examples preferred as a portion of the general formula (1-13-1) except for Dye and Y₀' include the following formulae (161) to (172). Among these, the formulae (162), (163), (166), (167), (170), and (171) are preferable, the formulae (170) and (171) are more preferable, and the formula (171) is particularly preferable.

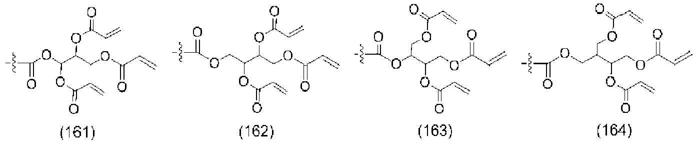

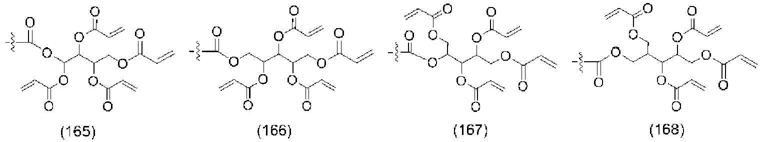

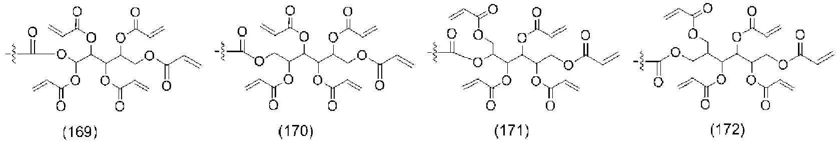

For "the preferred combination of A₁₋₅, A₃₋₄, A₄₋₄, n, and n₂' in the general formula (1-13-1)" and "specific examples preferred as a portion of the general formula (1-13-1) except for Dye and Y₀''', in a case where n in the general formula (1-13-1) is 1, the combinations of Dye and Y₀' combined as shown in the combinations 1 to 5 in the following table are preferable, and among these, the combinations of Dye and Y₀'s combined as show in the combinations 1 and 4 are more preferable. In a case where n in the general formula (1-13-1) is 2, the combinations of Dye and Y₀'s combined as shown in the combinations 6 to 11 in the following table are preferable, and among these, the combinations of Dye and Y₀'s combined as shown in the combinations 6 and 9 to 11 are more preferable. Among the combinations of Dye and Y₀'s, for example, the combinations 12 to 17 described in the following table are preferable, and among these, the combinations 12 to 14 are preferable.

| **Combination** | **Dye** | **(n pieces of) Y₀'** |
|---|---|---|
| **1** | General formula (I) | One single bond |
| **2** | General formula (III-1-1 ) | One single bond |
| **3** | General formula (III-1-2) | One -NH- |
| **4** | General formula (IV) | One methylene group |
| **5** | General formula (IV) | One single bond |
| **6** | General formula (I) | Two single bonds |
| **7** | General formula (III-1-1) | Two single bonds |
| **8** | General formula (III-1-2) | Two -NH- |
| **9** | General formula (IV) | Two methylene groups |
| **10** | General formula (IV) | One methylene group and one single bond |
| **11** | General formula (IV) | Two single bonds |
| **12** | General formula (II) | One single bond |
| **13** | General formula (V) | One methylene group |
| **14** | General formula (II) | Two single bonds |
| **15** | General formula (V) | Two methylene groups |
| **16** | General formula (V) | One methylene group and one single bond |
| **17** | General formula (V) | Two single bonds |

Examples of the preferred combination of A₁₋₆, A₃₋₂, A₄₋₄, A₄₋₆, Y₈, n, n₃', and n₄' in the general formula (1-13-2) include combinations 1 to 15 described in the following table.

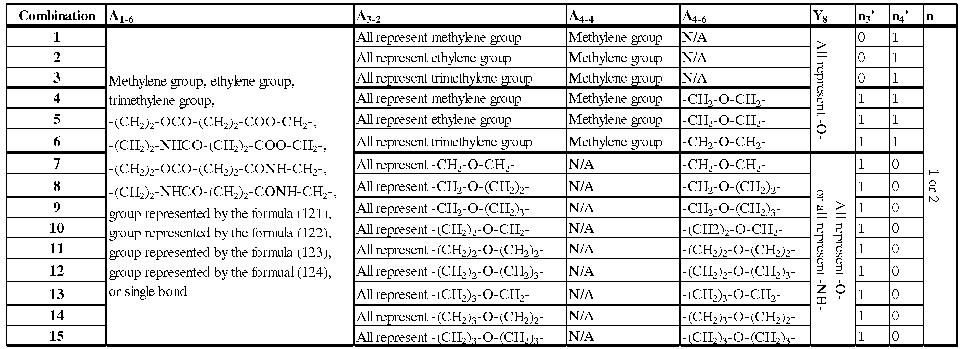

Among the preferred combinations, for example, the combinations 1 to 20 described in the following table are more preferable. Among these, the combinations 1 to 3, 5 to 7, 9 to 11, 16, and 20 are preferable, the combinations 1 to 3, 5, 9 to 11, and 20 are more preferable, the combinations 9 to 11 are even more preferable, and the combination 9 is particularly preferable.

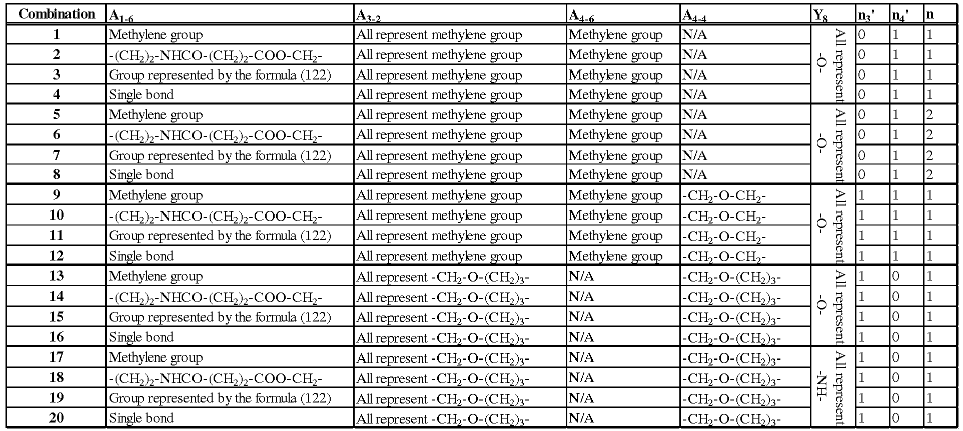

Specific examples preferred as a portion of the general formula (1-13-2) except for Dye and Y₀' include the following formulae (181) to (196). Among these, the formulae (181) to (183), (185) to (187), (192), and (196) are preferable, the formulae (181) to (183), (185) to (187), and (196) are more preferable, the formulae (185) to (187) are even more preferable, and the formulae (171) is particularly preferable.

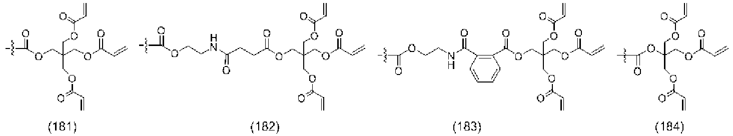

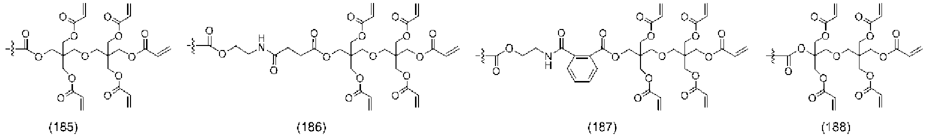

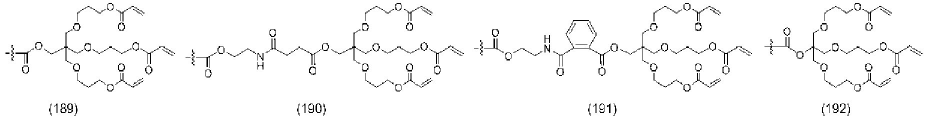

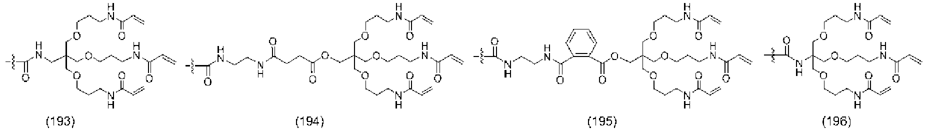

Examples of the combination of Dye and Y₀' combined with "the preferred combination of A₁₋₆, A₃₋₂, A₄₋₄, A₄₋₆, Y₈, n, n₃', and n₄' in the general formula (1-13-2)" or "specific examples preferred as a portion of the general formula (1-13-2) except for Dye and Y₀''' are the same as the examples of the combination of Dye and Y₀' combined with "the preferred combination of A₁₋₅, A₃₋₄, A₄₋₄, n, and n₂' in the general formula (1-13-1)" or "specific examples preferred as a portion of the general formula (1-13-1) except for Dye and Y₀''', and preferred examples are also the same.

### Production Method for the compound of the present invention

The compound of the present invention can be produced by a series of methods shown in the following reactions [I-I] and [I-II]. That is, in brief, a compound represented by the following general formula (21) may be subjected to a salt exchange reaction (reaction [I-I]) and then reacted with a compound represented by the following general formula (22) (reaction [I-II]).

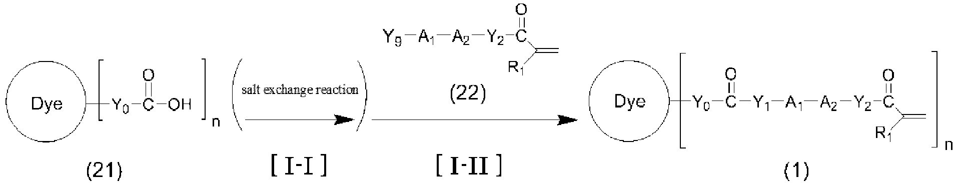

(In the formula, Y₉ represents a hydroxy group or an amino group, R₁, Y₀, Y₁, Y₂, A₁, A₂, n, and Dye are the same as described above. Here, a plurality of R₁'s, a plurality of Y₀'s, a plurality of Y₁'s, a plurality of Y₂'s, a plurality of A₁'s, and a plurality of A₂'s are the same as or different from each other respectively.)

In a case where Y₉ in the general formula (22) is a hydroxy group, Y₁ is -O-. In a case where Y₉ is an amino group, Y₁ is -NH-. As Y₉ in the general formula (22), the hydroxy group is preferable.

Specific examples preferred as the general formula (22) include the following general formula (22-1).

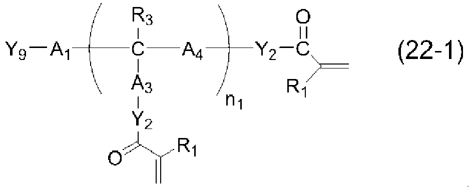

(In the formula, R₁, R₃, A₁, A₃, A₄, Y₂, Y₉, and n₁ are the same as described above. Here, a plurality of R₁'s, a plurality of R₃'s, a plurality of A₃'s, a plurality of A₄'s, and a plurality of Y₂'s are the same as or different from each other respectively, and the total number of carbon atoms in n₁ pieces of A₄ is 1 to 6. In addition, in a case where n₁ is 1, R₃ represents the group represented by the general formula (2-1).)

Specific examples preferred as the general formula (22-1) include the following general formula (22-2-1) or (22-2-2).

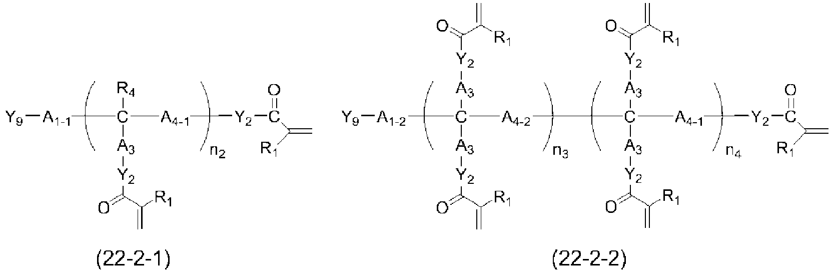

(In the formulae, R₁, R₄, A₁₋₁, A₁₋₂, A₃, A₄₋₁, A₄₋₂, Y₂, Y₉, n, and n₂ to n₄ are the same as described above. Here, a plurality of R₁'s, a plurality of R₄'s, a plurality of A₃'s, a plurality of A₄₋₁'s, a plurality of A₄₋₂'s, and a plurality of Y₂'s are the same as or different from each other respectively, the total number of carbon atoms in n₂ pieces of A₄₋₁ is 1 to 6, and the total number of carbon atoms in n₄ pieces of A₄₋₁ and n₃ pieces of A₄₋₂ is 1 to 6.)

As the compound represented by the general formula (21), a commercial compound or a compound which is appropriately synthesized by a known method may be used. Specific examples of the compound represented by the general formula (21) include compounds in which one or two -COOH groups are bonded to the specific examples of the dye residue represented by Dye. More specific examples thereof include compounds represented by the following general formulae (I-COOH), (III-COOH), and (IV-COOH) in which one or two -COOH groups are bonded to the dye residues represented by the general formulae (I), (III), and (IV).

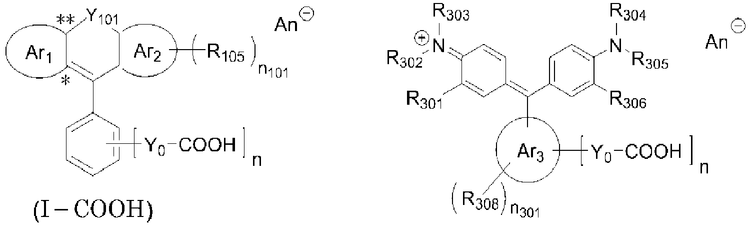

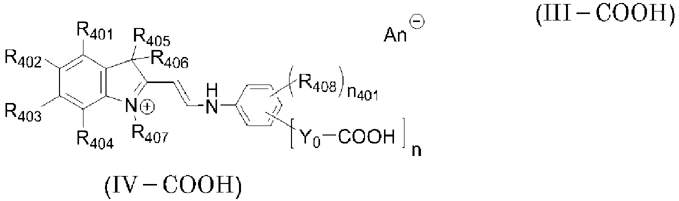

(In the formulae, R₁₀₅, R₃₀₁ to R₃₀₆, R₃₀₈, R₄₀₁ to R₄₀₈, Y₀, Y₁₀₁, Ar_{1,} Ar₂, Ar₃, An⁻, n, n₁₀₁, n₃₀₁, n₄₀₁, *, and ** are the same as described above. Here, a plurality of Y₀'s are the same as or different from each other.)

The salt exchange reaction in the reaction [I-I] is performed by bringing salts of the anions according to the present invention into contact with the compound represented by the general formula (21) in a solvent.

Examples of the solvent in the salt exchange reaction include organic solvents such as methanol, ethanol, isopropyl alcohol, tetrahydrofuran, 1,4-dioxane, N,N-dimethylformamide, dichloromethane, and dichloroethane. Among these, the dichloromethane is preferable. One kind of the solvent may be used singly, or two or more kinds of the solvents may be used by being appropriately combined. The amount of the reaction solvent used is generally 0.1 to 500 ml and preferably 1 to 100 ml, with respect to a weight of 1 g of the compound represented by the general formula (21).

The salt exchange reaction may be performed generally at a temperature of 0°C to 100°C and preferably at a temperature of 10°C to 50°C, generally for 1 to 12 hours and preferably for 1 to 6 hours.

Examples of the salts of the anions according to the present invention that are used in the salt exchange reaction, the anions according to the present invention, a sodium salt, a potassium salt, a lithium salt, and the like. Among these, the lithium salt is preferable. The amount of the used salts of the anions according to the present invention is generally 1 to 2 equivalents and preferably 1 to 1.5 equivalents, with respect to the mol number of the compound represented by the general formula (21).

In the reaction [I-II], the reaction between the compound represented by the general formula (21) or a compound obtained by the salt exchange reaction of the compound and the compound represented by the general formula (22) is performed in a solvent in the presence of a dehydrocondensation agent, generally at a temperature of 0°C to 100°C and preferably at a temperature of 10°C to 50°C generally for 1 to 24 hours and preferably for 1 to 12 hours.

Examples of the solvent include ethers such as ethyl ether, diisopropyl ether, ethyl methyl ether, tetrahydrofuran, 1,4-dioxane, and dimethoxyethane; ketones such as acetone, dimethyl ketone, methyl ethyl ketone, diethyl ketone, 2-hexanone, tert-butylmethyl ketone, cyclopentanone, and cyclohexanone; halogenated hydrocarbons such as chloromethane, chloroform, dichloromethane, dichloroethane, trichloroethane, carbon tetrachloride, and chlorobenzene; hydrocarbons such as n-hexane, benzene, toluene, and xylene; esters such as ethyl acetate, butyl acetate, and methyl propionate; nitriles such as acetonitrile; amides such as N,N-dimethylformamide; and the like. Among these, the ethers, the halogenated hydrocarbons, and the hydrocarbons are preferable, and the tetrahydrofuran, the dichloromethane, and the toluene are more preferable. One kind of the solvent may be used singly, or two or more kinds of the solvents may be used by being appropriately combined. The amount of the reaction solvent used is generally 0.1 to 500 ml and preferably 1 to 100 ml with respect to a total weight of 1 g of the compounds reacted.

As the dehydrocondensation agent, those generally used as a dehydrocondensation agent may be used, and examples thereof include inorganic dehydration agents such as diphosphorus pentoxide and anhydrous zinc chloride; carbodiimides such as dicyclohexyl carbodiimide, diisopropyl carbodiimide, and 1-ethyl-3-(3-dimethylaminopropyl)carbodiimide hydrochloride; polyphosphoric acid, acetic anhydride, sulfuric acid, carbonyl diimidazole, p-toluenesulfonic acid, and the like. Among these, the carbodiimides are preferable. The amount of the dehydrocondensation agent used is generally 1 to 20 equivalents and preferably 1 to 10 equivalents, with respect to the mol number of the compound represented by the general formula (21) or the compound obtained by the salt exchange reaction of the compound represented by the general formula (21).

In the reaction [I], in order to improve the efficiency of the dehydrocondensation agent, a catalyst such as dimethylaminopyridine may be used. The amount of the catalyst used is generally 0.1 to 10 equivalents with respect to the mol number of the compound represented by the general formula (21) or the compound obtained by the salt exchange reaction of the compound represented by the general formula (21).

In a case where n in the general formulae (1) and (21) is 1, the amount of the compound represented by general formula (22) used is generally 1 to 2 equivalents and preferably 1 to 1.5 equivalents, with respect to the mol number of the compound represented by the general formula (21) or the compound obtained by the salt exchange reaction of the compound represented by the general formula (21). In a case where n in the general formulae (1) and (21) is 2, the amount of the compound represented by the general formula (22) is generally 2 to 4 equivalents and preferably 2 to 3 equivalents, with respect to the mol number of the compound represented by the general formula (21) or the compound obtained by the salt exchange reaction of the compound represented by the general formula (21).

As the compound represented by the general formula (22), a commercial compound or a compound appropriately synthesized by a known method may be used. Specific examples of the compound represented by the general formula (22) include compounds described below.

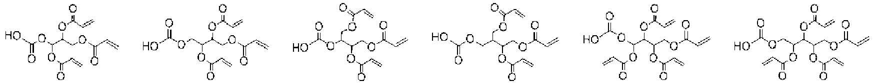

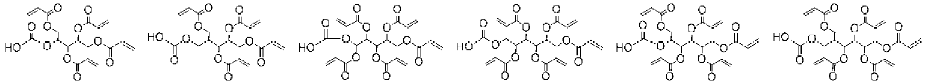

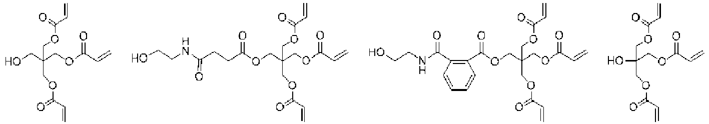

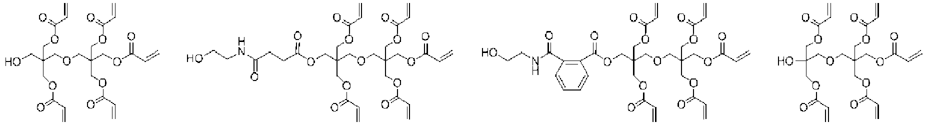

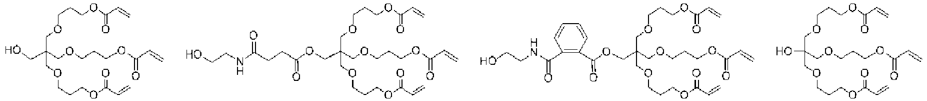

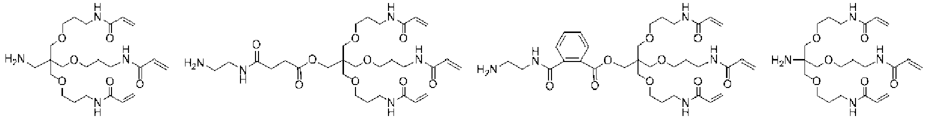

For example, in a case where a compound represented by the following general formula (27) is synthesized among the compounds represented by the general formula (22), the compound can be synthesized by a series of methods shown in the following reactions [II] to [IV]. That is, first, a compound represented by the following general formula (23) is reacted with a compound represented by the following general formula (24), thereby obtaining a compound represented by the following general formula (25) (reaction [II]). Then, by subjecting the compound represented by the general formula (25) to a reduction reaction, a compound represented by the following general formula (26) is obtained (reaction [III]). Thereafter, the obtained compound represented by the general formula (26) may be reacted with 3-chloropropionylchloride (reaction [IV]).

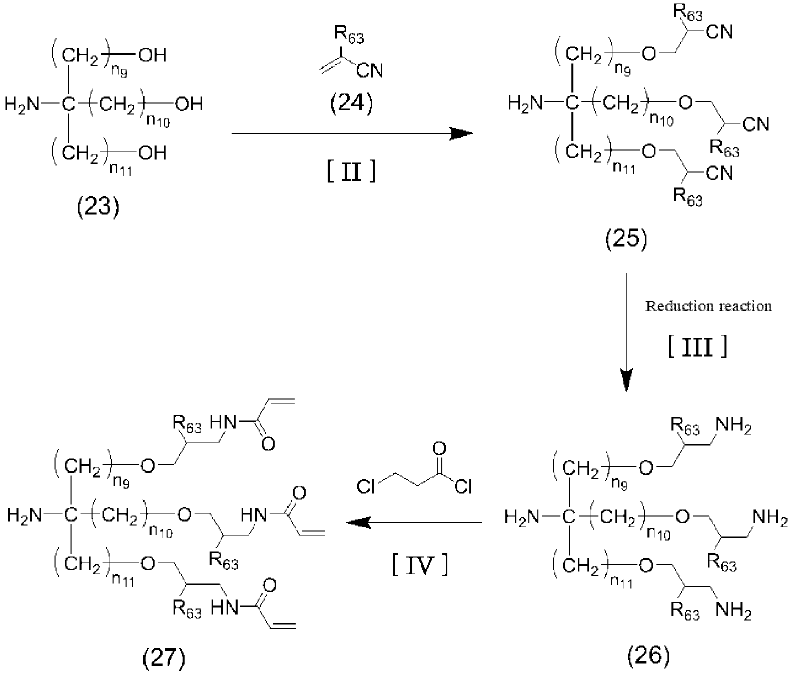

(In the formulae, R₆₃ represents a hydrogen atom or a methyl group, n₉, n₁₀, and n₁₁ each independently represent 1 or 2.)

As R₆₃ in the general formulae (24) to (27), a hydrogen atom is preferable.

It is preferable that all of n₉, n₁₀, and n₁₁ in the general formulae (23) and (25) to (27) are the same as each other. In addition, as n₉, n₁₀, and n₁₁, 1 is preferable.

As the compound represented by the general formula (23), a commercial compound or a compound appropriately synthesized by a known method may be used. Specific examples of the compound represented by the general formula (23) include tris(hydroxymethyl)aminomethane, 2-amino-2-hydroxymethyl-1,4-butanediol, 3-amino-3-(2-hydroxymethyl)-1,5-pentanediol, and 3-amino-3-(2-hydroxyethyl)-1,5-pentanediol.

In a case where R₆₃ is a hydrogen atom, the compound represented by the general formula (24) is acrylonitrile. In a case where R₆₃ is a methyl group, the compound represented by the general formula (24) is methacrylonitrile. As the compound, acrylonitrile is preferable.

The reaction between the compound represented by the general formula (23) and the compound represented by the general formula (24) in the reaction [II] is performed in a solvent in the presence of a base generally at a temperature of 0°C to 100°C and preferably at a temperature of 30°C to 80°C generally for 1 to 24 hours and preferably for 1 to 12 hours.

Examples of the solvent are the same as the examples of the solvent in the reaction [I-II]. Among these, nitriles are preferable, and acetonitrile is more preferable. One kind of the solvent may be used singly, or two or more kinds of the solvents may be used by being appropriately combined. The amount of the reaction solvent used is generally 0.1 to 500 ml and preferably 1 to 100 ml, with respect to a total weight of 1 g of the compound represented by the general formula (23) and the compound represented by the general formula (24).

Examples of the base include an alkali metal hydride such as sodium hydride or potassium hydride; an alkali metal alkoxide such as sodium methoxide, potassium methoxide, sodium ethoxide, potassium ethoxide, lithium-tert-butoxide, sodium-tert-butoxide, or potassium-tert-butoxide; an organic lithium compound such as n-butyl lithium, sec-butyl lithium, tert-butyl lithium, or n-hexyl lithium; an alkali metal hydroxide such as sodium hydroxide or potassium hydroxide; an alkali metal salt of carbonic acid such as sodium carbonate, potassium carbonate, and cesium carbonate; a tertiary amine such as triethylamine, pyridine, 1,8-diazabicyclo[5.4.0]undec-7-ene, and 1,5-diazabicyclo[4.3.0]non-5-ene; a metal amide such as lithium isopropylamide, lithium hexamethyldisilazane, sodium hexamethyldisilazane, and potassium hexamethyldisilazane; and the like. Among these, the alkali metal alkoxide is preferable, and the lithium-tert-butoxide, the sodium-tert-butoxide, and the potassium-tert-butoxide are more preferable. It should be noted that as the base, one kind of base may be used, or two or more kinds of bases may be used by being appropriately combined. The amount of the base used is generally 0.1 to 1 equivalent with respect to the mol number of the compound represented by the general formula (23).

The amount of the compound represented by general formula (24) used is generally 3 to 5 equivalents and preferably 3.5 to 4 equivalents, with respect to the mol number of the compound represented by the general formula (23).

The reduction reaction in the reaction [III] is performed by reacting the compound represented by the general formula (25) with hydrogen in a solvent in the presence of a catalyst generally at a temperature of 0°C to 100°C and preferably at a temperature of 10°C to 50°C generally for 1 to 24 hours and preferably for 1 to 12 hours.

As the hydrogen, it is preferable to use a hydrogen gas. The amount of the hydrogen used is generally 3 to 10 equivalents with respect to the mol number of the compound represented by the general formula (25).

Examples of the solvent include ethanol, methanol, isopropyl alcohol, tetrahydrofuran, 1,4-dioxane, N,N-dimethylformamide, dichloromethane, dichloroethane, ethyl acetate, ammonium hydroxide, and the like. Among these, the methanol and the ammonium hydroxide are preferable. One kind of the solvent may be used singly, or two or more kinds of the solvents may be used by being appropriately combined. The amount of the reaction solvent used is generally 0.1 to 500 ml and preferably 1 to 100 ml, with respect to a weight of 1 g of the compound represented by the general formula (25).

Examples of the catalyst include platinum (IV) oxide, palladium carbon, Raney nickel (Ni), Raney cobalt (Co), and the like. Among these, the Raney nickel and the Raney cobalt are preferable. The amount of the catalyst used is generally 3 to 10 equivalents with respect to the mol number of the compound represented by the general formula (25).

The reaction between the compound represented by the general formula (26) and 3-chloropropionyl chloride in the reaction [IV] is performed by causing a reaction in a solvent in the presence of a base generally at a temperature of 0°C to 50°C and preferably at a temperature of 0°C to 20°C generally for 1 to 24 hours and preferably for 1 to 12 hours, then further adding a base, and causing a reaction generally at a temperature of 10°C to 100°C and preferably at a temperature of 10°C to 50°C for 1 to 24 hours and preferably for 1 to 12 hours.

Examples of the solvent are the same as the examples of the solvent in the reaction [I-II]. Among these, nitriles are preferable, and acetonitrile is more preferable. One kind of the solvent may be used singly, or two or more kinds of the solvents may be used by being appropriately combined. The amount of the reaction solvent used is generally 0.1 to 500 ml and preferably 1 to 100 ml, with respect to a total weight of 1 g of the compound represented by the general formula (26) and the 3-chloropropionyl chloride.

Examples of the base are the same as the examples of the base in the reaction [II]. Among these, the alkali metal hydroxide is preferable, and the sodium hydroxide and the potassium hydroxide are more preferable. It should be noted that one kind of the base may be used singly, or two or more kinds of the bases may be used by being appropriately combined. The amount of the base used is generally 1 to 10 equivalents with respect to the mol number of the compound represented by the general formula (26).

The amount of the 3-chloropropionyl chloride used is generally 3 to 6 equivalents and preferably 4 to 5 equivalents, with respect to the mol number of the compound represented by the general formula (26).

For example, in a case where a compound represented by the following general formula (31) is synthesized among the compounds represented by the general formula (22), the compound can be synthesized by a series of methods shown in the following reactions [V] and [VI]. That is, first, a compound represented by the following general formula (28) is reacted with succinic anhydride, thereby obtaining a compound represented by the following general formula (29) (reaction [V]). Then, the obtained compound represented by the general formula (29) may be reacted with a compound represented by the following general formula (30) (reaction [VI]).

For example, in a case where a compound represented by the following general formula (33) is synthesized among the compounds represented by the general formula (22), the compound can be synthesized by a series of methods shown in the following reactions [VII] and [VIII]. That is, first, a compound represented by the following general formula (28) is reacted with phthalic anhydride, thereby obtaining a compound represented by the following general formula (32) (reaction [VII]). Then, the obtained compound represented by the general formula (32) may be reacted with a compound represented by a general formula (30) (reaction [VIII]) .

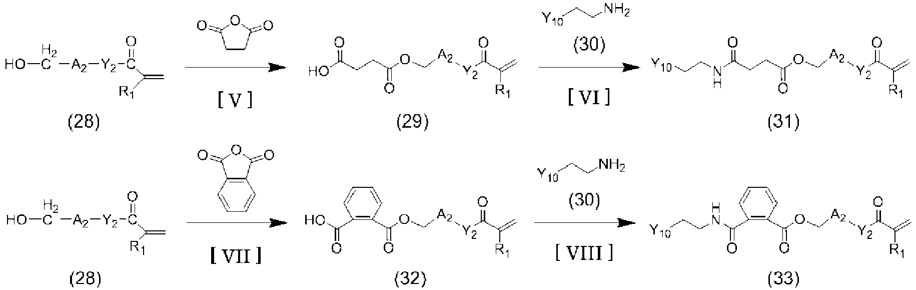

(In the formulae, R₁, Y₂, Y₁₀, and A₂ are the same as described above.)

Specific examples preferred as the general formula (28) include the following general formula (28-1).

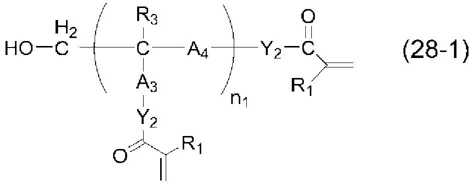

(In the formula, R₁, R₃, A₃, A₄, Y₂, and n₁ are the same described above. Here, a plurality of R₁'s, a plurality of R₃'s, a plurality of A₃'s, a plurality of A₄'s, and a plurality of Y₂'s are the same as or different from each other respectively, the total number of carbon atoms in n₁ pieces of A₄ is 1 to 6, and in a case where n₁ is 1, R₃ represents the group represented by the general formula (2-1).)

Specific examples preferred as the general formula (28-1) include the following general formula (28-2-1) or (28-2-2).

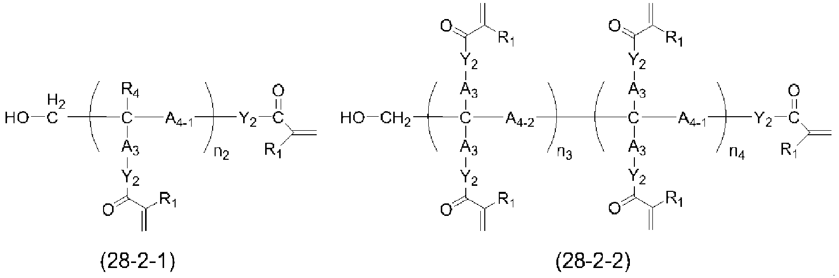

(In the formulae, R₁, R₄, A₃, A₄₋₁, A₄₋₂, Y₂, and n₂ to n₄ are the same as described above. Here, a plurality of R₁'s, a plurality of R₄'s, a plurality of A₃'s, a plurality of A₄₋₁'s, a plurality of A₄₋₂'s, and a plurality of Y₂'s are the same as or different from each other respectively. In addition, the total number of carbon atoms in n₂ pieces of A₄₋₁ is 1 to 6, and the total number of carbon atoms in n₄ pieces of A₄₋₁ and n₃ pieces of A₄₋₂ is 1 to 6.)

As the compound represented by the general formula (28), a commercial compound or a compound appropriately synthesized by a known method may be used. Specific examples of the compound represented by the general formula (28) include the following compounds.

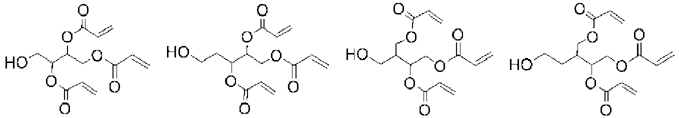

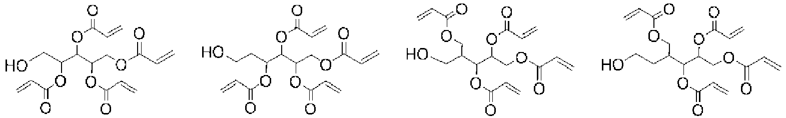

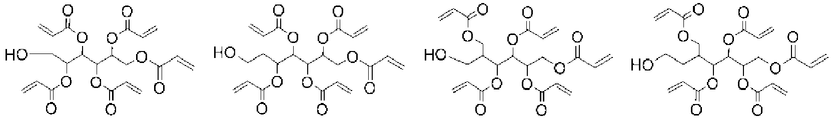

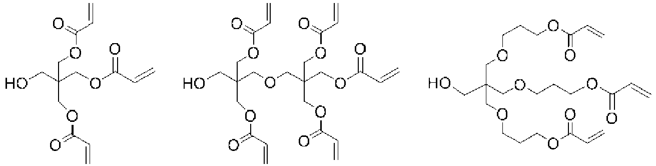

In a case where Y₁₀ is a hydroxy group, the compound represented by the general formula (30) is ethanolamine, and in a case where Y₁₀ is an amino group, the compound represented by the general formula (30) is ethylenediamine.

The reaction between the compound represented by the general formula (28) and succinic anhydride in the reaction [V] or the reaction between the compound represented by the general formula (28) and phthalic anhydride in the reaction [VII] is performed by causing the reaction in a solvent in the presence of a base generally at a temperature of 0°C to 100°C and preferably at a temperature of 10°C to 50°C generally for 1 to 48 hours and preferably for 12 to 36 hours.

Examples of the solvent are the same as the examples of the solvent in the reaction [I-II]. Among these, halogenated hydrocarbons are preferable, and dichloromethane is more preferable. One kind of the solvent may be used singly, or two or more kinds of the solvents may be used by being appropriately combined. The amount of the reaction solvent used is generally 0.1 to 500 ml and preferably 1 to 100 ml with respect to a total weight of 1 g of the compound represented by the general formula (28) and the succinic anhydride or the phthalic anhydride.

Examples of the base are the same as the examples of the base in the reaction [II]. Among these, a tertiary amine is preferable, and triethylamine is more preferable. It should be noted that as the base, one kind of base may be used singly, or two or more kinds of bases may be used by being appropriately combined. The amount of the base used is generally 0.1 to 1 equivalent with respect to the mol number of the compound represented by the general formula (28).

In the reaction [V] or [VII], in order to improve the reaction efficiency, a catalyst such as dimethylaminopyridine may be used. The amount of the catalyst used is generally 0.1 to 10 equivalents with respect to the mol number of the compound represented by the general formula (28).

The amount of the succinic anhydride or the phthalic anhydride used is generally 1 to 2 equivalents and preferably 1 to 1.5 equivalents with respect to the mol number of the compound represented by the general formula (28).

The reaction between the compound represented by the general formula (29) and the compound represented by the general formula (30) in the reaction [VI] or the reaction between the compound represented by the general formula (32) and the compound represented by the general formula (30) in the reaction [VIII] is performed by causing the reaction in a solvent in the presence of a dehydrocondensation agent generally at a temperature of 0°C to 100°C and preferably at a temperature of 10°C to 50°C generally for 1 to 24 hours and preferably for 1 to 12 hours.

Examples of the solvent include methanol, ethanol, isopropyl alcohol, tetrahydrofuran, 1,4-dioxane, N,N-dimethylformamide, dichloromethane, dichloroethane, ethyl acetate, ammonium hydroxide, and the like. Among these, the ethanol is preferable. One kind of the solvent may be used singly, or two or more kinds of the solvents may be used by being appropriately combined. The amount of the reaction solvent used is generally 0.1 to 500 ml and preferably 1 to 100 ml, with respect to a weight of 1 g of the compound represented by the general formula (29) or the compound represented by the general formula (32).

As the dehydrocondensation agent, those generally used as a dehydrocondensation agent may be used. Examples thereof include triazines such as 4-(4,6-dimethoxy-1,3,5-triazin-2-yl)-4-methyl morpholinium chloride n-hydride, and the like. The amount of the dehydrocondensation agent used is generally 1 to 20 equivalents and preferably 1 to 10 equivalents with respect to the mol number of the compound represented by the general formula (29) or the compound represented by the general formula (32).

The amount of the compound represented by the general formula (30) used is generally 1 to 2 equivalents and preferably 1 to 1.5 equivalents with respect to the mol number of the compound represented by the general formula (29) or the compound represented by the general formula (32).

The pressure at the time of the reaction in the reactions [I] to [VIII] is not particularly limited as long as the series of reactions can be smoothly performed. For example, the reactions may be performed under normal pressure.

If necessary, the reactants and the products obtained after the reactions in the reactions [I] to [VIII] may be isolated by general post-treatment operations or purification operations that are usually carried out in the field of the related art. Specifically, for example, the obtained reactants and products may be isolated by performing filtration, washing, extraction, concentration under reduced pressure, recrystallization, distillation, column chromatography, and the like.

### Colored composition of the present invention

The colored composition of the present invention comprises at least one kind of the compound of the present invention. The colored composition can form an excellent colored cured material having higher elution resistance than that of the related art. Therefore, the colored composition of the present invention can be used for forming colored pixels of color filters and the like, which are used in liquid crystal display (LCD) or a solid-state imaging device (CCD, CMOS, and the like), and can be used in printing ink, ink jet ink, paint, and the like. Particularly, the colored composition of the present invention is suitable for color filters of a liquid crystal display. Furthermore, the colored composition of the present invention can be used as a colored resin molded material by being molded into a sheet, a film, a bottle, a cup, and the like, by means of molding methods known in the related art. Accordingly, the colored composition of the present invention can also be used for eyeglasses, contact lenses, colored contact lenses, and the like. By being made into a multilayer structure with a known resin, the colored composition of the present invention can also be used for the same uses. In addition, for example, the colored composition of the present invention can be used for optical films, hair coloring agents, labeling substances for compounds or biological substances, materials for organic solar cells, and the like.

It is preferable that the colored composition of the present invention comprises one or more kinds of the compound of the present invention and one or more kinds of binder resins. It is more preferable that the colored composition of the present invention further comprises a polymerization initiator in addition to the compound of the present invention and the binder resins. If necessary, the colored composition may contain a silane coupling agent, a pigment, a solvent, and the like. In addition, the colored composition of the present invention may contain a cross-linking agent. However, even though the composition does not contain a cross-linking agent, the colored composition can form a colored cured material having excellent elution resistance. Therefore, it is preferable that the colored composition does not contain a cross-linking agent. Accordingly, it is preferable that the colored composition of the present invention comprises one or more kinds of the compound of the present invention and one or more kinds of binder resins and does not contain a cross-linking agent.

The content of the compound of the present invention in the colored composition of the present invention is 1% to 50% by weight and preferably 5% to 30% by weight with respect to the weight of the composition. It should be noted that the weight of the composition mentioned herein means the weight of solid components except for a solvent. Hereinbelow, in the present application, the weight of the composition means the same thing.

The binder resins are not particularly limited as long as they are binder resins generally used in the field of the related art, such as a polyolefin resin, a polystyrene resin, a polyester resin, a polyamide resin, a polyurethane resin, a polycarbonate resin, an epoxy resin, an acryl resin, an acrylonitrile resin, and an ABS resin. Among these, it is preferable that they are binder resins having a polymerizable unsaturated group. More specifically, a binder resin containing a monomer unit represented by the following general formula (5) as a constituent component is preferable.

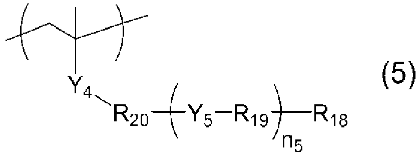

(In the formula, R₁₈ represents a group represented by any one of the following formulae (5-1) to (5-10),

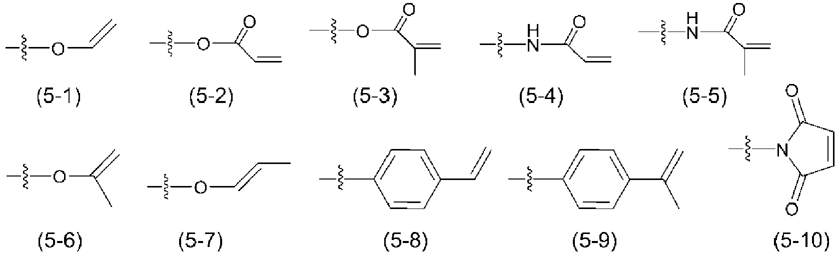

R₁₉ and R₂₀ each independently represent an alkylene group having 1 to 12 carbon atoms that may have a substituent, the substituent is selected from a hydroxy group, an alkyl group having 1 to 6 carbon atoms, an alkoxy group having 1 to 6 carbon atoms, an aryl group having 6 to 10 carbon atoms, an aryloxy group having 6 to 10 carbon atoms, an arylalkyl group having 7 to 13 carbon atoms, an aryloxyalkyl group having 7 to 13 carbon atoms, and an alicyclic hydrocarbon group having 6 to 12 carbon atoms that has or does not have an oxygen atom, Y₄ represents -COO-, -CONH-, -OCONH-, or -NHCONH-, Y₅ represents -O-, -OCO-, -COO-, -CONH-, -NHCO-, -OCONH-, or -NHCONH-, and n₅ represents an integer of 1 to 4. Here, a plurality of R₁₉'s and a plurality of Y₅'s are the same as or different from each other respectively.)

As R₁₈ in the general formula (5), formulae (5-2) and (5-3) are preferable, and the formula (5-2) is more preferable.

In a case where R₁₉ and R₂₀ in the general formula (5) represent "an alkylene group having 1 to 12 carbon atoms that may have a substituent", as the alkylene group having 1 to 12 carbon atoms, any of linear, branched, and cyclic alkylene groups are preferable, and linear alkylene groups or alkylene groups as a combination of a cyclic alkylene group and a linear alkylene group are preferable. In addition, among the alkylene groups having 1 to 12 carbon atoms, an alkylene group having 1 to 6 carbon atoms is preferable. Specific examples thereof include a methylene group, an ethylene group, a methyl methylene group, a trimethylene group, a propylene group, a dimethyl methylene group, an ethyl methylene group, a tetramethylene group, a 1-methyltrimethylene group, a 2-methyltrimethylene group, a 1,2-dimethylethylene group, a 1,1-dimethylethylene group, an ethyl ethylene group, an ethyl methyl methylene group, a propyl methylene group, a pentamethylene group, a 1-methyltetramethylene group, a 2-methyltetramethylene group, a 1-ethyltrimethylene group, a 2-ethyltrimethylene group, an n-propylethylene group, an isopropyl ethylene group, an n-butylmethylene group, an isobutyl methylene group, a tert-butylmethylene group, a hexamethylene group, a 1-methylpentamethylene group, a 2-methylpentamethylene group, a 3-methylpentamethylene group, a 1-ethyltetramethylene group, a 2-ethyltetramethylene group, a 1-n-propyltrimethylene group, a 1-isopropyltrimethylene group, a 2-n-propyltrimethylene group, a 2-isopropyltrimethylene group, an n-butylethylene group, an isobutyl ethylene group, a tert-butylethylene group, an n-pentylmethylene group, an isopentyl methylene group, a heptamethylene group, an octamethylene group, a nonamethylene group, a decamethylene group, an undecamethylene group, a dodecamethylene group, a -C₆H₁₀-CH₂- group, a -C₆H₁₀-C₂H₄- group, a -C₆H₁₀-C₃H₆- group, a -C₆H₁₀-C₄H₈- group, a -C₆H₁₀-C₅H₁₀- group, a -C₆H₁₀-C₆H₁₂- group, and the like. Among these, the methylene group, the ethylene group, the trimethylene group, the tetramethylene group, the pentamethylene group, the hexamethylene group, the heptamethylene group, the octamethylene group, the nonamethylene group, the decamethylene group, the undecamethylene group, the dodecamethylene group, the -C₆H₁₀-CH₂- group, the -C₆H₁₀-C₂H₄- group, the -C₆H₁₀-C₃H₆- group, the -C₆H₁₀-C₄H₈- group, the -C₆H₁₀-C₅H₁₀- group, and the -C₆H₁₀-C₆H₁₂- group are preferable, the methylene group, the ethylene group, the trimethylene group, the tetramethylene group, the pentamethylene group, the hexamethylene group, the -C₆H₁₀-CH₂- group, the -C₆H₁₀-C₂H₄- group, and the -C₆H₁₀-C₃H₆- group are more preferable.

As the alkylene group having 1 to 12 carbon atoms represented by R₁₉ and R₂₀ in the general formula (5) that has a substituent, the alkylene group having one or more kinds of 1 to 3 substituents is preferable, the alkylene group having one kind of 1 to 3 substituents is more preferable, and the alkylene group having one substituent is even more preferable. The substituent is selected from a hydroxy group, an alkyl group having 1 to 6 carbon atoms, an alkoxy group having 1 to 6 carbon atoms, an aryl group having 6 to 10 carbon atoms, an aryloxy group having 6 to 10 carbon atoms, an arylalkyl group having 7 to 13 carbon atoms, an aryloxyalkyl group having 7 to 13 carbon atoms, and an alicyclic hydrocarbon group having 6 to 12 carbon atoms that has or does not have an oxygen atom.

Examples of the alkyl group having 1 to 6 carbon atoms among the substituents are the same as the examples of the alkyl group having 1 to 6 carbon atoms represented by R₃ in the general formula (2). Among these, a methyl group, an ethyl group, an n-propyl group, an isopropyl group, an n-butyl group, an isobutyl group, a sec-butyl group, and a tert-butyl group are preferable, and the methyl group and the ethyl group are more preferable.

Examples of the alkoxy group having 1 to 6 carbon atoms among the substituents are the same as the examples of the alkoxy group having 1 to 6 carbon atoms represented by R₃₀₈ in the general formula (III). Among these, a methoxy group, an ethoxy group, an n-propoxy group, an isopropoxy group, an n-butoxy group, an isobutoxy group, a sec-butoxy group, and a tert-butoxy group are preferable, and the methoxy group and the ethoxy group are more preferable.

Examples of the aryl group having 6 to 10 carbon atoms among the substituents include a phenyl group, a naphthyl group, and the like. Among these, the phenyl group is preferable.

Examples of the aryloxy group having 6 to 10 carbon atoms among the substituents include a phenoxy group, a naphthyloxy group, and the like. Among these, the phenoxy group is preferable.

Examples of the arylalkyl group having 7 to 13 carbon atoms among the substituents are the same as the examples of the arylalkyl group having 7 to 13 carbon atoms in the substituent of the amino group having a substituent represented by R₁₀₅ in the general formula (I), and preferred examples are also the same.

Examples of the aryloxyalkyl group having 7 to 13 carbon atoms among the substituents include a phenoxymethyl group, a phenoxyethyl group, a phenoxypropyl group, a naphthyloxymethyl group, a naphthyloxyethyl group, a naphthyloxypropyl group, and the like.

Examples of the alicyclic hydrocarbon group having 6 to 12 carbon atoms and an oxygen atom among the substituents include a dicyclopentenyloxyethyl group and the like.

Examples of the alicyclic hydrocarbon group having 6 to 12 carbon atoms that does not have an oxygen atom among the substituents include a cyclohexyl group, an isobornyl group, a dicyclopentanyl group, and the like.

Examples of the alkylene group having 1 to 12 carbon atoms and a substituent that is represented by R₁₉ and R₂₀ in the general formula (5) include groups represented by the following general formulae (41) to (43). Among these, the group represented by the general formula (41) is preferable.

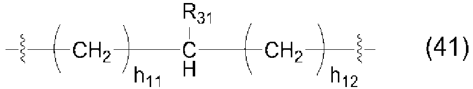

(In the formula, R₃₁ represents a hydroxy group, an alkyl group having 1 to 6 carbon atoms, an alkoxy group having 1 to 6 carbon atoms, an aryl group having 6 to 10 carbon atoms, an aryloxy group having 6 to 10 carbon atoms, an arylalkyl group having 7 to 13 carbon atoms, an aryloxyalkyl group having 7 to 13 carbon atoms, or an alicyclic hydrocarbon group having 6 to 12 carbon atoms that has or does not have an oxygen atom, and h₁₁ and h₁₂ each independently represent an integer of 0 to 6. Here, h₁₁ + h₁₂ equals an integer of 0 to 11.)

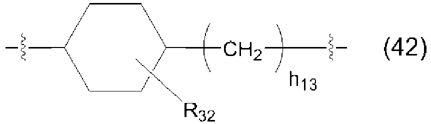

(In the formula, R₃₂ represents a hydroxy group, an alkyl group having 1 to 6 carbon atoms, an alkoxy group having 1 to 6 carbon atoms, an aryl group having 6 to 10 carbon atoms, an aryloxy group having 6 to 10 carbon atoms, an arylalkyl group having 7 to 13 carbon atoms, an aryloxyalkyl group having 7 to 13 carbon atoms, or an alicyclic hydrocarbon group having 6 to 12 carbon atoms that has or does not have an oxygen atom, and h₁₃ represents an integer of 1 to 6.)

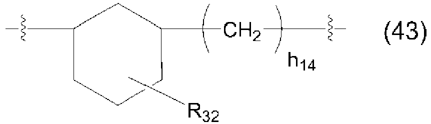

(In the formula, R₃₂ is the same as described above, and h₁₄ represents an integer of 1 to 6.)

Examples of the alkyl group having 1 to 6 carbon atoms, the alkoxy group having 1 to 6 carbon atoms, the aryl group having 6 to 10 carbon atoms, the aryloxy group having 6 to 10 carbon atoms, the arylalkyl group having 7 to 13 carbon atoms, the aryloxyalkyl group having 7 to 13 carbon atoms, and the alicyclic hydrocarbon group having 6 to 12 carbon atoms that has or does not have an oxygen atom represented by R₃₁ in the general formula (41) are the same as the examples of the substituents represented by R₁₉ and R₂₀ in the general formula (5), and preferred examples are also the same.

As R₃₁ in the general formula (41), a hydroxy group, an alkyl group having 1 to 6 carbon atoms, an alkoxy group having 1 to 6 carbon atoms, an aryl group having 6 to 10 carbon atoms, an aryloxy group having 6 to 10 carbon atoms, an arylalkyl group having 7 to 13 carbon atoms, and an aryloxyalkyl group having 7 to 13 carbon atoms are preferable. Specifically, a hydroxy group, a methyl group, an ethyl group, an n-propyl group, an isopropyl group, an n-butyl group, an isobutyl group, a sec-butyl group, a tert-butyl group, a methoxy group, an ethoxy group, an n-propoxy group, an isopropoxy group, an n-butoxy group, an isobutoxy group, a sec-butoxy group, a tert-butoxy group, a phenyl group, a phenoxy group, a benzyl group, a phenoxymethyl group, a phenoxyethyl group, and a phenoxypropyl group are preferable, the hydroxy group, the methyl group, the ethyl group, the methoxy group, the ethoxy group, the phenyl group, the phenoxy group, the benzyl group, and the phenoxymethyl group are more preferable, and the hydroxy group is particularly preferable.

It is preferable that h₁₁ and h₁₂ in the general formula (41) are the same as each other. As h₁₁ and h₁₂, an integer of 1 to 4 is preferable, 1 or 2 is more preferable, and 1 is particularly preferable.

Examples of the alkyl group having 1 to 6 carbon atoms, the alkoxy group having 1 to 6 carbon atoms, the aryl group having 6 to 10 carbon atoms, the aryloxy group having 6 to 10 carbon atoms, the arylalkyl group having 7 to 13 carbon atoms, the aryloxyalkyl group having 7 to 13 carbon atoms, and the alicyclic hydrocarbon group having 6 to 12 carbon atoms that has or does not have an oxygen atom represented by R₃₂ in the general formulae (42) and (43) are the same as the examples of the substituents represented by R₁₉ and R₂₀ in the general formula (5), and preferred examples are also the same.

As R₃₂ in the general formulae (42) and (43), a hydroxy group, an alkyl group having 1 to 6 carbon atoms, an alkoxy group having 1 to 6 carbon atoms, an aryl group having 6 to 10 carbon atoms, an aryloxy group having 6 to 10 carbon atoms, an arylalkyl group having 7 to 13 carbon atoms, and an aryloxyalkyl group having 7 to 13 carbon atoms are preferable. Specifically, a hydroxy group, a methyl group, an ethyl group, an n-propyl group, an isopropyl group, an n-butyl group, an isobutyl group, a sec-butyl group, a tert-butyl group, a methoxy group, an ethoxy group, an n-propoxy group, an isopropoxy group, an n-butoxy group, an isobutoxy group, a sec-butoxy group, a tert-butoxy group, a phenyl group, a phenoxy group, a benzyl group, a phenoxymethyl group, a phenoxyethyl group, and a phenoxypropyl group are preferable, the hydroxy group, the methyl group, the ethyl group, the methoxy group, the ethoxy group, the phenyl group, the phenoxy group, the benzyl group, and the phenoxymethyl group are more preferable, and the hydroxy group is particularly preferable.

As h₁₃ in the general formula (42) and h₁₄ in the general formula (43), an integer of 1 to 3 is preferable, and 1 is more preferable.

Specific examples preferred as the group represented by the general formula (41) include groups represented by the following formulae (221) to (238). Among these, the groups represented by the formulae (221) to (229) are preferable, and the group represented by the formula (221) is more preferable.

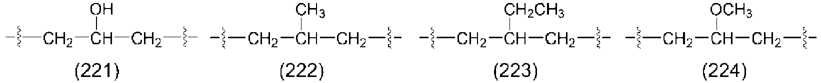

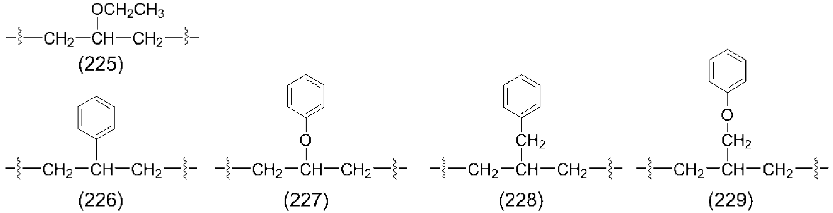

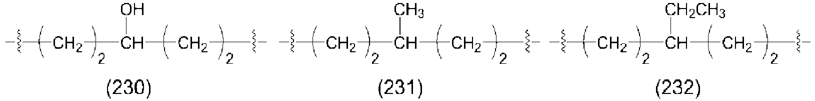

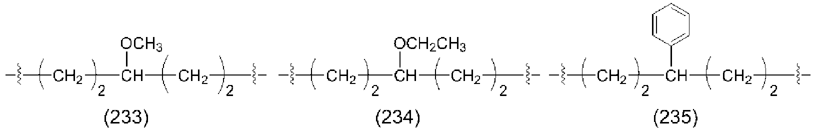

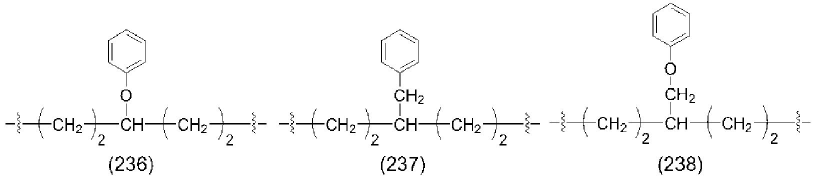

Specific examples of the group represented by the general formula (42) include groups represented by the following formulae (239) to (241). Among these, the group represented by the formula (239) is preferable.

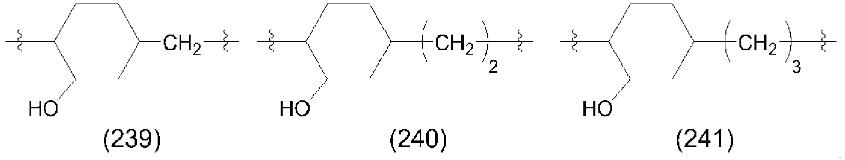

Specific examples of the group represented by the general formula (43) include groups represented by the following formulae (242) to (244). Among these, the group represented by the formula (242) is preferable.

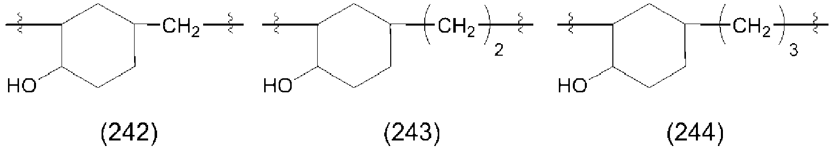

As R₁₉ and R₂₀ in the general formula (5), a linear alkylene group having 1 to 12 carbon atoms that has one substituent, which is selected from a hydroxy group, an alkyl group having 1 to 6 carbon atoms, an alkoxy group having 1 to 6 carbon atoms, an aryl group having 6 to 10 carbon atoms, an aryloxy group having 6 to 10 carbon atoms, an arylalkyl group having 7 to 13 carbon atoms, an aryloxyalkyl group having 7 to 13 carbon atoms, and an alicyclic hydrocarbon group having 6 to 12 carbon atoms that has or does not have an oxygen atom, and an unsubstituted linear alkylene group having 1 to 12 carbon atoms are preferable, and a linear alkylene group having 1 to 6 carbon atoms that has one substituent, which is selected from a hydroxy group, an alkyl group having 1 to 4 carbon atoms, an alkoxy group having 1 to 4 carbon atoms, a phenyl group, a phenoxy group, a phenylalkyl group having 7 to 9 carbon atoms, and a phenoxyalkyl group having 7 to 9 carbon atoms and an unsubstituted linear alkylene group having 1 to 6 carbon atoms are more preferable.

Specific examples of R₁₉ and R₂₀ in the general formula (5) include a methylene group, an ethylene group, a trimethylene group, a tetramethylene group, a pentamethylene group, a hexamethylene group, a -C₆H₁₀-CH₂- group, a -C₆H₁₀-C₂H₄- group, a -C₆H₁₀-C₃H₆- group, and the groups represented by the formulae (221) to (244). Among these, the methylene group, the ethylene group, the trimethylene group, the tetramethylene group, groups represented by the formulae (221) to (229), the group represented by the formula (239), and the group represented by the formula (242) are preferable, and the methylene group, the ethylene group, the group represented by the formula (221), the group represented by the formula (239), and the group represented by the formula (242) are more preferable.

As Y₄ in the general formula (5), -COO- is preferable.

As Y₅ in the general formula (5), -O-, -NHCO-, -OCONH-, and -NHCONH- are preferable, and -O- is more preferable.

As n₅ in the general formula (5), 1 or 2 is preferable, and 1 is more preferable.

Examples of the preferred combination of R₁₈, R₁₉, (n₅ pieces of) R₂₀, Y₄, (n₅ pieces of) Y₅, and n₅ in the general formula (5) include combinations described in the following table.

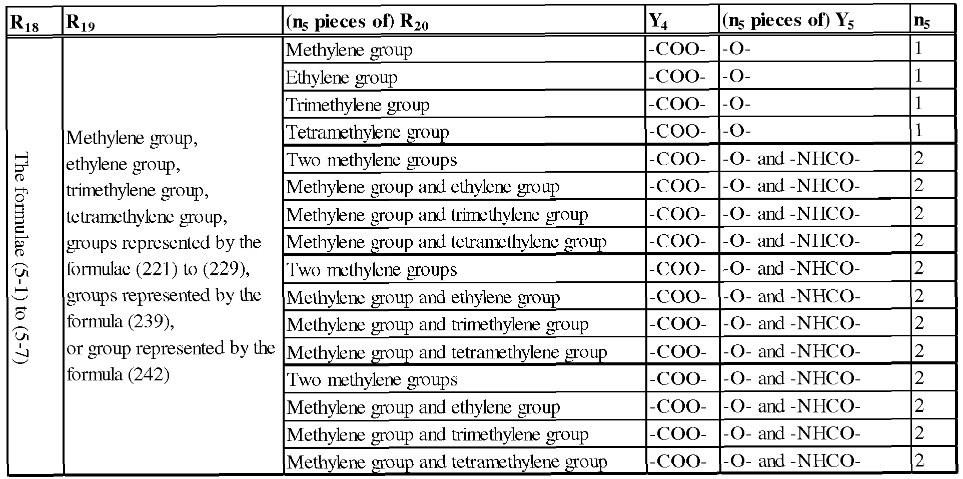

Among the preferred combinations, combinations described in the following table are more preferable.

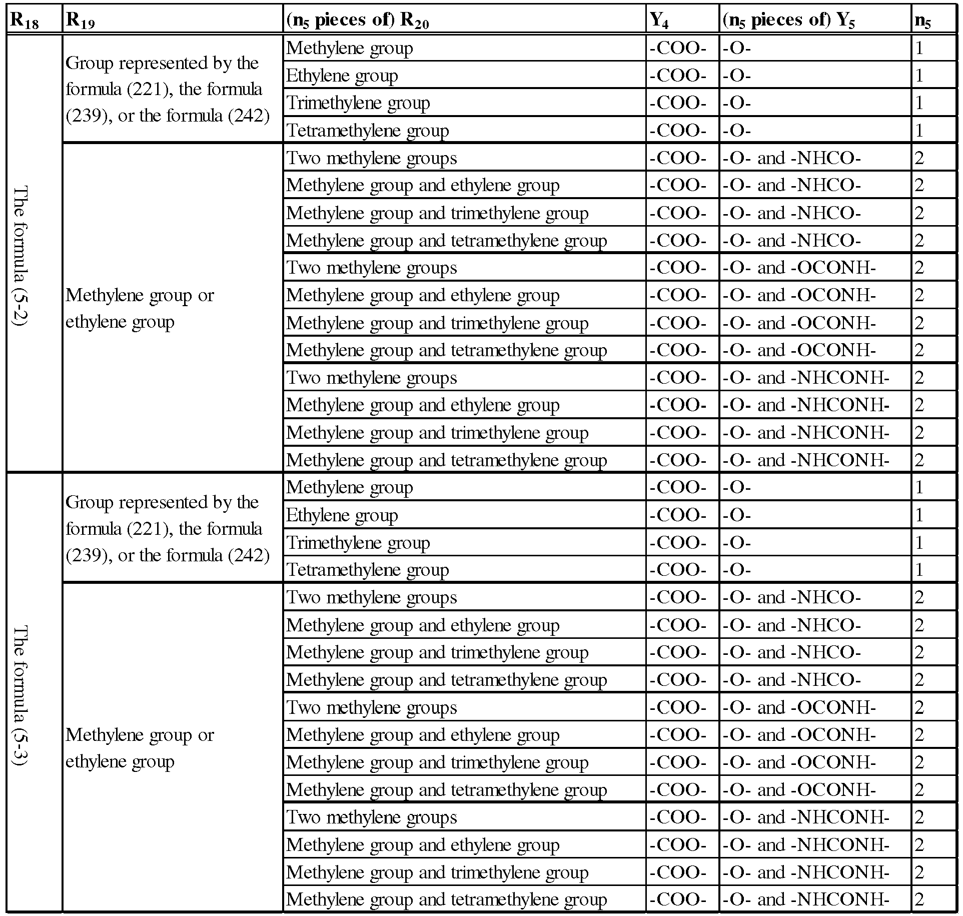

Among the more preferred combinations, combinations described in the following table are particularly preferable.

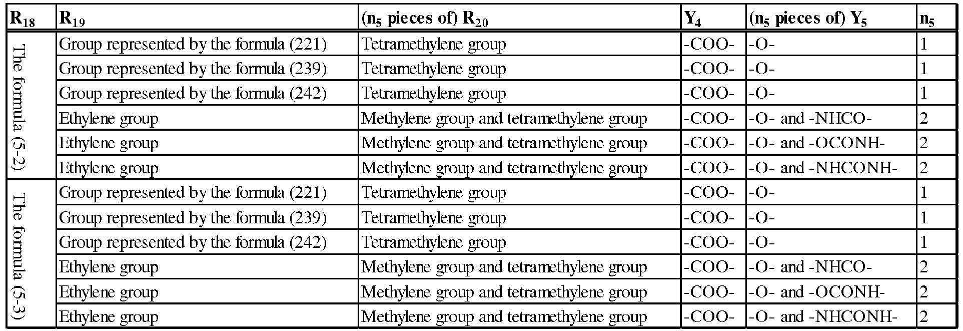

It is preferable that the binder resin containing the monomer unit represented by the general formula (5) as a constituent component further contains, as a constituent component, at least one kind of monomer unit derived from a compound represented by the following general formula (6), (10), (11), or (12).

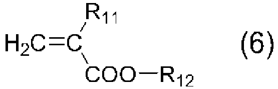

{In the formula, R₁₁ represents a hydrogen atom or a methyl group, R₁₂ represents a hydrogen atom, an alkyl group having 1 to 30 carbon atoms, a hydroxyalkyl group having 1 to 10 carbon atoms, an aryl group having 6 to 10 carbon atoms, an arylalkyl group having 7 to 13 carbon atoms, an alkoxyalkyl group having 2 to 9 carbon atoms, an alkoxyalkoxyalkyl group having 3 to 9 carbon atoms, an aryloxyalkyl group having 7 to 13 carbon atoms, a morpholinoalkyl group having 5 to 7 carbon atoms, a trialkylsilyl group having 3 to 9 carbon atoms, an alicyclic hydrocarbon group having 6 to 12 carbon atoms that has an oxygen atom, a dialkylaminoalkyl group having 3 to 9 carbon atoms, a fluoroalkyl group having 1 to 18 carbon atoms, an N-alkylenephthalimide group having 9 to 14 carbon atoms, a group represented by the following general formula (7), 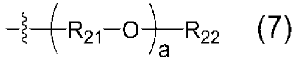 (in the formula, R₂₁ represents an alkylene group having 1 to 3 carbon atoms that has a hydroxy group as a substituent or is unsubstituted, R₂₂ represents a phenyl group that has a hydroxy group as a substituent or is unsubstituted, or represents an alkyl group having 1 to 3 carbon atoms, and a represents an integer of 1 to 3.),
a group represented by the following general formula (8), 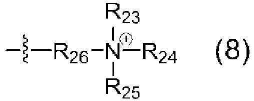 (in the formula, R₂₃ to R₂₅ each independently represent an alkyl group having 1 to 3 carbon atoms, and R₂₆ represents an alkylene group having 1 to 3 carbon atoms),
or a group represented by the following general formula (9), 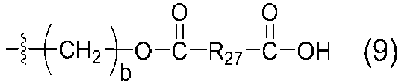 (in the formula, R₂₇ represents a phenylene group or a cyclohexylene group, and b represents an integer of 1 to 6.).} 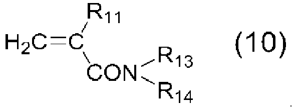 (in the formula, R₁₃ represents a hydrogen atom or an alkyl group having 1 to 3 carbon atoms, R₁₄ represents a hydrogen atom, an alkyl group having 1 to 3 carbon atoms, a dialkylaminoalkyl group having 3 to 9 carbon atoms, or a hydroxyalkyl group having 1 to 10 carbon atoms, and R₁₁ is the same as described above. R₁₃ and R₁₄ may form a morpholino group together with a nitrogen atom adjacent thereto.) 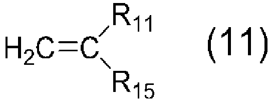 (in the formula, R₁₅ represents a phenyl group or a pyrrolidino group, and R₁₁ is the same as described above.) 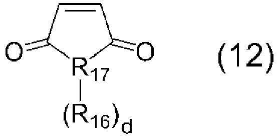 (in the formula, R₁₇ represents a nitrogen atom or an oxygen atom, R₁₆ represents a hydrogen atom, an alkyl group having 1 to 30 carbon atoms, a hydroxyalkyl group having 1 to 10 carbon atoms, a haloalkyl group having 1 to 10 carbon atoms, an aryl group having 6 to 10 carbon atoms, or an aryl group having 6 to 10 carbon atoms that has an alkyl group having 1 to 6 carbon atoms or a halogeno group as a substituent, d represents 0 in a case where R₁₇ is an oxygen atom, and represents 1 in a case where R₁₇ is a nitrogen atom.)

As R₁₁ in the general formula (6), a methyl group is preferable.

Examples of the alkyl group having 1 to 30 carbon atoms represented by R₁₂ in the general formula (6) and R₁₆ in the general formula (12) are the same as the examples of the alkyl group having 1 to 30 carbon atoms represented by R₁₀₅ in the general formula (I), and preferred examples are also the same.

Examples of the hydroxyalkyl group having 1 to 10 carbon atoms represented by R₁₂ in the general formula (6), R₁₄ in the general formula (10), and R₁₆ in the general formula (12) include a hydroxymethyl group, a hydroxyethyl group, a hydroxypropyl group, a hydroxybutyl group, a hydroxypentyl group, a hydroxyhexyl group, a hydroxyheptyl group, a hydroxyoctyl group, a hydroxynonyl group, a hydroxydecyl group, and the like.

Examples of the aryl group having 6 to 10 carbon atoms, the arylalkyl group having 7 to 13 carbon atoms, the aryloxyalkyl group having 7 to 13 carbon atoms, and the alicyclic hydrocarbon group having 6 to 12 carbon atoms and an oxygen atom represented by R₁₂ in the general formula (6) are the same as the examples of those represented by R₁₉ and R₂₀ in the general formula (5), and preferred examples are also the same.

Examples of the alkoxyalkyl group having 2 to 9 carbon atoms represented by R₁₂ in the general formula (6) include a methoxymethyl group, a methoxyethyl group, a methoxypropyl group, a methoxybutyl group, a methoxypentyl group, a methoxyhexyl group, a methoxyheptyl group, a methoxyoctyl group, an ethoxymethyl group, an ethoxyethyl group, an ethoxypropyl group, an ethoxybutyl group, an ethoxypentyl group, an ethoxyhexyl group, an ethoxyheptyl group, a propoxymethyl group, a propoxyethyl group, a propoxypropyl group, a propoxybutyl group, a propoxypentyl group, a propoxyhexyl group, and the like.

Examples of the alkoxyalkoxyalkyl group having 3 to 9 carbon atoms represented by R₁₂ in the general formula (6) include a methoxymethoxymethyl group, a methoxymethoxyethyl group, a methoxymethoxypropyl group, an ethoxyethoxymethyl group, an ethoxyethoxyethyl group, an ethoxyethoxypropyl group, a propoxymethoxymethyl group, a propoxymethoxyethyl group, a propoxymethoxypropyl group, an ethoxyethoxymethyl group, an ethoxyethoxyethyl group, an ethoxyethoxypropyl group, a propoxyethoxymethyl group, a propoxyethoxyethyl group, a propoxyethoxypropyl group, a propoxypropoxymethyl group, a propoxypropoxyethyl group, a propoxypropoxypropyl group, and the like.

Examples of the morpholinoalkyl group having 5 to 7 carbon atoms represented by R₁₂ in the general formula (6) include a morpholinomethyl group, a morpholinoethyl group, a morpholinopropyl group, and the like.

Examples of the trialkylsilyl group having 3 to 9 carbon atoms represented by R₁₂ in the general formula (6) include a trimethylsilyl group, a triethylsilyl group, a tripropylsilyl group, a dimethylethylsilyl group, a diethylmethylsilyl group, and the like.

Examples of the dialkylaminoalkyl group having 3 to 9 carbon atoms represented by R₁₂ in the general formula (6) and R₁₄ in the general formula (10) include a N,N-dimethylaminomethyl group, a N,N-dimethylaminoethyl group, a N,N-dimethylaminopropyl group, a N,N-diethylaminomethyl group, a N,N-diethylaminoethyl group, a N,N-diethylaminopropyl group, a N,N-dipropylaminomethyl group, a N,N-dipropylaminoethyl group, a N,N-dipropylaminopropyl group, and the like.

Examples of the fluoroalkyl group having 1 to 18 carbon atoms represented by R₁₂ in the general formula (6) include a 2,2,2-trifluoroethyl group, a 2,2,3,3-trifluoropropyl group, a 2,2,3,3,4,4-hexafluorobutyl group, a 2,2,3,3,4,4,5,5-octafluoropentyl group, 3,3,4,4,5,5,6,6,7,7,8,8,9,9,10,10,10-heptadecafluorodecyl group, a 2-(heptadecafluorooctyl)ethyl group, and the like.

Examples of the N-alkylenephthalimide group having 9 to 14 carbon atoms represented by R₁₂ in the general formula (6) include a 2-phthalimidoethyl group, a 2-tetrahydrophthalimidoethyl group, and the like.

Examples of the alkylene group having 1 to 3 carbon atoms represented by R₂₁ in the general formula (7) that has a hydroxy group as a substituent or is unsubstituted include a methylene group, an ethylene group, a trimethylene group, a hydroxymethylene group, a hydroxyethylene group, a 1-hydroxytrimethylene group, a 2-hydroxytrimethylene group, and the like. Among these, the ethylene group, the trimethylene group, and the 2-hydroxytrimethylene group are preferable.

Examples of the phenyl group represented by R₂₂ in the general formula (7) that has a hydroxy group as a substituent or is unsubstituted include a hydroxyphenyl group, a phenyl group, and the like.

Examples of the alkyl group having 1 to 3 carbon atoms represented by R₂₂ in the general formula (7), R₂₃ to R₂₅ in the general formula (8), and R₁₃ and R₁₄ in the general formula (10) include a methyl group, an ethyl group, an n-propyl group, and an isopropyl group.

Specific examples of the group represented by the general formula (7) include a (4-hydroxyphenoxy)methyl group, a (4-hydroxyphenoxy)ethyl group, a (4-hydroxyphenoxy)propyl group, a 1-hydroxy-1-phenoxymethyl group, a 1-hydroxy-2-phenoxyethyl group, a 2-hydroxy-3-phenoxypropyl group, a methyl trimethylene glycol group, a methyl triethylene glycol group, a methyl tripropylene glycol group, and the like. Among these, the (4-hydroxyphenoxy)propyl group, the 2-hydroxy-3-phenoxypropyl group, the methyl tripropylene glycol group, and the methyl triethylene glycol group are preferable.

Examples of the alkylene group having 1 to 3 carbon atoms represented by R₂₆ in the general formula (8) include a methylene group, an ethylene group, a trimethylene group, and the like.

Specific examples of the group represented by the general formula (8) include a trimethylammonium methyl group, a trimethylammonium ethyl group, a triethylammonium methyl group, a triethylammonium ethyl group, and the like.

Specific examples preferred as the group represented by the general formula (9) include the following groups.

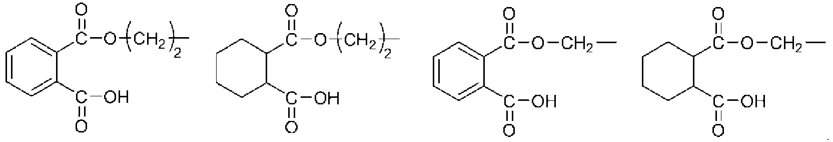

As R₁₂ in the general formula (6), a hydrogen atom, an alkyl group having 1 to 30 carbon atoms, a hydroxyalkyl group having 1 to 10 carbon atoms, an aryl group having 6 to 10 carbon atoms, an arylalkyl group having 7 to 13 carbon atoms, an alkoxyalkyl group having 2 to 9 carbon atoms, an aryloxyalkyl group having 7 to 13 carbon atoms, the group represented by the general formula (7), and the group represented by the general formula (9) are preferable. Among these, the hydrogen atom, the alkyl group having 1 to 30 carbon atoms, the hydroxyalkyl group having 1 to 10 carbon atoms, the aryl group having 6 to 10 carbon atoms, the arylalkyl group having 7 to 13 carbon atoms, and the alkoxyalkyl group having 2 to 9 carbon atoms are more preferable, the hydrogen atom, an alkyl group having 1 to 12 carbon atoms, and the arylalkyl group having 7 to 13 carbon atoms are even more preferable, and the hydrogen atom and the arylalkyl group having 7 to 13 carbon atoms are particularly preferable.

Specific examples preferred as the general formula (6) include an acrylic acid, benzyl acrylate, a methacrylic acid, benzyl methacrylate, hydroxyethyl methacrylate, methyl methacrylate, and the like. Among these, the acrylic acid, the benzyl acrylate, the methacrylic acid, and the benzyl methacrylate are preferable, and the methacrylic acid and the benzyl methacrylate are more preferable.

Specific examples preferred as the general formula (10) include acrylamide, methacrylamide, N,N-dimethylacrylamide, N,N-dimethylmethacrylamide, N,N-diethylacrylamide, N,N-diethylmethacrylamide, hydroxyethylacrylamide, hydroxyethylmethacrylamide, 4-acryloylmorpholine, and the like. Among these, the acrylamide, the methacrylamide, the N,N-dimethylacrylamide, and the N,N-diethylacrylamide are preferable, and the N,N-diethylacrylamide is more preferable.

Specific examples preferred as the general formula (11) include styrene, α-methylstyrene, N-vinylpyrrolidone, and the like. Among these, the styrene and the α-methylstyrene are preferable, and the styrene is more preferable.

Examples of the haloalkyl group having 1 to 10 carbon atoms represented by R₁₆ in the general formula (12) include a chloromethyl group, a chloroethyl group, a chloro-n-propyl group, a chloroisopropyl group, a chloro-n-butyl group, a chloro-tert-butyl group, a chloro-n-pentyl group, a chloro-n-hexyl group, a chloro-n-heptyl group, a chloro-n-octyl group, a chloro-n-nonyl group, a chloro-n-decyl group, a chlorocyclohexyl group, a chlorocycloheptyl group, a fluoromethyl group, a fluoroethyl group, a fluoro-n-propyl group, a fluoroisopropyl group, a fluoro-n-butyl group, a fluoro-tert-butyl group, a fluoro-n-pentyl group, a fluoro-n-hexyl group, a fluoro-n-heptyl group, a fluoro-n-octyl group, a fluoro-n-nonyl group, a fluoro-n-decyl group, a fluorocyclohexyl group, a fluorocycloheptyl group, and the like.

Examples of the aryl group having 6 to 10 carbon atoms represented by R₁₆ in the general formula (12) include a phenyl group, a naphthyl group, and the like.

Examples of the aryl group having 6 to 10 carbon atoms represented by R₁₆ in the general formula (12) that has an alkyl group having 1 to 6 carbon atoms or a halogeno group as a substituent include a methyl phenyl group, an ethyl phenyl group, an n-propylphenyl group, an n-butylphenyl group, an n-pentylphenyl group, an n-hexylphenyl group, a chlorophenyl group, a fluorophenyl group, a methyl naphthyl group, an ethyl naphthyl group, an n-propylnaphthyl group, a chloronaphthyl group, a fluoronaphthyl group, and the like.

Specific examples preferred as the general formula (12) include maleic anhydride, maleimide, N-methylmaleimide, N-ethylmaleimide, N-butylmaleimide, N-octylmaleimide, N-dodecylmaleimide, N-(2-ethylhexyl)maleimide, N-(2-hydroxyethyl)maleimide, N- (2-chlorohexyl)maleimide, N-cyclohexylmaleimide, N- (2-methylcyclohexyl)maleimide, N-(2-ethylcyclohexyl)maleimide, N-(2-chlorocyclohexyl)maleimide, N-phenylmaleimide, N-(2-methylphenyl)maleimide, N-(2-ethyphenyl)maleimide, N-(2-chlorophenyl)maleimide, and the like. Among these, the N-phenylmaleimide is preferable.

Specific examples of the binder resin containing, as constituent components, the monomer unit represented by the general formula (5) and at least one kind of monomer unit derived from the compound represented by the general formula (6), (10), (11), or (12) include combinations of monomer units described in the following table. Among these, the combinations 1 and 5 to 7 are preferable, and the combination 6 is more preferable. Among the combinations 6, a combination containing two kinds of monomer units derived from the compound represented by the general formula (6) is preferable.

| | **Monomer unit constituting binder resin** | | |
|---|---|---|---|
| **Combination 1** | The general formula (5) | Derived from the general formula (6) | |
| **Combination 2** | | Derived from the general formula (10) | |
| **Combination 3** | | Derived from the general formula (11) | |
| **Combination 4** | | Derived from the general formula (12) | |
| **Combination 5** | | Derived from the general formula (6) | Derived from the general formula (10) |
| **Combination 6** | | Derived from the general formula (6) | Derived from the general formula (11) |
| **Combination 7** | | Derived from the general formula (6) | Derived from the general formula (12) |

Among the combinations, a binder resin is preferable which contains, as constituent components, one kind of monomer unit represented by the general formula (5), one or two kinds of monomer units derived from a compound represented by the following general formula (6'), and one kind of monomer unit derived from the compound represented by the general formula (11).

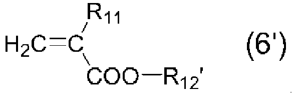

(In the formula, R₁₂' represents a hydrogen atom, an alkyl group having 1 to 30 carbon atoms, a hydroxyalkyl group having 1 to 10 carbon atoms, an aryl group having 6 to 10 carbon atoms, an arylalkyl group having 7 to 13 carbon atoms, or an alkoxyalkyl group having 2 to 9 carbon atoms, and R₁₁ is the same as described above.)

Specific examples of the alkyl group having 1 to 30 carbon atoms, the hydroxyalkyl group having 1 to 10 carbon atoms, the aryl group having 6 to 10 carbon atoms, the arylalkyl group having 7 to 13 carbon atoms, and the alkoxyalkyl group having 2 to 9 carbon atoms represented by R₁₂' in the general formula (6') are the same as the specific examples of those represented by R₁₂ in the general formula (6).

As R₁₂' in the general formula (6'), a hydrogen atom, an alkyl group having 1 to 12 carbon atoms, and an arylalkyl group having 7 to 13 carbon atoms are preferable, and the hydrogen atom and the arylalkyl group having 7 to 13 carbon atoms are more preferable.

Specific examples preferred as the general formula (6') include an acrylic acid, benzyl acrylate, a methacrylic acid, benzyl methacrylate, and the like. Among these, the methacrylic acid and the benzyl methacrylate are preferable.

The weight ratio between the monomer unit represented by the general formula (5) and the monomer unit derived from the compound represented by the general formula (6), (10), (11), or (12) may be appropriately set according to the type of the monomer unit used. The weight ratio of the monomer unit represented by the general formula (5) to the total weight of the obtained polymer is generally 10% to 90% by weight and preferably 30% to 70% by weight.

The weight-average molecular weight of the binder resin is generally 1,000 to 100,000, preferably 1,000 to 50,000, and more preferably 2,000 to 30,000. In addition, the content of the binder resin with respect to the weight of the composition is 5% to 90% by weight, and preferably 10% to 80% by weight.

As the polymerization initiator, it is possible to use a thermal polymerization initiator or a photopolymerization initiator known in the related art that are generally used in the field of the related art. Among these, the photopolymerization initiator is preferable. Specific examples thereof include an acetophenone-based photopolymerization initiator such as diethoxyacetophenone, 2-hydroxy-2-methyl-1-phenylpropan-1-one, benzyl dimethyl ketal, 1-(4-isopropylphenyl)-2-hydroxy-2-methylpropan-1-one, 4-(2-hydroxyethoxy)phenyl-(2-hydroxy-2-propyl)ketone, 1-hydroxycyclohexyl-phenylketone, 2-methyl-2-morpholino(4-thiomethylphenyl)propan-1-one, or 2-benzyl-2-dimethylamino-1-(4-morpholinophenyl)-butanone; a benzoin-based photopolymerization initiator such as benzoin, benzoin isopropyl ether, or benzoin isobutyl ether; an acylphosphine oxide-based photopolymerization initiator such as 2,4,6-trimethylbenzoyldiphenylphosphine oxide; a photopolymerization initiator based on benzyl or methyphenyl glyoxyester; a benzophenone-based photopolymerization initiator such as benzophenone, methyl o-benzoylbenzoate, 4-phenylbenzophenone, a 4,4'-dichlorobenzophenone, hydroxybenzophenone, 4-benzoyl-4'-methyl-diphenylsulfide, acrylated benzophenone, 3,3',4,4'-tetra(tert-butylperoxycarbonyl)benzophenone, or 3,3'-dimethyl-4-methoxybenzophenone; a thioxanthone-based photopolymerization initiator such as 2-isopropylthioxanthone, 2,4-dimethylthioxanthone, 2,4-diethylthioxanthone, or 2,4-dichlorothioxanthone; an aminobenzophenone-based photopolymerization initiator such as Michler's ketone or 4,4'-diethylaminobenzophenone; an oxime ester-based photopolymerization initiator such as 1-[4-(phenylthio)phenyl]-1,2-octanedione-2-(o-benzoyloxime) or 1-[6-(2-methylbenzoyl)-9-ethyl-9H-carbazol-3-yl]ethanone-o-acetyloxime; 10-butyl-2-chloroacridone, 2-ethylanthraquinone, 9,10-phenanthrenequinone, camphorquinone, and the like. The colored composition of the present invention may contain one kind of the polymerization initiator or two or more kinds of the photopolymerization initiator. The content of the photopolymerization initiator with respect to the weight of the composition is 0.1% to 20% by weight and preferably 1% to 10% by weight.

The silane coupling agent is used in a case where the colored composition is bonded to a substrate such as glass. As the silane coupling agent, it is possible to use silane coupling agents known in the related art that are generally used in the field of the related art. Examples thereof include silane coupling agents having, as a reactive organic functional group, an epoxy group, a thiol group, a hydroxy group, an amino group, a ureide group, a vinyl group, an acryloyl group, or the like. Specific examples thereof include β-(3,4-epoxycyclohexyl)ethyltrimethoxysilane, γ-glycidoxypropyltrimethoxysilane, γ-mercaptopropyltrimethoxysilane, γ-aminopropyltriethoxysilane, N-β-(aminoethyl)-γ-aminopropyltrimethoxysilane, γ-ureidopropyltriethoxysilane, vinyltriethoxysilane, vinyl-tris(β-methoxyethoxy)silane, and 3-(methacryloyloxy)propyltrimethoxysilane. The amount of the used silane coupling agent in a reaction solution is generally 0.1% to 20% by weight and preferably 1% to 10% by weight.

The pigment may be a pigment used for preparing a colored pattern of red, blue, or green, and examples thereof include a phthalocyanine-based pigment and the like. Examples of the phthalocyanine-based pigment include those containing magnesium, titanium, iron, cobalt, nickel, copper, zinc, or aluminum as a central metal. Specific examples thereof include C. I. Pigment red 1, C. I. Pigment red 2, C. I. Pigment red 5, C. I. Pigment red 17, C. I. Pigment red 31, C. I. Pigment red 32, C. I. Pigment red 41, C. I. Pigment red 122, C. I. Pigment red 123, C. I. Pigment red 144, C. I. Pigment red 149, C. I. Pigment red 166, C. I. Pigment red 168, C. I. Pigment red 170, C. I. Pigment red 171, C. I. Pigment red 175, C. I. Pigment red 176, C. I. Pigment red 177, C. I. Pigment red 178, C. I. Pigment red 179, C. I. Pigment red 180, C. I. Pigment red 185, C. I. Pigment red 187, C. I. Pigment red 202, C. I. Pigment red 206, C. I. Pigment red 207, C. I. Pigment red 209, C. I. Pigment red 214, C. I. Pigment red 220, C. I. Pigment red 221, C. I. Pigment red 224, C. I. Pigment red 242, C. I. Pigment red 243, C. I. Pigment red 254, C. I. Pigment red 255, C. I. Pigment red 262, C. I. Pigment red 264, C. I. Pigment red 272, C. I. Pigment blue 15, C. I. Pigment blue 15:1, C. I. Pigment blue 15:2, C. I. Pigment blue 15:3, C. I. Pigment blue 15:4, C. I. Pigment blue 15:5, C. I. Pigment blue 15:6, C. I. Pigment blue 16, C. I. Pigment blue 17:1, C. I. Pigment blue 75, C. I. Pigment blue 79, C. I. Pigment green 7, C. I. Pigment green 36, C. I. Pigment green 37, C. I. Pigment green 58, chloroalminum phthalocyanine, hydroxyaluminum phthalocyanine, aluminum phthalocyanine oxide, and zinc phthalocyanine. The content of these pigments with respect to the weight of the composition is 10% to 50% by weight and preferably 10% to 30% by weight.

In a case where the colored composition of the present invention contains the pigment, it is preferable that the colored composition contains a pigment dispersant. Examples of the pigment dispersant include polyamide amine or a salt thereof, a polycarboxylic acid or a salt thereof, a high-molecular weight unsaturated ester, modified polyurethane, modified polyester, modified poly(meth)acrylate, modified polymethacrylate, an acrylic copolymer, a methacrylic copolymer, a nathalene sulfonate formaldehyde condensate, a polyoxyethylene alkyl phosphoric acid ester, polyoxyethylene alkylamine, alkanolamine, and the like. One kind of pigment dispersant may be used singly, or two or more kinds of pigment dispersants may be used in combination. The content of the pigment dispersant with respect to the weight of the pigment is generally 1% to 80% by weight, and preferably 10% to 60% by weight.

The solvent may be appropriately selected according to the components contained in the colored composition of the present invention. Specific examples thereof include ethyl acetate, n-butyl acetate, isobutyl acetate, amyl formate, isoamyl acetate, butyl propionate, isopropyl butyrate, ethyl butyrate, butyl butyrate, methyl lactate, ethyl lactate, methyl oxyacetate, ethyl oxyacetate, butyl oxyacetate, methyl methoxyacetate, ethyl methoxyacetate, butyl methoxyacetate, methyl ethoxyacetate, ethyl ethoxyacetate, methyl 3-oxypropionate, ethyl 3-oxypropionate, methyl 3-methoxypropionate, ethyl 3-methoxypropionate, methyl 3-ethoxypropionate, ethyl 3-ethoxypropionate, methyl 2-oxypropionate, ethyl 2-oxypropionate, propyl 2-oxypropionate, methyl 2-methoxypropionate, ethyl 2-methoxypropionate, propyl 2-methoxypropionate, methyl 2-ethoxypropionate, ethyl 2-ethoxypropionate, methyl 2-oxy-2-methylpropionate, methyl 2-methoxy-2-methylpropionate, ethyl 2-ethoxy-2-methylpropionate, methyl pyruvate, ethyl pyruvate, propyl pyruvate, methyl acetoacetate, ethyl acetoacetate, methyl 2-oxobutanoate, ethyl 2-oxobutanoate, diethylene glycol dimethyl ether, tetrahydrofuran, ethylene glycol monomethyl ether, ethylene glycol monoethyl ether, methyl cellosolve acetate, ethyl cellosolve acetate, diethylene glycol monomethyl ether, diethylene glycol monoethyl ether, diethylene glycol monobutyl ether, propylene glycol monomethyl ether, propylene glycol monomethyl ether acetate, propylene glycol monoethyl ether acetate, propylene glycol monopropyl ether acetate, methyl ethyl ketone, cyclohexanone, 2-heptanone, 3-heptanone, and the like. The amount of the solvent is set such that the concentration of the colored composition of the present invention in the solvent becomes generally 5% to 80% by weight and preferably 10% to 60% by weight.

The cross-linking agent is not particularly limited as long as it enables a film to be cured by a cross-linking reaction. As the cross-linking agent, a commercial cross-linking agent or a cross-linking agent appropriately synthesized by a known method may be used. Specific examples thereof include (a) polyfunctional (meth)acrylate compound, (b) polyfunctional epoxy compound, (c) polyfunctional vinyl ether compound, (d) polyfunctional allyl ether compound, (e) polyfunctional thiol compound, (f) acid anhydride, (g) melamin compound, guanamine compound, glycoluril compound, or urea compound substituted with at least one substituent selected from a methylol group, an alkoxymethyl group, and an acyloxymethyl group, (h) phenol compound, naphthol compound, or hydroxyanthracene compound substituted with at least one substituent selected from a methylol group, an alkoxymethyl group, and an acyloxymethyl group, and the like. Among these, (a) to (f) are preferable, and (a) is more preferable. It should be noted that these compounds need to have at least two crosslink-forming substituents. The content of the cross-linking agent with respect to the weight of the composition is generally 20% to 80% by weight, and preferably 30% to 60% by weight.

Examples of (a) polyfunctional (meth)acrylate compound include polyethylene glycol di(meth)acrylate (having 2 to 14 ethylene groups), polypropylene glycol di(meth)acrylate (having 2 to 14 propylene groups), hexanediol di(meth)acrylate, decanediol di(meth)acrylate, trimethylolpropane di(meth)acrylate, trimethylolpropane tri(meth)acrylate, trimethylolpropane ethoxytri(meth)acrylate, trimethylolpropane propoxytri(meth)acrylate, tetramethylolmethane tri(meth)acrylate, tetramethylolmethane tetra(meth)acrylate, dipentaerythritol penta(meth)acrylate, dipentaerythritol hexa(meth)acrylate, ethoxylated pentaerythritol tetra(meth)acrylate (having 40 or less ethoxy groups), propoxylated pentaerythritol tetra(meth)acrylate (having 40 or less propoxy groups), ethoxylated trimethylolpropane tri(meth)acrylate (having 40 or less ethoxy groups), propoxylated trimethylolpropane tri(meth)acrylate (having 40 or less propoxy groups), bisphenol A dioxyethylene di(meth)acrylate, bisphenol A triethoxyethylene di(meth)acrylate, bisphenol A decaoxyethylene di(meth)acrylate, bisphenol A polyoxyethylene di(meth)acrylate, ethoxy isocyanurate-modified tri(meth)acrylate, and the like. Among these, the pentaerythritol penta(meth)acrylate and the dipentaerythritol hexa(meth)acrylate are preferable.

Examples of (b) polyfunctional epoxy compound include diglycidyl ether, ethylene glycol diglycidyl ether, polyethylene glycol diglycidyl ether (having 2 to 14 ethylene groups), propylene glycol diglycidyl ether, polypropylene glycol diglycidyl ether (having 2 to 14 propylene groups), butanediol diglycidyl ether, neopentyl glycol diglycidyl ether, spiroglycol diglycidyl ether, 1,6-hexanediol diglycidyl ether, trimethylolpropane polyglycidyl ether, sorbitol polyglycidyl ether, glycerol diglycidyl ether, glycerol polyglycidyl ether, diglycerol polyglycidyl ether, polyglycerol polyglycidyl ether, bisphenol A diglycidyl ether, bisphenol F diglycidyl ether, bisphenol AD diglycidyl ether, bisphenyl diglycidyl ether, glycidyl (meth)acrylate, and the like.

Examples of (c) polyfunctional vinyl ether compound include polyethylene glycol divinyl ether (having 2 to 14 ethylene groups), polypropylene glycol divinyl ether (having 2 to 14 propylene groups), hexanediol divinyl ether, decanediol divinyl ether, trimethylolpropane divinyl ether, trimethylolpropane trivinyl ether, trimethylolpropane ethoxytrivinyl ether, trimethylolpropane propoxytrivinyl ether, tetramethylolmethane trivinyl ether, tetramethylolmethane tetravinyl ether, dipentaerythritol pentavinyl ether, dipentaerythritol hexavinyl ether, ethoxylated pentaerythritol tetravinyl ether (having 40 or less epoxy groups), propoxylated pentaerythritol tetravinyl ether (having 40 or less propoxy groups), ethoxylated trimethylolpropane trivinyl ether (having 40 or less ethoxy groups), propoxylated trimethylolpropane trivinyl ether (having 40 or less propoxy groups), bisphenol A dioxyethylene divinyl ether, bisphenol A trioxyethylene divinyl ether, bisphenol A decaoxyethylene divinyl ether, bisphenol A polyoxyethylene divinyl ether, ethoxy isocyanurate-modified trivinyl ether, and the like.

Examples of (d) polyfunctional allyl ether compound include polyethylene glycol diallyl ether (having 2 to 14 ethylene groups), polypropylene glycol diallyl ether (having 2 to 14 propylene groups), hexanediol diallyl ether, decanediol diallyl ether, trimethylolpropane diallyl ether, trimethylolpropane triallyl ether, trimethylolpropane ethoxytriallyl ether, trimethylolpropane propoxytriallyl ether, tetramethylolmethane triallyl ether, tetramethylolmethane tetraallyl ether, dipentaerythritol pentaallyl ether, dipentaerythritol hexaallyl ether, ethoxylated pentaerythritol tetraallyl ether (having 40 or less ethoxy groups), propoxylated pentaerythritol tetraallyl ether (having 40 or less propoxy groups), ethoxylated trimethylolpropane triallyl ether (having 40 or less ethoxy groups), propoxylated trimethylolpropane triallyl ether (having 40 or less propoxy groups), bisphenol A dioxyethylene diallyl ether, bisphenol A trioxyethylene diallyl ether, bisphenol A decaoxyethylene diallyl ether, bisphenol A polyoxyethylene diallyl ether, ethoxy isocyanurate-modified triallyl ether, and the like.

Examples of (e) polyfunctional thiol compound include ethylene glycol bis(3-mercaptobutyrate), dutanediol bis(3-mercaptobutyrate), pentaerythritol tetrakis(3-mercaptobutyrate), dipentaerythritol hexakis(3-mercaptobutyrate), ethylene glycol bis(3-mercaptobutyrate), butanediol bis(3-mercaptoisobutyrate), pentaerythritol tetrakis(3-mercaptoisobutyrate), dipentaerythritol hexakis(3-mercaptoisobutyrate), trimethylolpropane tris(3-mercaptoisobutyrate), tris[(3-mercaptopropionyloxy)ethyl]isocyanurate, pentaerythritol tetrakis(3-mercaptopropionate), dipentaerythritol hexa(3-mercaptopropionate), trimethylolpropane tris(3-mercaptopropionate), diethylene glycol bis(3-mercaptopropionate), dipentaerythritol tetrakis(3-mercaptobutyrate), 1,4-bis(3-mercaptobutyryloxy)butane, 1,3,5-tris(3-mercaptobutyloxyethyl)-1,3,5-triazine-2,4,6-(1H,3H,5H)-trione, and the like.

Examples of (f) compound having an acid anhydride include phthalic anhydride, tetrahydrophthalic anhydride, hexahydrophthalic anhydride, methyl tetrahydrophthalic anhydride, methyl hexahydrophthalic anhydride, nadic anhydride, methyl nadic anhydride, maleic anhydride, succinic anhydride, octyl succinic anhydride, dodecyl succinic anhydride, chlorendic anhydride, pyromellitic anhydride, benzophenone tetracarboxylic anhydride, ethylene glycol bis(anhydrotrimate), methyl cyclohexene tetracarboxylic anhydride, trimellitic anhydride, polyazelaic anhydride, and the like.

The colored composition of the present invention may contain, in addition to the component described above, a polymerization inhibitor, a surfactant, and the like within a range that does not impair the object and effect of the present invention. Furthermore, according to the use, the colored composition of the present invention may contain additives generally used in the field of the related art, such as a lubricant, an antistatic agent, an anti-ultraviolet agent, an antioxidant, a light stabilizer, a dispersant, a processing stabilizer, a processing aid, an impact resistance enhancer, a filler, a stiffener, a flame retardant, a plasticizer, and a foaming agent. The additives are not particularly limited as long as they are known additives, and the amount thereof used is not limited as long as the amount is generally adopted in the field of the related art.

The colored composition of the present invention is prepared by mixing together the components described above.

The colored composition of the present invention can be molded in a desired shape by known molding methods by using various molds or rolling machines. Specifically, for example, the colored composition of the present invention can be formed in a desired shape by molding methods such as an injection molding method, a compression molding method, an injection compression molding method, an extrusion molding method, a blow molding method, a calender molding method, an inflation molding method, a T-die molding method, and a transfer molding method. In addition, the colored composition of the present invention can also coat other materials (metals, glass, wood, paper, bricks, concrete, polymer materials, and the like) or can be bonded to other materials by known coating and bonding methods. Specifically, for example, the colored composition of the present invention can coat other materials by a slit coating method, an injection method, a spin coating method, a cast coating method, a roll coating method, a screen printing method, and the like.

For example, in a case where a coloring curing film is formed using the colored composition of the present invention, first, a substrate such as a glass substrate is coated with the colored composition of the present invention by a known method, the coated surface is dried if necessary, and then the coated surface is photocured and/or thermally cured. In this way, a coloring curing film can be obtained.

The drying method is not particularly limited as long as it is a known method. For example, the drying is carried out by heating and drying performed using a hot plate, an oven, an infrared heater, or the like generally at a temperature of 50°C to 200°C and preferably at a temperature of 50°C to 150°C generally for 60 seconds to 5 hours and preferably for 60 seconds to 1 hour.

The photocuring method is not particularly limited as long as it is a known method, and the photocuring is performed by irradiating the coated surface with active energy rays such as ultraviolet rays or electron beams. Specifically, for example, the photocuring is performed by irradiating the coated surface with ultraviolet rays generally having a wavelength of 200 to 400 nm and preferably having a wavelength of 320 to 380 nm by using a light source such as a low-pressure mercury lamp, a medium-pressure mercury lamp, a high-pressure mercury lamp, a metal halide lamp, an LED lamp, or the like generally in an exposure amount of 100 to 3,000 mJ/cm² and preferably in an exposure amount of 500 to 2,000 mJ/cm².

The thermal curing method is not particularly limited as long as it is a known method. For example, the thermal curing is carried out by performing heating generally at a temperature of 100°C to 300°C and preferably at a temperature of 150°C to 250°C generally for 0.1 to 10 hours and preferably for 0.1 to 5 hours by using a hot plate, an oven, an infrared heater, or the like.

Hereinafter, the present invention will be more specifically described based on examples, but the present invention is not limited to the examples.

### Examples

### Synthesis Example 1 Synthesis of intermediate (compound 2)

Rhodamine B (2.0 g (3.9 mmol)) (compound 1: produced by Wako Pure Chemical Industries, Ltd.), 2.7 g (3.9 mmol) of a lithium salt of tetrakis(pentafluorophenyl)boron (IV) (LiFABA) (produced by Tosoh Finechem Corporation), and 30 ml of dichloromethane were put into a round-bottom flask equipped with a stirring device, and reacted for 1 hour at 25°C. The reaction solution was diluted with dichloromethane and then washed with water. The solvent was distilled away from the reaction solution by concentration under reduced pressure, thereby obtaining 4.1 g (yield: 100%) of a carboxylic acid substance (compound 2) of reddish-violet solids having a tetrakis(pentafluorophenyl)boron (IV) anion.

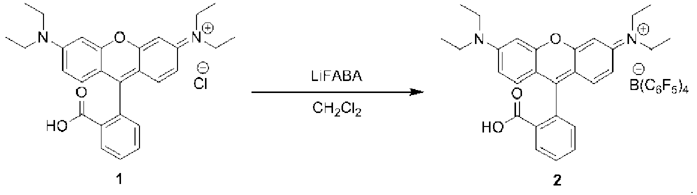

### Example 1 Synthesis of polyfunctional polymerizable dye (compound 4)

The intermediate (compound 2) (4.4 g (3.9 mmol)) obtained in Synthesis Example 1 and 30 ml of dichloromethane were put into a round-bottom flask equipped with a stirring device and dissolved. Then, 2.4 g (4.7 mmol) of pentaerythritol triacrylate {compound 3: trade name LIGHT ACRYLATE PE-3A (content of the compound 3: 60.0%), produced by Kyoeisha Chemical Co., Ltd.}, 0.1 g (1.2 mmol) of 4-dimethylaminopyridine (DMAP) (produced by Wako Pure Chemical Industries, Ltd.), and 1.3 g (6.7 mmol) of 1-ethyl-3-(3-dimethylaminopropyl)carbodiimide hydrochloride (WSC) (produced by Toyobo Co., Ltd.) were added thereto, and reacted for 3 hours at 25°C. The reaction solution was washed with water, and the solvent was distilled away from the reaction solution by concentration under reduced pressure, thereby obtaining reddish-violet solids. The solids were purified using a silica gel column, thereby obtaining reddish-violet solids. Methanol (100 ml) was added to the obtained solids so as to dissolve the solids, 40 ml of water was slowly added dropwise thereto so as to precipitate crystals, and then the solvent was distilled away by concentration under reduced pressure, thereby obtaining 1.15 g (yield: 21%) of a polyfunctional polymerizable dye as reddish-violet solids (compound 4).

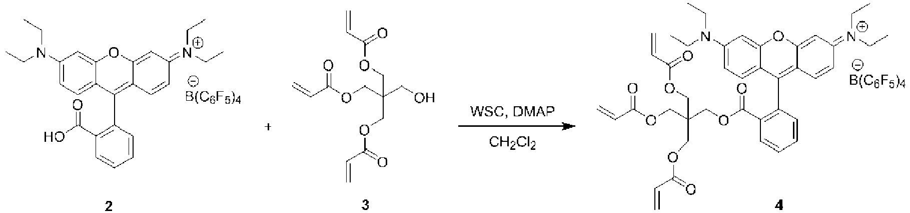

### Example 2 Synthesis of polyfunctional polymerizable dye (compound 6)

The intermediate (compound 2) (8.8 g (7.9 mmol)) obtained in Synthesis Example 1 and 60 ml of dichloromethane were put into a round-bottom flask equipped with a stirring device and dissolved. Then, 12.4 g (9.4 mmol) of dipentaerythritol pentaacrylate {compound 5: trade name NK ESTER A-9550 (content of the compound 5: 40.0%), produced by Shin-Nakamura Chemical Co., Ltd.}, 0.3 g (2.4 mmol) of DMAP (produced by Wako Pure Chemical Industries, Ltd.), and 2.6 g (13.4 mmol) of WSC (produced by Toyobo Co., Ltd.) were added thereto, and reacted for 3 hours at 25°C. The reaction solution was washed with water, and the solvent was distilled away from the reaction solution by concentration under reduced pressure, thereby obtaining reddish-violet solids. Methanol (1,300 ml) was added dropwise to the solids so as to dissolve the solids, 300 ml of water was slowly added thereto so as to precipitate crystals, and then the solvent was distilled away by concentration under reduced pressure, thereby obtaining 5.3 g (yield: 41%) of a polyfunctional polymerizable dye as reddish-violet solids (compound 6).

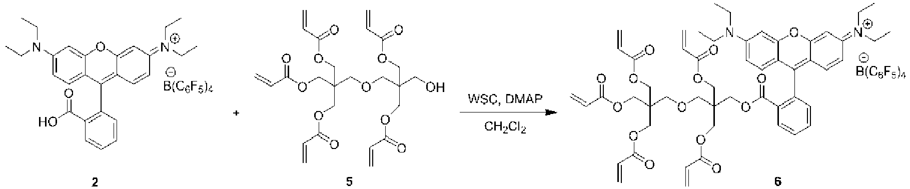

### Synthesis Example 2 Synthesis of intermediate (compound 9)

D(-)-sorbitol (7.2 g (40 mmol)) (compound 7: produced by Wako Pure Chemical Industries, Ltd.), 28.8 g (400 mmol) of an acrylic acid (compound 8: produced by Wako Pure Chemical Industries, Ltd.), 0.06 g (0.5 mmol) of p-methoxyphenol (MEHQ) (produced by Wako Pure Chemical Industries, Ltd.), 2.1 g (12.2 mmol) of p-toluenesulfonic acid (TsOH) (produced by Wako Pure Chemical Industries, Ltd.), and 30 ml of toluene were put into a round-bottom flask equipped with a stirring device, an air introduction pipe, and a Dean-Stark apparatus, and reacted for 8 hours at 110°C. Toluene was distilled away by pressure reduction, and 50 ml of toluene was added thereto for dissolution. Then, the reaction solution was washed with water, and the solvent was distilled away again, thereby obtaining a crude substance. The obtained crude substance was purified by silica gel column chromatography, and then the solvent was distilled away by concentration under reduced pressure, thereby obtaining 1.4 g (yield: 7.7%) of a colorless liquid intermediate (compound 9).

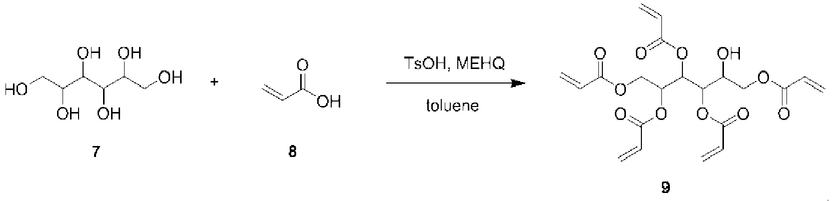

### Example 3 Synthesis of polyfunctional polymerizable dye (compound 10)

The intermediate (compound 2) (2.9 g (2.6 mmol)) obtained in Synthesis Example 1 and 20 ml of dichloromethane were put into a round-bottom flask equipped with a stirring device and dissolved, and 1.4 g (3.1 mmol) of the intermediate (compound 9) obtained in Synthesis Example 2, 0.1 g (0.8 mmol) of DMAP (produced by Wako Pure Chemical Industries, Ltd.), and 0.9 g (4.5 mol) of WSC (produced by Toyobo Co., Ltd.) were added thereto and reacted for 5 hours at 25°C. The reaction solution was washed with water, the solvent was distilled away from the reaction solution by concentration under reduced pressure, thereby obtaining reddish-violet solids. Methanol (400 ml) was added to the solids so as to dissolve the solids, 100 ml of water was slowly added dropwise thereto so as to precipitate crystals, and then the solvent was distilled away by concentration under reduced pressure, thereby obtaining 0.8 g (yield: 19%) of a polyfunctional polymerizable dye (compound 10) as reddish-violet solids.

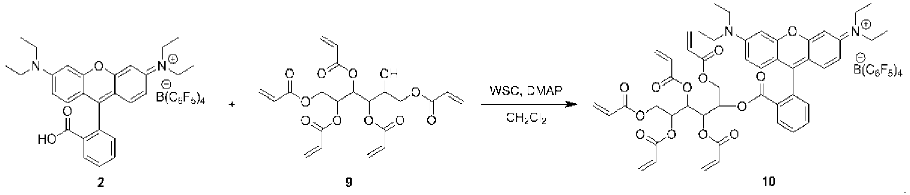

### Synthesis Example 3 Synthesis of intermediate (compound 12)

Succinic anhydride (1.71 g, (17.1 mmol)) (compound 11: produced by Wako Pure Chemical Industries, Ltd.) and 100 ml of dichloromethane were put into a round-bottom flask equipped with a stirring device. Then, dipentaerythritol pentaacrylate (24.8 g (18.8 mmol) {compound 5: trade name NK ESTER A-9550 (content of the compound 5: 40.0%), produced by Shin-Nakamura Chemical Co., Ltd.}, 18 mg (0.15 mmol) of DMAP (produced by Wako Pure Chemical Industries, Ltd.), and 1.9 g (18.8 mmol) of triethylamine (Et₃N) (produced by Wako Pure Chemical Industries, Ltd.) were added thereto and reacted for 20 hours at room temperature. The reaction solution was washed with dichloromethane and a 5% aqueous sodium hydrogen carbonate solution, and the aqueous layer was made into acidic so as to have a pH of about 2. The acidic aqueous layer was extracted using ethyl acetate, the obtained organic layer was dried over sodium sulfate and concentrated, thereby obtaining a crude substance. The crude substance was purified by column chromatography, and then the solvent was distilled away by concentration under reduced pressure, thereby obtaining 3.0 g (yield: 28%) of a colorless liquid intermediate (compound 12).

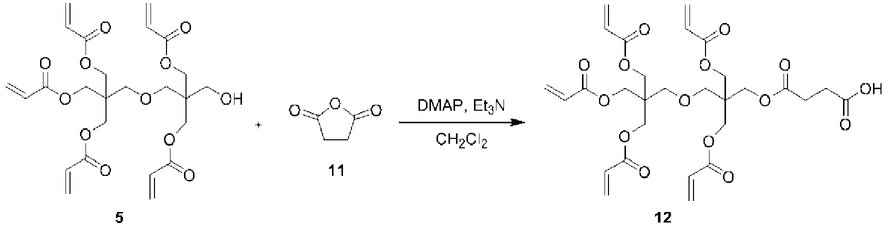

### Synthesis Example 4 Synthesis of intermediate (compound 14)

The intermediate (compound 12) (3.0 g (4.8 mmol)) obtained in Synthesis Example 3 and 30 ml of ethanol were put into a round-bottom flask equipped with a stirring device and dissolved, and 0.4 g (5.8 mmol) of ethanolamine (compound 13) (produced by Wako Pure Chemical Industries, Ltd.) and 2.3 g (8.2 mmol) of 4-(4,6-dimethoxy-1,3,5-triazin-2-yl)-4-methylmorpholinium chloride n-hydrate (DMT-MM) (produced by Wako Pure Chemical Industries, Ltd.) were added thereto and reacted for 5 hours at 25°C. The reaction solution was washed with water, and the solvent was distilled away from the reaction solution by concentration under reduced pressure, thereby obtaining a crude substance. The crude substance was purified by column chromatography, and then the solvent was distilled away by concentration under reduced pressure, thereby obtaining 1.1 g (yield: 34%) of a colorless liquid intermediate (compound 14).

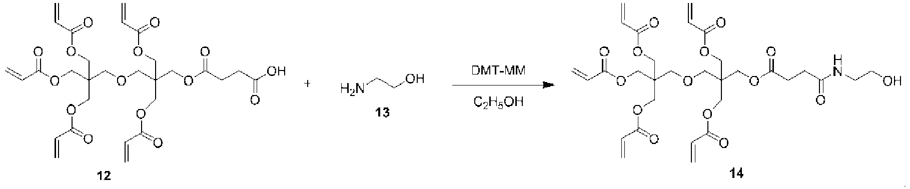

### Example 4 Synthesis of polyfunctional polymerizable dye (compound 15)

The intermediate (compound 2) (1.5 g (1.3 mmol)) obtained in Synthesis Example 1 and 15 ml of dichloromethane were put into a round-bottom flask equipped with a stirring device and dissolved, 1.1 g (1.6 mmol) of the intermediate (compound 14) obtained in Synthesis Example 4, 59 mg (0.5 mmol) of DMAP (produced by Wako Pure Chemical Industries, Ltd.), and 0.4 g (2.2 mol) of WSC (produced by Toyobo Co., Ltd.) were added thereto and reacted for 5 hours at 25°C. The reaction solution was washed with water, the solvent was distilled away from the reaction solution by concentration under reduced pressure, thereby obtaining reddish-violet solids. Methanol (210 ml) was added to the solids so as to dissolve the solids, 50 ml of water was slowly added dropwise thereto so as to precipitate crystals, and then the solvent was distilled away by concentration under reduced pressure, thereby obtaining 0.5 g (yield: 22%) of a polyfunctional polymerizable dye (compound 15) as reddish-violet solids.

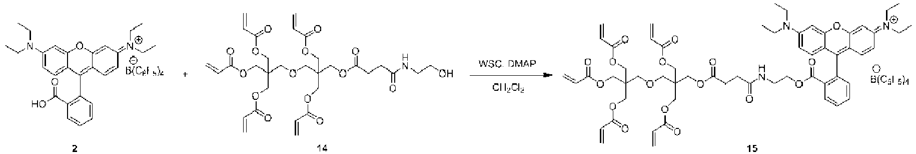

### Synthesis Example 5 Synthesis of intermediate (compound 17)

Phthalic anhydride (2.65 g, (17.9 mmol)) (compound 16: produced by Wako Pure Chemical Industries, Ltd.) and 100 ml of dichloromethane were put into a round-bottom flask equipped with a stirring device. Then, dipentaerythritol pentaacrylate (24.8 g (18.8 mmol)) {compound 5: trade name NK ESTER A-9550 (content of the compound 5: 40.0%), produced by Shin-Nakamura Chemical Co., Ltd.}, 18 mg (0.15 mmol) of DMAP (produced by Wako Pure Chemical Industries, Ltd.), and 1.9 g (18.8 mmol) of Et₃N (produced by Wako Pure Chemical Industries, Ltd.) were added thereto and reacted for 28 hours at room temperature. The reaction solution was washed with dichloromethane and a 5% aqueous sodium hydrogen carbonate solution, and the aqueous layer was made into acidic so as to have a pH of about 2. The acidic aqueous layer was extracted using ethyl acetate, the obtained organic layer was dried over sodium sulfate and concentrated, thereby obtaining a crude substance. The crude substance was purified by column chromatography, and then the solvent was distilled away by concentration under reduced pressure, thereby obtaining 3.6g (yield: 31%) of an intermediate (compound 17).

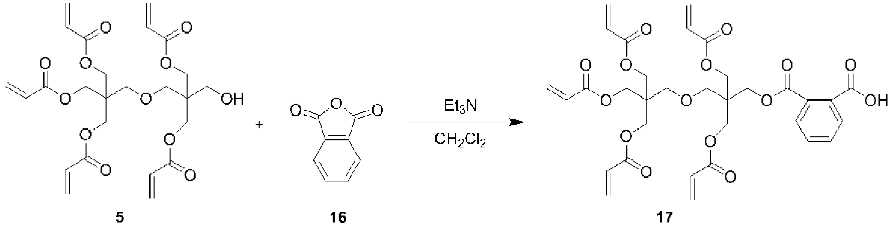

### Synthesis Example 6 Synthesis of intermediate (compound 18)

The intermediate (compound 17) (3.6 g (5.4 mmol)) obtained in Synthesis Example 5 and 36 ml of ethanol were put into a round-bottom flask equipped with a stirring device and dissolved, and 0.4 g (6.5 mmol) of ethanolamine (compound 13) (produced by Wako Pure Chemical Industries, Ltd.) and 2.5 g (9.2 mmol) of 4-(4,6-dimethoxy-1,3,5-triazin-2-yl)-4-methylmorpholinium chloride n-hydrate (DMT-MM) (produced by Wako Pure Chemical Industries, Ltd.) were added thereto and reacted for 5 hours at 25°C. The reaction solution was washed with water, and the solvent was distilled away from the reaction solution by concentration under reduced pressure, thereby obtaining a crude substance. The crude substance was purified by column chromatography, and then the solvent was distilled away by concentration under reduced pressure, thereby obtaining 1.2 g (yield: 31%) of a colorless liquid intermediate (compound 18).

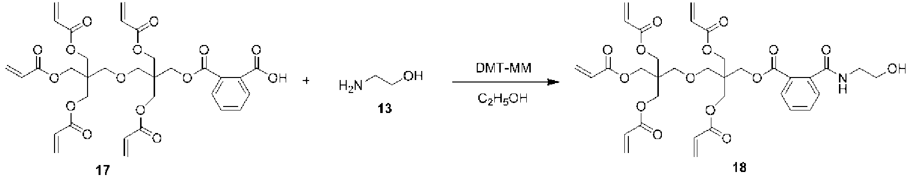

### Example 5 Synthesis of polyfunctional polymerizable dye (compound 19)

The intermediate (compound 2) (1.6 g (1.4 mmol)) obtained in Synthesis Example 1 and 15 ml of dichloromethane were put into a round-bottom flask equipped with a stirring device and dissolved, 1.2 g (1.7 mmol) of the intermediate (compound 18) obtained in Synthesis Example 6, 49 mg (0.4 mmol) of DMAP (produced by Wako Pure Chemical Industries, Ltd.), and 0.5 g (2.4 mol) of WSC (produced by Toyobo Co., Ltd.) were added thereto and reacted for 5 hours at 25°C. The reaction solution was washed with water, the solvent was distilled away from the reaction solution by concentration under reduced pressure, thereby obtaining reddish-violet solids. Methanol (230 ml) was added to the solids so as to dissolve the solids, 55 ml of water was slowly added dropwise thereto so as to precipitate crystals, and then the solvent was distilled away by concentration under reduced pressure, thereby obtaining 0.6 g (yield: 24%) of a polyfunctional polymerizable dye (compound 19) as reddish-violet solids.

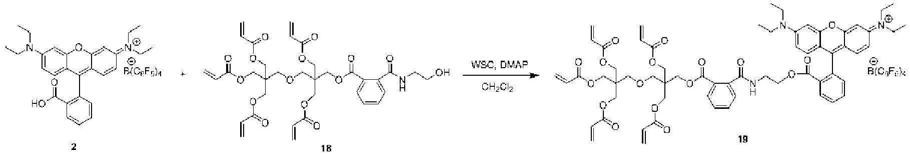

### Synthesis Example 7 Synthesis of intermediate (compound 23)

According to the method described in JP2015-30796A, a polyamine intermediate (compound 20) was synthesized. The synthesized polyamine intermediate (compound 20) (10.0 g (34.2 mmol)), 30 ml of a 5 N aqueous sodium hydroxide solution, and 70 ml of acetonitrile were put into a flask equipped with a stirrer, and in an ice bath, 13.0 g (102.6 mmol) of 3-chloropropionic acid chloride (compound 21: produced by Wako Pure Chemical Industries, Ltd.) was added dropwise thereto for 30 minutes. Then, the solution was stirred for 1 hour at room temperature. After liquid separation, 50 ml of 5 N aqueous potassium hydroxide solution was added thereto, and the solution was stirred for 5 hours at 30°C. The solution was cooled to room temperature, subjected to liquid separation, and neutralized using a concentrated hydrochloric acid. As a polymerization inhibitor, 18 mg (0.1 mmol) of a 4-hydroxy-2,2,6,6-tetramethyl-1-piperidinyloxy radical (produced by Wako Pure Chemical Industries, Ltd.) was added thereto, and the solution was concentrated under reduced pressure, thereby obtaining a crude substance. The obtained crude substance was purified column chromatography, and then the solvent was distilled away by concentration under reduced pressure, thereby obtaining 6.2 g (yield: 40%) of an intermediate (compound 23).

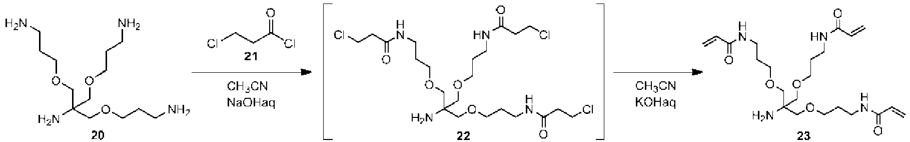

### Example 6 Synthesis of polyfunctional polymerizable dye (compound 24)

The intermediate (compound 2) (8.8 g (7.9 mmol)) obtained in Synthesis Example 1 and 60 ml of dichloromethane were put into a round-bottom flask equipped with a stirring device and dissolved, 4.3 g (9.4 mmol) of the intermediate (compound 23) obtained in Synthesis Example 7, 0.3 g (2.4 mmol) of DMAP (produced by Wako Pure Chemical Industries, Ltd.), and 2.6 g (13.4 mol) of WSC (produced by Toyobo Co., Ltd.) were added thereto and reacted for 5 hours at 25°C. The reaction solution was washed with water, the solvent was distilled away from the reaction solution by concentration under reduced pressure, thereby obtaining reddish-violet solids. Methanol (1,300 ml) was added to the solids so as to dissolve the solids, 300 ml of water was slowly added dropwise thereto so as to precipitate crystals, and then the solvent was distilled away by concentration under reduced pressure, thereby obtaining 4.0 g (yield: 32%) of a polyfunctional polymerizable dye (compound 24) as reddish-violet solids.

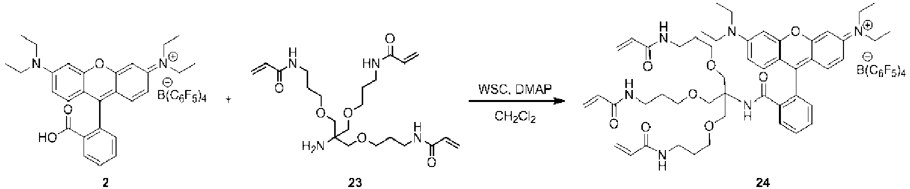

### Synthesis Example 8 Synthesis of intermediate (compound 27)

4-Aminophenyl acetate (3.7 g (25.0 mmol)) (compound 25: produced by Wako Pure Chemical Industries, Ltd.), 5.0 g (25.0 mmol) of 2-(1,3,3-trimethylindoline-2-indene)acetaldehyde (compound 26: produced by Wako Pure Chemical Industries, Ltd.), and 35 ml of acetonitrile were put into a round-bottom flask equipped with a stirring device. Then, 8.67 g (produced by Wako Pure Chemical Industries, Ltd.) of concentrated sulfuric acid was slowly added thereto at room temperature and then reacted for 16 hours at 50°C. The reaction solution was cooled to room temperature, and 10 g of sodium chloride and 90 ml of ethyl acetate (EtOAc) were added thereto, thereby precipitating yellow solids. After 30 minutes of stirring, the solids were collected by filtration, thereby obtaining 6.8 g (yield: 77%) of an intermediate (compound 27) as yellow solids.

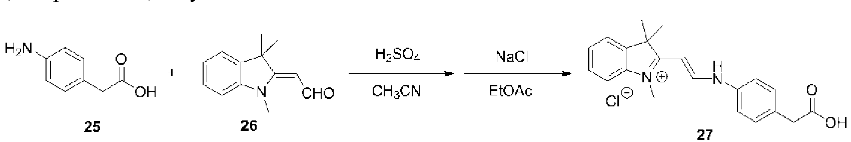

### Synthesis Example 9 Synthesis of intermediate (compound 28)

The intermediate (compound 27) (2.0 g (5.6 mmol)) obtained in Synthesis Example 8, 4.3 g (5.6 mmol) of LiFABA (produced by Tosoh Finechem Corporation), and 15 ml of dichloromethane were put into a round-bottom flask equipped with a stirring device, and reacted for 1 hour at 25°C. The reaction solution was diluted with dichloromethane and then washed with water. The solvent was distilled away from the reaction solution by concentration under reduced pressure, thereby obtaining 5.6 g (yield: 100%) of a carboxylic acid substance (compound 28) as yellow solids having a tetrakis(pentafluorophenyl)boron (IV) anion.

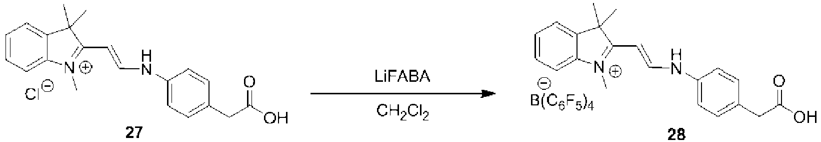

### Example 7 Synthesis of polyfunctional polymerizable dye (compound 29)

The intermediate (compound 28) (5.6 g (5.6 mmol)) obtained in Synthesis Example 9 and 30 ml of dichloromethane were put into a round-bottom flask equipped with a stirring device and dissolved. Then, 8.8 g (6.7 mmol) of dipentaerythritol pentaacrylate {compound 5: trade name NK ESTER A-9550 (content of the compound 5: 40.0%), produced by Shin-Nakamura Chemical Co., Ltd.}, 0.2 g (1.7 mmol) of DMAP (produced by Wako Pure Chemical Industries, Ltd.), and 1.8 g (9.5 mol) of WSC (produced by Toyobo Co., Ltd.) were added thereto, and reacted for 7 hours at 25°C. The reaction solution was washed with water, and the solvent was distilled away from the reaction solution by concentration under reduced pressure, thereby obtaining yellow solids. Methanol (1,300 ml) was added to the solids so as to dissolve the solids, 300 ml of water was slowly added dropwise thereto so as to precipitate crystals, and then the solvent was distilled away by concentration under reduced pressure, thereby obtaining 2.0 g (yield: 24%) of a polyfunctional polymerizable dye as yellow solids (compound 29).

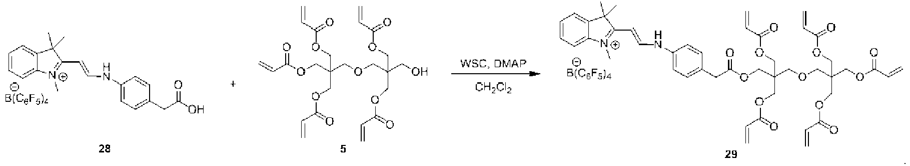

### Synthesis Example 10 Synthesis of intermediate (compound 32)

N,N-diethyl-3-aminophenol (1.3 g (8.0 mmol)) (compound 30: produced by Tokyo Chemical Industry Co., Ltd.), 3.1 g (16.0 mmol) of trimellitic anhydride (compound 31: produced by Wako Pure Chemical Industries, Ltd.), and 100 ml of toluene were put into a round-bottom flask equipped with a stirring device, and reacted for 72 hours under reflux. The solution was cooled to room temperature, and then the precipitated reddish-violet solids were collected by filtration. The obtained solids were purified by column chromatography, and then the solvent was distilled away by concentration under reduced pressure, thereby obtaining 2.3 g (yield: 59%) of an intermediate (compound 32).

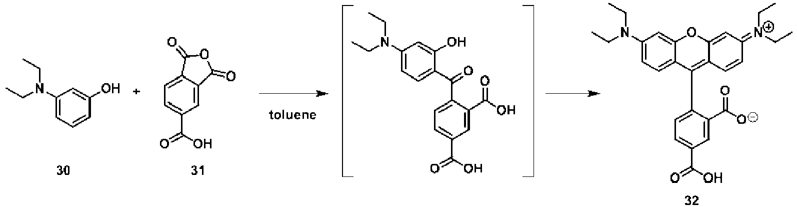

### Synthesis Example 11 Synthesis of intermediate (compound 33)

The intermediate (compound 32) (2.3 g (4.7 mmol)) obtained in Synthesis Example 10 and 50 ml of ethanol were put into a round-bottom flask equipped with a stirring device and dissolved. LiFABA (3.2 g (4.7 mmol)) (produced by Tosoh Finechem Corporation) and 12 ml of a 1 mol/L hydrochloric acid added thereto and reacted for 5 hours at 40°C. The reaction solution was diluted with 100 ml of dichloromethane and then washed with deionized water. The solvent was distilled away by concentration under reduced pressure, thereby obtaining 5.5 g (yield: 100%) of a dye monomer (compound 33) as reddish-violet solids having a tetrakis(pentafluorophenyl)boron (IV) anion.

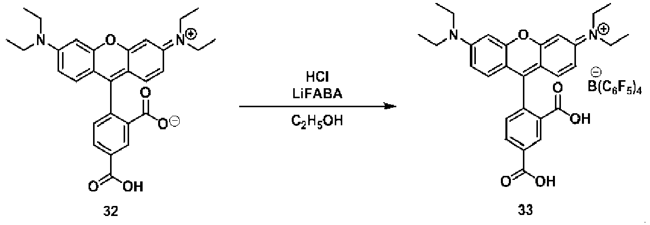

### Example 8 Synthesis of polyfunctional polymerizable dye (compound 34)

The intermediate (compound 33) (5.5 g (4.7 mmol)) obtained in Synthesis Example 11 and 40 ml of dichloromethane were put into a round-bottom flask equipped with a stirring device and dissolved. Then, 4.5 g (9.4 mmol) of pentaerythritol triacrylate {compound 3: trade name LIGHT ACRYLATE PE-3A (content of the compound 3: 60.0%), produced by Kyoeisha Chemical Co., Ltd.}, 0.2 g (1.4 mmol) of DMAP (produced by Wako Pure Chemical Industries, Ltd.), and 1.5 g (8.0 mol) of WSC (produced by Toyobo Co., Ltd.) were added thereto, and reacted for 8 hours at 25°C. The reaction solution was washed with water, and the solvent was distilled away from the reaction solution by concentration under reduced pressure, thereby obtaining reddish-violet solids. Methanol (1,300 ml) was added to the obtained solids so as to dissolve the solids, 300 ml of water was slowly added dropwise thereto so as to precipitate crystals, and then the solvent was distilled away by concentration under reduced pressure, thereby obtaining 1.5 g (yield: 18%) of a polyfunctional polymerizable dye (compound 34) as reddish-violet solids.

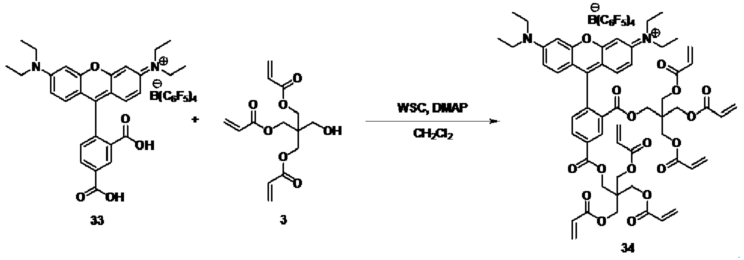

### Synthesis Example 12 Synthesis of monofunctional polymerizable dye (compound I)

A monofunctional polymerizable dye (compound I) was synthesized according to the method described in WO2014/126167.

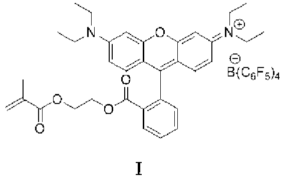

### Synthesis Example 13 Synthesis of difunctional polymerizable dye (compound II)

The intermediate (compound 2) (4.4 g (3.9 mmol)) obtained in Example 1 and 20 ml of dichloromethane were put into a round-bottom flask equipped with a stirring device and dissolved. Then, 1.1 g (4.7 mmol) of glycerol dimethacrylate (compound 35: trade name LIGHT ESTER G101P, produced by Kyoeisha Chemical Co., Ltd.), 0.1 g (1.2 mmol) of DMAP (produced by Wako Pure Chemical Industries, Ltd.), and 1.3 g (6.7 mmol) of WSC (produced by Toyobo Co., Ltd.) were added thereto, and reacted for 6 hours at 25°C. The reaction solution was washed with water, and the solvent was distilled away from the reaction solution by concentration under reduced pressure, thereby obtaining reddish-violet solids. The solids were purified using a silica gel column, thereby obtaining 2.90 g (yield: 55%) of a difunctional polymerizable dye (compound II) as reddish-violet solids.

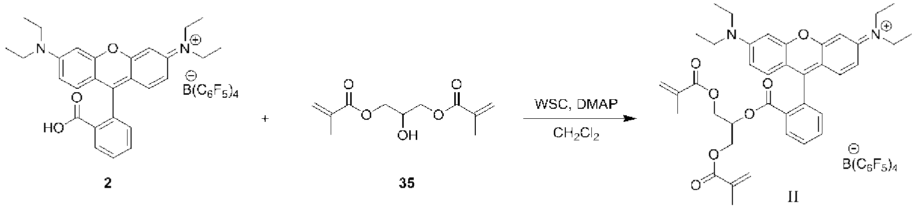

### Synthesis Example 14 Synthesis of monofunctional polymerizable dye (compound III)

A monofunctional polymerizable dye (compound III) was synthesized according to the method described in WO2014/133578.

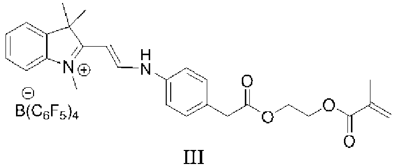

### Experimental Example 1 Evaluation of elution resistance of colored composition by using polyfunctional polymerizable dye

The elution resistance of the colored composition comprising the compound 4 obtained in Example 1, the compound 6 obtained in Example 2, or the compound 29 obtained in Example 7 was evaluated as below.

### (1) Preparation of colored composition

### (1-1) Synthesis of binder A

Propylene glycol monomethyl ether acetate (PGMEA) (98.5 g, produced by Wako Pure Chemical Industries, Ltd.) was put into a round-bottom flask equipped with a stirring device, a cooling pipe, a thermometer, and a nitrogen introduction pipe, and heated in a nitrogen stream until the internal temperature became 90°C. Then, a solution, which was obtained by mixing together 186.2 g of benzyl methacrylate (produced by Wako Pure Chemical Industries, Ltd.), 25.6 g of a methacrylic acid (produced by Wako Pure Chemical Industries, Ltd.), and 33.9 g of dimethyl 2,2'-azobis(2-methylpropionate) (trade name: V-601, produced by Wako Pure Chemical Industries, Ltd.), was added dropwise to heated PGMEA for 2 hours. Thereafter, the obtained solution was reacted for 2 hours at 90°C. Subsequently, the solution was heated to 100°C and reacted for 1 hour. After the reaction, the solution was cooled to room temperature, 171.5 g of PGMEA was added thereto for dilution, thereby obtaining a transparent light yellow polymer solution. The solution was used as a binder A. It should be noted that the concentration of nonvolatile components in the binder A was 36.1%.

### (1-2) Preparation of colored composition

The following components were mixed together in the amount described in Table 1, thereby obtaining colored compositions having the same dye concentration represented by % by mass.

### Components of colored composition

- Polymerizable dye: any one of compounds 4, 6, and 29
- Binder: binder A
- Cross-linking agent: dipentaerythritol hexaacrylate (DPHA) (produced by Nippon Kayaku Co., Ltd.)
- Solvent: PGMEA (produced by Wako Pure Chemical Industries, Ltd.)
- Photopolymerization initiator: 1-[4-(phenylthio)phenyl]-1,2-octanedione-2-(o-benzoyloxime) (trade name: IRGACURE OXE-01, produced by BASF SE)
- Silane coupling agent: 3-(methacryloyloxy)propyltrimethoxysilane (trade name: LS-3380, produced by Shin-Etsu Chemical Co., Ltd.)

### (2) Creation of coloring curing film

A 3-inch glass wafer (EAGLE XG produced by Corning Incorporated) was coated with the colored composition obtained in (1) by using a spin coater such that the film thickness became 1.3 to 1.8 µm, and the composition was dried for 90 seconds at 100°C. Then, by using an exposure device, the coating film was irradiated with light having a wavelength of 365 nm in an exposure amount of 1,000 mJ/cm². After the exposure, the composition was dried for 30 minutes at 230°C, thereby obtaining a coloring curing film.

### (3) Evaluation of elution resistance

PGMEA (20 g) was put into a glass petri dish, and the coloring curing film obtained in (2) was immersed in PGMEA for 2 hours for each of the glass wafers. After the immersion, the glass wafer was taken out of the PGMEA solution, and by using a spectrophotometer (spectrophotometer UV-2550 produced by Shimadzu Corporation), an absorbance (λa) of the PGMEA solution, in which the coloring curing film had been immersed, at a maximum absorption wavelength was measured.

### Comparative Example 1 Evaluation of elution resistance of colored composition by using monofunctional polymerizable dye

The elution resistance of the colored composition comprising the compound I or the compound III was evaluated in the same manner as that in Experimental Example 1, except that the compound I obtained in Synthesis Example 12 or the compound III obtained in Synthesis Example 14 was used instead of the compound 4, 6, or 29.

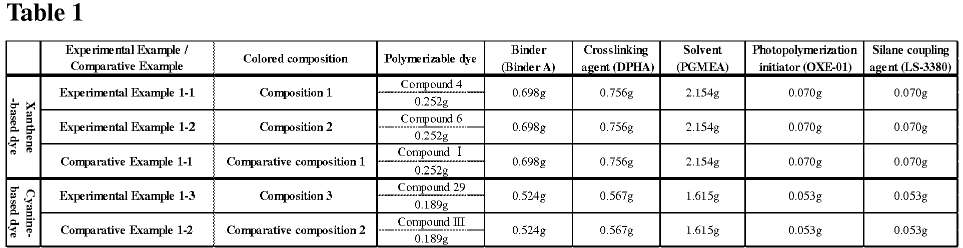

The absorbance λa obtained in Experimental Example 1 and Comparative Example 1 is shown in Table 2.

In addition, by regarding the absorbance λa in Comparative Example 1-1 as 100, an absorbance ratio in Experimental Examples 1-1 and 1-2 was calculated and taken as an elution ratio of a dye eluted from the same simple resist composition. Similarly, by regarding the absorbance λa in Comparative Example 1-2 as 100, the absorbance ratio in Experimental Example 1-3 was calculated and taken as an elution ratio of a dye eluted from the same simple resist composition. The obtained elution ratio of the dye is shown in Table 2.

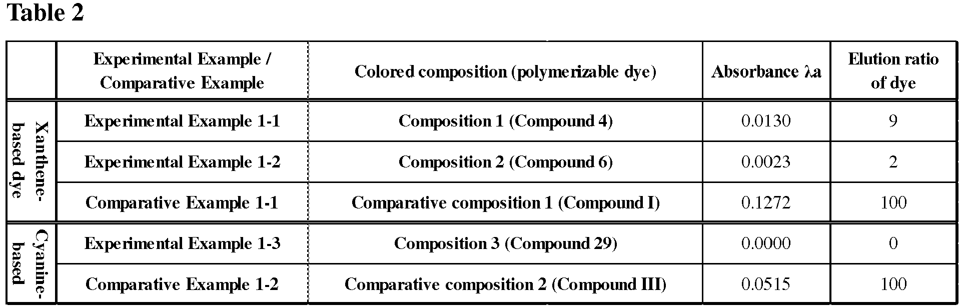

As a result of observing the PGMEA solution in which the coloring curing film had been immersed, it was found that the solution turned reddish violet in Comparative Example 1-1 while turned yellow in Comparative Example 1-2, and the elution of the dye could be confirmed with unaided eyes in both the comparative examples. In contrast, in Experimental Examples 1-2 and 1-3, the solution remained colorless and transparent, and the elution of the dye could not be confirmed with unaided eyes. From these observation results and the result of the degree of dye elution shown in Table 2, it was concluded that the colored composition comprising the compound (polyfunctional polymerizable dye) of the present invention can form a colored cured material having better elution resistance, compared to a colored composition of the related art containing a polymerizable dye (monofunctional polymerizable dye) having one polymerizable group.

### Experimental Example 2 Evaluation 2 of elution resistance of colored composition by using polyfunctional polymerizable dye

The elution resistance of the colored composition comprising the compound 4 obtained in Example 1, the compound 6 obtained in Example 2, the compound 10 obtained in Example 3, the compound 15 obtained in Example 4, the compound 19 obtained in Example 5, the compound 24 obtained in Example 6, or the compound 34 obtained in Example 8 was evaluated as below.

### (1) Preparation of colored composition

### (1-1) Synthesis of binder B

PGMEA (53.4 g, produced by Wako Pure Chemical Industries, Ltd.) was put into a round-bottom flask equipped with a stirring device, a cooling pipe, a thermometer, and a nitrogen introduction pipe, and heated in a nitrogen stream until the internal temperature became 95°C. Then, a solution, which was obtained by mixing together 32.5 g of benzyl methacrylate (produced by Wako Pure Chemical Industries, Ltd.), 35.0 g of a methacrylic acid (produced by Wako Pure Chemical Industries, Ltd.), 32.5 g of styrene (produced by Wako Pure Chemical Industries, Ltd.), and 15.0 g of dimethyl 2,2'-azobis(2-methylpropionate) (trade name: V-601, produced by Wako Pure Chemical Industries, Ltd.), was added dropwise to heated PGMEA for 2 hours. Thereafter, the obtained solution was reacted for 2 hours at 95°C. Subsequently, the solution was heated to 100°C and reacted for 1 hour. After the reaction, the solution was cooled to room temperature and left to stand overnight. The solution was heated again to 90°C, and 67.1 g of 4-hydroxybutylacrylate glycidyl ether (produced by Nippon Kasei Chemical Co., Ltd.), 2.34 g of tributylamine (produced by Wako Pure Chemical Industries, Ltd.), and 0.34 g of paramethoxyphenol (produced by Wako Pure Chemical Industries, Ltd.) were added thereto and reacted for 6 hours, thereby obtaining a transparent light yellow polymer solution. The solution was used as a binder B. It should be noted that the concentration of nonvolatile components in the binder B was 39.0%.

### (1-2) Preparation of colored composition

The following components were mixed together in the amount described in Table 3, thereby obtaining colored compositions having the same dye concentration represented by % by mass.

### Components of colored composition

- Polymerizable dye: any one of compounds 4, 6, 10, 15, 19, 24, and 34
- Binder: binder B
- Solvent: PGMEA (produced by Wako Pure Chemical Industries, Ltd.)
- Photopolymerization initiator: OXE-01 (produced by BASF SE)
- Silane coupling agent: LS-3380 (produced by Shin-Etsu Chemical Co., Ltd.)

### (2) Creation of coloring curing film

A 3-inch glass wafer (EAGLE XG produced by Corning Incorporated) was coated with the colored composition obtained in (1) by using a spin coater such that the film thickness became 1.3 to 1.8 µm, and the composition was dried for 90 seconds at 100°C. Then, by using an exposure device, the coating film was irradiated with light having a wavelength of 365 nm in an exposure amount of 1,000 mJ/cm². After the exposure, the composition was dried for 30 minutes at 230°C, thereby obtaining a coloring curing film.

### (3) Evaluation of elution resistance

PGMEA (20 g) was put into a glass petri dish, and the coloring curing film obtained in (2) was immersed in PGMEA for 2 hours for each of the glass wafers. After the immersion, the glass wafer was taken out of the PGMEA solution, and by using a spectrophotometer (spectrophotometer UV-2550 produced by Shimadzu Corporation), an absorbance (λa) of the PGMEA solution, in which the coloring curing film had been immersed, at a maximum absorption wavelength was measured.

### Comparative Example 2 Evaluation of elution resistance of colored composition by using difunctional polymerizable dye

The elution resistance of the colored composition comprising the compound II was evaluated in the same manner as that in Experimental Example 2, except that the compound II obtained in Synthesis Example 13 was used instead of the compound 4, 6, 10, 15, 19, 24, or 34.

**Table 3**

| **Experimental Example / Comparative Example** | **Colored composition** | **Polymerizable dye** | **Binder (Binder B)** | **Solvent (PGMEA)** | **Photopolymerization initiator (OXE-01)** | **Silane coupling agent (LS-3380)** |
|---|---|---|---|---|---|---|
| **Experimental Example 2-1** | **Composition 4** | Compound 4 | 2.585g | 1.023g | 0.070g | 0.070g |
| | | 0.252g | | | | |
| **Experimental Example 2-2** | **Composition 5** | Compound 6 | 2.585g | 1.023g | 0.070g | 0.070g |
| | | 0.252g | | | | |
| **Experimental Example 2-3** | **Composition 6** | Compound 10 | 2.585g | 1.023g | 0.070g | 0.070g |
| | | 0.252g | | | | |
| **Experimental Example 2-4** | **Composition 7** | Compound 15 | 2.585g | 1.023g | 0.070g | 0.070g |
| | | 0.252g | | | | |
| **Experimental Example 2-5** | **Composition 8** | Compound 19 | 2.585g | 1.023g | 0.070g | 0.070g |
| | | 0.252g | | | | |
| **Experimental Example 2-6** | **Composition 9** | Compound 24 | 2.585g | 1.023g | 0.070g | 0.070g |
| | | 0.252g | | | | |
| **Experimental Example 2-7** | **Composition 10** | Compound 34 | 2.585g | 1.023g | 0.070g | 0.070g |
| | | 0.252g | | | | |
| **Comparative Example 2-1** | **Comparative composition 3** | Compound II | 2.585g | 1.023g | 0.070g | 0.070g |
| | | 0.252g | | | | |

The absorbance λa obtained in Experimental Example 2 and Comparative Example 2 is shown in Table 4.

In addition, by regarding the absorbance λa in Comparative Example 2-1 as 100, an absorbance ratio in Experimental Examples 2-1 to 2-7 was calculated and taken as an elution ratio of a dye eluted from the same simple resist composition. The obtained elution ratio of the dye is shown in Table 4.

**Table 4**

| **Experimental Example / Comparative Example** | **Colored composition (polymerizable dye)** | **Absorbance λa** | **Elution ratio of dye** |
|---|---|---|---|
| **Experimental Example 2-1** | **Composition 4 (Compound 4)** | 0.0717 | 13 |
| **Experimental Example 2-2** | **Composition 5 (Compound 6)** | 0.0026 | 0.5 |
| **Experimental Example 2-3** | **Composition 6 (Compound 10)** | 0.0042 | 0.8 |
| **Experimental Example 2-4** | **Composition 7 (Compound 15)** | 0.0038 | 0.7 |
| **Experimental Example 2-5** | **Composition 8 (Compound 19)** | 0.0031 | 0.6 |
| **Experimental Example 2-6** | **Composition 9 (Compound 24)** | 0.0521 | 10 |
| **Experimental Example 2-7** | **Composition 10 (Compound 34)** | 0.0025 | 0.5 |
| **Comparative Example 2-1** | **Comparative composition 3 (Compound II)** | 0.5270 | 100 |

As a result of observing the PGMEA solution in which the coloring curing film had been immersed, it was found that the solution turned reddish violet in Comparative Example 2-1, and the elution of the dye could be confirmed with unaided eyes. In contrast, in Experimental Examples 2-2 to 2-7, the solution remained colorless and transparent, and the elution of the dye could not be confirmed with unaided eyes. From these observation results and the result of the degree of dye elution shown in Table 4, it was concluded that the colored composition comprising the compound of the present invention can form a colored cured material having excellent elution resistance even though the colored composition does not contain a cross-linking agent.

## Claims

1. A compound represented by the following general formula (1), 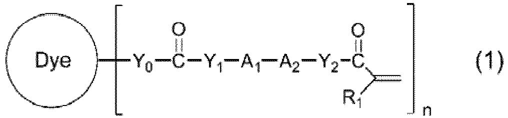 in the formula, Dye represents a dye residue, R₁ represents a hydrogen atom or a methyl group, Y₀ represents a linear alkylene group having 1 to 3 carbon atoms, -NH-, or a single bond, Y₁ and Y₂ each independently represent -O- or -NH-, A₁ represents an alkylene group having 1 to 6 carbon atoms that may have -O-, -OCO-, -COO-, -NHCO-, -CONH-, -NHCONH-, and/or a phenylene group in a chain, or represents a single bond, A₂ represents an alkylene group having 1 to 6 carbon atoms that may have -O- in a chain and has 1 to 6 groups represented by the following general formula (2) in the chain or on the terminal of the chain, and n represents 1 or 2; 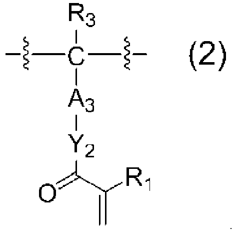 in the formula, R₁ and Y₂ are the same as described above, R₃ represents a hydrogen atom, an alkyl group having 1 to 6 carbon atoms, or a group represented by the following general formula (2-1), and A₃ represents an alkylene group having 1 to 6 carbon atoms that may have -O- in a chain, or represents a single bond; 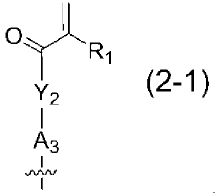 in the formula, R₁, A₃, and Y₂ are the same as described above,
a plurality of R₁'s, a plurality of Y₀'s, a plurality of Y₁'s, a plurality of Y₂'s, a plurality of A₁'s, and a plurality of A₂'s are the same as or different from each other respectively, and in a case where the number of the groups represented by the general formula (2) in A₂ is 1, R₃ in the general formula (2) represents the group represented by the general formula (2-1).

2. The compound according to claim 1,
wherein in a case where R₃ in the general formula (2) is a hydrogen atom or an alkyl group having 1 to 6 carbon atoms, the number of the groups represented by the general formula (2) in A₂ in the general formula (1) is 2 to 6, or in a case where R₃ in the general formula (2) is the group represented by the general formula (2-1), the number of the groups represented by the general formula (2) in A₂ in the general formula (1) is 1 to 3.

3. The compound according to claim 1 or 2,
wherein A₂ in the general formula (1) is represented by the following general formula (3), 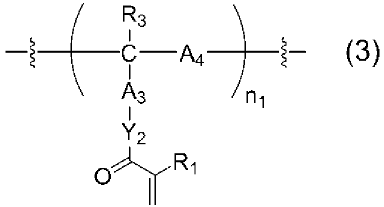 in the formula, R₁, R₃, A₃, and Y₂ are the same as described above, A₄ represents an alkylene group having 1 to 6 carbon atoms that may have -O- in a chain or represents a single bond, n₁ represents an integer of 1 to 6, a plurality of R₁'s, a plurality of R₃'s, a plurality of A₃'s, a plurality of A₄'s, and a plurality of Y₂'s are the same as or different from each other respectively, the total number of carbon atoms in the plurality of A₄'s is 1 to 6, and in a case where n₁ is 1, R₃ represents the group represented by the general formula (2-1).

4. The compound according to any one of claims 1 to 3,
wherein A₂ in the general formula (1) is represented by the following general formula (4-1) or (4-2); 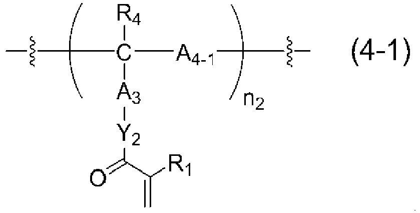 in the formula, R₁, A₃, and Y₂ are the same as described above, R₄ represents a hydrogen atom or an alkyl group having 1 to 6 carbon atoms, A₄₋₁ represents an alkylene group having 1 to 6 carbon atoms or a single bond, and n₂ represents an integer of 2 to 6, a plurality of R₁'s, a plurality of R₄'s, a plurality of A₃'s, a plurality of A₄₋₁'s, and a plurality of Y₂'s are the same as or different from each other respectively, and the total number of carbon atoms in the plurality of A₄₋₁'s is 1 to 6, 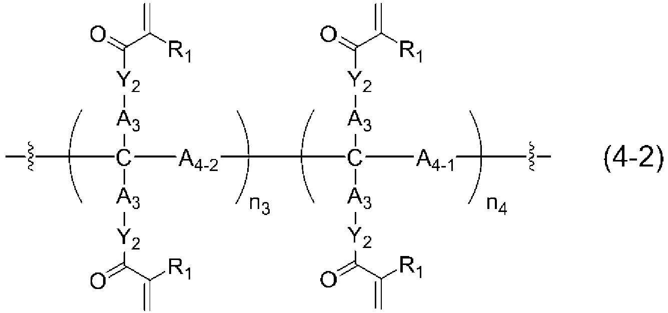 in the formula, R₁, A₃, A₄₋₁, and Y₂ are the same as described above, A₄₋₂ represents an alkylene group having 1 to 6 carbon atoms that has -O- in a chain, n₃ and n₄ each independently represent an integer of 0 to 3, and n₃ + n₄ equals an integer of 1 to 3, a plurality of R₁'s, a plurality of A₃'s, a plurality of A₄₋₁'s, a plurality of A₄₋₂'s, and a plurality of Y₂'s are the same as or different from each other respectively, and the total number of carbon atoms in the plurality of A₄₋₁'s and the plurality of A₄₋₂'s is 1 to 6.

5. The compound according to any one of claims 1 to 4,
wherein -A₁-A₂- in the general formula (1) is represented by any one of the following formulae (101) to (114).

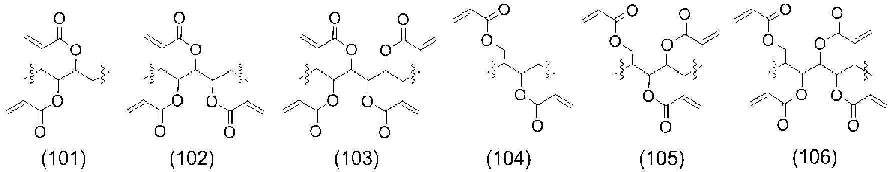

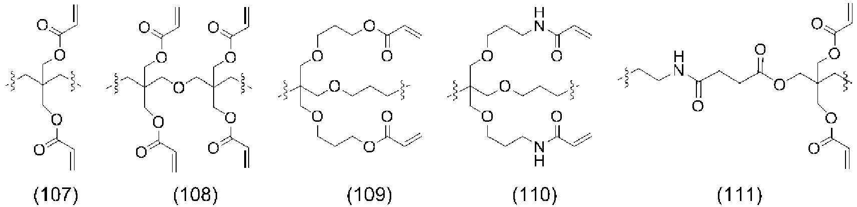

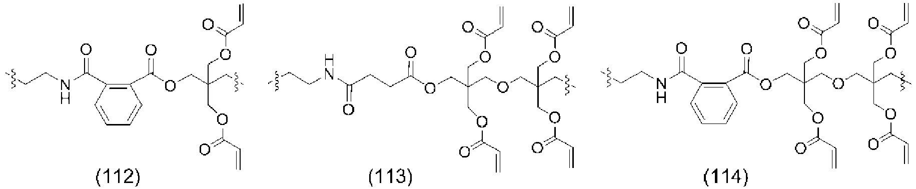

6. The compound according to any one of claims 1 to 5,
wherein Dye in the general formula (1) is a residue derived from a xanthene-based dye, a triarylmethane-based dye, or a cyanine-based dye.

7. The compound according to any one of claims 1 to 6,
wherein Dye in the general formula (1) is a residue derived from the xanthene-based dye or the cyanine-based dye.

8. The compound according to any one of claims 1 to 7,
wherein Dye in the general formula (1) is a dye residue represented by the following general formula (I) or the following general formula (IV); 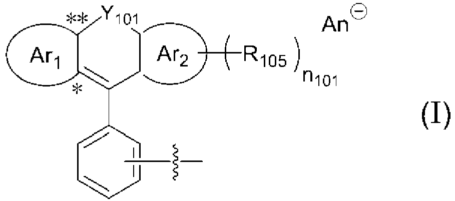 in the formula, n₁₀₁ pieces of R₁₀₅ each independently represent a halogeno group, an alkyl group having 1 to 30 carbon atoms, an alkoxy group having 1 to 20 carbon atoms, an alkylthio group having 1 to 20 carbon atoms, an amino group which has a substituent or is unsubstituted, a hydroxy group, an aryl group having 6 to 14 carbon atoms, an aryloxy group having 6 to 14 carbon atoms, or an arylalkyl group having 7 to 20 carbon atoms, Y₁₀₁ represents an oxygen atom, a sulfur atom, or -NR₁₃₂-, R₁₃₂ represents an alkyl group having 1 to 6 carbon atoms, An⁻ represents an anion, and Ar₁ represents a ring structure represented by any one of the following general formulae (I-1-1) to (I-1-7); 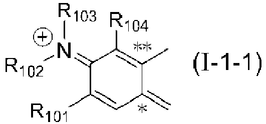 in the formula, R₁₀₁ and R₁₀₄ each independently represent a hydrogen atom or a methyl group, R₁₀₂ and R₁₀₃ each independently represent an alkyl group having 1 to 30 carbon atoms or an aryl group having 6 to 14 carbon atoms that has a substituent or is unsubstituted, R₁₀₁ and R₁₀₂ may form an alkylene group having 2 to 4 carbon atoms together, and R₁₀₃ and R₁₀₄ may form an alkylene group having 2 to 4 carbon atoms together, 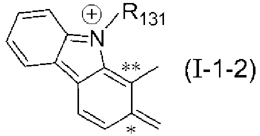 in the formula, R₁₃₁ represents an alkyl group having 1 to 30 carbon atoms, 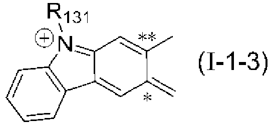 in the formula, R₁₃₁ is the same as described above, 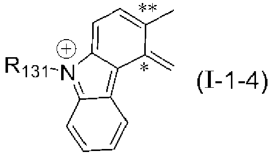 in the formula, R₁₃₁ is the same as described above, 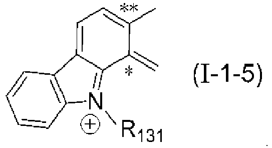 in the formula, R₁₃₁ is the same as described above, 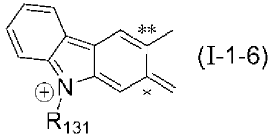 in the formula, R₁₃₁ is the same as described above, 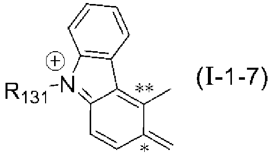 in the formula, R₁₃₁ is the same as described above,
* and ** represent each binding position, Ar₂ represents a benzene ring, a naphthalene ring, or an anthracene ring, in a case where Ar₂ is the benzene ring, n₁₀₁ represents an integer of 0 to 4, in a case where Ar₂ is the naphthalene ring, n₁₀₁ represents an integer of 0 to 6, and in a case where Ar₂ is the anthracene ring, n₁₀₁ represents an integer of 0 to 8, 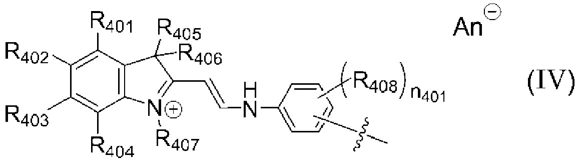 in the formula, An⁻ is the same as described above, R₄₀₁ to R₄₀₄ each independently represent a hydrogen atom, an alkyl group having 1 to 6 carbon atoms, an alkoxy group having 1 to 6 carbon atoms, an alkyloxycarbonyl group having 2 to 4 carbon atoms, an alkylcarbonyloxy group having 2 to 4 carbon atoms, a phenylcarbonyl group, a naphthylcarbonyl group, a halogeno group, a carboxy group, a nitro group, a cyano group, or an amino group, R₄₀₅ and R₄₀₆ each independently represent a hydrogen atom; an alkyl group having 1 to 6 carbon atoms; a phenylalkyl group having 7 to 9 carbon atoms that has an alkyl group having 1 to 6 carbon atoms, a nitro group, a halogeno group, or a cyano group, or is unsubstituted; or a naphthylalkyl group having 11 to 13 carbon atoms, R₄₀₇ represents a hydrogen atom; an alkyl group having 1 to 6 carbon atoms that has an alkoxy group having 1 to 6 carbon atoms, an alkyloxycarbonyl group having 2 to 4 carbon atoms, a hydroxy group, a carboxy group, a halogeno group, a cyano group, or an amino group, or is unsubstituted; a phenylalkyl group having 7 to 9 carbon atoms that has an alkoxy group having 1 to 6 carbon atoms, a halogeno group, or an amino group, or is unsubstituted; or a naphthylalkyl group having 11 to 13 carbon atoms, n₄₀₁ pieces of R₄₀₈ each independently represent an alkyl group having 1 to 6 carbon atoms, an alkoxy group having 1 to 6 carbon atoms, an alkyloxycarbonyl group having 2 to 4 carbon atoms, an alkylcarbonyloxy group having 2 to 4 carbon atoms, a phenylcarbonyl group, a naphthylcarbonyl group, a halogeno group, a carboxy group, a nitro group, a cyano group, or an amino group, in a case where n in the general formula (1) is 1, n₄₀₁ represents an integer of 0 to 4, and in a case where n in the general formula (1) is 2, n₄₀₁ represents an integer of 0 to 3.

9. The compound according to any one of claims 1 to 8,
wherein Dye in the general formula (1) is a dye residue represented by the following general formula (II) or the following general formula (V); 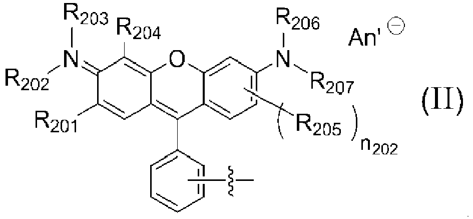 in the formula, R₂₀₁ and R₂₀₄ each independently represent a hydrogen atom or a methyl group, R₂₀₂ and R₂₀₃ each independently represent an alkyl group having 1 to 12 carbon atoms, or a phenyl group that has an alkyl group having 1 to 6 carbon atoms or is unsubstituted, n₂₀₂ pieces of R₂₀₅ represent a halogeno group; an alkyl group having 1 to 12 carbon atoms; an alkoxy group having 1 to 12 carbon atoms; an alkylthio group having 1 to 12 carbon atoms; an amino group having an alkyl group having 1 to 30 carbon atoms, a haloalkyl group having 1 to 20 carbon atoms, an aryl group having 6 to 10 carbon atoms, or an arylalkyl group having 7 to 13 carbon atoms; a hydroxy group; an aryl group having 6 to 14 carbon atoms; an aryloxy group having 6 to 14 carbon atoms; and an arylalkyl group having 7 to 20 carbon atoms, R₂₀₆ and R₂₀₇ each independently represent an alkyl group having 1 to 12 carbon atoms, a phenyl group, or a phenylalkyl group having 7 to 9 carbon atoms, An'⁻ represents an anion containing an aryl group having an electron-withdrawing substituent, a sulfonyl group having an electron-withdrawing substituent, a haloalkyl group, or a halogeno group, n₂₀₂ represents an integer of 0 to 3, R₂₀₁ and R₂₀₂ may form an alkylene group having 2 to 4 carbon atoms together, and R₂₀₃ and R₂₀₄ may form an alkylene group having 2 to 4 carbon atoms together, 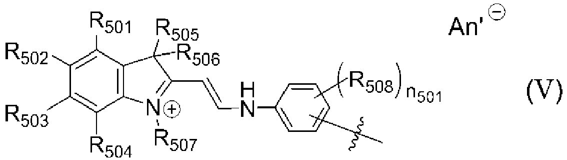 in the formula, An'⁻ is the same as described above, R₅₀₁ to R₅₀₆ each independently represent a hydrogen atom or an alkyl group having 1 to 6 carbon atoms, R₅₀₇ represents an alkyl group having 1 to 6 carbon atoms that has an alkoxy group having 1 to 6 carbon atoms, an alkyloxycarbonyl group having 2 to 4 carbon atoms, a hydroxy group, a carboxy group, a halogeno group, a cyano group, or an amino group, or is unsubstituted, n₅₀₁ pieces of R₅₀₈ each independently represent an alkyl group having 1 to 6 carbon atoms or an alkoxy group having 1 to 6 carbon atoms, in a case where n in the general formula (1) is 1, n₅₀₁ represents an integer of 0 to 2, and in a case where n in the general formula (1) is 2, n₅₀₁ represents an integer of 0 to 3.

10. A colored composition comprising:
the compound according to any one of claims 1 to 9.

11. The colored composition according to claim 10,
wherein the composition does not contain a cross-linking agent.

12. The colored composition according to claim 10 or 11, further comprising:
a binder resin.

13. The colored composition according to claim 12,
wherein the binder resin is a binder resin having a polymerizable unsaturated group.

14. The colored composition according to claim 13,
wherein the binder resin having the polymerizable unsaturated group is a binder resin containing a monomer unit represented by the following general formula (5) as a constituent component; 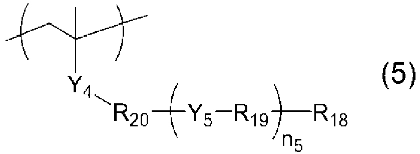 in the formula, R₁₈ represents a group represented by any one of the following formulae (5-1) to (5-10), 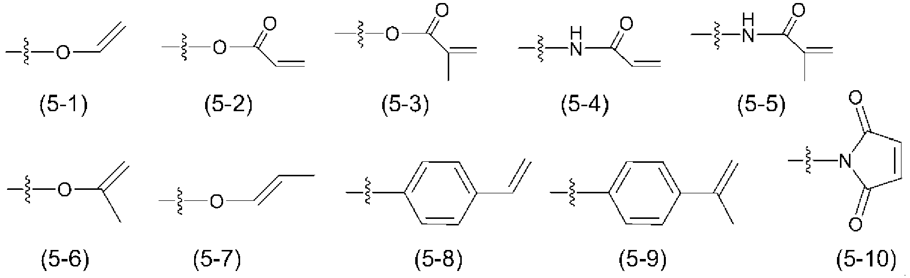 R₁₉ and R₂₀ each independently represent an alkylene group having 1 to 12 carbon atoms that may have a substituent, the substituent is selected from a hydroxy group, an alkyl group having 1 to 6 carbon atoms, an alkoxy group having 1 to 6 carbon atoms, an aryl group having 6 to 10 carbon atoms, an aryloxy group having 6 to 10 carbon atoms, an arylalkyl group having 7 to 13 carbon atoms, an aryloxyalkyl group having 7 to 13 carbon atoms, and an alicyclic hydrocarbon group having 6 to 12 carbon atoms that has or does not have an oxygen atom, Y₄ represents -COO-, -CONH-, -OCONH-, or -NHCONH-, Y₅ represents -O-, -OCO-, -COO-, -CONH-, -NHCO-, -OCONH-, or -NHCONH-, n₅ represents an integer of 1 to 4, and a plurality of R₁₉'s and a plurality of Y₅'s are the same as or different from each other respectively.

15. The colored composition according to claim 14,
wherein the binder resin having the polymerizable unsaturated group is a binder resin further containing, as a constituent component, at least one kind of monomer unit derived from a compound represented by the following general formula (6), (10), (11), or (12); 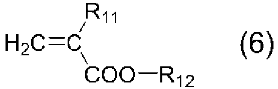 in the formula, R₁₁ represents a hydrogen atom or a methyl group, R₁₂ represents a hydrogen atom, an alkyl group having 1 to 30 carbon atoms, a hydroxyalkyl group having 1 to 10 carbon atoms, an aryl group having 6 to 10 carbon atoms, an arylalkyl group having 7 to 13 carbon atoms, an alkoxyalkyl group having 2 to 9 carbon atoms, an alkoxyalkoxyalkyl group having 3 to 9 carbon atoms, an aryloxyalkyl group having 7 to 13 carbon atoms, a morpholinoalkyl group having 5 to 7 carbon atoms, a trialkylsilyl group having 3 to 9 carbon atoms, an alicyclic hydrocarbon group having 6 to 12 carbon atoms that has an oxygen atom, a dialkylaminoalkyl group having 3 to 9 carbon atoms, a fluoroalkyl group having 1 to 18 carbon atoms, an N-alkylenephthalimide group having 9 to 14 carbon atoms, a group represented by the following general formula (7), 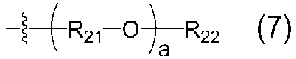 in the formula, R₂₁ represents an alkylene group having 1 to 3 carbon atoms that has a hydroxy group as a substituent or is unsubstituted, R₂₂ represents a phenyl group that has a hydroxy group as a substituent or is unsubstituted, or represents an alkyl group having 1 to 3 carbon atoms, and a represents an integer of 1 to 3,
a group represented by the following general formula (8), 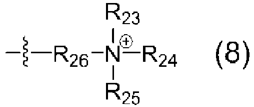 in the formula, R₂₃ to R₂₅ each independently represent an alkyl group having 1 to 3 carbon atoms, and R₂₆ represents an alkylene group having 1 to 3 carbon atoms, or
a group represented by the following general formula (9), 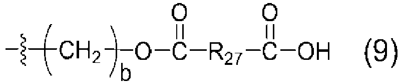 in the formula, R₂₇ represents a phenylene group or a cyclohexylene group, and b represents an integer of 1 to 6, 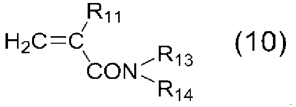 in the formula, R₁₃ represents a hydrogen atom or an alkyl group having 1 to 3 carbon atoms, R₁₄ represents a hydrogen atom, an alkyl group having 1 to 3 carbon atoms, a dialkylaminoalkyl group having 3 to 9 carbon atoms, or a hydroxyalkyl group having 1 to 10 carbon atoms, R₁₁ is the same as described above, and R₁₃ and R₁₄ may form a morpholino group together with a nitrogen atom adjacent thereto, 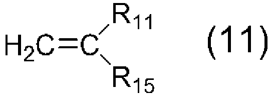 in the formula, R₁₅ represents a phenyl group or a pyrrolidino group, and R₁₁ is the same as described above, 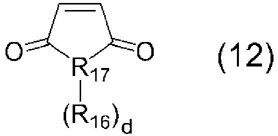 in the formula, R₁₇ represents a nitrogen atom or an oxygen atom, R₁₆ represents a hydrogen atom, an alkyl group having 1 to 30 carbon atoms, a hydroxyalkyl group having 1 to 10 carbon atoms, a haloalkyl group having 1 to 10 carbon atoms, an aryl group having 6 to 10 carbon atoms, or an aryl group having 6 to 10 carbon atoms that has an alkyl group having 1 to 6 carbon atoms or a halogeno group as a substituent, d represents 0 in a case where R₁₇ is an oxygen atom, and represents 1 in a case where R₁₇ is a nitrogen atom.
